# EUROPÄISCHE PATENTANMELDUNG

(11) **EP 2 380 885 A1**
(43) Veröffentlichungstag der Anmeldung: **26.10.2011**
(21) Anmeldenummer: 11002549.1
(22) Anmeldetag: 15.02.2005
(51) Int. Cl.: C07D 277/48, C07D 417/04, C07D 277/82, C07D 285/12, C07D 285/08, C07D 249/14, C07D 237/22, C07D 231/56, C07D 263/28, C07D 261/14, A61K 31/425

(54) **Guanidinverbindungen und ihre Verwendung als Bindungspartner fuer 5-HT5-Rezeptoren**

(30) Priorität: 19.02.2004 DE 102004008141
(62) Teilanmeldung aus: 05707406.4
(71) Anmelder: Abbott GmbH & Co. KG, 65205 Wiesbaden (DE)
(72) Erfinder: Netz, Astrid, 68199 Mannheim (DE); Amberg, Wilhelm, 68239 Mannheim (DE); Lange, Udo, 68199 Mannheim (DE); Ochse, Michael, 67061 Ludwigshafen (DE); Hutchins, Charles, W., Green Oaks, Illinois 60058 (US); Garcia-Ladona, Francisco-Xavier, 76870 Kandel (DE); Wernert, Wolfgang, 67434 Neustadt/Weinstrasse (DE); Kling, Andreas, 68239 Mannheim (DE)
(74) Vertreter: Grünecker, Kinkeldey, Stockmair & Schwanhäusser

(57) **Zusammenfassung**

Die vorliegende Erfindung betrifft Guanidinverbindungen der allgemeinen Formel I 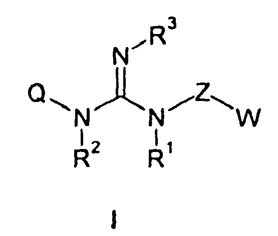 entsprechende enantiomere, diastereomere und/oder tautomere Formen davon sowie pharmazeutisch annehmbare Salze davon. Weiterhin betrifft die vorliegende Verbindung die Verwendung von Guanidinverbindungen als Bindungspartner für 5-HT5-Rezeptoren zur Behandlung von Krankheiten, die durch eine 5-HT5-Rezeptoraktivität moduliert werden, insbesondere zur Behandlung von neurodegenerativen und neuropsychiatrischen Störungen sowie den damit zusammenhängenden Anzeichen, Symptomen und Fehlfunktionen.

## Beschreibung

### Gebiet der Erfindung

Die vorliegende Erfindung betrifft Guanidinverbindungen und die Verwendung von Guanidinverbindungen als Bindungspartner für 5-HT5-Rezeptoren zur Behandlung von Krankheiten, die durch eine 5-HT5-Rezeptoraktivität moduliert werden, insbesondere zur Behandlung von neurodegenerativen und neuropsychiatrischen Störungen sowie den damit zusammenhängender Anzeichen, Symptomen und Fehlfunktionen.

### Hintergrund der Erfindung

Wenigstens sieben verschiedene Rezeptorklassen vermitteln die physiologischen Aktivitäten, die einer Beteiligung des Neurotransmitters Serotonin (5-Hydroxytryptamin, kurz 5-HT) zugeschrieben werden. Sie werden entsprechend einer international anerkannten Klassifikation mit 5-HT1, 5-HT2, 5-HT3, 5-HT4, 5-HT5, 5-HT6 und 5-HT7 bezeichnet. Die meisten dieser Klassen umfassen darüber hinaus weitere unterscheidbare Rezeptorsubtypen. So gehören zur 5-HT1-Klasse Rezeptoren, die sich wiederum in wenigstens fünf Unterklassen einteilen lassen, und als 5-HT1A, 5-HT1 B, 5-HT1 C 5-HT1 D und 5-HT1 E bezeichnet werden (Boess, Martin; Neuropharmacology 33:275-317(1994).

Die 5-HT5-Klasse wurde erstmals von Plassat et al., The EMBO Journal Bd. 11 Nr. 13, S. 4779-4786 (1992) beschrieben. Man unterscheidet 5-HT5A- und 5-HT5B-Rezeptoren (Erlander et al., Proc. Natl. Acad. Sci. USA 90:3452-3456 (1993). Es bestehen nur geringe Sequenzhomologien zwischen 5-HT5 und anderen 5-HT-Rezeptoren, und das pharmakologische Profil dieser Rezeptoren ist deutlich unterschiedlich. 5-HT5-Rezeptoren konnten mithilfe molekularbiologischer Techniken im Riechkolben, im Hippocampus, im Cortex, in den Zerebralventrikeln, im Corpus Callosum und im Kleinhirn lokalisiert werden. Mittels immunhistochemischer Methoden wurde gezeigt, dass 5-HT5-Rezeptoren von Neuronen in verschiedenen Hirnregionen exprimiert werden (Oliver et al. Brain Res 2000, 867, 131-142 ; Pasqualetti et al. Mol Brain Res 1998, 56, 1-8)) Diese 5-HT5-Rezeptoren können zum einen direkt oder indirekt wichtige Funktionen des Hirns modulieren, andererseits aber auch an Mechanismen beteiligt sein, die bei neuropathologischen, neurodegenerativen und neuropsychiatrischen Erkrankungen involviert sind. 5HT5-Rezeptoren wurden auch in Astrocyten lokalisiert (Carson et al., GLIA 17:317-326 (1996). Astrocyten liegen direkt an der Basalmembran von Gehirnkapillaren der Bluthirnschranke an, und eine anormale Astrocyten-Endothelium-Struktur geht mit einem Verlust der Bluthirnschranke einher. Die genaue Bedeutung der Astrocyten ist unklar, sie scheinen Transportaufgaben und konnektive Funktionen wahrzunehmen. Reaktive Astrocyten wurden in Zusammenhang mit reaktiver Gliosis bei einer Reihe von pathologischen Gehirnveränderungen und neuropsychiatrischen Erkrankungen beobachtet. Infolge von Gehirnverletzungen verändern diese Astrocyten ihre Morphologie. Das Protein-Expressionsmuster ändert sich und Wachstumsfaktoren werden produziert. In-vitro Untersuchungen an kultivierten Astrocyten zeigten 5-HT5-Rezeptor-vermittelte Antworten. Aus diesem Grund wird einerseits vermutet, dass der 5-HT5-Rezeptor an Erholungsprozessen des Gehirns nach Störungen beteiligt sind, andererseits ist aber auch nicht auszuschließen, dass sie zur Schadensentstehung oder sogar zu einer Schadensvermehrung beitragen.

Erkrankungen des ZNS betreffen heutzutage große Bevölkerungsteile. Insbesondere aufgrund der Zunahme älterer Menschen steigen die Patientenzahlen ständig. Neuropathologische Zustände wie cerebrale Ischämie, Schlaganfall, Epilepsie und Anfälle im allgemeinen, chronische Schizophrenie, andere psychotischen Erkrankungen, Depression, Angstzustände, bipolare Störungen, Demenz, insbesondere Alzheimer Demenz, demyelinisierende Erkrankungen, insbesondere Multiple Sklerose, und Gehirntumore führen zu Schädigungen des Gehirns und zu den damit verbundenen neuronalen Defiziten. Therapeutische Behandlungen der geschilderten neurodegenerativen und neuropsychiatrischen Störungen richteten sich bislang auf verschiedene Membranrezeptoren mit dem Ziel, Defizite in Neurotransmissionsvorgängen zu kompensieren. Zwar konnten neuroprotektive Wirkungen mit verschiedenen serotonergen Verbindungen in Tiermodellen für neuropathologische Zustände, wie Ischämie, Hirnschlag und Excitotoxizität, erzielt werden; teilweise konnten auch günstige Wirkungen auf Gemütsstörungen, wie Depression oder Angstzustände, beobachtet werden. Zu nennen sind hier beispielsweise 5-HT1A-Agonisten wie Buspiron, oder die als selektiver 5-HT1A-Rezeptor-Ligand charakterisierte Verbindung 8-Hydroxy-2-(di-n-propylamino)tetralin (8-OH-DPAT). Diese Wirkstoffe mindern die beschriebenen neurologischen Defizite allerdings nur bedingt, eine effektive Therapie für diese Erkrankungen gibt es derzeit noch nicht.

Eine weitere neuropathologische Erkrankung, die große Bevölkerungsteile betrifft, ist Migräne. Migräne äußert sich in den meisten Fällen durch immer wiederkehrende Kopfschmerzen, von denen schätzungsweise 8 Mio Personen, d.h. 3-5 % aller Kinder, 7% aller Männer und 14% aller Frauen, betroffen sind. Obwohl eine genetische Prädisposition propagiert wird, scheinen die Ursachen doch vielschichtig zu sein (Diener H.C. et al., Arzneimitteltherapie 15:387-394 (1997).Es dominieren zwei Hypothesen. Die schon seit langem bekannte Gefäß-Theorie schlägt als Ursache einen Dilatationsvorgang des inneren und äußeren zerebralen Gefäßsystems vor. Die neurogene Theorie stützt sich auf eine Ausschüttung vasoaktiver Neurotransmitter, vornehmlich Neuropeptide, wie Substanz P and Neurokinin, aus Axonen der Vaskulatur infolge einer Stimulierung bestimmter Gehirngewebe innervierender Ganglien, was zu entzündlichen Reaktionen und somit zu Schmerz führen soll.

Eine Kausaltherapie zur Behandlung von Migräne gibt es derzeit noch nicht. Zwei verschiedene Behandlungsmethoden kommen momentan zur Anwendung: eine erste, prophylaktische Therapie zur Vorbeugung gegen wiederkehrende Migräneattacken und eine zweite, symptomatische Therapie zur Unterdrückung akuter Symptome bei Attacken. Prophylaktisch werden migränespezifische Wirkstoffe, wie Sanmigran^{R}, Nocerton^{R}, Desernil^{R} und Vidora^{R}, aber auch gewöhnlich für andere Indikationenverwendete Wirkstoffe, wie Beta-Blocker, antiemetische Wirkstoffe wie Sibelium^{R}, Antidepressiva wie Laroxyl^{R}, oder antiepileptsche Wirkstoffe wie Depakin^{R}, verabreicht. Im Rahmen der Akuttherapie gibt man Analgetika, wie Aspirin^{R} Paracetamol^{R} oder Optalidon^{R}, nichtsteroidale Antiinflammatika, wie Cebutid^{R}, Voltaren^{R}, Brufen^{R}, Ponstyl^{R}, Profenid^{R}, Apranx^{R} und Naprosin^{R} gegen den Schmerz und Entzündung, Ergotalkaloide, wie Ergotamin, Dihydroergotamin, die eine Vasokonstriktion auslösen können, oder Substanzen der Triptan-Familie, wie Sumatriptan, Naramig^{R}, und AscoTop^{R} mit hoher Affinität für 5-HT1 D-Rezeptoren. Letztere Substanzen wirken als Agonisten und blockieren die Vasodilatation.

Die genannten Wirkstoffe sind allerdings nicht optimal für die Behandlung von Migräne geeignet. Nichtopioide Analgetika haben häufig Nebenwirkungen. Der komplexe Wirkungsmechanismus der Ergotalkaloide führt infolge der starken peripheren Vasokonstriktion zu Nebenwirkungen wie Hypertonie and Gangrän. Zu der Triptan-Familie gehörende Verbindungen wirken ebenfalls nicht völlig zufriedenstellend (Pfaffenrath V. Münch. Med. Wschr. 625-626 (1998).

Die Verwendung von 5-HT5-Rezeptor Liganden allgemein zur Behandlung von Migräne und anderen cerebrovaskulären Erkrankungen ist in WO 00/041472, zur Behandlung von neurodegenerativen und neuropsychiatrischen Erkrankungen in WO 00/041696 beschrieben.

Guanidinverbindungen wurden bisher nicht als 5-HT5 Liganden verwendet.

Substiuierte Guanidine sind generell bekannt als H2-Antagonisten, als Inhibitoren der H+K+-ATPase, Magensäure-Sekretionshemmer, und in diesen Eigenschaften als Mittel zur Behandlung des PUD-Syndroms (Peptic Ulcer Disease). Verschiedenste substituierte Thiazol-Guanidine sind generell in der Literatur beschrieben als Verbindungen mit antiviraler, bakterizider, antimikrobieller und/oder antiinflammatorischer Wirkung, als Protease-Inhibitoren oder Vitronectin-Antagonisten.

WO-9911637 beschreibt allgemein substituierte *N*-{4-[Anilinoalkyl)phenyl]-1,3-thiazol-2-yl}-*N'*-benzylguanidine und ihre Verwendung als Protease-Inhibitoren. WO-9850373 beschreibt N- substituierte *N*-[4-(Phenoxyphenyl)-1,3-thiazol-2-yl]guanidine und ihre Verwendung als Bakterizide. In WO-9605187 und EP-545376 wird die Herstellung von substituierten 4-(3-Aminomethylphenyl)-2-thiazolylguanidinen und ihre Verwendung als H2-Rezeptorantagonisten beschrieben. JP-59225172 beschreibt allgemein N-Alkylsubstituierte 4-Phenylthiazolguanidine als H1- und H2-Rezeptorantagonisten und ihre Verwendung als Magensäure Sekretionshemmer. NL-7700083, US-4089965, DE-2700951, BE-850148 beschreiben N-Aryl- substituierte 4-Phenyl-thiazolguanidine mit antiviralen Eigenschaften, speziell als Antirhinovirus-Agentien. EP-3640 beschreibt allgemein substituierte *N*-[4-(3-aminophenyl)-1,3-thiazol-2-yl]guanidine und ihre antisekretorischen Eigenschaften. JP-0817614 beschreibt die Herstellung von 2-[(Diaminomethylen)amino]-4-pyrimidinylthiazolguanidinen mit H2-antagonistischen, antiulcer und antibakteriellen Eigenschaften.

In WO-9518126, JP-09040671, WO-9403450, WO-9303028, EP-355612, US-4814341 werden N-substituierte 4-Furylthiazolguanidine mit antibaktieriellen Eigenschaften (besonders Heliobacter pylori) und ihre Verwendung zur Behandlung von Gastritis und allgemeinem PUD Syndrom (Ulcer, Zollinger-Ellison Syndrom, Oseophagititis, gastrointestinalen Blutungen) beschrieben. WO-9429304 und JP-08245621 beschreiben entsprechende 4-Thienylthiazolguanidine mit ähnlichem Verwendungszweck. WO-9216526, EP-417751 beschreiben generell die Herstellung von N-substituierten 4-Hetaryl-substituierten Thiazolguanidinen, speziell entsprechende Pyridyl- und Thiazolyl-Derivate, H2-antagonistische Eigenschaften und ihre Verwendung als Antiulcer- und antimikrobielle Agentien. EP-259085 beschreibt ebenfalls die Herstellung von 4-Hetarylsubstituierten Thiazolguanidinen, speziell entsprechende Pyrrolyl- und Indolyl-Derivate, zur Behandlung des PUD Syndroms. JP-59225186 und JP-59036674 beschreiben 4-Hetaryl-substituierte Thiazolguanidine, speziell 2-Furyl- und 2-Pyridyl-Derivate, und ihre antisekretorischen Eigenschaften.

WO-9324485, JP-07188197 beschreibt 4-Phenyloxazolguanidine als H2-Rezeptorantagonisten mit zusätzlich antibakteriellen Eigenschaften zur Behandlung von gastrointestinalen Krankheiten.

In Journal of Chemical and Engineering Data 1978, 23 (2), 177-8 werden N-Aryl-N'-2-(thiazolyl-, napthothiazolyl-, benzothiazolyl)guanidine als Substanzen mit Antimalaria-Wirkung und/oder analgetischen Eigenschaften beschrieben. In Bioorg.Med.Chem.Lett. 2000 (10), 265-268, wird *N*-[2-(2-methoxyphenyl)ethyl]-*N'*-1,3-thiazol-2-ylguanidin im Rahmen bioisosterer Modifikationen von PETT-HIV-1 RT-Inhibitoren beschrieben, die Verbinung zeigte aber keine nachweisbare biologische Aktivität. Unterschiedlich substituierte Thiazolguanidine werden in folgenden Literaturstellen als antimikrobielle Substanzen mit Aktivität gegen Heliobacter pyloris beschrieben: 2-(Substituierte Guanidino)-4-arylthiazole and Aryloxazole in J. Med. Chem. 2002, 45(1), 143-150; 2-( substituierte Guanidino)-4-phenylthiazole und rigidisierte Derivative in J.Med.Chem. 2000 ,17,3315-3321; 2-[(Arylalkyl)guanidino]-4-furylthiazole in J.Med.Chem. 1999, 42(15), 2920-2926; Alkylguanidino-4-furylthiazole in Bioorg.Med.Chem. Lett. 1998, 8(11), 1307-1312; 2-(Alkylguanidino)-4-furylthiazole und Analoge in J.Med.Chem. 1997, 40(16), 2462-2465. Als Inhibitoren der H⁺, K⁺-ATPase werden 4-substituierte 2-Guanidinothiazole in J.Med.Chem. 1990, 33, 543-552; und 4-Indolyl-2-guanidinothiazole in THEOCHEM 2001, 539, 245-251; THEOCHEM 2002, 580, 263-270; beschrieben.

### Zusammenfassung der Erfindung

Aufgabe der vorliegenden Erfindung ist es, Verbindungen zur Verfügung zu stellen, die die Behandlung neuropathologischer, neuropsychiatrischer und neurodegenerativer Störungen mit ausreichender Wirksamkeit und geringen Nebenwirkungen zu ermöglichen.

Überraschenderweise wurde nun gefunden, dass Substanzen der allgemeinen Formel I oder IA als Liganden des 5-HT5 Rezeptors wirken und deshalb eine Behandlung der damit verbundenen, oben beschriebenen Krankheitszustände sowie der damit zusammenhängenden Symptome und Fehlfunktionen ermöglicht wird.

Die vorliegende Erfindung betrifft daher eine Guanidinverbindüng der allgemeinen Formel I 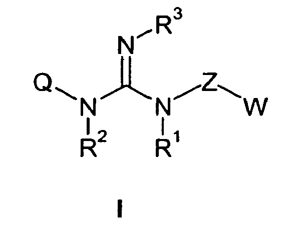 entsprechende enantiomere, diastereomere und/oder tautomere Formen davon sowie pharmazeutisch annehmbare Salze davon, wobei die angegebenen Reste die folgenden Definitionen besitzen:
**W:**
   einen Rest der allgemeinen Formel **W1** oder **W2** 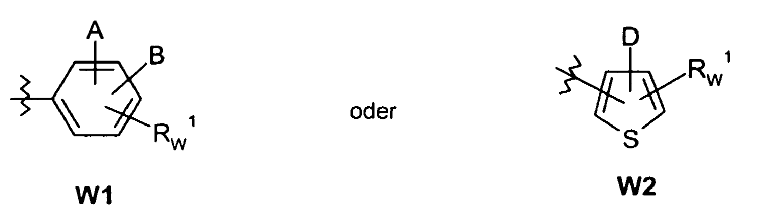 worin
**A:**
   NO₂, NH₂, OH, CN, CF₃, OCF₃, CHF₂, OCHF₂, COOH, O-CH₂-COOH, Halogen, SH, oder
   jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocyclo-alkyl, Aryl, Hetaryl, Heterocycloalkyl, C₁-C₄-Alkylen-Hetaryl oder C₁-C₄-Alkylen-Aryl, oder
   O-R_{A}¹, CO-R_{A}¹, S-R_{A}¹, SO-R_{A}¹, CO-O-R_{A}¹, NR_{A}⁴-CO-O-R_{A}¹, O-CH₂-COO-R_{A}¹, NRA²R_{A}³, CONH₂, SO₂NH₂, NR_{A}⁴-CO-R_{A}¹, SO₂-R_{A}¹, NR_{A}⁴-SO₂-R_{A}¹, SO₂-NR_{A}²R_{A}³ oder CO-NR_{A}²R_{A}³;
**R_{A}¹:**
   jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇Cycloalkyl, C₁-C₄-Alkylen-Heterocyclo-alkyl, Aryl, Hetaryl, Heterocycloalkyl, C₁-C₄-Alkylen-Aryl, C₂-C₆-Alkenylen-Aryl oder C₁-C₆-Alkylen-Hetaryl;
**R_{A}²:**
   Wasserstoff, OH, CN, oder
   jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-AlkylenHetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
**R_{A}³:**
   jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-AlkylenHetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
   oder die Reste **R_{A}²** und **R_{A}³** zusammen mit dem Stickstoff einen 3 bis 7-gliedrigen, gegebenenfalls substituierten, gesättigten oder aromatischen Hetero-cyclus, der ein, zwei oder drei weitere verschiedene oder gleiche Heteroatome aus der Gruppe O, N, S enthalten kann, bilden; wobei gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und wobei der dadurch gebildete Cyclus gegebenenfalls substituiert sein kann oder an diesem Cyclus ein weiterer, gegebenenfalls substituierter Cyclus ankondensiert sein kann;
**R_{A}⁴:**
   Wasserstoff, oder
   jeweils gegebenenfalls substituiertes C₁-C₆-Alkyl, C₁-C₆-Alkylen-O-C₁-C₆-Alkyl, C₂-C₆-Alkenyl, C₃-C₁₂-Alkinyl, CO-C₁-C₆-Alkyl, CO-O-C₁-C₆-Alkyl, SO₂-C₁-C₆-Alkyl, C₃-C₇-Cycloalkyl, Aryl, C₁-C₄-Alkylen-Aryl, CO-O-Arylalkyl, CO-C₁-C₄-Alkylen-Aryl, CO-Aryl, SO₂-Aryl, Hetaryl, CO-Hetaryl oder SO₂-C₁-C₄-Alkylen-Aryl;
**B:**
   Wasserstoff oder wie Rest **A** definiert ist,
   oder jeweils unabhängig voneinander zwei der Reste **A, B** oder **R_{w}¹** zusammen einen 3 bis 7-gliedrigen, gegebenenfalls substituierten, gesättigten oder ungesättigten Carbocyclus oder einen gegebenenfalls substituierten, gesättigten oder ungesättigten oder aromatischen Heterocyclus, der ein, zwei oder drei weitere verschiedene oder gleiche Heteroatome aus der Gruppe O, N, S enthalten kann, bilden; wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
**R_{w}¹:**
   Wasserstoff, OH, Halogen, NO₂, NH₂, CN, CF₃, CHF₂, O-CF₃, O-CHF₂, oder
   jeweils gegebenenfalls substituiertes C₁-C₆-Alkyl, C₃-C₇-Cycloalkyl, C₁-C₆-Alkylen-O-C₁-C₆-Alkyl, C₁-C₆-Thioalkyl, Aryl, Hetaryl, O-C₁-C₆-Alkyl, O-Aryl, O-Benzyl, C₁-C₆-Alkylamino, C₁-C₆-Dialkylamino, Pyrrolidinyl, Piperidinyl, Morpholinyl, CO-C₁-C₆-Alkyl, SO₂-C₁-C₆-Alkyl, CO-Aryl, SO₂-Aryl, CO-C₁-C₄-Alkylen-Aryl, SO₂-C₁-C₄-Alkylen-Aryl, SO-Aryl, CONH₂, CONH-C₁-C₆-Alkyl, SO₂NH-C₁-C₆-Alkyl, CON-(C₁-C₆-Alkyl)₂, SO₂N-(C₁-C₆-Alkyl)₂, NH-SO₂-C₁-C₆-Alkyl oder NH-CO-C₁-C₆-Alkyl;
**D:**
   wie Rest **A** definiert ist;
**Z:**
   einen Rest der allgemeinen Formel **Z1** 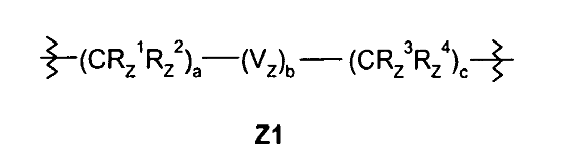 mit den Indizes
      a=0-4
      b = 0, 1
      c=0-4
      wobei die Summe aus a, b und c mindestens 1 und höchstens 5 beträgt;
**R_{z}¹, R_{z}², R_{z}³, R_{z}⁴** unabhängig voneinander :
   Wasserstoff, Halogen, OH, oder
   jeweils gegebenenfalls substituiertes C₁-C₆-Alkyl, C₂-C₆-Alkenyl, C₂-C₆-Alkinyl, C₁-C₆-Alkylen-C₃-C₇-Cycloalkyl, C₃-C₇-Cycloalkyl, Aryl, C₁-C₄-Alkylen-Aryl, Het- aryl oder C₁-C₄-Alkylen-Hetaryl, oder
   jeweils unabhängig voneinander zwei Reste **R_{z}¹** und **R_{z}²** oder **R_{z}³** und **R_{z}⁴** zusammen einen 3 bis 7-gliedrigen, gegebenenfalls substituierten, gesättigten oder ungesättigten Carbo- oder Heterocyclus bilden, wobei der Heterocyclus bis zu drei Heteroatome aus der Gruppe O, N oder S enthalten kann;
**V**_{z}**:**
   -CO-, -CO-NR_{z}⁵-, -NR_{z}⁵-CO-, -O-, -S-, -SO-, -SO₂-, -SO₂-NR_{z}⁵-, -NR_{z}⁵-SO₂-, -CS-, -CS-NR_{z}⁵-, -NR_{z}⁵-CS-, -CS-O-, -O-CS-, -CO-O-, -O-CO-, -O-, Ethinylen, -C(=CR_{z}⁶R_{z}⁷)-, -CR_{z}⁶=CR_{z}⁷-, -NR_{z}⁵-CO-NR_{z}^{5*}-, -O-CO-NR_{z}⁵-, - NR_{z}⁵-;
**R_{z}⁵, R_{z}⁵*** unabhängig voneinander:
   Wasserstoff oder
   jeweils gegebenenfalls substituiertes C₁-C₆-Alkyl, C₁-C₆-Alkylen-O-C₁-C₆-Alkyl, C₂-C₆-Alkenyl, C₃-C₁₂-Alkinyl, CO-C₁-C₆-Alkyl, CO-O-C₁-C₆-Alkyl, SO₂-C₁-C₆-Alkyl, C₃-C₇-Cycloalkyl, Aryl, C₁-C₄-Alkylen-Aryl, CO-O-C₁-C₄-Alkylen-Aryl, CO-C₁-C₄-Alkylen-Aryl, CO-Aryl, SO₂-Aryl, Hetaryl, CO-Hetaryl oder SO₂-C₁-C₄-Alkylen-Aryl;
**R_{z}⁶, R_{z}⁷** unabhängig voneinander:
   Wasserstoff, OH oder
   jeweils gegebenenfalls substituiertes C₁-C₆-Alkyl, C₁-C₄-Alkoxy, C₂-C₆-Alkenyl, C₂-C₆-Alkinyl, C₁-C₆-Alkylen-C₃-C₇-Cycloalkyl, C₃-C₇-Cycloalkyl, Aryl, C₁-C₄-Alkylen-Aryl, Hetaryl oder C₁-C₄-Alkylen-Hetaryl;
**R¹, R², R³** unabhängig voneinander:
   Wasserstoff, OH, CN, oder
   jeweils gegebenenfalls substituiertes C₁-C₆-Alkyl, O-C₁-C₆-Alkyl, C₁-C₆-Alkylen-O-C₁-C₆-Alkyl, C₃-C₇-Cycloalkyl, O-C₃-C₇-Cycloalkyl, Aryl, Hetaryl, C₁-C₄-Alkylen-Aryl, C₁-C₄-Alkylen-Hetaryl, O-Aryl, O-C₁-C₄-Alkylen-Aryl, O-Hetaryl, O-C₁-C₄-Alkylen-Hetaryl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C6-Alkyl, CO-O-Aryl, CO-O-Hetaryl, CO-O-C₁-C₄-Alkylen-Aryl, SO₂-C₁-C₆-Alkyl, SO₂-Aryl, SO₂-Hetaryl, SO₂- C₁-C₄-Alkylen-Aryl, OCO-C₁-C₆-Alkyl, OCO-Aryl, OCO-Hetaryl, OCO-C₁-C₄-Alkylen-Aryl, OCO-C₁-C₄-Alkylen-Hetaryl, SO₂-C₁-C₆-Alkyl, SO₂-Aryl, SO₂-Hetaryl oder SO₂-C₁-C₄-Alkylen-Aryl, oder
   jeweils unabhängig vom dritten Rest zwei Reste von **R¹, R²** oder **R³** zusammen einen 5 bis 7-gliedrigen, gegebenenfalls substituierten, gesättigten oder ungesättigten Carbocyclus, oder einen gegebenenfalls substituierten, gesättigten oder ungesättigten Heterocyclus, der ein, zwei oder drei weitere verschiedene oder gleiche Heteroatome aus der Gruppe O, N, S enthalten kann, bilden, wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
**Q:**
   einen zweifach substituierten 5-gliedrigen Hetaryl-Rest, ausgewählt aus **Q1** bis **Q7** 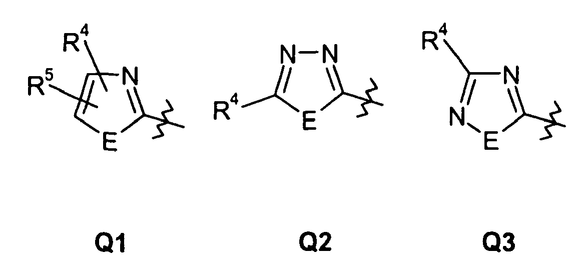 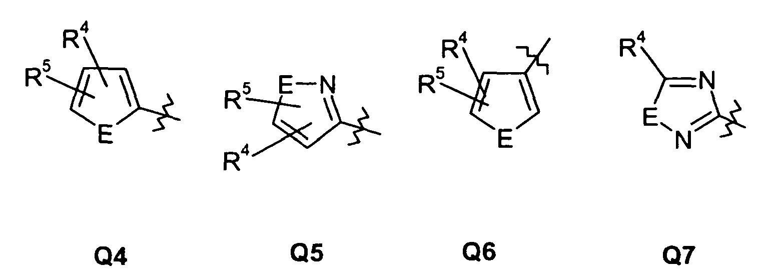
**E:** O, N-R_{Q}¹ oder S;
**R_{Q}¹:**
   Wasserstoff, oder
   jeweils gegebenenfalls substituiertes C₁-C₄-Alkyl, CO-C₁-C₄-Alkyl, SO₂-C₁-C₄-Alkyl, CO-O-C₁-C₄-Alkyl, Aryl, C₁-C₄-Alkylen-Aryl, CO-Aryl, CO-Hetaryl, SO₂-Aryl, SO₂-Hetaryl, CO-O-Aryl, CO-C₁-C₄-Alkylen-Aryl, SO₂-C₁-C₄-Alkylen-Aryl oder CO-O-C₁-C₄-Alkylen-Aryl;
**R⁴, R⁵** jeweils unabhängig voneinander einen Rest, ausgewählt aus den Gruppen **1.), 2.), 3.), 4.), 5.), 6.)** oder **7.):**
   **1.)** Wasserstoff, Halogen, CN, CF₃, CHF₂, oder
      jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryl, C₁-C₄-Alkylen-Hetaryl, C₁-C₆-Alkylen-O-C₁-C₆-Alkyl, C₁-C₆-Alkylen-O-Aryl, COO-C₁-C₄-Alkyl oder C₁-C₄-Alkylen-COO-C₁-C₄-Alkyl;
   **2.)** Phenyl oder Naphthyl, die jeweils mit **R_{Q}², R_{Q}³** und **R_{Q}⁴** substituiert sind, wobei
**R_{Q}², R_{Q}³** und **R_{Q}⁴ jeweils** unabhängig voneinander einen Substituenten aus der folgenden Gruppe darstellen:
   Wasserstoff, NO₂, NH₂, OH, CN, CF₃, CHF₂, OCF₃, OCHF₂, COOH, O-CH₂-COOH, SH, Halogen, oder
      jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-Alkylen-Hetaryl, oder
      O-R_{Q}⁵, S-R_{Q}⁵, NR_{Q}⁷R_{Q}⁸, CO-OR_{Q}⁶, NR_{Q}⁸-CO-O-R_{Q}⁶, O-CH₂-COO-R_{Q}⁶, NR_{Q}⁸-CO-R_{Q}⁶, SO₂-R_{Q}⁶, NR_{Q}⁸-SO₂-R_{Q}⁶, SO₂NH₂, CONH₂, SO₂-NR_{Q}⁷R_{Q}⁸ oder CO-NR_{Q}⁷R_{Q}⁸, oder
   jeweils zwei der Reste aus **R_{Q}², R_{Q}³** oder **R_{Q}⁴** zusammen einen 3 bis 7-gliedrigen, gegebenenfalls substituierten, gesättigten, ungesättigten oder aromatischen Carbocyclus oder einen gegebenenfalls substituierten, gesättigten, ungesättigten aromatischen Heterocyclus, der bis zu drei weitere verschiedene oder gleiche Heteroatome O, N, S enthalten kann, bilden, und gegebenenfalls zwei an diesem Heterocyclus substituierte Reste können zusammen einen anellierten, gesättigten, ungesättigten oder aromatischen Carbocyclus oder Heterocyclus bilden, wobei der Heterocyclus bis zu drei verschiedene oder gleiche Heteroatome O, N, S enthalten kann, und der gebildete Cyclus kann gegebenenfalls substituiert sein oder an diesem Cyclus kann ein weiterer, gegebenenfalls substituierter Cyclus ankondensiert sein.
**R_{Q}⁵** jeweils gegebenenfalls substituiertes C₂-C₆-Alkenyl, C₂-C₆-Alkinyl, C₁-C₄-Alkylen-C₃-C₇-Cycloalkyl, C₁-C₄-Alkylen-Heterocycloalkyl, Heterocycloalkyl oder Hetaryl, oder
   C₁-C₆-Alkyl, das gegebenenfalls substituiert ist mit einem Substituenten aus der Gruppe, bestehend aus Halogen, NO₂, NH₂, OH, CN, CF₃, CHF₂, OCF₃, OCHF₂, NH-(C₁-C₆-Alkyl) und N(C₁-C₆-Alkyl)₂;
**R_{Q}⁶** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl oder C₁-C₆-Alkylen-O-C₁-C₆-Alkyl;
**R_{Q}⁷** Wasserstoff, OH, CN, oder
   jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, C₁-C₄-Alkylen-Aryl, C₁-C₄-Alkylen-Hetaryl, CO-Aryl, CO-Hetaryl, CO-C₁-C₄-AlkylenAryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-AlkylenHetaryl, SO₂-C₁-C₆-Alkyl, SO₂-Aryl, SO₂Hetaryl, SO₂-C₁-C₄-Alkylen-Aryl oder SO₂-C₁-C₄-Alkylen-Hetaryl;
**R_{Q}⁸** Wasserstoff oder
   jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
   oder die Reste **R_{Q}⁷** und **R_{Q}⁸** zusammen mit dem Stickstoff einen 3 bis 7-gliedrigen, gegebenenfalls substituierten, gesättigten oder aromatischen Heterocyclus, der ein, zwei oder drei weitere verschiedene oder gleiche Heteroatome O, N, S enthalten kann, bilden; und gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und der gebildete Cyclus gegebenenfalls substituiert sein kann oder an diesem Cyclus ein weiterer, gegebenenfalls substituierter Cyclus ankondensiert sein kann;
**3.)** einen 5- oder 6-gliedrigen, gegebenenfalls mit 1 oder 2 Substituenten substituierten Hetaryl-Rest aus der Gruppe, bestehend aus:
   2-Pyrrolyl, 3-Pyrrolyl, 2-Thiazolyl, 4-Thiazolyl, 5-Thiazolyl, 2-Oxazolyl, 4-Oxazolyl, 5-Oxazolyl, 2-Pyrimidyl, 4-Pyrimidyl, 5-Pyrimidyl, 6-Pyrimidyl, 3-Pyrazolyl, 4-Pyrazolyl, 5-Pyrazolyl, 3-Isothiazolyl, 4-Isothiazolyl, 5-Isothiazolyl, 2-Imidazolyl, 4-Imidazolyl, 5-Imidazolyl, 3-Pyridazinyl, 4-Pyridazinyl, 5-Pyridazinyl, 6-Pyridazinyl, 3-Isoxazolyl, 4-Isoxazolyl, 5-Isoxazolyl, Thiadiazolyl, Oxadiazolyl oder Triazinyl oder deren anellierten Derivate Indazolyl, Benzothiophenyl, Benzofuranyl, Indolinyl, Benzimidazolyl, Benzthiazolyl, Benzoxazolyl, Chinolinyl und Isochinolinyl; oder
   gegebenenfalls mit 1 oder 2 Substituenten substituiertes 2-Thienyl oder 3-Thienyl, wobei die Substituenten ausgewählt sind aus der Gruppe, bestehend aus Halogen, NO₂, NH₂, OH, CN, CF₃, OCF₃, CHF₂, O-CHF₂, C₁-C₆-Alkyl, O-C₁-C₆-Alkyl, NH-(C₁-C₆-Alkyl), N(C₁-C₆-Alkyl)₂, NHCO-C₁-C₄-Alkyl, NHSO₂-C₁-C₄-Alkyl und SO₂-C₁-C₄-Alkyl;
**4.)** beide Reste **R⁴** und **R⁵** zusammen einen 4 bis 7-gliedrigen, gegebenenfalls substituierten, gesättigten oder ungesättigten oder aromatischen Carbocyclus oder einen 5- oder 6-gliedrigen gegebenenfalls substituierten, gesättigten oder ungesättigten oder aromatischen Heterocyclus, der bis zu drei weitere verschiedene oder gleiche Heteroatome O, N, S enthalten kann, bilden, und mit bis zu zwei weiteren Resten substituiert sein kann, wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
**5.)** einen C₅-C₁₈- bi- oder tricyclischen, gesättigten Kohlenwasserstoffrest.
**6.)** jeweils gegebenenfalls substituiertes C₁-C₈-Alkyl-NH₂, C₁-C₈-Alkyl-NR_{Q}⁷R_{Q}⁸, C₁-C₈-Alkyl-CO-NR_{Q}⁷R_{Q}⁸, C₁-C₈-Alkyl-SO₂NQ(NR_{Q}⁸, C₁-C₈-Alkyl-CO-NH₂, C₁-C₈-Alkyl-SO₂NH₂, CO-NH₂, CO-NR_{Q}⁷R_{Q}⁸, SO₂NH₂, SO₂NR_{Q}⁷R_{Q}⁸, NR_{Q}⁷R_{Q}⁸;
**7.)** einen 4-7-gliedrigen mono- oder bicyclischen gesättigten oder ungesättigten Heterocyclus, der bis zu zwei verschiedene oder gleiche Heteroatome aus der Gruppe O, N oder S enthalten kann, wobei dieser Cyclus ebenfalls mehrfach substituiert sein kann. Für den Fall, dass der Heterocyclus ein N-Atom enthält, kann dies mit einem Rest R_{Q}⁷ substituiert sein.

In einer Ausführungsform betrifft die vorliegende Erfindung die folgenden Guanidinverbindungen der Formel (I), wobei
**W:**
   einen Rest der allgemeinen Formel **W1** oder **W2** 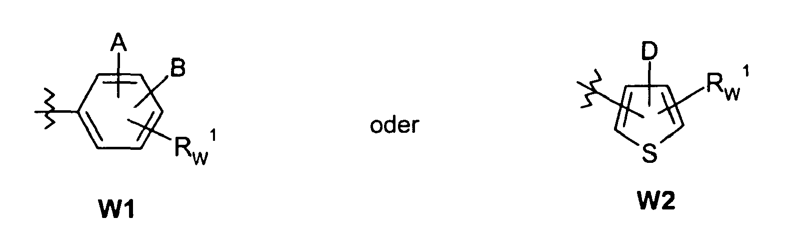 worin
**A:**
   NO_{2,} NH₂, OH, CN, CF₃, OCF₃, CHF₂, OCHF₂, COOH, O-CH₂-COOH, Halogen, SH, oder
   jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocyclo-alkyl, Aryl, Hetaryl, Heterocycloalkyl, C₁-C₄-Alkylen-Hetaryl oder C₁-C₄-Alkylen-Aryl, oder
   O-R_{A}¹, CO-R_{A}¹, S-R_{A}¹, SO-R_{A}¹, CO-O-R_{A}¹, NR_{A}⁴-CO-O-R_{A}¹, O-CH₂-COO-R_{A}¹, NR_{A}²R_{A}³, CONH₂, SO₂NH₂, NR_{A}⁴-CO-R_{A}¹, SO₂-R_{A}¹, NR_{A}⁴-SO₂-R_{A}¹, SO₂-NRA₂²R_{A}³ oder CO-NR_{A}²R_{A}³;
**R_{A}¹:**
   jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocyclo-alkyl, Aryl, Hetaryl, Heterocycloalkyl, C₁-C₄-Alkylen-Aryl, C₂-C₆-Alkenylen-Aryl oder C₁-C₆-Alkylen-Hetaryl;
**R_{A}²:**
   Wasserstoff, OH, CN, oder
   jeweils gegebenenfalls substituiertes C₁-C₆-Alkyl, C₂-C₆-Alkenyl, C₂-C₆-Alkinyl, C₁-C₄-Alkylen-C₃-C₇-Cyclalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-AlkylenHetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
**R_{A}³:**
   jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-AlkylenHetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
   oder die Reste **R_{A}²** und **R_{A}³** zusammen mit dem Stickstoff einen 3 bis 7-gliedrigen, gegebenenfalls substituierten, gesättigten oder aromatischen Heterocyclus, der ein, zwei oder drei weitere verschiedene oder gleiche Heteroatome aus der Gruppe O, N, S enthalten kann, bilden; wobei
   gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und wobei der dadurch gebildete Cyclus gegebenenfalls substituiert sein kann oder an diesem Cyclus ein weiterer, gegebenenfalls substituierter Cyclus ankondensiert sein kann;
**R_{A}⁴:**
   Wasserstoff, oder
   jeweils gegebenenfalls substituiertes C₁-C₆-Alkyl, C₁-C₆-Alkylen-O-C₁-C₆-Alkyl, C₂-C₆-Alkenyl, C₃-C₁₂-Alkinyl, CO-C₁-C₆-Alkyl, CO-O-C₁-C₆-Alkyl, SO₂-C₁-C₆-Alkyl, C₃-C₇-Cycloalkyl, Aryl, C₁-C₄-Alkylen-Aryl, CO-O-Arylalkyl, CO-C₁-C₄-Alkylen-Aryl, CO-Aryl, SO₂-Aryl, Hetaryl, CO-Hetaryl oder SO₂-C₁-C₄-Alkylen-Aryl;
**B:**
   Wasserstoff oder wie Rest **A** definiert ist,
   oder jeweils unabhängig voneinander zwei der Reste **A, B** oder **R_{w}¹** zusammen einen 3 bis 7-gliedrigen, gegebenenfalls substituierten, gesättigten oder ungesättigten Carbocyclus oder einen gegebenenfalls substituierten, gesättigten oder ungesättigten oder aromatischen Heterocyclus, der ein, zwei oder drei weitere verschiedene oder gleiche Heteroatome aus der Gruppe O, N, S enthalten kann, bilden; wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
**R_{w}¹:**
   Wasserstoff, OH, Halogen, NO₂, NH₂, CN, CF₃, CHF₂, OCF₃, OCHF₂, oder jeweils gegebenenfalls substituiertes C₁-C₆-Alkyl, C₃-C₇-Cycloalkyl, C₁-C₆-Alkylen-O-C₁-C₆-Alkyl, C₁-C₆-Thioalkyl, Aryl, Hetaryl, O-C₁-C₆-Alkyl, O-Aryl, O-Benzyl, C₁-C₆-Alkylamino, C₁-C₆-Dialkylamino, Pyrrolidinyl,
   Piperidinyl, Morpholinyl, CO-C₁-C₆-Alkyl, SO₂-C₁-C₆-Alkyl, CO-Aryl, SO₂-Aryl, CO-C₁-C₄-Alkylen-Aryl, SO₂-C₁-C₄-Alkylen-Aryl, SO-Aryl, CONH₂, CONH-C₁-C₆-Alkyl, SO₂NH-C₁-C₆-Alkyl, CON-(C₁-C₆-Alkyl)₂, SO₂N-(C₁-C₆-Alkyl)₂, NH-SO₂-C₁-C₆-Alkyl oder NH-CO-C₁-C₆-Alkyl;
**D:**
   wie Rest **A** definiert ist;
**Z:**
   einen Rest der allgemeinen Formel **Z1** 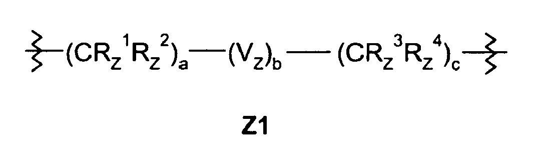 mit den Indizes
      a=0-4
      b=0, 1
      c=0-4
      wobei die Summe aus a, b und c mindestens 1 und höchstens 5 beträgt;
**R_{z}¹, R_{z}², R_{z}³, R_{z}⁴** unabhängig voneinander :
   Wasserstoff, Halogen, OH, oder
   jeweils gegebenenfalls substituiertes C₁-C₆-Alkyl, C₂-C₆-Alkenyl, C₂-C₆-Alkinyl, C₁-C₆-Alkylen-C₃-C₇-Cycloalkyl, C₃-C₇-Cycloalkyl, Aryl, C₁-C₄-Alkylen-Aryl, Hetaryl oder C₁-C₄-Alkylen-Hetaryl, oder
   jeweils unabhängig voneinander zwei Reste **R_{z}¹** und **R_{z}²** oder **R_{z}³** und **R_{z}⁴** zusammen einen 3 bis 7-gliedrigen, gegebenenfalls substituierten, gesättigten oder ungesättigten Carbo- oder Heterocyclus bilden, wobei der Heterocyclus bis zu drei Heteroatome aus der Gruppe O, N oder S enthalten kann;
**V_{z}:**
   -CO-, -CO-NR_{z}⁵-, -NR_{z}⁵-CO-, -O-, -S-, -SO-, -SO₂-, -SO₂-NR_{z}⁵-, -NR_{z}⁵-SO₂-, -CS-, -CS-NR_{z}⁵-, -NR_{z}⁵-CS-, -CS-O-, -O-CS-, -CO-O-, -O-CO-, -O-, Ethinylen, -C(=CR_{z}⁶R_{z}⁷)-, -CR_{z}⁶=CR_{z}⁷-, -NR_{z}⁵-CO-NR_{z}^{5*}-, -O-CO-NR_{z}⁵-, NR_{z}⁵-;
**R_{z}⁵**, **R_{z}^{5*}** unabhängig voneinander:
   Wasserstoff oder
   jeweils gegebenenfalls substituiertes C₁-C₆-Alkyl, C₁-C₆-Alkylen-O-C₁-C₆-Alkyl, C₂-C₆-Alkenyl, C₃-C₁₂-Alkinyl, CO-C₁-C₆-Alkyl, CO-O-C₁-C₆-Alkyl, SO₂-C₁-C₆-Alkyl, C₃-C₇-Cycloalkyl, Aryl, C₁-C₄-Alkylen-Aryl, CO-O-C₁-C₄-Alkylen-Aryl, CO-C₁-C₄-Alkylen-Aryl, CO-Aryl, SO₂-Aryl, Hetaryl, CO-Hetaryl oder SO₂-C₁-C₄-Alkylen-Aryl;
**R_{z}⁶**, **R_{z}⁷** unabhängig voneinander:
   Wasserstoff, OH oder
   jeweils gegebenenfalls substituiertes C₁-C₆-Alkyl, C₁-C₄-Alkoxy, C₂-C₆-Alkenyl, C₂-C₆-Alkinyl, C₁-C₆-Alkylen-C₃-C₇-Cycloalkyl, C₃-C₇-Cycloalkyl, Aryl, C₁-C₄-Alkylen-Aryl, Hetaryl oder C₁-C₄-Alkylen-Hetaryl;
**R¹**, **R²**, **R³** unabhängig voneinander:
   Wasserstoff, OH, CN, oder
   jeweils gegebenenfalls substituiertes C₁-C₆-Alkyl, O-C₁-C₆-Alkyl, C₁-C₆-Alkylen-O-C₁-C₆-Alkyl, C₃-C₇-Cloalkyl, O-C₃-C₇-Cycloalkyl, Aryl, Hetaryl, C₁-C₄-Alkylen-Aryl, C₁-C₄-Alkylen-Hetaryl, O-Aryl, O-C₁-C₄-Alkylen-Aryl, O-Hetaryl, O-C₁-C₄-Alkylen-Hetaryl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-Hetaryl, CO-O-C₁-C₄-Alkylen-Aryl, SO₂-C₁-C₆-Alkyl, SO₂-Aryl, SO₂-Hetaryl, SO₂- C₁-C₄-Alkylen-Aryl, OCO-C₁-C₆-Alkyl, OCO-Aryl, OCO-Hetaryl, OCO-C₁-C₄-Alkylen-Aryl, OCO-C₁-C₄-Alkylen-Hetaryl, SO₂-C₁-C₆-Alkyl, SO₂-Aryl, SO₂-Hetaryl oder SO₂-C₁-C₄-Alkylen-Aryl, oder
   jeweils unabhängig vom dritten Rest zwei Reste von **R¹, R²** oder **R³** zusammen einen 5 bis 7-gliedrigen, gegebenenfalls substituierten, gesättigten oder ungesättigten Carbocyclus, oder einen gegebenenfalls substituierten, gesättigten oder ungesättigten Heterocyclus, der ein, zwei oder drei weitere verschiedene oder gleiche Heteroatome aus der Gruppe O, N, S enthalten kann, bilden, wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
**Q:**
   einen zweifach substituierten 5-gliedrigen Hetaryl-Rest, ausgewählt aus **Q1** bis **Q6** 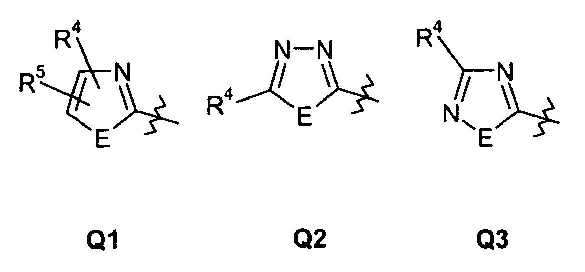 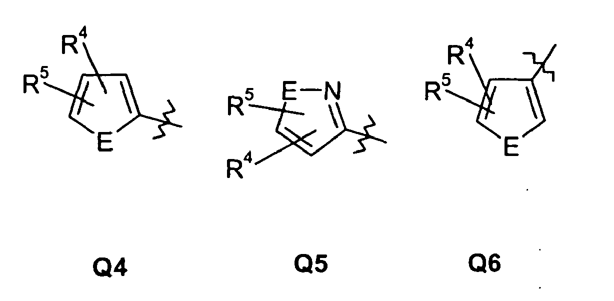
**E:** O, N-R_{Q}¹ oder S;
**R_{Q}¹:**
   Wasserstoff, oder
   jeweils gegebenenfalls substituiertes C₁-C₄-Alkyl, CO-C₁-C₄-Alkyl, SO₂-C₁-C₄-Alkyl, CO-O-C₁-C₄-Alkyl, Aryl, C₁-C₄-Alkylen-Aryl, CO-Aryl, CO-Hetaryl, SO₂-Aryl, SO₂-Hetaryl, CO-O-Aryl, CO-C₁-C₄-Alkylen-Aryl, SO₂-C₁-C₄-Alkylen-Aryl oder CO-O-C₁-C₄-Alkylen-Aryl;
**R⁴, R⁵** jeweils unabhängig voneinander einen Rest, ausgewählt aus den Gruppen **1.), 2.), 3.), 4.)** oder **5.):**
   **1.)** Wasserstoff, Halogen, CN, CF₃, CHF₂, oder jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryl, C₁-C₄-Alkylen-Hetaryl, C₁-C₆-Alkylen-O-C₁-C₆-Alkyl, C₁-C₆-Alkylen-O-Aryl, COO-C₁-C₄-Alkyl oder C₁-C₄-Alkylen-COO-C₁-C₄-Alkyl;
   **2.)** Phenyl oder Naphthyl, die jeweils mit **R_{Q}², R_{Q}³** und **R_{Q}⁴** substituiert sind, wobei
      **R_{Q}², R_{Q}³** und **R_{Q}⁴** jeweils unabhängig voneinander einen Substituenten aus der folgenden Gruppe darstellen:
         Wasserstoff, NO₂, NH₂, OH, CN, CF₃, CHF₂, OCF₃, OCHF₂, COOH, O-CH₂-COOH, SH, Halogen, oder
            jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-Alkylen-Hetaryl, oder
            O-R_{Q}⁵, S-R_{Q}⁵, NR_{Q}⁷R_{Q}⁸, CO-OR_{Q}⁶, NR_{Q}⁸CO-O-R_{Q}⁶, O-CH₂-COO-R_{Q}⁶, NR_{Q}⁸-CO-R_{Q}⁶, SO₂-R_{Q}⁶, NR_{Q}⁸-SO₂-R_{Q}⁶, SO₂NH₂, CONH₂, SO₂-NRQ⁷R_{Q}⁸ oder CO-NR_{Q}⁷R_{Q}⁸, oder
         jeweils zwei der Reste aus **R_{Q}², R_{Q}³** oder **R_{Q}⁴** zusammen einen 3 bis 7-gliedrigen, gegebenenfalls substituierten, gesättigten, ungesättigten oder aromatischen Carbocyclus oder einen gegebenenfalls substituierten, gesättigten, ungesättigten aromatischen Heterocyclus, der bis zu drei weitere verschiedene oder gleiche Heteroatome O, N, S enthalten kann, bilden, und gegebenenfalls zwei an diesem Heterocyclus substituierte Reste können zusammen einen anellierten, gesättigten, ungesättigten oder aromatischen Carbocyclus oder Heterocyclus bilden, wobei der Heterocyclus bis zu drei verschiedene oder gleiche Heteroatome O, N, S enthalten kann, und der gebildete Cyclus kann gegebenenfalls substituiert sein oder an diesem Cyclus kann ein weiterer, gegebenenfalls substituierter Cyclus ankondensiert sein;
      **R_{Q}⁵** jeweils gegebenenfalls substituiertes C₂-C₆-Alkenyl, C₂-C₆-Alkinyl, C₁-C₄-Alkylen-C₃-C₇-Cycloalkyl, C₁-C₄-Alkylen-Heterocycloalkyl, Heterocycloalkyl oder Hetaryl, oder C₁-C₆-Alkyl, das gegebenenfalls substituiert ist mit einem Substituenten aus der Gruppe, bestehend aus Halogen, NO₂, NH₂, OH, CN, CF₃, CHF₂, OCF₃, OCHF₂, NH-(C₁-C₆-Alkyl) und N(C₁-C₆-Alkyl)₂;
      **R_{Q}⁶** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl oder C₁-C₆-Alkylen-O-C₁-C₆-Alkyl;
      **R_{Q}⁷** Wasserstoff, OH, CN, oder
         jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, C₁-C₄-Alkylen-Aryl, C₁-C₄-Alkylen-Hetaryl, CO-Aryl, CO-Hetaryl, CO-C₁-C₄-AlkylenAryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-AlkylenHetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
      **R_{Q}⁸** jeweils gegebenenfalls substituiertes C₁-C₆-Alkyl, C₂-C₆-Alkenyl,
         C₂-C₆-Alkinyl, C₃-C₇-Cycloalkyl, C₁-C₄-Alkylen-C₃-C₇Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
         oder die Reste **R_{Q}⁷** und **R_{Q}⁸** zusammen mit dem Stickstoff einen 3 bis 7-gliedrigen, gegebenenfalls substituierten, gesättigten oder aromatischen Heterocyclus, der ein, zwei oder drei weitere verschiedene oder gleiche Heteroatome O, N, S enthalten kann, bilden; und gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und der gebildete Cyclus gegebenenfalls substituiert sein kann oder an diesem Cyclus ein weiterer, gegebenenfalls substituierter Cyclus ankondensiert sein kann;
   **3.)** einen 5- oder 6-gliedrigen, gegebenenfalls mit 1 oder 2 Substituenten substituierten Hetaryl-Rest aus der Gruppe, bestehend aus:
      2-Pyrrolyl, 3-Pyrrolyl, 2-Thiazolyl, 4-Thiazolyl, 5-Thiazolyl, 2-Oxazolyl, 4-Oxazolyl, 5-Oxazolyl, 2-Pyrimidyl, 4-Pyrimidyl, 5-Pyrimidyl, 6-Pyrimidyl, 3-Pyrazolyl, 4-Pyrazolyl, 5-Pyrazolyl, 3-Isothiazolyl, 4-Isothiazolyl, 5-Isothiazolyl, 2-Imidazolyl, 4-Imidazolyl, 5-Imidazolyl, 3-Pyridazinyl, 4-Pyridazinyl, 5-Pyridazinyl, 6-Pyridazinyl, 3-Isoxazolyl, 4-Isoxazolyl, 5-Isoxazolyl, Thiadiazolyl, Oxadiazolyl oder Triazinyl oder deren anellierten Derivate Indazolyl, Benzothiophenyl, Benzofuranyl, Indolinyl, Benzimidazolyl, Benzthiazolyl, Benzoxazolyl, Chinolinyl und
      Isochinolinyl; oder
      gegebenenfalls mit 1 oder 2 Substituenten substituiertes 2-Thienyl oder 3-Thienyl, wobei die Substituenten ausgewählt sind aus der Gruppe, bestehend aus Halogen, NO₂, NH₂, OH, CN, CF₃, OCF₃, CHF₂, O-CHF₂, C₁-C₆-Alkyl, O-C₁-C₆-Alkyl, NH-(C₁-C₆-Alkyl), N(C₁-C₆-Alkyl)₂, NHCO-C₁-C₄-Alkyl, NHSO₂-C₁-C₄-Alkyl und SO₂-C₁-C₄-Alkyl;
   4.) beide Reste **R⁴** und **R⁵** zusammen einen 4 bis 7-gliedrigen, gegebenenfalls substituierten, gesättigten oder ungesättigten oder aromatischen Carbocyclus oder einen 5- oder 6-gliedrigen gegebenenfalls substituierten, gesättigten oder ungesättigten oder aromatischen Heterocyclus, der bis zu drei weitere verschiedene oder gleiche Heteroatome O, N, S enthalten kann, bilden, und mit bis zu zwei weiteren Resten substituiert sein kann, wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
   5.) einen C₅-C₁₈- bi- oder tricyclischen, gesättigten Kohlenwasserstoffrest.

Es ist bevorzugt, dass die Guanidinverbindungen Reste mit den folgenden Definitionen besitzen:
**W: W1;**
**A:** Halogen, OH, CN, CF₃, -CHF₂, OCF₃, OCHF₂, oder jeweils gegebenenfalls substituiertes C₁-C₆-Alkyl oder C₂-C₆-Alkenyl, -O-CH₂-COO-R_{A}¹,- O-R_{A}¹, S-R_{A}¹, NR_{A}²R_{A}³, -NR_{A}⁴-CO-R_{A}¹ oder-CO-NR_{A}⁴-R_{A}¹;
**R_{A}¹:** jeweils gegebenenfalls substituiertes C₁-C₄-Alkyl, C₃-C₇-Cycloalkyl, Phenyl oder Benzyl;
**R_{A}²:** Wasserstoff, oder
   jeweils gegebenenfalls substituiertes C₁-C₄-Alkyl, Phenyl, Benzyl, Phenethyl, CO-C₁-C₄-Alkyl, CO-Aryl, CO-O-C₁-C₄-Alkyl, SO₂-C₁-C₄-Alkyl, SO₂-Aryl, SO₂-Hetaryl, oder SO₂-C₁-C₄-Alkylen-Aryl;
**R_{A}³:** jeweils gegebenenfalls substituiertes C₁-C₄-Alkyl, Phenyl, Benzyl, Phenethyl, CO-C₁-C₄-Alkyl, CO-Aryl, CO-O-C₁-C₄-Alkyl, SO₂-C₁-C₄-Alkyl, SO₂-Aryl, SO₂-Hetaryl, oder SO₂- C₁-C₄-Alkylen-Aryl; oder beide Reste **R_{A}²** Und **R_{A}³** bilden zusammen einen gegebenenfalls substituierten 5-oder 6-gliedrigen gesättigten oder ungesättigten Ring, der bis zu zwei gleiche oder verschiedene Heteroatome aus der Gruppe O und N enthalten kann.
**R_{A}⁴:** Wasserstoff oder einen gegebenenfalls substituierten C₁-C₄-Alkylrest,
**B:** Wasserstoff, oder wie Rest **A**;
**R_{w}¹:** Wasserstoff, F, Cl, CN, CF₃, O-CF₃, oder
   jeweils gegebenenfalls substituiertes C₁-C₄-Alkyl, Aryl, C₁-C₆-Alkylamino oder C₁-C₆-Dialkylamino;
   bei der Formel **Z1** ist die Summe aus a, b, c 1, 2 oder 3;
**R_{z}^{1,} R_{z}², R_{z}^{3,} R_{z}⁴** unabhängig voneinander:
   Wasserstoff, Halogen, OH, gegebenenfalls substituiertes C₁-C₆-Alkyl;
**V_{z}:** -CO-, -CO-NR_{z}⁵-, -NR_{z}⁵-CO-, -O-, -S-;
**R_{z}⁵:** Wasserstoff, CH₃;
**R¹, R² R³** unabhängig voneinander:
   Wasserstoff, OH, CN, C₁₋₄-Alkyl, C₁-C₆-Alkylen-O-C₁-C₆-Alkyl, substituiertes Aryl, Benzyl, CO-C₁-C₆-Alkyl, CO-Aryl, CO-C₁-C₄-Alkylen-Aryl, OCO-C₁-C₆-Alkyl, OCO-Aryl oder OCO-C₁-C₄-AlkylenHetaryl;
**Q** ist ausgewählt aus der Gruppe, bestehend aus **Q1**, **Q2** und **Q3**;
**RQ¹:** Wasserstoff, gegebenenfalls substituiertes C₁-C₄-Alkyl, im Arylrest gegebenenfalls substituiertes Benzyl, CO-C₁-C₄-Alkyl, gegebenenfalls substituiertes Benzoyl, SO₂-C₁-C₄-Alkyl oder im Arylrest gegebenenfalls substituiertes SO₂-Aryl.

Es ist noch bevorzugter, dass die Guanidinverbindungen Reste mit den folgenden Definitionen besitzen:
**A:** OH, F, Cl, OCF₃, OCHF₂, gegebenenfalls substituiertes C₁-C₄-Alkyl, O-C₁-C₄-Alkyl oder S-C₁-C₄-Alkyl;
**B:** Wasserstoff, OH, F, Cl, CF₃, OCF₃, OCHF₂, gegebenenfalls substituiertes C₁-C₄-Alkyl, O-C₁-C₄-Alkyl oder S-C₁-C₄-Alkyl;
**R_{w}¹:** Wasserstoff, F, Cl, CN, CF₃ oder O-CF₃;
**Z:** jeweils gegebenenfalls substituiertes C₁₋₄-Alkyl oder C₁-C₄-Alkylen-O-C₁-C₄-Alkyl;
**R_{z}¹' R_{z}², R_{z}³' R_{z}⁴** jeweils unabhängig voneinander Wasserstoff, F, CH₃;
**R¹**, **R²**, **R³** unabhängig voneinander:
   Wasserstoff, OH, CN, O-Methyl, O-Phenyl, Acetyl, Benzoyl, O-Acetyl,
   O-Benzoyl;
**Q** ist ausgewählt aus der Gruppe, bestehend aus 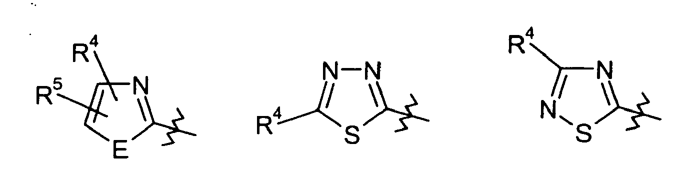
**R_{Q}¹:** Wasserstoff, CH₃, Methansulfonyl, Phenylsulfonyl oder Tosyl.

In einer weiteren bevorzugten Ausführungsform besitzen die Guanidinverbindungen Reste mit den folgenden Definitionen:
**A:** OH, OCF₃, OCH₃, O-Ethyl, O-Propyl oder O-iPropyl;
**Z:** -CH₂-, -CH₂-O-, -CH₂-CH₂- oder -CH₂-CH₂-O-;
jeweils zwei der Reste **R¹**, **R²**, oder **R³** sind Wasserstoff, und der dritte Rest ist Wasserstoff, OH, Acetyl oder Benzoyl;
**Q:**

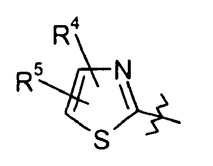

In einer anderen bevorzugten Ausführungsform stellen **R⁴** und/oder **R⁵** jeweils unabhängig voneinander einen Rest ausgewählt aus den Gruppen 1.), 2.), 3.), 4.) oder 5.) dar:
**1.)** Wasserstoff, F, Cl, CN, CF₃, oder jeweils gegebenenfalls substituiertes C₁-C₆-Alkyl, C₂-C₆-Alkenyl, C₁-C₆-Alkylen-O-C₁-C₆-Alkyl oder C₃-C₇-Cycloalkyl;
**2.) R_{Q}¹**, **R_{Q}²** und **R_{Q}³** unabhängig voneinander
   Wasserstoff, -CN, -CF₃, -CHF₂, -OCF₃, -OCHF₂, F, Cl, OH oder jeweils gegebenenfalls substituiertes Phenyl oder Hetaryl, C₁-C₄-Alkyl, C₅-C₇-Cycloalkyl, O-R_{Q}⁵, -NR_{Q}⁷ R_{Q}⁸, -CO-OR_{Q}⁶, -NR_{Q}⁸-CO-O-R_{Q}⁶, O-CH₂-COO-R_{Q}⁶, -NR_{Q}⁸-CO-R_{Q}⁶, -SO₂-R_{Q}⁶, -NR_{Q}⁸-SO₂R_{Q}⁶, - NR_{Q}⁸-CO-O-R_{Q}⁶, -SO₂NH₂, -CONH₂, -SO₂NR_{Q}⁷R_{Q}⁸ oder -CO-NR_{Q}⁷R_{Q}⁸.
   **R_{Q}⁵** C₁-C₄-Alkyl, das gegebenenfalls substituiert ist mit einem Substituenten aus der Gruppe, bestehend aus F, Cl, -OH, -CN, -CF₃, - OCF₃, NH-(C₁-₄-Alkyl) und N(C₁-₄-Alkyl)₂;
   **R_{Q}⁶** jeweils gegebenenfalls substituiertes C₁-C₆-Alkyl, Aryl, Hetaryl oder Phenyl;
   **R_{Q}⁷:** Wasserstoff, jeweils gegebenenfalls substituiertes C₁-C₄-Alkyl, Allyl, Aryl, Hetaryl, Benzyl, Phenethyl oder CH₂-Hetaryl;
   **R_{Q}⁸_{:}** jeweils gegebenenfalls substituiertes C₁-C₄-Alkyl, Allyl, Aryl, Hetaryl, Benzyl, Phenethyl oder CH₂-Hetaryl;
      oder R_{Q}⁷ und R_{Q}⁸ bilden einen gegebenenfalls substituierten 3-oder 7-gliedrigen gesättigten oder ungesättigten Ring, der bis zu zwei gleiche oder verschiedene Heteroatome aus der Gruppe O und N enthalten kann;
3.) Benzothiophenyl, Benzofuranyl, Chinolinyl oder Isochinolinyl;
4.) beide Reste **R⁴** und **R⁵** bilden zusammen einen der folgenden Ringe: 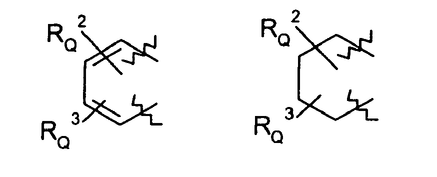 wobei R_{Q}² und R_{Q}³ wie unter 2.) definiert sind;
**5.)** Adamantyl.

Es ist bevorzugt, dass ein Rest von **R⁴** und **R⁵** ausgewählt wird aus Gruppe 1, und der andere von **R⁴** und **R⁵** ausgewählt wird aus Gruppe 1, 2 oder 3.

Die vorliegende Erfindung betrifft außerdem die Verwendung dieser Guanidinverbindungen als Arzneimittel sowie pharmazeutische Zusammensetzungen, enthaltend mindestens eine dieser Guanidinverbindungen, sowie einen pharmazeutisch annehmbaren Träger oder Verdünnungsmittel.

Die vorliegende Erfindung betrifft außerdem die Verwendung dieser Guanidinverbindungen zur Herstellung eines Medikaments zur Behandlung von Krankheiten, die durch eine 5-HT5-Rezeptoraktivität moduliert werden, wie nachstehend ausführlich dargelegt wird.

Weiterhin betrifft die vorliegende-Erfindung die Verwendung von Verbindungen der allgemeinen Formel IVa zur Herstellung von 5HT5-Rezeptorliganden:

W-Z-NH₂ **IVA**

Dabei ist es bevorzugt, dass diese Verbindungen zur Herstellung von erfindungsgemäßen Guanidinverbindungen verwendet werden.

Ferner betrifft die vorliegende Erfindung dieVerwendung einer Guanidinverbindung der allgemeinen Formel IA 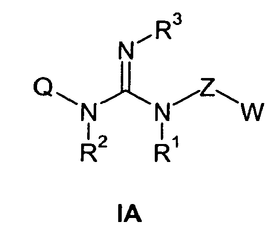 entsprechende enantiomere, diastereomere und/oder tautomere Formen davon sowie pharmazeutisch annehmbare Salze davon,
wobei die angegebenen Reste die folgenden Definitionen besitzen:
**W:**
   einen Rest der allgemeinen Formel **W1** oder **W2** 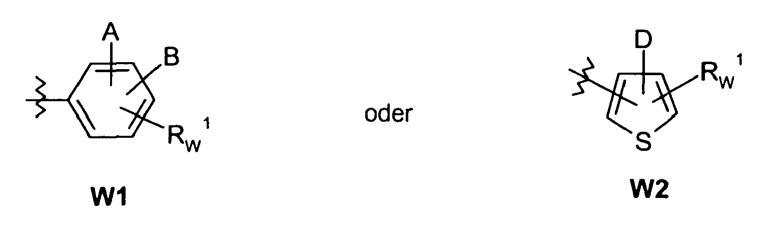
**A:**
   NO₂, NH₂, OH, CN, CF₃, OCF₃, CHF₂, OCHF₂, COOH, O-CH₂-COOH, Halogen, SH, oder
   jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl oder C₁-C₄-Alkylen-Hetero-cycloalkyl, Aryl, Hetaryl, Heterocycloalkyl, C₁-C₄-AlkylenHetaryl oder C₁-C₄-Alkylen-Aryl, oder
   O-R_{A}¹, CO-R_{A}¹, S-R_{A}¹, SO-R_{A}¹, CO-O-R_{A}¹, NR_{A}⁴-CO-O-R_{A}¹, O-CH₂-COO-R_{A}¹, NR_{A}²R_{A}³, CONH₂, SO₂NH₂, NR_{A}⁴-CO-R_{A}¹, SO₂-R_{A}¹, NR_{A}⁴-SO₂-R_{A}¹, SO₂-NR_{A}²RA³ oder CO-NR_{A}²R_{A}³;
**R_{A}¹:**
   jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocyclo-alkyl, Aryl, Hetaryl, Heterocycloalkyl, C₁-C₄-Alkylen-Aryl, C₂-C₆-Alkenylen-Aryl oder C₁-C₆-Alkylen-Hetaryl;
**R_{A}²:**
   Wasserstoff, OH, CN, oder
   jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-AlkylenHetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
**R_{A}³:**
   jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-AlkylenHetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
   oder die Reste **R_{A}²** und **R_{A}³** zusammen mit dem Stickstoff einen 3 bis 7-gliedrigen, gegebenenfalls substituierten, gesättigten oder aromatischen Heterocyclus, der ein, zwei oder drei weitere verschiedene oder gleiche Heteroatome aus der Gruppe O, N, S enthalten kann, bilden; wobei gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und wobei der dadurch gebildete Cyclus gegebenenfalls substituiert sein kann oder an diesem Cyclus ein weiterer, gegebenenfalls substituierter Cyclus ankondensiert sein kann;
**R_{A}⁴:**
   Wasserstoff, oder
   jeweils gegebenenfalls substituiertes C₁-C₆-Alkyl, C₁-C₆-Alkylen-O-C₁-C₆-Alkyl, C₂-C₆-Alkenyl, C₃-C₁₂-Alkinyl, CO-C₁-C₆-Alkyl, CO-O-C₁-C₆-Alkyl, SO₂-C₁-C₆-Alkyl, C₃-C₇-Cycloalkyl, Aryl, C₁-C₄-Alkylen-Aryl, CO-O-Arylalkyl, CO-C₁-C₄-Alkylen-Aryl, CO-Aryl, SO₂-Aryl, Hetaryl, CO-Hetaryl oder SO₂-C₁-C₄-Alkylen-Aryl;
**B:**
   Wasserstoff oder wie Rest **A** definiert ist,
   oder jeweils unabhängig voneinander zwei der Reste **A**, **B** oder **R_{w}¹** zusammen einen 3 bis 7-gliedrigen, gegebenenfalls substituierten, gesättigten oder ungesättigten Carbocyclus oder einen gegebenenfalls substituierten, gesättigten oder ungesättigten oder aromatischen Heterocyclus, der ein, zwei oder drei weitere verschiedene oder gleiche Heteroatome aus der Gruppe O, N, S enthalten kann, bilden; wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
**R_{w}¹:**
   Wasserstoff, OH, Halogen, NO₂, NH₂, CN, CF₃, CHF₂, OCF₃, OCHF₂, oder jeweils gegebenenfalls substituiertes C₁-C₆-Alkyl, C₃-C₇-Cycloalkyl, C₁-C₆-Alkylen-O-C₁-C₆-Alkyl, C₁-C₆-Alkylen-S-C₁-C₆-Alkyl, Aryl, Hetaryl, O-C₁-C₆-Alkyl, O-Aryl, O-Benzyl, C₁-C₆-Alkylamino, C₁-C₆-Dialkylamino,
   Pyrrolidinyl, Piperidinyl, Morpholinyl, CO-C₁-C₆-Alkyl, SO₂-C₁-C₆-Alkyl, CO-Aryl, SO₂-Aryl, CO-C₁-C₄-Alkylen-Aryl, SO₂-C₁-C₄-Alkylen-Aryl, SO-Aryl, CONH₂, CONH-C₁-C₆-Alkyl, SO₂NH-C₁-C₆-Alkyl, CON(C₁-C₆-Alkyl)₂, SO₂N(C₁-C₆-Alkyl)₂, NH-SO₂-C₁-C₆-Alkyl oder NH-CO-C₁-C₆-Alkyl;
**D:**
   wie Rest **A** definiert ist;
**Z:**
   einen Rest der allgemeinen Formel **Z1** 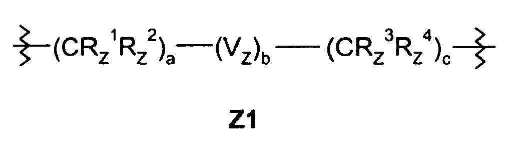 mit den Indizes
      a = 0 - 4
      b = 0, 1
      c = 0 - 4
      wobei die Summe aus a, b und c höchstens 5 beträgt;
**R_{z}¹**, **R_{z}²**, **R_{z}³**, **R_{z}⁴** unabhängig voneinander :
   Wasserstoff, Halogen, OH, oder
   jeweils gegebenenfalls substituiertes C₁-C₆-Alkyl, C₂-C₆-Alkenyl, C₂-C₆-Alkinyl, C₁-C₆-Alkylen-C₃-C₇-Cycloalkyl, C₃-C₇-Cycloalkyl, Aryl, C₁-C₄-Alkylen-Aryl, Het- aryl oder C₁-C₄-Alkylen-Hetaryl, oder jeweils unabhängig voneinander zwei Reste **R_{z}¹** und **R_{z}²** oder **R_{z}³** und **R_{z}⁴** zusammen einen 3 bis 7-gliedrigen, gegebenenfalls substituierten, gesättigten oder ungesättigten Carbo- oder Heterocyclus, der bis zu drei Heteroatome aus der Gruppe O, N oder S enthalten kann, bilden;
**V_{z}:**
   -CO-, -CO-NR_{z}⁵-, -NR_{z}⁵-CO-, -O-, -S-, -SO-, -SO₂-, -SO₂-NR_{z}⁵-, -NR_{z}⁵-SO₂-, -CS-, -CS-NR_{z}⁵-, -NR_{z}⁵-CS-, -CS-O-, -O-CS-, -CO-O-, -O-CO-, -O-, Ethinylen, -C(=CR_{z}⁶R_{z}⁷)-, -CR_{z}⁶=CR_{z}⁷-, -NR_{z}⁵-CO-NR_{z}^{5*}-, -O-CO-NR_{z}⁵-, -NR_{z}⁵-;
**R_{z}⁵**, **R_{z}⁵*** unabhängig voneinander:
   Wasserstoff, oder
   jeweils gegebenenfalls substituiertes C₁-C₆-Alkyl, C₁-C₆-Alkylen-O-C₁-C₆-Alkyl, C₂-C₆-Alkenyl, C₃-C₁₂-Alkinyl, CO-C₁-C₆-Alkyl, CO-O-C₁-C₆-Alkyl, SO₂-C₁-C₆-Alkyl, C₃-C₇-Cycloalkyl, Aryl, C₁-C₄-Alkylen-Aryl, CO-O-C₁-C₄-Alkylen-Aryl, CO-C₁-C₄-Alkylen-Aryl, CO-Aryl, SO₂-Aryl, Hetaryl, CO-Hetaryl oder SO₂-C₁-C₄-Alkylen-Aryl;
**R_{z}⁶**, **R_{z}⁷** unabhängig voneinander:
   Wasserstoff, OH, oder
   jeweils gegebenenfalls substituiertes C₁-C₆-Alkyl, C₁-C₄-Alkoxy, C₂-C₆-Alkenyl, C₂-C₆-Alkinyl, C₁-C₆-Alkylen-C₃-C₇-Cycloalkyl, C₃-C₇-Cycloalkyl, Aryl, C₁-C₄-Alkylen-Aryl, Hetaryl oder C₁-C₄-Alkylen-Hetaryl;
**R¹**, **R²**, **R³** unabhängig voneinander:
   Wasserstoff, OH, CN, oder
   jeweils gegebenenfalls substituiertes C₁-C₆-Alkyl, O-C₁-C₆-Alkyl, C₁-C₆-Alkylen-O-C₁-C₆-Alkyl, C₃-C₇-Cycloalkyl, O-C₃-C₇-Cycloalkyl, Aryl, Hetaryl, C₁-C₄-Alkylen-Aryl, C₁-C₄-Alkylen-Hetaryl, O-Aryl, O-C₁-C₄-Alkylen-Aryl, O-Hetaryl, O-C₁-C₄-Alkylen-Hetaryl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-Hetaryl, CO-O-C₁-C₄-Alkylen-Aryl, SO₂-C₁-C₆-Alkyl, SO₂-Aryl, SO₂-Hetaryl, SO₂-C₁-C₄-Alkylen-Aryl, OCO-C₁-C₆-Alkyl, OCO-Aryl, OCO-Hetaryl, OCO-C₁-C₄-Alkylen-Aryl, OCO-C₁-C₄-Alkylen-Hetaryl, SO₂-C₁-C₆-Alkyl, SO₂-Aryl, SO₂-Hetaryl oder SO₂-C₁-C₄-Alkylen-Aryl, oder
   jeweils unabhängig vom dritten Rest zwei Reste von **R¹**, **R²** oder **R³** zusammen einen 5 bis 7-gliedrigen, gegebenenfalls substituierten, gesättigten oder ungesättigten Carbocyclus, oder einen gegebenenfalls substituierten, gesättigten oder ungesättigten, der ein, zwei oder drei weitere verschiedene oder gleiche Heteroatome aus der Gruppe O, N, S enthalten kann, bilden, wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
**Q:**
   einen zweifach substituierten 5-gliedrigen Hetaryl-Rest, augewählt aus **Q1** bis **Q6** 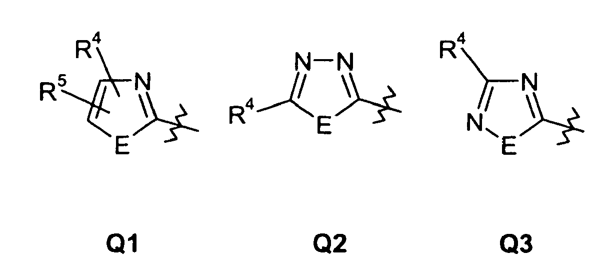 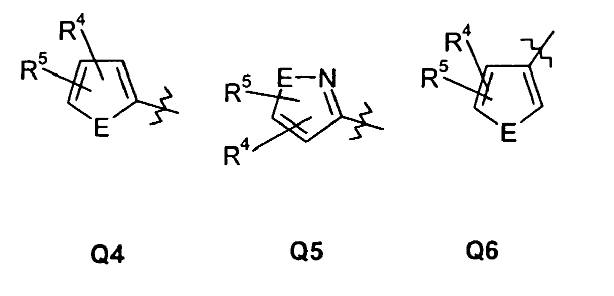
**E:** O, N-R_{Q}¹ oder S;
**R_{Q}¹:**
   Wasserstoff, oder
   jeweils gegebenenfalls substituiertes C₁-C₄-Alkyl, CO-C₁-C₄-Alkyl, SO₂-C₁-C₄-Alkyl, CO-O-C₁-C₄-Alkyl, Aryl, C₁-C₄-Alkylen-Aryl, CO-Aryl, CO-Hetaryl, SO₂-Aryl, SO₂-Hetaryl, CO-O-Aryl, CO-C₁-C₄-Alkylen-Aryl, SO₂-C₁-C₄-Alkylen-Aryl oder CO-O-C₁-C₄-Alkylen-Aryl;
**R⁴, R⁵** jeweils unabhängig voneinander einen Rest ausgewählt aus den Gruppen **1.)**, **2.)**, **3.)**, **4.)** oder **5.)**:
   **1.)** Wasserstoff, Halogen, CN, CF₃, CHF₂, oder jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryl, C₁-C₄-Alkylen-Hetaryl, C₁-C₆-Alkylen-O-C₁-C₆-Alkyl, C₁-C₆-Alkylen-O-Aryl, COO-C₁-C₄-Alkyl oder C₁-C₄-Alkylen-COO-C₁-C₄-Alkyl;
   **2.)** Phenyl oder Naphthyl, die jeweils mit **R_{Q}²**, **R_{Q}³** und **R_{Q}⁴** substituiert sind, wobei
      **R_{Q}²**, **R_{Q}³** und **R_{Q}⁴** jeweils unabhängig voneinander einen Substituenten aus der folgenden Gruppe darstellen:
         Wasserstoff, NO₂, NH₂, OH, CN, CF₃, CHF₂, OCF₃, OCHF₂, COOH, O-CH₂-COOH, SH, Halogen, oder
         jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-Alkylen-Hetaryl, oder
         O-R_{Q}⁵, S-R_{Q}⁵, NR_{Q}⁷R_{Q}⁸, CO-OR_{Q}⁶, NR_{Q}⁸-CO-O-R_{Q}⁶, O-CH₂-COO-R_{Q}⁶, NR_{Q}⁸-CO-R_{Q}⁶, SO₂-R_{Q}⁶, NR_{Q}⁸-SO₂-R_{Q}⁶, SO₂NH₂, CONH₂, SO₂-NR_{Q}⁷R_{Q}⁸ oder CO-NR_{Q}⁷R_{Q}⁸, oder jeweils zwei der Reste aus **R_{Q}²**, **R_{Q}³** oder **R_{Q}⁴** zusammen einen 3 bis 7-gliedrigen, gegebenenfalls substituierten, gesättigten, ungesättigten Carbocyclus oder einen gegebenenfalls substituierten, gesättigten, ungesättigten aromatischen Heterocyclus, der bis zu drei weitere verschiedene oder gleiche Heteroatome O, N, S enthalten kann, bilden, und gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und der gebildete Cyclus gegebenenfalls substituiert sein kann oder an diesem Cyclus ein weiterer, gegebenenfalls substituierter Cyclus ankondensiert sein kann;
      **R_{Q}⁵** jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Heterocycloalkyl, Aryl oder Hetaryl;
      **R_{Q}⁶** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇Cycloalkyl, C₁-C₄-Alkylen-Heterocycloalkyl, Aryl, Hetaryl, Heterocycloalkyl oder C₁-C₆-Alkylen-O-C₁-C₆-Alkyl;
      **R_{Q}⁷** Wasserstoff, OH, CN, oder
         jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, C₁-C₄-Alkylen-Aryl, C₁-C₄-Alkylen-Hetaryl, CO-Aryl, CO-Hetaryl, CO-C₁-C₄-AlkylenAryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-AlkylenHetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
      **R_{Q}⁸** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
      oder beide Reste **R_{Q}⁷** und **R_{Q}⁸** zusammen mit dem Stickstoff einen 3 bis 7-gliedrigen, gegebenenfalls substituierten, gesättigten oder aromatischen Heterocyclus, der ein, zwei oder drei weitere verschiedene oder gleiche Heteroatome O, N, S enthalten kann- bilden; und gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und der gebildete Cyclus gegebenenfalls substituiert sein kann oder an diesem Cyclus ein weiterer, gegebenenfalls substituierter Cyclus ankondensiert sein kann;
   **3.)** einen 5- oder 6-gliedrigen, gegebenenfalls mit 1 oder 2 Substituenten substituierten Hetaryl-Rest aus der Gruppe, bestehend aus:
      2-Furyl, 3-Furyl, 2-Pyrrolyl, 3-Pyrrolyl, 2-Thienyl, 3-Thienyl, 2-Pyridyl, 3-Pyridyl, 4-Pyridyl, 2-Thiazolyl, 4-Thiazolyl, 5-Thiazolyl, 2-Oxazolyl, 4-Oxazolyl, 5-Oxazolyl, 2-Pyrimidyl, 4-Pyrimidyl, 5-Pyrimidyl, 6-Pyrimidyl, 3-Pyrazolyl, 4-Pyrazolyl, 5-Pyrazolyl, 3-Isothiazolyl, 4-Isothiazolyl, 5-Isothiazolyl, 2-Imidazolyl, 4-Imidazolyl, 5-Imidazolyl, 3-Pyridazinyl, 4-Pyridazinyl, 5-Pyridazinyl, 6-Pyridazinyl, 3-Isoxazolyl, 4-Isoxazolyl, 5-Isoxazolyl, Thiadiazolyl, Oxadiazolyl oder Triazinyl oder deren anellierten Derivate Indazolyl, Indolyl, Benzothiophenyl, Benzofuranyl, Indolinyl, Benzimidazolyl, Benzthiazolyl, Benzoxazolyl, Chinolinyl und Isochinolinyl;
   **4.)** beide Reste **R⁴** und **R⁵** zusammen einen 4 bis 7-gliedrigen, gegebenenfalls substituierten, gesättigten oder ungesättigten oder aromatischen Carbocyclus oder einen 5- oder 6-gliedrigen gegebenenfalls substituierten, gesättigten oder ungesättigten oder aromatischen Heterocyclus, der bis zu drei weitere verschiedene oder gleiche Heteroatome O, N, S enthalten kann, bilden, und mit bis zu zwei weiteren Resten substituiert sein kann, wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
   **5.)** einen C₆-C₁₀- bi- oder tricyclischen, gesättigten Kohlenwasserstoffrest;

zur Herstellung eines Medikaments zur Behandlung von Krankheiten, die durch eine 5-HT5-Rezeptoraktivität moduliert werden.

Dabei ist die Behandlung von neuropathologischen, neuropsychiatrischen und neurodegenerativen Störungen, Symptomen und Fehlfunktionen bevorzugt, insbesondere die Behandlung von Migräne und Gehirnschädigungen. Als Beispiele der Gehirnschädigungen und/oder Störungen können cerebrale Ischämie, Schlaganfall, Epilepsie und Anfälle im allgemeinen, Psychosen, Schizophrenie, Autismus, OCD-Syndrom, cognitive Erkrankungen, Aufmerksamkeitsstörungen, Depressionen, bipolare und/oder unipolare Depressionen, Angstzustände, Demenz, seniler Demenz, Alzheimer Demenz, demyelinisierende Erkrankungen, Multiple Sklerose und Gehirntumore genannt werden. Ebenfalls bevorzugt ist die Behandlung von cerebrovaskulären Störungen, Schmerz, Schmerz-bedingten Störungen, Abhängigkeit, Drogen-bedingten Störungen, Amnesie, Alkoholmissbrauch, Drogenmissbrauch, Störungen des circadianen Rhythmus und dem Cushing Syndrom.

### Ausführliche Beschreibung der Erfindung

In bevorzugten Ausführungsformen besitzen die Reste der Formeln I oder IA die folgenden Bedeutungen:
W ist wie vorstehend definiert und stellt vorzugsweise W1 dar.
A ist wie vorstehend definiert und bedeutet vorzugsweise
   Halogen, OH, CN, CF₃, CHF₂, OCF₃, OCHF₂, oder
   jeweils gegebenenfalls substituiertes C₁-C₆-Alkyl oder C₂-C₆-Alkenyl,
   -CH₂-COO-R_{A}¹. O-R_{A}¹, S-R_{A}¹, NR_{A}² R_{A}³, -NR_{A}⁴-CO-R_{A}¹, SO₂NH₂, NR_{A}⁴-SO₂₋R_{A}¹, SO₂-NR_{A}²R_{A}³ oder -CO-NR_{A}⁴-R_{A}¹.

In einer Ausführungsform ist A vorzugsweise
Halogen, OH, CN, CF₃, CHF₂, OCF₃, OCHF₂, oder
jeweils gegebenenfalls substituiertes C₁-C₆-Alkyl oder C₂-C₆-Alkenyl,
-O-CH₂-COO-R_{A}¹, O-R_{A}¹, S-R_{A}¹, NR_{A}²RA₃, -NR_{A}⁴-CO-R_{A}¹ oder -CO-NR_{A}⁴-R_{A}¹.

Besonders bevorzugt ist A Halogen, OH, OCF₃, OCHF₂, gegebenenfalls substituiertes C₁-C₄-Alkyl, O-R_{A}¹ oder S-R_{A}¹. Noch bevorzugter ist A Halogen, OH, OCF₃, OCHF₂, jeweils gegebenenfalls substituiertes C₁-C₄-Alkyl, O-C₁-C₄-Alkyl, O-Benzyl, O-Phenyl oder S-C₁-C₄-Alkyl. Davon noch bevorzugter ist A OH, F, Cl, OCF₃, OCHF₂, C₁-C₄-Alkyl, O-C₁-C₄-Alkyl oder S-C₁-C₄-Alkyl. Am meisten bevorzugt ist A OH, OCF₃, OCH₃, O-Ethyl, O-n-Propyl oder O-i-Propyl.

A befindet sich vorzugsweise in der 2- oder 4-Position am Ring, noch bevorzugter in der 2-Position.

R_{A}¹ ist wie vorstehend definiert und bedeutet vorzugsweise jeweils gegebenenfalls substituiertes C₁-C₄-Alkyl, C₃-C₇-Cycloalkyl, Phenyl oder Benzyl. Noch bevorzugter ist R_{A}¹ Methyl, Ethyl, n-Propyl oder i-Propyl. Am meisten bevorzugt ist R_{A}¹ Methyl oder Ethyl.

R_{A}² ist wie vorstehend definiert und bedeutet vorzugsweise
Wasserstoff oder jeweils gegebenenfalls substituiertes C₁-C₄-Alkyl, Phenyl, Benzyl, Phenethyl, CO-C₁-C₄-Alkyl, CO-Aryl, CO-O-C₁-C₄-Alkyl, SO₂-C₁-C₄-Alkyl, SO₂-Aryl, SO₂-Hetaryl, oder SO₂-C₁-C₄-Alkylen-Aryl.
Noch bevorzugter ist R_{A}² Wasserstoff, C₁-C₄-Alkyl, Phenyl oder Benzyl.

In einer Ausführungsform ist R_{A}² vorzugsweise Wasserstoff, Methyl, Ethyl, n-Propyl, i-Propyl oder Phenyl.

R_{A}³ ist wie vorstehend definiert und bedeutet vorzugsweise jeweils gegebenenfalls substituiertes C₁₋₄-Alkyl, Phenyl, Benzyl, Phenethyl, CO-C₁-C₄-Alkyl, CO-Aryl, CO-O-C₁-C₄-Alkyl, SO₂-C₁-C₄-Alkyl, SO₂-Aryl, SO₂-Hetaryl, oder SO₂-C₁-C₄-Alkylen-Aryl.

Noch bevorzugter ist R_{A}³ C₁-C₄-Alkyl, Phenyl oder Benzyl, am meisten bevorzugt Methyl , Ethyl, n-Propyl oder i-Propyl oder Phenyl.

Die beiden Reste R_{A}² und R_{A}³ können auch, wie vorstehend beschrieben, zusammen mit dem Stickstoff einen 3-7-gliedrigen Heterocyclus bilden. Dabei bilden beide Reste R_{A}² und R_{A}³ vorzugsweise zusammen einen gegebenenfalls substituierten 5-oder 6-gliedrigen gesättigten oder ungesättigten Ring, der ein oder zwei weitere gleiche oder verschiedene Heteroatome aus der Gruppe O, N und S enthalten kann.

RA⁴ ist wie vorstehend definiert und bedeutet vorzugsweise Wasserstoff oder einen gegebenenfalls substituierten C₁-C₄-Alkyl-Rest. Am meisten bevorzugt ist R_{A}⁴ Wasserstoff, Methyl, Ethyl, n-Propyl oder i-Propyl.

B ist wie vorstehend definiert und bedeutet vorzugsweise
Wasserstoff, Halogen, OH, CN, CF₃, CHF₂, OCF₃, OCHF₂,
jeweils gegebenenfalls substituiertes C₁-C₆-Alkyl oder C₂-C₆-Alkenyl,
-O-CH₂-COO-R_{A}¹, O-R_{A}¹, S-R_{A}¹, NR_{A}²R_{A}³, -NR_{A}⁴-CO-R_{A}¹ oder-CO-NR_{A}⁴-R_{A}¹.

Besonders bevorzugt ist B
Wasserstoff, Halogen, OH, OCF₃, OCHF₂, gegebenenfalls substituiertes C₁-C₄-Alkyl, O-R_{A}¹ oder S-R_{A}¹.

Noch bevorzugter ist B Wasserstoff, Halogen, OH, OCF₃, OCHF₂, jeweils gegebenenfalls substituiertes C₁-C₄-Alkyl, O-C₁-C₄-Alkyl, O-Benzyl, O-Phenyl oder S-C₁-C₄-Alkyl. Davon noch bevorzugter ist B Wasserstoff, OH, F, Cl, OCF₃, OCHF₂, C₁-C₄-Alkyl, O-C₁-C₄-Alkyl oder S-C₁-C₄-Alkyl. Am meisten bevorzugt ist B Wasserstoff, OH, OCF₃, OCH₃, O-Ethyl, O-n-Propyl oder O-i-Propyl.

B befindet sich vorzugsweise in der 5- oder 6-Position am Ring, noch bevorzugter in der 6-Position.

R_{w}¹ ist wie vorstehend definiert und bedeutet vorzugsweise Wasserstoff, F, Cl, CN, CF₃, CHF₂, O-CF₃, OCHF₂, gegebenenfalls substituiertes C₁-C₄-Alkyl, OC₁-C₄-Alkyl, Aryl, C₁-C₆-Alkylamino oder C₁-C₆-Dialkylamino. Noch bevorzugter ist R_{w}¹ Wasserstoff, F, Cl, CN, CF₃ oder O-CF₃, OMe, am meisten bevorzugt Wasserstoff.

D ist wie vorstehend definiert und bedeutet vorzugsweise
Halogen, OH, CN, CF₃, CHF₂, OCF₃, OCHF₂,
jeweils gegebenenfalls substituiertes C₁-C₆-Alkyl oder C₂-C₆-Alkenyl,
-O-CH₂-COO-R_{A}¹, O-R_{A}¹, S-R_{A}¹, NR_{A}²R_{A}³, -NR_{A}⁴-CO-R_{A}¹ oder -CO-NR_{A}⁴-R_{A}¹.

Besonders bevorzugt ist D
Halogen, OH, OCF₃, OCHF₂, gegebenenfalls substituiertes C₁-C₄-Alkyl, O-R_{A}¹ oder S-R_{A}¹.

Noch bevorzugter ist D Halogen, OH, OCF₃, OCHF₂,
jeweils gegebenenfalls substituiertes C₁-C₄-Alkyl, O-C₁-C₄-Alkyl, O-Benzyl, O-Phenyl oder S-C₁-C₄-Alkyl.

Davon noch bevorzugter ist D OH, F, Cl, OCF₃, OCHF₂, C₁-C₄-Alkyl, O-C₁-C₄-Alkyl oder S-C₁-C₄-Alkyl. Am meisten bevorzugt ist D OH, OCF₃, OCH₃, O-Ethyl, O-n-Propyl oder O-i-Propyl.

D befindet sich vorzugsweise in der 3 oder 4-Position am Ring, noch bevorzugter in der 3-Position.

Insgesamt stellt W vorzugsweise einen Rest dar, der sich aus den bevorzugten Kombinationen von A, B, R_{A}¹, R_{A}² , R_{A}³, R_{A}⁴ und R_{W}¹ zusammensetzt.

Z ist wie vorstehend definiert, wobei die Summe der Indizes a, b und c 1, 2, 3, 4 oder 5, vorzugsweise 1, 2 oder 3, noch bevorzugter 1 oder 2 beträgt. Insbesondere ist a 0, 1, 2, 3 oder 4, vorzugsweise 1, 2 oder 3, noch bevorzugter 1 oder 2. Index b ist 0 oder 1, vorzugsweise 0. Index c ist 0, 1, 2, 3 oder 4, vorzugsweise 0, 1 oder 2, am meisten bevorzugt 0.

Z ist vorzugsweise jeweils gegebenenfalls substituiertes C₁₋₄-Alkylen oder C₁₋₄-Alkylenoxy, noch bevorzugter -CH₂-, -CH₂-O-, -CH₂-CH₂-, -CH₂-CH₂-CH₂- oder -CH₂-CH₂-O-, noch bevorzugter -CH₂-, -CH₂-O-, -CH₂-CH₂- oder -CH₂-CH₂-O-, am meisten bevorzugt -CH₂-.

R_{z}^{1,} R_{z}², R_{z}³, R_{z}⁴ sind wie vorstehend definiert und bedeuten vorzugsweise unabhängig voneinander Wasserstoff, Halogen, OH oder gegebenenfalls substituiertes C₁-C₆-Alkyl, noch bevorzugter Wasserstoff, F oder CH₃. Am meisten bevorzugt sind R_{z}^{1,} R_{z}², R_{z}³ und R_{z}⁴ gleichzeitig Wasserstoff.

V_{z} ist wie vorstehend definiert und bedeutet vorzugsweise -CO-, -CO-NR_{z}⁵-, -NR_{z}⁵-CO-, -O-, -S-, noch bevorzugter -O- oder -S-, am meisten bevorzugt -O-.

R_{z}⁵ und R_{z}^{5*} sind wie vorstehend definiert und bedeuten vorzugsweise unabhängig voneinander Wasserstoff oder CH₃.

R_{z}⁶ und R_{z}⁷ sind wie vorstehend definiert und bedeuten vorzugsweise unabhängig voneinander Wasserstoff oder CH₃, am meisten bevorzugt Wasserstoff.

R¹, R², R³ sind wie vorstehend definiert und bedeuten vorzugsweise unabhängig voneinander Wasserstoff, OH, CN, C₁-C₄-Alkyl, C₁-C₆-Alkylen-O-C₁-C₆-Alkyl, gegebenenfalls substituiertes Aryl, Benzyl, CO-C₁-C₆-Alkyl, CO-Aryl, CO-C₁-C₄-AlkylenAryl, OCO-C₁-C₆-Alkyl, O-CO-Aryl oder O-CO-C₁-C₄-Alkylen-Hetaryl, CO-O-C₁-C₄-Alkyl, O-CO-C₁-C₄-Alkyl ,CO-OBenzyl, O-CO-Benzyl, noch bevorzugter Wasserstoff. OH, CN, O-Methyl, O-Phenyl, Acetyl, Benzoyl, O-Acetyl, O-Benzoyl, CO-O-C₁-C₄-Alkyl, O-CO-C₁-C₄-Alkyl ,CO-OBenzyl, O-CO-Benzyl. Besonders bevorzugt sind jeweils zwei der Reste R¹, R², oder R³ Wasserstoff, und der dritte Rest Wasserstoff, OH, Acetyl, Benzoyl, CO-O-C₁-C₄-Alkyl, O-CO-C₁-C₄-Alkyl, am meisten bevorzugt sind alle Reste R¹, R² und R³ Wasserstoff.

Q ist wie vorstehend definiert und bedeutet vorzugsweise einen Rest der Formeln Q1, Q2, Q3 oder Q5. In einer Ausführungsform bedeutet Q einen Rest der Formeln Q1, Q2 oder Q3. Besonders bevorzugt sind die Reste der Formeln 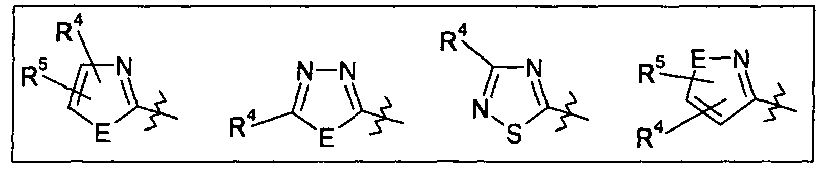 noch bevorzugter sind die Reste der Formeln

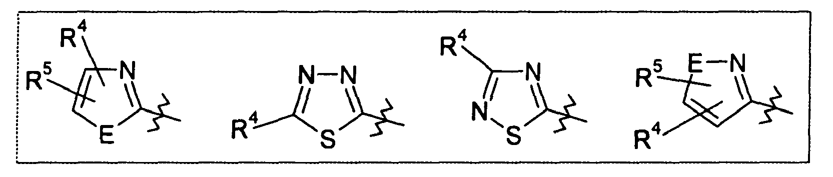

Am meisten bevorzugt ist Q 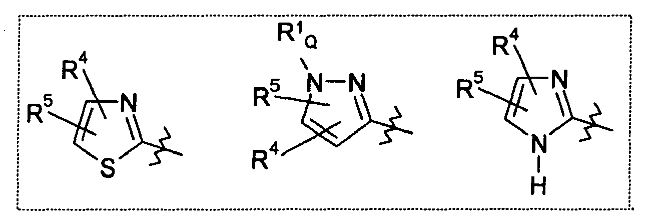 In einer Ausführungsform sind die Reste der Formeln 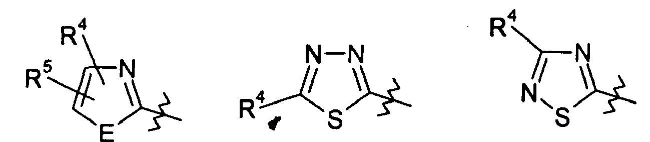 bevorzugt. In dieser Ausführungsform ist Q am meisten bevorzugt

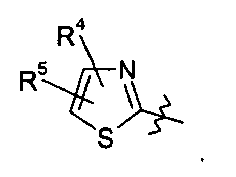

E ist wie vorstehend definiert und bedeutet vorzugsweise S oder O, noch bevorzugter S.

R_{Q}¹ ist wie vorstehend definiert und bedeutet vorzugsweise Wasserstoff, gegebenenfalls substituiertes C₁-C₄-Alkyl, im Arylrest gegebenenfalls substituiertes Benzyl, CO-C₁-C₄-Alkyl, gegebenenfalls substituiertes Benzoyl, SO₂-C₁-C₄-Alkyl oder im Arylrest gegebenenfalls substituiertes SO₂-Aryl. Noch bevorzugter ist R_{Q}¹ Wasserstoff, CH₃, Phenyl, Benzyl, Methansulfonyl, Phenylsulfonyl oder Tosyl, am meisten bevorzugt Wasserstoff.

In einer weiteren Ausführungsform ist R_{Q}¹ wie vorstehend definiert und bedeutet vorzugsweise Wasserstoff, gegebenenfalls substituiertes C₁-C₄-Alkyl, im Arylrest gegebenenfalls substituiertes Benzyl, CO-C₁-C₄-Alkyl, gegebenenfalls substituiertes Benzoyl, SO₂-C₁-C₄-Alkyl oder im Arylrest gegebenenfalls substituiertes SO₂-Aryl. Noch bevorzugter ist R_{Q}¹ Wasserstoff, CH₃, Methansulfonyl, Phenylsulfonyl oder Tosyl, am meisten bevorzugt Wasserstoff.

R⁴ und R⁵ sind wie vorstehend definiert und besitzen vorzugsweise die folgenden Definitionen:

Für den Fall 1.) sind R⁴ und/oder R⁵ wie vorstehend definiert, vorzugsweise jeweils unabhängig voneinander jeweils ein Rest ausgewählt aus der Gruppe, bestehend aus Wasserstoff, F, Cl, CN, CF₃, CHF₂,
jeweils gegebenenfalls substituiertes C₁-C₆-Alkyl, C₂-C₆-Alkenyl, C₁-C₆-Alkylen-O-C₁-C₆-Alkyl oder C₃-C₇-Cycloalkyl. Besonders bevorzugt sind Wasserstoff, C₁-C₄-Alkyl, z. B. Methyl, Ethyl, n-Propyl, i-Propyl oder tert-Butyl, Cyclopentyl- oder Cyclohexyl oder CF₃.

Für den Fall 2.) sind R⁴ und/oder R⁵ wie vorstehend definiert, vorzugsweise Phenyl, das mit R_{Q}², R_{Q}³ und R_{Q}⁴ substituiert ist.

R_{Q}², R_{Q}³ und R_{Q}⁴ sind wie vorstehend definiert und bedeuten vorzugsweise jeweils unabhängig voneinander einen Substituenten aus der folgenden Gruppe:

Wasserstoff, -NO₂, -NH₂, -OH, -CN, -CF₃, -CHF₂, -OCF₃, -OCHF₂, Halogen, jeweils gegebenenfalls substituiertes Aryl, Hetaryl, C₁-C₆-Alkyl, C₂-C₆-Alkenyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-Alkylen-Hetaryl;
O-R_{Q}⁵, NR_{Q}⁷R_{Q}⁸, -CO-OR_{Q}⁶, -NR_{Q}⁸-CO-O-R_{Q}⁶, -O-CH₂-COO-R_{Q}⁶, -NR_{Q}⁸-CO-R_{Q}⁶, -SO₂-R_{Q}⁶, -NR_{Q}⁸-SO₂-R_{Q}⁶, -NR_{Q}⁸-CO-O-R_{Q}⁶, -SO₂NH₂, -CONH₂, -SO₂-NR_{Q}⁷R_{Q}⁸ oder-CO-NR_{Q}⁷R_{Q}⁸. Besonders bevorzugt sind Wasserstoff, NH₂, -CHF₂, -CF₃, -OCF₃, O-R_{Q}⁵, C₁-C₄-Alkyl, -NR_{Q}⁷R_{Q}⁸ und Halogen.

In einer Ausführungsform sind Wasserstoff, CF₃, -OCF₃, O-CH₃, -OCHF₂, OH, N(CH₃)₂, Cl und F bevorzugt. In einer anderen Ausführungsform sind Wasserstoff, CF₃, -O.CF₃, O-CH₃, Cl und F bevorzugt.

Es ist auch möglich, dass jeweils zwei der Reste aus R_{Q}², R_{Q}³ oder R_{Q}⁴ zusammen einen 3 bis 7-gliedrigen, gegebenenfalls substituierten, gesättigten, ungesättigten oder aromatischen Carbocyclus oder einen gegebenenfalls substituierten, gesättigten, ungesättigten aromatischen Heterocyclus bilden, der bis zu drei weitere verschiedene oder gleiche Heteroatome O, N, S enthalten kann, und gegebenenfalls zwei an diesem Heterocyclus substituierte Reste können zusammen einen anellierten, gesättigten, ungesättigten oder aromatischen Carbocyclus oder Heterocyclus bilden, der bis zu drei verschiedene oder gleiche Heteroatome O, N, S enthalten kann, und der gebildete Cyclus kann gegebenenfalls substituiert sein oder an diesem Cyclus kann ein weiterer, gegebenenfalls substituierter Cyclus ankondensiert sein. In dieser Ausführungsform bilden R_{Q}², R_{Q}³ oder R_{Q}⁴ vorzugsweise zusammen einen 5- oder 6-gliedrigen, noch bevorzugter 5-gliedrigen, Heterocyclus, der ein weiteres Heteroatom O, N, S, vorzugsweise O, enthält. Der Heterocyclus ist vorzugsweise gesättigt.

In einer Ausführungsform sind R_{Q}², R_{Q}³ und R_{Q}⁴ vorzugsweise jeweils Wasserstoff oder zwei der Substituenten sind Wasserstoff und der dritte Substituent ist ein Rest außer Wasserstoff.

R_{Q}⁵ ist wie vorstehend definiert und bedeutet vorzugsweise jeweils gegebenenfalls substituiertes C₁-C₆-Alkyl oder C₂-C₆-Alkenyl, noch bevorzugter C₁-C₄-Alkyl, das gegebenenfalls substituiert ist mit einem Substituenten aus der Gruppe, bestehend aus F, Cl, -OH, -CN, -CF₃, -CHF₂, -OCF₃, -OCHF₂, NH-(C₁-₄-Alkyl) und N(C₁-₄-Alkyl)₂, am meisten bevorzugt Methyl oder Ethyl.

In einer Ausführungsform ist R_{Q}⁵ C₁-C₄-Alkyl-Heterocycloalkyl, noch bevorzugter C₁-C₂-Alkyl-Heterocycloalkyl, wobei das Heterocycloalkyl vorzugsweise ein 5- oder 6-gliedriger Ring ist mit 1 bis 3, noch bevorzugter 1 oder 2 Heteroatomen, ausgewählt aus N, O oder S, noch bevorzugter N oder O. In einer Ausführungsform ist R_{Q}⁵ Morpholino.

R_{Q}⁶ ist wie vorstehend definiert und bedeutet vorzugsweise jeweils gegebenenfalls substituiertes C₁-C₆-Alkyl, C₂-C₆-Alkenyl, C₃-C₇-Cycloalkyl, Aryl, Hetaryl oder C₁-C₆-Alkylen-O-C₁-C₆-Alkyl, noch bevorzugter jeweils gegebenenfalls substituiertes C₁-C₆-Alkyl, Aryl, Hetaryl, noch bevorzugter C₁-C₄-Alkyl, C₃-C₇-Cycloalkyl, Aryl, oder C₁-C₆-Alkylen-O-C₁-C₆-Alkyl, am meisten bevorzugt Methyl, Ethyl, Cyclohexyl oder Phenyl.

R_{Q}⁷ ist wie vorstehend definiert und bedeutet vorzugsweise
Wasserstoff, OH, oder
jeweils gegebenenfalls substituiertes C₁-C₆-Alkyl, C₂-C₆-Alkenyl, C₃-C₇-Cycloalkyl, Aryl, Hetaryl, C₁-C₄-Alkylen-Aryl, C₁-C₄-Alkylen-Hetaryl oder C₁-C₆-Alkylen-O-C₁-C₆-Alkyl, noch bevorzugter Wasserstoff, jeweils gegebenenfalls substituiertes C₁-C₄-Alkyl, Allyl, Aryl, Hetaryl, Benzyl, Phenethyl oder CH₂-Hetaryl.

Davon noch bevorzugter ist Wasserstoff, C₁-C₄-Alkyl, Phenyl, oder Benzyl, am meisten bevorzugt Wasserstoff, Methyl, Ethyl, oder Phenyl.

R_{Q}⁸ ist wie vorstehend definiert und bedeutet vorzugsweise Wasserstoff, jeweils gegebenenfalls substituiertes C₁-C₆-Alkyl, C₂-C₆-Alkenyl, C₃-C₇-Cycloalkyl, Aryl, Hetaryl, C₁-C₄-Alkylen-Aryl, C₁-C₄-Alkylen-Hetaryl oder C₁-C₆-Alkylen-O-C₁-C₆-Alkyl, CO-C₁-C₄-Alkyl, SO₂-C₁-C₄-Alkyl.

Noch bevorzugter sind Wasserstoff, jeweils gegebenenfalls substituiertes C₁-C₄-Alkyl, Aryl, Hetaryl, Benzyl, Phenethyl oder CH₂-Hetaryl. Davon noch bevorzugter sind Wasserstoff, C₁-C₄-Alkyl, Phenyl, oder Benzyl, am meisten bevorzugt Wasserstoff, Methyl, Ethyl, oder Phenyl.

Es ist ebenfalls bevorzugt, dass die beiden Reste R_{Q}⁷ und R_{Q}⁸ zusammen mit dem Stickstoff einen gegebenenfalls substituierten 3-oder 7-gliedrigen gesättigten oder ungesättigten Ring, der ein N oder zwei N oder jeweils ein O und ein N enthalten kann, bilden. Noch bevorzugter bilden die beiden Reste R_{Q}⁷ und R_{Q}⁸ zusammen mit dem Stickstoff einen 5- oder 6-gliedrigen, gegebenenfalls substituierten, gesättigten Heterocyclus, der ein weiteres Heteroatom O, N, oder S, vorzugsweise O oder N enthalten kann, bilden. Bevorzugt sind ein 5-gliedriger gesättigter Heterocyclus mit einem N und ein 6-gliedriger gesättigter Heterocyclus mit 2 N oder 1 N und 1 O.

Für den Fall 3.) sind R⁴ und/oder R⁵ vorzugsweise jeweils unabhängig voneinander jeweils ein Rest ausgewählt aus der Gruppe, bestehend aus
jeweils gegebenenfalls mit 1 oder 2 Substituenten substituiertes 2-Pyrrolyl, 3-Pyrrolyl, Benzothiophenyl, Benzofuranyl, Chinolinyl, Isochinolinyl; oder
jeweils gegebenenfalls mit 1 oder 2 Substituenten substituiertes 2-Thienyl, oder 3-Thienyl, wobei die Substituenten ausgewählt sind aus der Gruppe, bestehend aus Halogen, insbesondere Cl, -NO₂, -NH₂, -OH, -CN, -CF₃,-OCF₃, -CHF₂, O-CHF₂, C₁-C₆-Alkyl, insbesondere Methyl oder Ethyl, O-C₁-C₆-Alkyl, NH-(C₁-C₆-Alkyl) und N(C₁-C₆-Alkyl)₂, NHCO-C₁-C₄-Alkyl, NHSO₂-C₁-C₄-Alkyl und SO₂-C₁-C₄-Alkyl.

Besonders bevorzugt sind Benzothiophenyl, Benzofuranyl, Chinolinyl, Isochinolinyl, 2-Thienyl, oder 3-Thienyl, wobei die beiden Letzteren vorzugsweise substituiert sind mit Halogen, insbesondere Cl, C₁-C₆-Alkyl, insbesondere Methyl oder Ethyl.

In einer Ausführungsform sind R⁴ und/oder R⁵ vorzugsweise jeweils unabhängig voneinander jeweils 2-Pyridyl, 3-Pyridyl, 4-Pyridyl, 2-Pyrimidyl, 4-Pyrimidyl, 5-Pyrimidyl, 6-Pyrimidyl, 2-Thienyl, 3-Thienyl, Benzothiophenyl, Benzofuranyl, Benzimidazolyl, Chinolinyl oder Isochinolinyl, noch bevorzugter 2-Pyridyl, 3-Pyridyl, 4-Pyridyl, 2-Thienyl, 3-Thienyl, Benzothiophenyl, Benzofuranyl, Chinolinyl, oder Isochinolinyl, die gegebenenfalls mit 1 oder 2 Resten substituiert sein können. Die Reste sind vorzugsweise ausgewählt aus der Gruppe, bestehend aus Halogen, insbesondere Cl oder F, -NO₂, -NH₂, -OH, -CN, -CF₃,-OCF₃, -CHF₂, O-CHF₂, C₁-C₆-Alkyl, insbesondere Methyl oder Ethyl, O-C₁-C₆-Alkyl, NH-(C₁-C₆-Alkyl) und N(C₁-C₆-Alkyl)₂, NHCO-C₁-C₄-Alkyl, NHSO₂-C₁-C₄-Alkyl und SO₂-C₁-C₄-Alkyl, am meisten bevorzugt Halogen, insbesondere Cl oder F, C₁-C₆-Alkyl, insbesondere Methyl oder Ethyl.

Dabei sind 2-Pyridyl, 3-Pyridyl, 4-Pyridyl oder 2-Pyrimidyl, inbesondere 2-Pyridyl, 3-Pyridyl oder 4-Pyridyl besonders bevorzugt.

Für den Fall 4.) bilden beide Reste R⁴ und R⁵ vorzugsweise zusammen einen der folgenden Ringe:

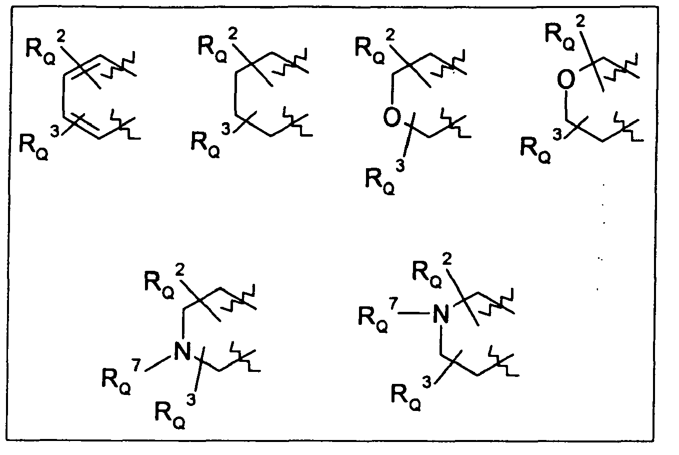

In einer Ausführungsform bilden beide Reste R⁴ und R⁵ vorzugsweise zusammen einen der folgenden Ringe: 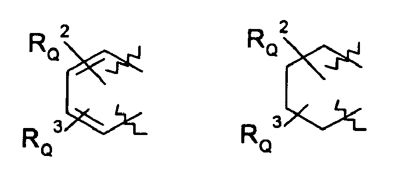 wobei R_{Q}², R_{Q}³ und R_{Q}⁷ wie unter 2) definiert sind, einschließlich der bevorzugten Ausführungsformen.

Am meisten bevorzugt sind beide Substituenten R_{Q}² und R_{Q}³ Wasserstoff, oder ein Substituent ist Wasserstoff und der andere ist ein Substituent außer Wasserstoff. Dabei ist der andere Substituent ist bevorzugt Methyl oder O-C₁-C₃-Alkyl. Es ist ebenfalls bevorzugt, dass beide Substituenten Methyl sind oder ein Substituent Methyl und der andere Halogen ist.

In einer Ausführungsform bilden R_{Q}² und R_{Q}³ zusammen einen Phenylring.

Wenn beide Reste R⁴ und R⁵ zusammen einen der vorstehend genannten stickstoffhaltigen Ringe bilden, dann ist R_{Q}⁷ wie unter 2) definiert, vorzugsweise Wasserstoff, C(O)-C₁-C₄-Alkyl, SO₂-Aryl oder C₁-C₄-Alkylen-Aryl, noch bevorzugter Wasserstoff, C(O)-CH₃, SO₂-Phenyl oder Benzyl. Am stickstoffhaltigen Ring sind beide Substituenten R_{Q}² und R_{Q}³ dabei vorzugsweise Wasserstoff.Für den Fall 5.) sind R⁴ und/oder R⁵ vorzugsweise Adamantyl.

Vorzugsweise wird einer der beiden Reste R⁴ und R⁵ aus der Gruppe 1.), einschließlich der bevorzugten Ausführungsformen davon, ausgewählt, und der andere Rest wird aus der Gruppe 1.), 2.) oder 3.), einschließlich der jeweiligen bevorzugten Ausführungsformen davon, ausgewählt. Dabei ist der erste Rest von R⁴ und R⁵ vorzugsweise Methyl oder Wasserstoff.

Für den Fall 6.) sind **R⁴, R⁵** vorzugsweise C₁-C₄-Alkyl-NH₂, C₁-C₄-Alkyl-NR_{Q}⁷R_{Q}⁸, C₁-C₄-Alkyl-CO-NR_{Q}⁷R_{Q}⁸, CO-NR_{Q}⁷R_{Q}⁸ oder NR_{Q}⁷R_{Q}⁸,

Für den Fall C₁-C₄-Alkyl-NR_{Q}⁷R_{Q}⁸ oder C₁-C₄-Alkyl-CO-NR_{Q}⁷R_{Q}⁸ sind R_{Q}⁷ und R_{Q}³ wie unter 2) definiert, einschließlich der bevorzugten Ausführungsformen. Besonders bevorzugt ist R_{Q}⁷ Wasserstoff und R_{Q}⁸ ist C(O)O-C₁-C₄-Alkyl, C(O)-Aryl, oder SO₂-C₁-C₆-Alkyl. Alternativ können beide R_{Q}⁷ und R_{Q}⁸ C₁-C₄-Alkyl sein. Bei diesen Definitionen bedeutet C₁-C₄-Alkyl vorzugsweise einen Methylen- oder Ethylenrest.

Für den Fall CO-NR_{Q}⁷R_{Q}⁸ sind R_{Q}⁷ und R_{Q}⁸ wie unter 2) definiert, einschließlich der bevorzugten Ausführungsformen. Besonders bevorzugt ist R_{Q}⁷ Wasserstoff und R_{Q}⁸ ist C₁-C₄-Alkyl, C₂-C₆-Alkenyl, oder C₁-C₄-Alkylen-Aryl, noch bevorzugter Isopropyl, Propenyl oder Benzyl.

Besonders bevorzugt sind die Reste CH₂NH₂, CH₂-NH-C(O)O-tert-Butyl, CH₂-NH-C(O)O-Methyl, CH₂-NH-C(O)-Phenyl, CH₂-NH-SO₂-n-Butyl, CH₂-N(Me)₂, C(O)-NH-CH(CH₃)₂, C(O)-NH-CH₂CHCH₂, C(O)-NH-CH₂-Phenyl.

Für den Fall 7.) sind **R⁴, R⁵** vorzugsweise jeweils gegebenenfalls substituiertes Azetidin-3-yl, Pyrrolidin-2-yl, Pyrrolidin-3-yl, Piperidin-2-yl, Piperidin-3-yl, Piperidin-4-yl, Tetrahydro-2H-pyran-4-yl, Tetrahydrofuran-3-yl, Azepan-4-yl, Azepan-3-yl, Azepan-2-yl, 1,4-Diazepan-5-yl, 1,2,3,6-Tetrahydropyridin-4-yl, 2,5-Dihydro-1H-pyn-ol-3-yl, besonders bevorzugt:

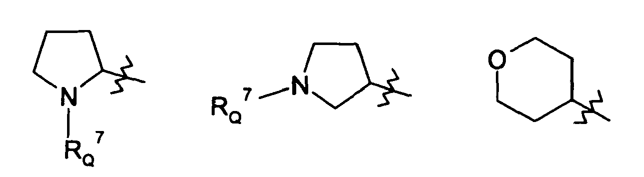

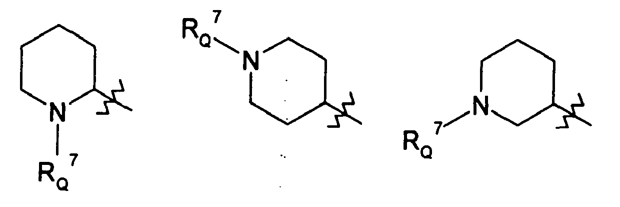

Noch bevorzugter sind

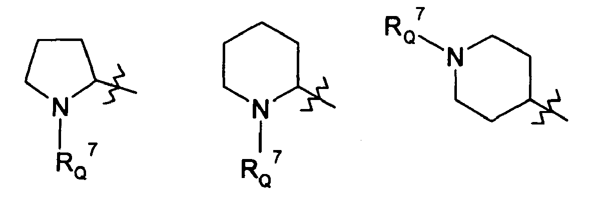

In diesem Fall ist R_{Q}⁷ wie unter 2) definiert, einschließlich der bevorzugten Ausführungsformen. Besonders bevorzugt ist R_{Q}⁷ Wasserstoff, C₁-C₄-Alkyl, C(O)-C₁-C₄-Alkyl, C₁-C₄-Alkylen-Aryl, SO₂-Aryl oder SO₂-C₁-C₄-Alkyl. Am meisten bevorzugt ist R_{Q}⁷ Wasserstoff, Methyl, Isopropyl, C(O)-Me, SO₂-Phenyl, SO₂-Me oder Benzyl.

In einer bevorzugten Ausführungsform wird einer der beiden Reste Reste R⁴ und R⁵ aus der Gruppe 1) ausgewählt, einschließlich der bevorzugten Ausführungsformen aus der Gruppe 1, und der andere Rest von R⁴ und R⁵ ist vorzugsweise Methyl oder Wasserstoff.

In einer weiteren bevorzugten Ausführungsform wird einer der beiden Reste Reste R⁴ und R⁵ aus der Gruppe 2) ausgewählt, einschließlich der bevorzugten Ausführungsformen aus der Gruppe 2, und der andere Rest von R⁴ und R⁵ ist vorzugsweise Methyl oder Wasserstoff.

In einer bevorzugten Ausführungsform wird einer der beiden Reste Reste R⁴ und R⁵ aus der Gruppe 3) ausgewählt, einschließlich der bevorzugten Ausführungsformen aus der Gruppe 3, und der andere Rest von R⁴ und R⁵ ist vorzugsweise Methyl oder Wasserstoff.

In einer weiteren bevorzugten Ausführungsform wird einer der beiden Reste Reste R⁴ und R⁵ aus der Gruppe 4) ausgewählt, einschließlich der bevorzugten Ausführungsformen aus der Gruppe 4, und der andere Rest von R⁴ und R⁵ ist vorzugsweise Methyl oder Wasserstoff.

In einer weiteren bevorzugten Ausführungsform wird einer der beiden Reste Reste R⁴ und R⁵ aus der Gruppe 6) ausgewählt, einschließlich der bevorzugten Ausführungsformen aus der Gruppe 6, und der andere Rest von R⁴ und R⁵ ist vorzugsweise Methyl oder Wasserstoff.

In einer weiteren bevorzugten Ausführungsform wird einer der beiden Reste Reste R⁴ und R⁵ aus der Gruppe 7) ausgewählt, einschließlich der bevorzugten Ausführungsformen aus der Gruppe 7, und der andere Rest von R⁴ und R⁵ ist vorzugsweise Methyl oder Wasserstoff.

Insgesamt stellt Q vorzugsweise einen Rest dar, der sich aus den bevorzugten Kombinationen von E, R_{Q}¹, R⁴, R⁵, R_{Q}², R_{Q}³, R_{Q}⁴, R_{Q}⁵, R_{Q}⁶, R_{Q}⁷ und R_{Q}⁸ zusammensetzt.

Die vorstehend erläuterten Ausführungsformen eines jeden Restes, einschließlich der bevorzugten Ausführungsformen, sind mit den jeweiligen Ausführungsformen der anderen Reste beliebig kombinierbar.

In der vorliegenden Erfindung besitzen die verwendeten Ausdrücke die nachstehend erläuterten Bedeutungen:

Alkyl ist eine geradkettige oder verzweigte gesättigte Kohlenwasserstoffkette mit der jeweils angegebenen Anzahl von Kohlenstoffatomen, vorzugsweise 1 bis 6, noch bevorzugter 1 bis 4 Kohlenstoffatomen, wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 1,2-Dimethylpropyl, 1,1-Dimethylpropyl, 2,2-Dimethylpropyl, 1-Ethylpropyl, n-Hexyl, 1-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl oder 1-Ethyl-2-methylpropyl, vorzugsweise Methyl, Ethyl, Propyl, n-Butyl oder i-Butyl.

Alkylen ist eine Alkylgruppe, die wie vorstehend definiert ist, bei der ein Wasserstoffatom durch eine Bindung ersetzt ist. Insbesondere sind Methylen, Eth-1,2-ylen, Prop-1,2-ylen, Prop-1,3-ylen, But-1,2-ylen, But-1,3-ylen, But-2,3-ylen, But-1,4-ylen, 2-Methylprop-1,3-ylen, Pent-1,2-ylen, Pent-1,3-ylen, Pent-1,4-ylen, Pent-1,5-ylen, Pent-2,3-ylen, Pent-2,4-ylen, 1-Methylbut-1,4-ylen, 2-Methylbut-1,4-ylen, 2-Methylbut-1,3-ylen, 2-Ethylprop-1,3-ylen, Hex-3,4-ylen, 3-Methylpent-2,4-ylen, Hept-3,5-ylen, 2-Ethylpent-1,3-ylen, 3-Ethylhept-3,5-ylen, etc., vorzugsweise Methylen, Eth-1,2-ylen und Prop-1,2-ylen, zu nennen

Cycloalkyl ist ein gesättigter Kohlenwasserstoffring mit 3 bis 7, vorzugsweise 3 bis 6 Kohlenstoffatomen, wie Cyclopropyl, Cyclobutyl, Cyclopentyl, Cyclohexyl oder Cycloheptyl.

Alkylen-O-Alkyl ist eine geradkettige oder verzweigte gesättigte Alkyletherkette, wobei sowohl der Alkylenrest als auch der Alkylrest unabhängig voneinander 1 bis 6, noch bevorzugter 1 bis 4, am meisten bevorzugt 1 oder 2 Kohlenstoffatome enthalten, wobei beide Reste wie vorstehend definiert sind. die bis insgesamt 2 bis 12 Kohlenstoffatome und ein Sauerstoffatom enthält: Bevorzugte Beispiele von Alkylen-O-Alkyl beinhalten Methoxymethylen, Ethoxymethylen, t-Butoxymethylen, Methoxyethylen oder Ethoxyethylen.

Thioalkyl ist eine geradkettige oder verzweigte Alkylensulfanylkette, die 1 bis 6 Kohlenstoffatome und ein Schwefelatom enthält. Vorzugsweise enthält der Alkylenrest 1 bis 4, noch bevorzugter 1 oder 2 Kohlenstoffatome, wobei Alkylen wie vorstehend definiert ist. Beispiele von Thioalkyl beinhalten Thiomethyl oder Thio-tert-butyl.

Alkenyl ist eine verzweigte oder unverzweigte Kohlenwasserstoffkette, enthaltend mindestens eine Doppelbindung, mit 2 bis 6, vorzugsweise 2 bis 4 Kohlenstoffatomen. Vorzugsweise enthält Alkenyl eine oder zwei Doppelbindungen, am meisten bevorzugt eine Doppelbindung. Beispiele der Alkenylgruppen sind jene, wie sie vorstehend für Alkyl genannt werden, wobei diese Gruppen eine oder zwei Doppelbindungen enthalten, wie beispielsweise Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-entenyl, 4-Methyl-4-pentenyl, 1,1-Dimethyl-2-butenyl, 1;1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere 2-Propenyl, 2-Butenyl, 3-Methyl-2-butenyl oder 3-Methyl-2-pentenyl.

Alkinyl ist eine verzweigte oder unverzweigte Kohlenwasserstoffkette, enthaltend mindestens eine Dreifachbindung mit 2 bis 6, vorzugsweise 2 bis 4 Kohlenstoffatomen. Vorzugsweise enthält Alkinyl eine oder zwei Dreifachbindungen, am meisten bevorzugt eine Dreifachbindung. Beispiele der Alkinylgruppen sind jene, wie sie vorstehend für Alkyl genannt werden, wobei diese Gruppen eine oder zwei Dreifachbindungen enthalten, wie beispielsweise Ethinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise Ethinyl, 2-Propinyl, 2-Butinyl, 1-Methyl-2-propinyl oder 1-Methyl-2-butinyl.

Heterocycloalkyl ist ein gesättigter Alkylring oder ein Alkylring, an den ein weiterer gesättigter Alkylring anelliert ist, mit vorzugsweise 3 bis 10 Ringatomen insgesamt, noch bevorzugter 3 bis 6 Ringatomen, am meisten bevorzugt 5 oder 6 Ringatomen, wobei dieser Heterocycloalkyl mindestens ein Heteroatom, ausgewählt aus der Gruppe O, N oder S, enthält und 1 bis 6, vorzugsweise 1 bis 5 Kohlenstoffatome enthält. Vorzugsweise enthält Heterocycloalkyl 1 oder 2 Heteroatome, die vorzugsweise aus N und/oder O werden. Beispiele einer Heterocycloalkylgruppe beinhalten beispielsweise N-Pyrrolidinyl, N-Piperidinyl, N-Hexahydroazepinyl, N-Morpholinyl oder N-Piperazinyl, wobei bei Heterocyclen, die Aminogruppen enthalten, wie beispielsweise N-Piperazinyl, diese Aminogruppen durch gängige Reste, wie beispielsweise Methyl, Benzyl, Boc (tert.-Butoxycarbonyl), Benzyloxycarbonyl, Tosyl (p-Toluoltsulfonyl), -SO₂-C₁-C₄-Alkyl, -SO₂-Phenyl oder -SO₂-Benzyl ersetzt sein können.

Aryl ist ein aromatischer mono-, bi- oder polycyclischer Rest mit vorzugsweise 6 bis 20 Kohlenstoffatomen, noch bevorzugter 6 bis 10 Kohlenstoffatomen und wird vorzugsweise ausgewählt aus Phenyl, Biphenyl, Naphthyl, Tetrahydronaphthyl, Fluorenyl, Indenyl und Phenanthrenyl, noch bevorzugter aus Phenyl und Naphthyl, wie 1-Naphthyl oder 2-Naphthyl. Am meisten bevorzugt ist Phenyl.

Alkylenaryl ist ein über C₁-C₆-, noch bevorzugter C₁-C₄-Alkylen gebundenes, im Arylrest gegebenenfalls substituiertes Aryl, wobei Alkylen und Aryl wie vorstehend definiert sind. Alkylenaryl ist insbesondere im Arylrest gegebenenfalls substituiertes Benzyl oder Phenethyl.

Aryloxy oder -O-Aryl ist ein über Sauerstoff gebundenes Aryl, das wie vorstehend definiert ist, insbesondere -O-Phenyl.

Hetaryl ist ein aromatischer Ring, enthaltend wenigstens ein Heteroatom, vorzugsweise 1 oder 2 Heteroatome, ausgewählt aus der Gruppe O, N oder S und vorzugsweise 1 bis 6, noch bevorzugter 1 bis 5 Kohlenstoffatome. Der aromatische Ring ist vorzugsweise 5-oder 6-gliedrig. Hetaryl umfasst außerdem die mit Aryl anellierten Derivate davon, nämlich einen aromatischen Rest mit vorzugsweise 6 bis 20 Kohlenstoffatomen, noch bevorzugter 6 bis 10 Kohlenstoffatomen, am meisten bevorzugt Phenyl, der mit diesem aromatischen Ring, enthaltend wenigstens ein Heteroatom, anelliert ist. Hetaryl kann auch ausgewählt werden aus einem aromatischen Rest mit vorzugsweise 6 bis 20, noch bevorzugter 6 bis 10 Kohlenstoffatomen, am meisten bevorzugt Phenyl, mit einer Heterocycloalkylgruppe, die daran anelliert ist. Dabei ist die Heterocycloalkylgruppe wie vorstehend definiert. Hetaryl wird vorzugsweise ausgewählt aus 2-Furyl, 3-Furyl, 2-Pyrrolyl, 3-Pyrrolyl, 2-Thienyl, 3-Thienyl, 2-Pyridyl, 3-Pyridyl, 4-Pyridyl, 2-Thiazolyl, 4-Thiazolyl, 5-Thiazolyl, 2-Oxazolyl, 4-Oxazolyl, 5-Oxazolyl, 2-Pyrimidyl, 4-Pyrimidyl, 5-Pyrimidyl, 6-Pyrimidyl, 3-Pyrazolyl, 4-Pyrazolyl, 5-Pyrazolyl, 3-Isothiazolyl, 4-Isothiazolyl, 5-Isothiazolyl, 2-Imidazolyl, 4-Imidazolyl, 5-Imidazolyl, 3-Pyridazinyl, 4-Pyridazinyl, 5-Pyridazinyl, 6-Pyridazinyl, 3-Isoxazolyl, 4-Isoxazolyl, 5-Isoxazolyl, Thiadiazolyl, Oxadiazolyl, Triazinyl, Indolinyl, Benzothienyl, Naphthothienyl, Benzofuranyl, Chromenyl, Indolyl, Isoindolyl, Indazolyl, Chinolyl, Isochinolyl, Phthalazinyl, Chinoxalinyl, Benzimidazolyl und Benzoxazolyl, 2,3-Dihydro-1,4-benzodioxinyl, 1,3-Benzodioxolyl-, 2,1,3-Benzothiadiazolyl.

Alkylenhetaryl ist ein über C₁-C₆-, noch bevorzugter C₁-C₄-Alkylen gebundenes, im Hetarylrest gegebenenfalls substituiertes Hetaryl, wobei Alkylen und Hetaryl wie hier definiert sind. Alkylenhetaryl ist vorzugsweise gegebenenfalls substituiertes -CH₂-2-Pyridyl, -CH₂-3-Pyridyl, -CH₂-4-Pyridyl, -CH₂-2-Thienyl, -CH₂-3-Thienyl, -CH₂-2-Thiazolyl, -CH₂-4-Thiazolyl, CH₂-5-Thiazolyl, -CH₂-CH₂-2-Pyridyl, -CH₂-CH₂-3-Pyridyl, -CH₂-CH₂-4-Pyridyl, -CH₂-CH₂-2-Thienyl, -CH₂-CH₂-3-Thienyl, -CH₂-CH₂-2-Thiazolyl, -CH₂-CH₂-4-Thiazolyl oder -CH₂-CH₂-5-Thiazolyl.

Ein bi- oder tricyclischer, gesättigter Kohlenwasserstoffrest ist ein Bicycloalkyl- oder Tricycloalkylrest und besitzt 5 bis 18 Kohlenstoffatome. Bei einem Bicycloalkylrest enthält das Ringsystem vorzugsweise 5 bis 12, noch bevorzugter 6 bis 10 Kohlenstoffatome, Bei einem Tricycloalkylrest enthält das Ringsystem vorzugsweise 6 bis 16, noch bevorzugter 6 bis 12 Kohlenstoffatome. Beispiele eines Bicycloalkylrestes beinhalten Indanyl, Camphyl und Norbomyl. Beispiele eines Tricycloalkylrestes beinhalten Adamantyl.

Halogen ist ein Halogenatom ausgewählt aus Fluor, Chlor, Brom oder lod, vorzugsweise Fluor, Chlor oder Brom, noch bevorzugter Fluor oder Chlor.

Mit Halogen substituiertes Alkyl bezeichnet einen Alkylrest, wie vorstehend definiert, der partiell oder vollständig durch Fluor, Chlor, Brom und/oder Iod substituiert ist, also z.B. CH₂F, CHF₂, CH₂Cl, 2-Fluorethyl, 2-Chlorethyl, 2,2,2-Trifluorethyl.

Falls erwähnt, können die Reste und Gruppen vorzugsweise ein- oder mehrfach, noch bevorzugter ein-, zwei- oder dreifach, am meisten bevorzugt ein- oder zweifach substituiert sein. Der Ausdruck "jeweils gegebenenfalls substituiert" soll verdeutlichen, dass nicht nur der direkt darauf folgende Rest sondern alle in der jeweiligen Gruppe genannten Reste substituiert sein können.

Beispiele der Substituenten beinhalten: Halogen, CN, CF₃, CHF₂, OCF₃, OCHF₂, NO₂, NH₂, OH, COOH, jeweils verzweigtes oder unverzweigtes, gegebenenfalls substituiertes C₁-C₆-Alkyl, C₃-C₇-Cycloalkyl, C₁-C₆-Alkylen-O-C₁-C₆-Alkyl oder C₁-C₆-Thioalkyl, O-C₁-C₄-Alkyl, N(C₁-C₄-Alkyl)₂, NH(C₁-C₄-Alkyl), Aryl, -O-Aryl, C₁-C₄-Alkylen-O-Aryl, NHCO-C₁-C₄-Alkyl, NH-SO₂-C₁-C₄-Alkyl, CO-C₁₋₄-Alkyl, SO₂-C₁-C₄-Alkyl, im Arylrest gegebenenfalls substituiertes NHCO-Aryl, NHSO₂-Aryl, CONH₂, SO₂NH₂, SO₂-Aryl, SO-C₁-C₄-Alkyl, SO-Aryl, N-Pyrrolidinyl, N-Piperidinyl, und N-Morpholinyl. Bevorzugte Substituenten sind F, Cl, CF₃, OCF₃, NH₂, NO₂, OH, COOH, C₁-C₄-Alkyl, Methoxy, Acetyl, NH-Acetyl und SO₂NH₂.

### Optische Isomere - Diastereomere - Geometrische Isomere - Tautomere

Die Guanidinverbindungen der Formel I oder IA bzw. deren Salze können mindestens ein asymmetrisches Zentrum besitzen und können als Racemate und racemische Gemische, einzelne Enantiomere, diastereomere Gemische und einzelne Diastereomere vorliegen. Die vorliegende Erfindung umfasst alle diese stereoisomeren Formen der Guanidinverbindungen der Formel I oder IA.

Die Guanidinverbindungen der Formel I oder IA können in ihre einzelnen Stereoisomere durch herkömmliche Verfahren aufgespalten werden durch z.B. fraktionierte Kristallisation aus einem geeigneten Lösungsmittel, z.B. Methanol oder Ethylacetat oder einem Gemisch davon oder durch chirale Chromatographie unter Verwendung einer optisch aktiven stationären Phase. Die absolute Stereochemie kann durch Röntgenkristallographie der kristallinen Produkte oder kristallinen Zwischenprodukte ermittelt werden, die, falls notwendig, mit einem Reaktionsmittel derivatisiert werden, das ein asymmetrisches Zentrum einer bekannten absoluten Konfiguration enthält. Alternativ kann ein beliebiges Stereoisomer eines Guanidins der Formel I oder IA erhalten werden durch stereospezifische Synthese unter Verwendung von optisch reinen Ausgangsmaterialien oder Reaktionsmitteln mit bekannter absoluter Konfiguration oder durch asymmetrische Synthesemethoden.

Bevorzugt ist die Verwendung einer enantiomeren- bzw. diastereomerenreinen Verbindung.

Insbesondere können die hier beschriebenen Guanidinverbindungen der Formel I oder IA auch als verschiedene Tautomere der Guanidingruppe vorliegen, wobei, wie es für den Fachmann ersichtlich ist, die Art der Tautomerie von der Natur der Reste R1, R2 und R3 abhängt. Auch andere Tautomere, wie Keto-Enol-Tautomere, können vorliegen. Alle einzelnen möglichen Tautomere sowie Gemische davon sind durch die Guanidinverbindungen der Formel I oder IA umfasst.

### Salze

Der Begriff "pharmazeutisch annehmbare Salze" bezieht sich auf Salze, die aus pharmazeutisch annehmbaren physiologisch verträglichen Basen oder Säuren, einschließlich anorganischen oder organischen Basen und anorganischen oder organischen Säuren, hergestellt werden.

Salze, die sich aus anorganischen Basen ableiten beinhalten Aluminium, Ammonium, Calcium, Kupfer, Eisen(II), Eisen(III), Lithium, Magnesium, Mangan, Kalium, Natrium, Zink und Ähnliche. Besonders bevorzugt sind die Ammonium-, Calcium-, Lithium-, Magnesium-, Kalium- und Natriumsalze. Salze, die sich von pharmazeutisch annehmbaren organischen nicht toxischen Basen ableiten, beinhalten Salze von primären, sekundären und tertiären Aminen, substituierten Aminen, einschließlich natürlich vorkommenden substituierten Aminen, cyclischen Aminen und basischen lonenaustauscherharzen, wie Arginin, Betain, Coffein, Cholin, N,N'-Dibenzylethylendiamin, Diethylamin, 2-Diethylaminomethanol, 2-Dimethylaminoethanol, Ethanolamin, Ethylendiamin, N-Ethylmorpholin, N-Ethylpiperidin, Glucamin, Glucosamin, Histidin, Hydrabamin, Isopropylamin, Lysin, Methylglucamin, Morpholin, Piperazin, Piperidin, Polyaminharze, Procain, Purine, Theobromin, Triethylamin, Trimethylamin, Tripropylamin, Tromethamin und ähnliche.

Wenn das Guanidin der vorliegenden Erfindung basisch ist, können Salze aus pharmazeutisch annehmbaren physiologisch verträglichen Säuren, einschließlich anorganischer und organischer Säuren, hergestellt werden. Solche Säuren beinhalten unter anderem Essigsäure, Benzolsulfonsäure, Benzoesäure, Kampfersulfonsäure, Citronensäure, Ethansulfonsäure, Ameisensäure, Fumarsäure, Gluconsäure, Glutaminsäure, Bromwasserstoffsäure, Chlorwasserstoffsäure, Milchsäure, Äpfelsäure, Maleinsäure, Mandelsäure, Methansulfonsäure, Malonsäure, Salpetersäure, Pantotheninsäure, Phosphorsäure, Propionsäure, Bernsteinsäure, Schwefelsäure, Weinsäure, p-Toluolsulfonsäure, Trifluoressigsäure und ähnliche. Besonders bevorzugt sind Citronensäure, Fumarsäure, Bromwasserstoffsäure, Chlorwasserstoffsäure, Maleinsäure, Phosphorsäure, Schwefelsäure und Weinsäure.

Wenn Bezug genommen wird auf die Guanidinverbindungen der Formel I oder IA, soll dies bedeuten, dass auch die pharmazeutisch annehmbaren Salze davon beinhaltet sind.

### Verwendung, Einsatzgebiete und Wirkungen

Gegenstand der Erfindung ist auch die Verwendung der Guanidinverbindungen der Formel I oder IA zur Behandlung von:
- Depressionen und/oder bipolaren Störungen wie zum Beispiel dysthemische Störungen, jahreszeitlich bedingte Störungen und/oder psychotische Störungen.
- Angst und/oder stress-bedingte Störungen wie zum Beispiel generelle Angststörungen, Panikstörungen, Zwangsstörungen, posttraumatische Störungen, akute Stress-Störungen und/oder soziale Phobie
- Gedächtnisstörungen und/oder Alzheimer Krankheit
- Schizophrenie, Psychosen, psychotische Störungen und/oder psychotisch bedingte Störungen
- Cerebrovaskuläre Störungen
- Schmerz und/oder Schmerz-bedingte Störungen, Abhängigkeit und Drogenbedingte Störungen, einschließlich Medikamenten-bedingte Störungen
- Amnesie
- Alkohol- und/oder Drogenmissbrauch, einschließlich Medikamentenmißbrauch
- Störungen des circadianen Rythmus
   und/oder
- Cushing Syndrom.

Der Begriff "Störung" im erfindungsgemäßen Sinne bezeichnet Anomalien, die in der Regel als krankhafte Zustände angesehen werden und sich in Form bestimmter Anzeichen, Symptome und/oder Fehlfunktionen zu erkennen geben können. Die Behandlung kann auf einzelne Störungen sprich Anomalien bzw. krankhafte Zustände gerichtet sein, es können aber auch mehrere gegebenenfalls ursächlich miteinander verbundene Anomalien zu Mustern, d.h. Syndromen, zusammengefasst sein, die erfindungsgemäß behandelt werden können. Dieser Zustand kann vorübergehend, fortschreitend oder dauerhaft bestehen.

Verbindungen der vorliegenden Erfindung können verwendet werden zur Behandlung oder Prävention von verschiedenen Erkrankungen, an deren Entstehung und/oder Verlauf 5-HT5-Rezeptoren beteiligt sind, d.h. Erkrankungen, die durch eine 5-HT5-Rezeptoraktivität moduliert werden, wie mentale Störungen. Beispiele solch mentaler Störungen sind nach dem "American Psychatric Assiciation DSM-IV, Diagnostic and Statistical Manual of Mental Disorders, 4th ed., 1994: Aufmerksamkeitsstörungen und sozial störendes Verhalten; Lemstörungen, Delir, Demenz und amnestische und andere kognitive Störungen; Störungen im Zusammenhang mit verschiedenen Substanzen, wie zum Beispiel Störungen im Zusammenhang mit Alkoholkonsum und Alkohol-induzierte Störungen, Entzugserscheinungen; Schizophrenie und andere psychotische Störungen wie z.B. schizophrenieforme Störung, schizoaffektive Störung, und wahnhafte Störung; Substanz-induzierte Psychosen; paranoide Störungen; Neuroleptika-induzierte Störungen; affektive Störungen, wie zum Beispiel depressive Störungen (Major Depression, dysthemische Störung, jahreszeitlich bedingte Störung, nicht näher bezeichnete depressive Störung), bipolare Störungen (bipolar I Störung, bipolar II Störung, zyklothyme Störung, nicht näher bezeichnete bipolare Störung, Substanz (Amphetamin oder amphetaminähnliche Substanzen) induzierte affektive Störung, nicht näher bezeichnete affektive Störung); Störungen im Zusammenhang mit Stress, wie zum Beispiel akute Belasturigsstörung; Angststörungen, wie zum Beispiel Panikstörungen ohne Agoraphobie, Panikstörung mit Agoraphobie, Agoraphobie ohne Panikstörung in der Vorgeschichte, spezifische Phobie, soziale Phobie, Zwangsstörung, posttraumatische Belastungsstörung, akute Belastungsstörung, generalisierte Angststörung, substanzinduzierte Angststörung; somatoforme Störungen wie zum Beispiel Somatisierungsstörung, nicht näher bezeichnete somatoforme Störung, Konversionsstörung, Schmerzstörung; Eßstörungen; Schlafstörungen wie zum Beispiel primäre Schlafstörungen (Dyssomnie, Parasomnie), Schlafstörungen im Zusammenhang mit einer anderen mentalen Störung.

Gegenstand der Erfindung ist insbesondere auch die Verwendung der Guanidinverbindungen I und IA für die Behandlung neuropathologischer, neuropsychiatrischer und neurodegenerativer Störungen.

Unter neuropathologischen Störungen versteht man Störungen, die von neurologischen Defiziten begleitet sind, d. h. einen durch neurologische Ausfallerscheinungen gekennzeichneten Zustand.

Erfindungsgemäß bevorzugt ist die Behandlung neurodegenerativer und/oder neuropsychiatrischer Störungen. Diese Störungen treten insbesondere bei neuropathologischen, in der Regel Gehimschädigungen verursachenden Krankheitsbildern auf, beispielsweise cerebraler Ischämie, Schlaganfall, Epilepsie und Anfällen im allgemeinen, chronischer Schizophrenie, anderen psychotischen Erkrankungen, Depression, Angstzuständen, bipolaren Störungen, Demenz, insbesondere Alzheimer Demenz, demyelinisierenden Erkrankungen, insbesondere Multipler Sklerose, Gehirntumore und generelle Entzündungsprozesse. Eine weitere neuropathologische Störung ist Migräne, sowie die damit zusammenhängenden Anzeichen, Symptome und Fehlfunktionen.

Gemäß eines weiteren Aspekts der vorliegenden Erfindung werden neuropathologische Störungen behandelt, die mit einer glialen Reaktion einhergehen. Die erfindungsgemäße Verwendung betrifft insbesondere die Modulation einer glialen Reaktion. Eine vorteilhafte Wirkung der Bindungspartner zeigt sich bei der präventiven oder akuten Behandlung neurologischer Defizite, die an Patienten beobachtet werden, die unter psychiatrischen Erkrankungen leiden, wie Epilepsie, Psychose, z.B. Psychosen vom akuten exogenen Reaktionstyp oder Begleitpsychosen organischer bzw. exogener Ursache, z.B. nach Trauma, vor allem Hirnläsionen und diffusen Hirnschädigungen, bei Stoffwechselstörungen, Infektionen, und Endokrinopathien; endogene Psychosen, wie Schizophrenie sowie schizotype und wahnhafte Störungen; affektive Störungen, wie Depression, Manie bzw. manisch-depressive Zustände; sowie Mischformen der zuvor geschilderten Psychosen; seniler Demenz und seniler Demenz vom Alzheimer Typ, sowie bei der Behandlung oder Prävention von Demyelinisationsvorgängen.

Wirksam sind die erfindungsgemäßen Guanidinverbindungen insbesondere im Hinblick auf die Behandlung ischämischer Schäden, z.B. infolge von Hirn- und Rückenmarkstrauma sowie Gefäßverschluß oder Herzversagen. Zu nennen ist hier vor allem der Schlaganfall (Synonym: Apoplexia cerebri, cerebraler oder apoplektischer Insult, Gehimschlag). Erfindungsgemäß behandelbar sind transitorisch-ischämische Attacken, reversible ischämische neurologische Defizite, prolongierte reversible ischämische neurologische Defizite, partiell reversible ischämische neurologische Symptomatiken und auch persistierende komplette Himinfarkte. Besonders vorteilhaft ist erfindungsgemäß die Behandlung akuter Formen.

Den erfindungsgemäß bevorzugt behandelten Formen neuropathologischer Störungen liegen eine oder mehrere der nachfolgend aufgezählten Veränderungen von Nervengeweben zugrunde: Degeneration oder Absterben von Neuronen, insbesondere der Ganglienzellen, zB. Tigrolyse, Kernmembranunschärfe, Zellschrumpfung, Zytoplasmavakuolisierung und -inkrustation, Parenchymnekrosen des Gehirns, Himödeme, durch Sauerstoffmangel verursachte Veränderungen von Neuronen, Atrophie, morphologische Veränderungen, wie Demyelinisierungen, insbesondere ein Markscheidenzerfall, perivaskuläre Infiltrate, gliöse Proliferation und/oder Glianarben; Degeneration der Substantia nigra.

Die erfindungsgemäß zu behandelnde Indikation ist häufig gekennzeichnet durch eine progressive Entwicklung, d.h. die vorstehend beschriebenen Zustände verändern sich im Laufe der Zeit, in der Regel nimmt der Schweregrad zu und gegebenenfalls können Zustände ineinander übergehen oder weitere Zustände zu bereits bestehenden Zuständen hinzutreten. Durch die erfindungsgemäße Behandlung neuropathologischer, neuropsychiatrischer oder neurodegenerativer Störungen bzw. den ihr zugrunde liegenden Zuständen lassen sich eine Reihe weiterer Anzeichen, Symptome und/oder Fehlfunktionen behandeln, die mit diesen Störungen zusammenhängen, d.h. insbesondere die oben beschriebenen Erkrankungszustände begleiten. Hierzu gehören beispielsweise Schocklunge; Himnervenausfälle, z.B. retrobulbäre Neuritis, Augenmuskellähmungen, skandierende Sprache, spastische Lähmungen, Kleinhirnsymptome, Sensibilitäts-, Blasen- und Mastdarmstörungen, Euphorie, Demenz; Hypo- und Akinese, fehlende Mitbewegung, kleinschrittiger Gang, Beugehaltung von Rumpf und Gliedern, Pro-, Retro- und Lateropulsion, Tremor, Mimikarmut, monotone Sprache, Depressionen, Apathie, labile oder starre Affektivität, erschwerte Spontaneität und Entschlußkraft, verlangsamtes Denken, verarmte Assoziationsfähigkeit; Muskelatrophie.

Eine Behandlung im erfindungsgemäßen Sinne umfasst nicht nur die Behandlung akuter oder chronischer Anzeichen, Symptome und/oder Fehlfunktionen sondern auch eine vorbeugende Behandlung (Prophylaxe) insbesondere als Rezidiv- oder Phasen-Prophylaxe. Die Behandlung kann symptomatisch, beispielsweise als Symptomsuppression ausgerichtet sein. Sie kann kurzzeitig erfolgen, mittelfristig ausgerichtet sein, oder es kann sich auch um eine Langzeitbehandlung, beispielsweise im Rahmen einer Erhaltungstherapie, handeln.

Der Begriff "Bindungspartner für 5-HT5-Rezeptoren" beschreibt Substanzen, welche an 5-HT5-Rezeptoren binden und daher auch als 5-HT5-Rezeptorliganden bezeichnet werden können.

Unter Bindung versteht man jede molekulare Wechselwirkung zwischen dem Bindungspartner und dem Rezeptor, insbesondere unter physiologischen Bedingungen. Dies sind in der Regel klassische Wechselwirkungen, zu denen elektrostatische Anziehung, Wasserstoffbrücken-Bindung, hydrophobe Bindungen, van-der-Waals-Kräfte oder metallkomplexartige koordinative Bindungen gehören. Zusätzlich zu den vorstehend genannten, reversiblen molekularen Wechselwirkungen können auch irreversible Wechselwirkungen zwischen Bindungspartner und Rezeptor in Betracht kommen, wie z. B. kovalente Bindungen.

Erfindungsgemäße Guanidinverbindungen können die Bindung von Vergleichsbindungspartnern, wie 5-HT (5-Hydroxytryptamin) oder 5-CT (5-Carboxamidotryptamin), an 5-HT5-Rezeptoren kompetitiv hemmen. Unter kompetitiver Hemmung versteht man, dass die erfindungsgemäße Guanidinverbindungen mit einem Vergleichsbindungspartner, im vorliegenden Fall Z.B. 5-HT oder 5-CT, um die Bindung an den Rezeptor konkurrieren.

Gemäß einer weiteren Ausführungsform hemmen erfindungsgemäße Guanidinverbindungen die Bindung von Vergleichsbindungspartnem, wie 5-HT (5-Hydroxytryptamin) oder 5-CT (5-Carboxamidotryptamin), an 5-HT5-Rezeptoren nichtkompetitiv. Unter nicht-kompetitiver Hemmung versteht man, dass erfindungsgemäße Guanidinverbindungen über ihre Bindung an den Rezeptor die Bindung eines Vergleichsbindungspartner, im vorliegenden Fall z.B. 5-HT oder 5-CT, modulieren, insbesondere dessen Bindungsaffinität verringern.

Zumindest für den Fall der kompetitiven Hemmung, also der reversiblen Bindung, gilt der Grundsatz, dass die Verdrängung eines Bindungspartners durch einen anderen mit abnehmender Bindungsaffinität des einen bzw. zunehmender Bindungsaffnität des anderen im Hinblick auf den Rezeptor zunimmt. Zweckmäßigerweise besitzen daher erfindungsgemäße Guanidinverbindungen eine hohe Bindungsaffinität für 5-HT5-Rezeptoren. Eine derartige Bindungsaffinität gestattet einerseits eine wirksame Verdrängung natürlich vorkommender Bindungspartner für 5-HT5-Rezeptoren, wie beispielsweise Serotonin (5-Hydroxytryptamin, 5-HT) selbst, wobei die erforderliche Konzentration an erfindungsgemäßer Guanidinverbindung zur Bindung einer bestimmten Menge dieses Bindungspartners an 5-HT5-Rezeptoren mit zunehmender Bindungsaffinität abnimmt. Im Hinblick auf die medizinische Anwendung werden daher Guanidinverbindungen bevorzugt, deren Bindungsaffinität so groß ist, dass diese als Wirkstoff im Rahmen einer wirksamen medizinischen Behandlung in vertretbaren Mengen verabreicht werden können.

Eine Möglichkeit, die Bindungsaffinität auszudrücken, bieten die oben angesprochenen Kompetitionsexperimente, mit denen man in-vitro diejenige Konzentration an erfindungsgemäßer Guanidinverbindung ermittelt, die einen anderen Vergleichsbindungspartner zu 50% von der Rezeptorbindungsstelle verdrängt (IC50-Werte). So lässt sich auch die kompetitive Hemmung der Bindung von 5-CT an 5-HT5-Rezeptoren dahingehend auswerten, dass bevorzugte erfindungsgemäße Guanidinverbindungen halbmaximale Hemmkonstanten IC50 von weniger als 10-5 M, vorzugsweise von weniger als 10-6 M und insbesondere von weniger als 10-7 M aufweisen. Die Bindungsaffinität erfindungsgemäßer Guanidinverbindungen kann auch über die Hemmkonstante Ki ausgedrückt werden, die man im allgemeinen ebenfalls mit Kompetitionsexperimenten in-vitro bestimmt. Für die Bindung an 5-HT5-Rezeptoren weisen erfindungsgemäße Guanidinverbindungen vorzugsweise Ki-Werte von weniger als 10-6 M, vorteilhafterweise von weniger als 10-7 M und insbesondere bevorzugt von weniger als 10-8 M auf.

Brauchbare Bindungspartner können mit einer geringeren, einer im wesentlichen gleichen, oder einer höheren Affinität an 5-HT5 binden als an einen bestimmten, von 5-HT5 verschiedenen Rezeptor. So gehören zu Bindungspartnern für 5-HT5-Rezeptoren im Hinblick auf die erfindungsgemäße Verwendung insbesondere diejenigen, deren Bindungsaffinität zu 5-HT5-Rezeptoren verglichen mit der Affinität zu 5-HT-Rezeptoren so hoch ist, dass sie für die erfindungsgemäße Verwendung in vorteilhafter Weise geeignet sind. Dies setzt nicht notwendigerweise eine vergleichsweise selektivere Bindung an 5-HT5-Rezeptoren voraus, wenngleich selektive Bindungspartner für 5-HT5-Rezptoren eine besondere Ausführungsform der vorliegenden Erfindung sind.

Beispielsweise kann man Bindungspartner verwenden, die hochaffin sowohl zu 5-HT5 als auch zu anderen 5-HT-Rezeptoren sind. Hochaffin bedeutet in diesem Zusammenhang Ki-Werte in der Regel im Bereich von 1-10-10 M bis 1-10-6 M. Gemäß einer besonderen Ausführungsform besitzen Guanidinverbindungen im hochaffinen Bereich zu 5-HT-Rezeptoren ein Bindungsprofil, dass durch eine Bindungsaffinität zu 5-HT5 gekennzeichnet ist, die im Vergleich zu anderen Bindungsaffinitäten dieses Bereichs im wesentlichen gleich oder nur wenig geringer ist. Faktoren von 10 oder weniger können von Vorteil sein.

Erfindungsgemäße Guanidinverbindungen besitzen Bindungsaffinitäten für 5-HT5-Rezeptoren, die größer sind als für einen oder mehrere von 5-HT5 verschiedene 5-HT-Rezeptoren, also insbesondere den obengenannten 5-HT-Rezeptorklassen 5-HT1, 5-HT2, 5-HT3, 5-HT4, 5-HT6 und 5-HT7 zuzuordnenden Rezeptoren. Ist die Bindungsaffinität für 5-HT5-Rezeptoren eines Bindungspartners größer als die eines von 5-HT5 verschiedenen 5-HT-Rezeptors, so spricht man von einer in Bezug auf den von 5-HT5 verschiedenen 5-HT-Rezeptor selektiven Bindung dieser Bindungspartner an 5-HT5-Rezeptoren. Besondere Bindungspartner sind diejenigen, deren Bindungsaffinität für 5-HT5-Rezeptoren größer ist als für wenigstens einen 5-HT-Rezeptor. Guanidinverbindungen, deren Bindungsaffinität für 5-HT5-Rezeptoren größer ist als für sämtliche von 5-HT5 verschiedene 5-HT-Rezeptoren, stellen eine weitere besondere Klasse erfindungsgemäßer Guanidinverbindungen dar.

Unter Selektivität versteht man die Eigenschaft eines Bindungspartners, vorzugsweise an 5-HT5-Rezeptoren zu binden. Für die vorstehend geschilderte Selektivität ist maßgebend, dass sich die Bindungsaffinitäten für 5-HT5-Rezeptoren einerseits und für einen oder mehrere von 5-HT5 verschiedene 5-HT-Rezeptoren andererseits hinreichend unterscheiden. Bevorzugt sind Affinitätsunterschiede, wonach Bindungsaffinitäts-Verhältnisse von wenigstens 2, vorteilhafter von wenigstens 5, besonders vorteilhaft von wenigstens 10, vorzugsweise von wenigstens 20, besonders bevorzugt von wenigstens 50 und insbesondere von wenigstens 100 vorliegen.

Gemäß einer weiteren Ausführungsform binden erfindungsgemäße Guanidinverbindungen in Bezug auf einen oder mehrere von 5-HT5 verschiedene 5-HT-Rezeptoren selektiv an 5-HT5-Rezeptoren mit den oben beschriebenen vorteilhaften Bindungsaffinitäten.

Gemäß einer weiteren Ausführungsform binden erfindungsgemäße Guanidinverbindungen in Bezug auf alle von 5-HT5 verschiedenen 5-HT-Rezeptoren selektiv an 5-HT5-Rezeptoren mit den oben beschriebenen vorteilhaften Bindungsaffinitäten.

Besonders vorteilhaft sind Guanidinverbindungen, die mit den vorstehend beschriebenen Affinitäten und Selektivitäten an 5-HT5-Rezeptoren binden, die von Gliazellen und insbesondere von Astrocyten exprimiert werden. Erfindungsgemäß ist die humane Rezeptorvariante ein bevorzugtes Target für die erfindungsgemäßen Guanidinverbindungen.

Die Bindung erfindungsgemäßer Guanidinverbindungen an 5-HT5-Rezeptoren ist an eine Effektorfunktion gekoppelt. Bindungspartner können agonistisch oder antagonistisch sowie teilagonistisch und/oder teilantagonistisch wirken. Als Agonisten werden erfindungsgemäß Verbindungen bezeichnet, die ganz oder teilweise die Aktivität von 5-HT an 5-HT5-Rezeptoren nachahmen. Als Antagonisten werden erfindungsgemäße Guanidinverbindungen bezeichnet, welche die agonistische Aktivität von 5-HT an 5-HT5-Rezeptoren blockieren können.

Gemäß einer besonderen Ausführungsform der vorliegenden Erfindung werden Guanidinverbindungen eingesetzt, deren Bindung zumindest an 5-HT5-Rezeptoren h5-HT5-transfizierter CHO- oder HEK 293- oder SHSY-5Y-Zellen eine Veränderung der Agonist-induzierten Stimulierung der GTP-Bindung an membrangebundene G-Proteine, eine Veränderung intrazellulärer Calcium-Spiegel, eine Veränderung der Agonist-induzierten Induktion der Phospholipase C-Aktivität und/oder eine Veränderung der cAMP-Produktion bewirkt. Was die Veränderung intrazellulärer Calcium-Spiegel angeht, so stellt die Verwendung von Guanidinverbindungen, die eine Erhöhung intrazellulärer Calcium-Spiegel bewirken, eine besondere Ausführungsform der Erfindung dar. Zu dieser Ausführungsform gehören auch Guanidinverbindungen, die in bekannten Tiermodellen für neurodegenerative und neuropsychiatrische Vorgänge wirksam sind.

Bevorzugt sind Guanidinverbindungen, die auch in Bezug auf ihre Effektorfunktion im oben beschriebenen Sinn selektiv für 5-HT5-Rezeptoren sind.

### Darreichungsformen und Formulierung

Aufgrund ihrer pharmakologischen Eigenschaften sind die erfindungsgemäßen Guanidinverbindungen als Wirkstoffe für therapeutische Zwecke brauchbar. Dabei werden die erfindungsgemäßen Guanidinverbindungen vorzugsweise vor der Verabreichung in eine geeignete Darreichungsform gebracht. Ein weiterer Gegenstand der vorliegenden Erfindung sind daher auch Zusammensetzungen, insbesondere pharmazeutische Zusammensetzungen, die wenigstens eine erfindungsgemäße Guanidinverbindung und sowie einen pharmazeutisch annehmbaren Träger oder Verdünnungsmittel enthalten.

Pharmazeutisch annehmbar sind die im Bereich der Pharmazie und angrenzenden Gebieten bekanntermaßen verwendbaren Träger oder Hilfstoffe, insbesondere die in einschlägigen Arzneibüchern (z.B. DAB (Deutsches Arzneimittelbuch), Ph. Eur. (Pharmacopoeia Europaea), BP (Baccalaureus Pharmaciae), NF (National Formulary), USP (United States Pharmacopoeia) gelisteten, und auch andere Träger, deren Eigenschaften einer physiologischen Anwendung nicht entgegenstehen.

Geeignete Träger und Hilfsstoffe können sein: Netzmittel; emulgierende und suspendierende Mittel; konservierende Mittel; Antioxidantien; Antireizstoffe; Chelatbildner; Dragierhilfsmittel; Emulsionsstabilisatoren; Filmbildner; Gelbildner; Geruchsmaskierungsmittel, Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle. Eine diesbezügliche Ausgestaltung beruht auf fachmännischem Wissen, wie es beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, dargestellt ist.

Beispiele geeigneter Träger und Verdünnungsmittel beinhalten Lactose, Dextrose, Sucrose, Sorbitol, Mannitol, Stärken, Gum Acacia, Calciumphosphat, Alginate, Tragant, Gelatine, Calciumsilicat, microkristalline Cellulose, Polyvinylpyrrolidon, Cellulose, Wassersirup, Methylcellulose, Methyl- and Propylhydroxybenzoate, Talkum, Magnesiumstearat and Mineralöl.

Die erfindungsgemäßen Guanidinverbindungen können formuliert werden, um eine sofortige oder eine verzögerte Freigabe des Wirkstoffes an den Patienten zu gewährleisten.

Beispiele geeigneter pharmazeutischer Zusammensetzungen sind feste Arzneiformen, wie Pulver, Puder, Granulate, Tabletten, insbesondere Filmtabletten, Pastillen, Sachets, Cachets, Dragees, Kapseln wie Hart- und Weichgelatinekapseln, Suppositorien oder vaginale Arzneiformen, halbfeste Arzneiformen, wie Salben, Cremes, Hydrogele, Pasten oder Pflaster, sowie flüssige Arzneiformen, wie Lösungen, Emulsionen, insbesondere Öl-in-Wasser- Emulsionen, Suspensionen, beispielsweise Lotionen, Injektions- und Infusionszubereitungen, Augen- und Ohrentropfen. Auch implantierte Abgabevorrichtungen können zur Verabreichung der erfindungsgemäßen Guanidinverbindungen verwendet werden. Ferner können auch Liposomen oder Mikrosphären zur Anwendung kommen.

Die erfindungsgemäßen Zusammensetzungen können beispielsweise auf üblichem Wege verabreicht werden.

Bei der Herstellung erfindungsgemäßen Zusammensetzungen werden die Wirkstoffe gewöhnlich mit einem geeigneten Hilfsstoff, in diesem Fall auch als Exzipient zu bezeichnen, vermischt oder verdünnt. Exzipienten können feste, halbfeste oder flüssige Materialien sein, die als Vehikel, Träger oder Medium für den Wirkstoff dienen. Die Zumischung weiterer Hilfsstoffe erfolgt erforderlichenfalls in an sich bekannter Weise. Es können Formgebungsschritte, gegebenenfalls in Verbindung mit Mischvorgängen, durchgeführt werden, z.B. eine Granulierung, Komprimierung und ähnliches.

Die erfindungsgemäße Verwendung der erfindungsgemäßen Wirkstoffe beinhaltet im Rahmen der Behandlung ein Verfahren. Dabei wird dem zu behandelnden Individuum, vorzugsweise einem Säuger, insbesondere einem Menschen, und auch einem Nutz-oder Haustier, eine wirksame Menge wenigstens einer Guanidinverbindung der Formel I oder IA, in der Regel der pharmazeutischen Praxis entsprechend formuliert, verabreicht.

Die Erfindung betrifft auch die Herstellung von Mitteln zur Behandlung eines Individuums, vorzugsweise eines Säugers, insbesondere eines Menschen, Nutz- oder Haustieres.

Die Guanidinverbindung der Formel I oder IA oder die entsprechende pharmazeutische Zusammensetzung können oral, rektal, topisch, parenteral, einschließlich subkutan, intravenös und intramuskulär, okular, pulmonar oder nasal verabreicht werden. Bevorzugt ist eine orale Verabreichung.

Eine wirksame Dosierung des Wirkstoffes kann von der Art der Guanidinverbindung, der Verabreichungsart, der zu behandelnden Erkrankung und der Schwere der zu behandelnden Erkrankung abhängig sein. Eine derartige wirksame Dosierung des Wirkstoffes kann von dem Fachmann auf dem Gebiet ohne Schwierigkeiten ermittelt werden.

Die Dosierung hängt vom Alter, Zustand und Gewicht des Patienten sowie von der Applikationsart ab. In der Regel beträgt die tägliche Wirkstoffdosis zwischen etwa 0,5 und 100 mg/kg Körpergewicht bei oraler Gabe und zwischen etwa 0,1 und 10 mg/kg Körpergewicht bei parenteraler Gabe.

### Herstellung der Guanidinverbindungen

Die erfindungsgemäßen Guandinverbindungen lassen sich analog zu literaturbekannten Methoden herstellen, wie sie dem Fachmann bekannt sind. So ist die Synthese von Guanidine allgemein beschrieben in J. Org. Chem. 1997, 9, 1053; Tetrahedron 1999, 55 (10), 713; Tetrahedron Letters 1999, 40, 53; J. Org. Chem. 2000, 65, 8080 und den dort angegebenen Literaturstellen.Die Synthese der erfindungsgemäßen Guanidinverbindungen kann gemäß Schemata 1 oder 2 unter üblichen Reaktionsbedingungen erfolgen, wie sie beispielsweise bei Journal of Medicinal Chemistry 1997, 40, S. 2462-2465, Journal of Medicinal Chemistry 1999, 42, S. 2920-2926, Bioorganic & Medicinal Chemistry Letters 2001, 11, S. 523-528, Journal of Medicinal Chemistry 2000, 43, S.3315-3321, Journal of Organic Chemistry 1991, 56, S. 2139-2143 oder Bioorganic and Medicinal Chemistry 2003, 11, 1319-1341 beschrieben werden.

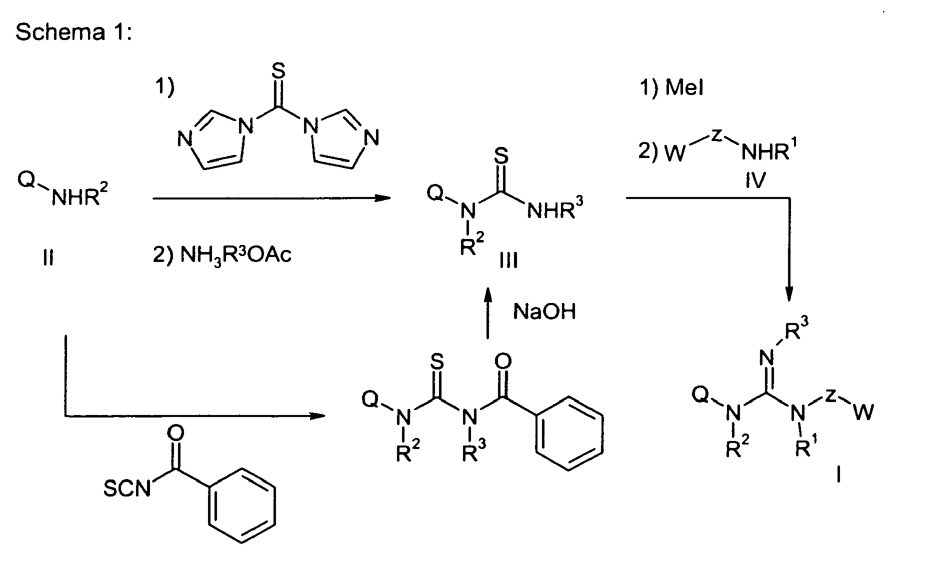

Hetarylamine II sind kommerziell erhältlich oder nach literaturbekannten Methoden (z. B. Houben-Weyl, Methoden der organischen Chemie, Band E8b und E8c, Stuttgart, 1994; M.B. Smith, J. March, March's Advanced Organic Chemistry, New York, 2001) herstellbar. Die nach dem in Schema 1 abgebildeten Syntheseweg verwendeten Amine IV sind ebenfalls kommerziell erhältlich oder beispielsweise nach bekannten Vorschriften (z. B. Houben-Weyl, Methoden der organischen Chemie, 4. Auflage, Band XI/1, Stuttgart, 1957) herstellbar.

Für den Fall, dass es sich bei dem Rest Q um einen substituierten Thiazol-Rest handelt, können die erfindungsgemäßen Guanidinverbindungen der allgemeinen Formel I in einem der letzten Schritte gemäß Schema 2 aufgebaut werden. Dazu setzt man kommerziell erhältliche oder nach der Literatur herstellbare (z. B. Houben-Weyl, Methoden der organischen Chemie, 4. Auflage, Band VII/2c/, Stuttgart, 1977) α-Haloketone V ein.

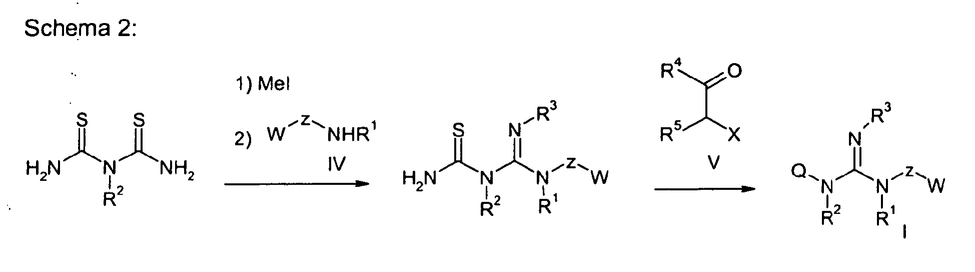

Die Verwendung des Zwischenprodukts IVA zur Herstellung der erfindungsgemäßen Guanidine erfolgt nach dem Fachmann geläufigen Methoden, wie sie bespielsweise in den oben angegebenen Literaturstellen beschrieben sind.

Die erfindungsgemäßen Guanidinverbindungen können genauso wie die gegebenenfalls anfallenden Zwischenprodukte auf herkömmliche Art und Weise gewonnen sowie erforderlichenfalls aufgereinigt werden, beispielsweise durch Umkristallisieren aus üblichen organischen Lösungsmitteln, vorzugsweise einem kurzkettigen Alkohol wie Ethanol, oder mit Hilfe chromatographischer Techniken.

Je nach Einsatzstoffen fallen die erfindungsgemäßen Guanidinverbindungen der Formel in freier Form oder bereits als Säureadditionssalze an. Sowohl die Verbindungen in freier Form als auch verfahrensgemäß resultierende Salze dieser Verbindungen können in an sich bekannter Weise in gewünschte Säureadditionssalze bzw. in die freie Form überführt werden.

Die folgenden Beispiele veranschaulichen die Erfindung ohne sie einzuschränken. Es ist zu beachten, dass Bezeichnung und formelmäßige Darstellung von Salzen mit protoniertem Stickstoff lediglich eine von mehreren allesamt erfassten Möglichkeiten hinsichtlich der Ladungsverteilung wiedergibt. Dies gilt auch für tautomere Formen.

### Herstellungsbeispiele

### Beispiel 1: N-(2-Methoxybenzyl)-N'-1,3-thiazol-2-ylguanidin

### 1.1. N-1,3-Thiazol-2-yl-1H-imidazole-1-carbothioamid

35 g (349.5 mmol) 2-Aminothiazol und 62.3g (349.5 mmol) Thiocarbonyldiimidazol in 1300 ml Acetonitril wurden insgesamt 4 Tage bei Raumtemparatur gerührt. Filtration des gebildeten Niederschlages und Trocknen ergab 65.5g leicht gelbliche Festkörper.

### 1.2. N-1,3-Thiazol-2-yl-thioharnstoff

Eine Mischung aus 65 g (309.1 mmol) N-1,3-Thiazol-2-yl-1H-imidazole-1-carbothioamid und 260 g Ammoniumacetat in 400 ml Ethanol wurde 1.5 Stunden auf 80°C erwärmt, nach beendeter Reaktion das Lösungsmittel abdestilliert und der erhaltene Rückstand mit Wasser versetzt. Nach Extraktion mit CH₂Cl₂ und Trocknen der organischen Phase mit Na₂SO₄ wurden 59.6g des Zielprodukts erhalten.

### 1.3. N-(2-Methoxybenzyl)-N'-1,3-thiazol-2-ylguanidin

400 mg (2.51 mmol) N-1,3-Thiazol-2-yl-thioharnstoff wurden in 20 ml Methanol suspendiert und mit 392 mg (2.76 mmol) Methyliodid versetzt. Man rührte das Reaktionsgemisch 4 Stunden unter Rückfluss. Nach destillativem Entfernen des Lösungsmittels im Vakuum wurde der so erhaltene Rückstand in 20 ml Ethanol aufgenommen, mit 1.72 g (12.6 mmol) 2-Methoxybenzylamin versetzt und 20 Stunden unter Rückfluss gerührt. Das Lösungsmittel wurde am Rotationsverdampfer entfernt. Den Rückstand trennte man mittels präparativer HPLC (Säule RP-18, Fließmittel Wasser/Acetonitril/0.1 % Essigsäure) auf und man isolierte 380 mg *N*-(2-Methoxybenzyl)-*N'*-1,3-thiazol-2-ylguanidin. ESI-MS [M+H⁺] = 263.15

### Beispiel 2: N-(2,6-Dimethoxybenzyl)-N'-(4-phenyl-1,3-thiazol-2-yl)guanidin

### 2.1 N-{[(4-Phenyl-1,3-thiazol-2-yl)amino]carbonothioyl}benzamid

3.10 g 2-Amino-4-phenylthiazol (176 mmol) und 3.00 g Benzoylisothiocyanat wurden in 50 ml Aceton 2 Stunden unter Rückfluss erhitzt, wobei sich ein gelber Feststoff bildete. Die Reaktionsmischung wurde anschließend 30 Minuten bei 5 °C gerührt, der Feststoff abgesaugt und mehrmals mit n-Pentan nachgewaschen. Nach dem Trocknen erhielt man 4.20 g der Zielstruktur als amorphen gelben Feststoff.
ESI-MS [M+H⁺] = 340.05

### 2.2 N-(4-Phenyl-1,3-thiazol-2-yl)thioharnstoff

4.20 g *N*-{[(4-Phenyl-1,3-thiazol-2-yl)amino]carbonothioyl}benzamid (339 mmol) wurden in 40 ml Methanol suspendiert, in wässriger Natronlauge gelöst (550 mg NaOH gelöst in 3 ml H₂O) und 3 Stunden auf Rückfluss erhitzt. Die Reaktionsmischung wurde eingedampft, der erhaltene Rückstand mit Wasser verrührt und der ausgefallene Feststoff abgesaugt. Nach Trocknen wurden 2.90 g eines leicht gelben Feststoffes erhalten.
ESI-MS [M+H⁺] = 236.05

### 2.3 Methyl N'-(4-phenyl-1,3-thiazol-2-yl)imidothiocarbamat Hydroiodid

2.54 g *N*-(4-Phenyl-1,3-thiazol-2-yl)thioharnstoff (235 mmol) in 50ml Methanol wurden mit 2.24 g Methyliodid versetzt und 3 Stunden lang unter Rückfluss gerührt. Anschließend wurde die Reaktionsmischung eingeengt, der erhaltene Rückstand mit n-Pentan verrührt und getrocknet. Man erhielt 3.90 g Produkt als gelben Feststoff, die ohne weitere Aufreinigung weiter umgesetzt wurden.
ESI-MS [M+H⁺] = 250.15

### 2.4 N-(2,6-Dimethoxybenzyl)-N'-(4-phenyl-1,3-thiazol-2-yl)guanidin

3.00 g Methyl-*N*'-(4-phenyl-1,3-thiazol-2-yl)imidothiocarbamat Hydroiodid (377 mmol) und 3.60 g 2,6-Dimethoxybenzylamin (167 mmol) wurden in 30 ml n-Propanol gelöst und 2 Stunden bei 95°C in der Mikrowelle (Einstrahlung: 300Watt) erhitzt. Die Mischung wurde anschließend eingeengt, der Rückstand in CH₂Cl₂ aufgenommen, jeweils mit H₂O, 5 % NaHCO₃- und ges. NaCl-Lösung gewaschen, über MgSO₄ getrocknet, filtriert und eingedampft. Nach Chromatographie an Kieselgel (Eluent: CH₂Cl₂/Methanol 96:4) wurde der so erhaltene Feststoff aus Methanol umkristallisiert, und man erhielt 1.25 g eines weißen amorphen Feststoffes.
ESI-MS [M+H⁺] = 369.15
¹H-NMR (400 MHz, CDCl₃), δ (ppm): 3.90 (s, 6H), 4.45 (d, 2H), 6.58 (d, 2H), 6.78 (s, 1H), 7.2-7.3 (m, 5H), 7.78 (m, 2H), 7.85 (m, 2H).

### Beispiel 3: N-(2,6-Dimethoxybenzyl)-N'-(4-ethyl-1,3-thiazol-2-yl)-guanidin

### 3.1 N-[[(2,6-Dimethoxybenzyl)amino](imino)methyl]thioharnstoff

2.00 g (14.8 mmol) Dithiobiuret wurden in 25 ml Methanol vorgelegt und bei RT 2.10 g (14.8 mmol) Methyliodid zugegeben. Die Mischung wurde 3 Stunden auf Rückfluss erhitzt, dann die Lösung eingeengt, mit 25 ml Ethanol verdünnt und 2.47 g (14.8 mmol) 2,6-Dimethoxybenzylamin zugegeben. Anschließend wurde erneut 2 Stunden unter Rückfluss und anschließend 30 Minuten bei 5°C gerührt. Filtration des entstandenen Niderschlags ergab 970 mg *N*-[[(2,6-dimethoxybenzyl)amino](imino)methyl]thioharnstoff.
ESI-MS [M+H⁺] = 268.3

### 3.2 N-(2,6-Dimethoxy-benzyl)-N'-(4-ethyl-thiazol-2-yl)-guanidin

200 mg (0.75 mmol) *N*-[[(2,6-Dimethoxybenzyl)amino](imino)methyl]thioharnstoff, 130mg (0.77 mmol) 1-Brom-2-butanon und 104 mg (0.80 mmol) Diisopropylethylamin wurden in 10 ml Dioxan suspendiert und 2 Stunden bei 100°C gerührt. Nach Einengen des Reaktionsgemisches wurde mit Dichlormethan verdünnt, mit wässriger Natriumchlorid-Lösung gewaschen und die organische Phase mit Magnesiumsulfat getrocknet. Nach Entfernen des Trocken- und Lösungsmittels wurde ein öliger Rückstand erhalten, den man an Kieselgel mit Dichlormethan/Methanol reinigte. Durch Verrühren mit n-Pentan wurden 100 mg *N*-(2,6-Dimethoxy-benzyl)-*N'*-(4-ethyl-thiazol-2-yl)-guanidin als weißer Feststoff erhalten.
ESI-MS [M+H⁺] = 269.05

### Die Herstellung der Endprodukte der Formel I bzw. IA

Die Verbindungen 4, 5, 6, 7, 8, 9, 10, 11, 12, 13, 14, 15, 16 17, 18, 20, 22, 23, 24, 26, 27, 28, 30, 92 und 93 wurden durch Umsetzung geeigneter Ausgangsmaterialien der Formeln II und IV analog zu Beispiel 1 und 2, die Verbindungen 19, 21, 25, 31-104 durch Umsetzung geeigneter Ausgangsmaterialien der Formeln IV und V analog zu Beispiel 3 hergestellt:

### Beispiel 4:

*N*-(2,5-Dimethylbenzyl)-*N'*-1,3-thiazol-2-ylguanidin Hydrochlorid
ESI-MS [M+H⁺] = 261.25

### Beispiel 5:

*N*-(2,6-Dimethoxybenzyl)-*N'*-1,3-thiazol-2-ylguanidin
ESI-MS [M+H⁺] = 293.25

### Beispiel 6:

*N*-(2-Chloro-6-methoxybenzyl)-*N*'-1,3-thiazol-2-ylguanidin Hydrochlorid
ESI-MS [M+H⁺] = 297.05

### Beispiel 7:

*N*-(2-Chlorobenzyl)-*N'*-1,3-thiazol-2-ylguanidin Hydrochlorid
ESI-MS [M+H⁺] = 267.05

### Beispiel 8:

*N*-(2-Ethoxybenzyl)-*N'*-1,3-thiazol-2-ylguanidin Hydrochlorid
ESI-MS [M+H⁺] = 277.05

### Beispiel 9:

*N*-(2-Fluoro-6-methoxybenzyl)-*N'*-1,3-thiazol-2-ylguanidin
ESI-MS [M+H⁺] = 281.05

### Beispiel 10:

*N*-(2-Hydroxybenzyl)-*N'*-1,3-thiazol-2-ylguanidin Acetat
ESI-MS [M+H⁺] = 249.1

### Beispiel 11:

*N*-(2-Methoxybenzyl)-*N'*-1,3-thiazol-2-ylguanidin Hydrochlorid
ESI-MS [M+H⁺] = 277.05

### Beispiel 12:

*N*-(2-Methylbenzyl)-*N'*-1,3-thiazol-2-ylguanidin
ESI-MS [M+H⁺] = 247.05

### Beispiel 13

*N*-(3-Chlorobenzyl)-*N'*-1,3-thiazol-2-ylguanidin Hydrochlorid
ESI-MS [M+H⁺] = 267.0

### Beispiel 14:

*N*-(3-Methoxybenzyl)-*N'*-1,3-thiazol-2-ylguanidin Hydrochlorid
ESI-MS [M+H⁺] = 263.1

### Beispiel 15:

*N*-(4-Methoxybenzyl)-*N'*-1,3-thiazol-2-ylguanidin
ESI-MS [M+H⁺] = 263.05

### Beispiel 16:

*N*-[2-(2-Methoxyphenyl)ethyl]-*N'*-1,3-thiazol-2-ylguanidin Hydrochlorid
ESI-MS [M+H⁺] = 277.1

### Beispiel 17:

*N*-[2-(Benzyloxy)benzyl]-*N'*-1,3-thiazol-2-ylguanidin
ESI-MS [M+H⁺] = 339.05

### Beispiel 18:

*N*-1,3-Thiazol-2-yl-*N'*-[2-(trifluoromethyl)benzyl]guanidin
ESI-MS [M+H⁺] = 301.0

### Beispiel 19:

*N*-{4-[3,5-Bis(trifluoromethyl)phenyl]-1,3-thiazol-2-yl}-N'-(2,6-dimethoxybenzyl)guanidin Hydrobromid
ESI-MS [M+H⁺] = 505.15

### Beispiel 20:

*N*-[Imino(1,3-thiazol-2-ylamino)methyl]-2-methoxybenzamid
ESI-MS [M+H⁺] = 277.05

### Beispiel 21:

*N*-[4-(2,5-Dichlorophenyl)-1,3-thiazol-2-yl]-*N'*-(2,6-dimethoxybenzyl)guanidin Hydrobromid
ESI-MS [M+H⁺] = 437.15/439.15

### Beispiel 22:

*N*-[3-(3-{[Imino(1,3-thiazol-2-ylamino)methyl]amino}propoxy)phenyl]acetamid
ESI-MS [M+H⁺] = 334.1

### Beispiel 23:

*N*-[3-(3-Acetylphenoxy)propyl]-*N'*-1,3-thiazol-2-ylguanidin (2E)-But-2-endioat
ESI-MS [M+H⁺] = 319.1

### Beispiel 24:

*N*-(2-Methoxybenzyl)-*N'*-methyl-N"-1,3-thiazol-2-ylguanidin (2E)-But-2-endioat
ESI-MS [M+H⁺] = 277.0

### Beispiel 25:

*N*-[4-(2,3-Dihydro-1,4-benzodioxin-6-yl)-1,3-thiazol-2-yl]-*N'*-(2,6-dimethoxybenzyl)guanidin Hydrobromid
ESI-MS [M+H⁺] = 427.15

### Beispiel 26:

*N*-Ethyl-*N'*-(2-methoxybenzyl)-*N"*-1,3-thiazol-2-ylguanidin (2E)-But-2-endioat
ESI-MS [M+H⁺] = 291.0

### Beispiel 27:

*N*-1,3-Benzothiazol-2-yl-*N'*-(2,6-dimethoxybenzyl)guanidin (2E)-But-2-endioat
ESI-MS [M+H⁺] = 343.1

### Beispiel 28:

*N*-1,3-Benzothiazol-2-yl-*N'*-(2-methoxybenzyl)guanidin (2E)-But-2-endioat
ESI-MS [M+H⁺] = 313.0

### Beispiel 29:

*N*-[2-(2-Chlorophenoxy)ethyl]-*N*'-1,3-thiazol-2-ylguanidin (2E)-But-2-endioat
ESI-MS [M+H⁺] = 297.0

### Beispiel 30:

*N*-(2-Methoxy-benzyl)-*N*'-thiophen-3-yl-guanidine Acetat
ESI-MS [M+H⁺] = 262.25

### Beispiel 31:

*N*-(2-Methoxybenzyl)-*N*'-(4-phenyl-1,3-thiazol-2-yl)guanidin
ESI-MS [M+H⁺] = 339.05

### Beispiel 32:

*N*-(2,6-Dimethoxybenzyl)-*N*'-(4-methyl-1,3-thiazol-2-yl)guanidin
ESI-MS [M+H⁺] = 307.25

### Beispiel 33:

*N*-(2-Methoxybenzyl)-*N*'-[4-(2-naphthyl)-1,3-thiazol-2-yl]guanidin
ESI-MS [M+H⁺] = 389.05

### Beispiel 34:

*N*-(2,6-Dimethoxybenzyl)-*N*'-[4-(2-naphthyl)-1,3-thiazol-2-yl]guanidin
ESI-MS [M+H⁺] = 419.15

### Beispiel 35:

*N*-[4-(4-Chlorophenyl)-1,3-thiazol-2-yl]-*N*'-(2-methoxybenzyl)guanidin
ESI-MS [M+H⁺] = 373.05

### Beispiel 36:

*N*-(4-Tert-butyl-1,3-thiazol-2-yl)-*N*'-(2,6-dimethoxybenzyl)guanidin
ESI-MS [M+H⁺] = 349.15

### Beispiel 37:

*N*-(4-Tert-butyl-1,3-thiazol-2-yl)-*N*'-(2-methoxybenzyl)guanidin (2E)-But-2-endioat
ESI-MS [M+H⁺] = 319.15

### Beispiel 38:

*N*-[4-(4-Chlorophenyl)-1,3-thiazol-2-yl]-*N*'-(2,6-dimethoxybenzyl)guanidin
ESI-MS [M+H⁺] = 403.25

### Beispiel 39:

*N*-(2-Methoxybenzyl)-*N*'-(4-methyl-1,3-thiazol-2-yl)guanidin (2E)-But-2-endioat
ESI-MS [M+H⁺] = 277.05

### Beispiel 40:

*N*-(4,5-Dimethyl-1,3-thiazol-2-yl)-*N*'-(2-methoxybenzyl)guanidin
ESI-MS [M+H⁺] = 291.15

### Beispiel 41:

*N*-(2-Methoxybenzyl)-*N*'-(4-pyridin-2-yl-1,3-thiazol-2-yl)guanidin
ESI-MS [M+H⁺] = 340.15

### Beispiel 42:

*N*-(2,6-Dimethoxybenzyl)-*N*'-(4-pyridin-2-yl-1,3-thiazol-2-yl)guanidin (2E)-But-2-endioat
ESI-MS [M+H⁺] = 370.15

### Beispiel 43:

*N*-[4-(2-Chlorophenyl)-1,3-thiazol-2-yl]-*N*'-(2-methoxybenzyl)guanidin (2E)-But-2-endioat
ESI-MS [M+H⁺] = 373.05

### Beispiel 44:

*N*-[4-(2-Chlorophenyl)-1,3-thiazol-2-yl]-*N*'-(2,6-dimethoxybenzyl)guanidin
ESI-MS [M+H⁺] = 403.05

### Beispiel 45:

*N*-(2,6-Dimethoxybenzyl)-*N*'-(4-pyridin-4-yl-1,3-thiazol-2-yl)guanidin
ESI-MS [M+H⁺] = 370.0

### Beispiel 46:

*N*-(2-Methoxybenzyl)-*N*'-(4-pyridin-4-yl-1,3-thiazol-2-yl)guanidin
¹H-NMR (400 MHz, CDCl₃), δ (ppm): 3.85 (s, 3H), 4.40 (d, 2H), 6.95 (m, 1H), 7.15
(m, 1H), 7.25-7.80 (m, 6H), 8.55 (m, 2H).

### Beispiel 47:

Methyl- [2-({imino[(2-methoxybenzyl)amino]methyl}amino)-1,3-thiazol-4-yl]acetat
ESI-MS [M+H⁺] = 440.25

### Beispiel 48:

*N*-(2-Methoxybenzyl)-*N*'-(4-pyridin-3-yl-1,3-thiazol-2-yl)guanidin
ESI-MS [M+H⁺] = 340.15

### Beispiel 49:

Methyl-(2-{[[(2,6-dimethoxybenzyl)amino](imino)methyl]amino}-1,3-thiazol-4-yl)acetat
ESI-MS [M+H⁺] = 365.15

### Beispiel 50:

*N*-(2,6-Dimethoxybenzyl)-*N*'-(4-pyridin-3-yl-1,3-thiazol-2-yl)guanidin Acetat
ESI-MS [M+H⁺] = 370.25

### Beispiel 51:

2-(2-{[[(2,6-Dimethoxybenzyl)amino](imino)methyl]amino}-1,3-thiazol-4-yl)-*N*-(2-methoxybenzyl)acetamide (2E)-But-2-endioat
ESI-MS [M+H⁺] = 440.25

### Beispiel 52:

*N*-(2-Methoxybenzyl)-*N*'-[4-(trifluoromethyl)-1,3-thiazol-2-yl]guanidin (2E)-But-2-endioat
ESI-MS [M+H⁺] = 331.05

### Beispiel 53:

*N*-(2,6-Dimethoxybenzyl)-*N*'-[4-(trifluoromethyl)-1,3-thiazol-2-yl]guanidin
ESI-MS [M+H⁺] = 361.05

### Beispiel 54:

*N*-(2-Methoxybenzyl)-*N*'-(5-methyl-1,3-thiazol-2-yl)guanidin (2E)-But-2-endioat
ESI-MS [M+H⁺] = 277.25

### Beispiel 55:

*N*-(2,6-Dimethoxybenzyl)-*N*'-(5-methyl-1,3-thiazol-2-yl)guanidin
ESI-MS [M+H⁺] = 307.25

### Beispiel 56:

*N*-(2,6-Dimethoxybenzyl)-*N*'-[4-(4-fluorophenyl)-1,3-thiazol-2-yl]guanidin
ESI-MS [M+H⁺] = 387.15

### Beispiel 57:

*N*-(2,6-Dimethoxybenzyl)-*N*'-[4-(4-methylphenyl)-1,3-thiazol-2-yl]guanidin
ESI-MS [M+H⁺] = 383.15

### Beispiel 58:

*N*-(2,6-Dimethoxybenzyl)-*N*'-[4-(4-methoxyphenyl)-1,3-thiazol-2-yl]guanidin
ESI-MS [M+H⁺] = 399.15

### Beispiel 59:

*N*-(2-Fluoro-6-methoxybenzyl)-*N*'-(4-phenyl-1,3-thiazol-2-yl)guanidin
ESI-MS [M+H⁺] = 357.05

### Beispiel 60:

*N*-[4-(4-Cyanophenyl)-1,3-thiazol-2-yl]-*N*'-(2,6-dimethoxybenzyl)guanidin
ESI-MS [M+H⁺] = 394.05

### Beispiel 61:

*N*-(2,6-Dimethoxybenzyl)-*N*'-[4-(3-methoxyphenyl)-1,3-thiazol-2-yl]guanidin
ESI-MS [M+H⁺] = 399.15

### Beispiel 62:

*N*-{4-[4-(Diethylamino)phenyl]-1,3-thiazol-2-yl}-*N*'-(2,6-dimethoxybenzyl)guanidin
ESI-MS [M+H⁺] = 440.2

### Beispiel 63:

*N*-(2,6-Dimethoxybenzyl)-*N*'-[4-(4-pyrrolidin-1-ylphenyl)-1,3-thiazol-2-yl]guanidin Hydrobromid
ESI-MS [M+H⁺] = 438.3

### Beispiel 64:

*N*-(2,6-Dimethoxybenzyl)-*N*'-{4-[4-(trifluoromethoxy)phenyl]-1,3-thiazol-2-yl}guanidin Hydrobromid
ESI-MS [M+H⁺] = 453.05

### Beispiel 65:

*N*-(2,6-Dimethoxybenzyl)-*N*'-[4-(4-morpholin-4-ylphenyl)-1,3-thiazol-2-yl]guanidin Hydrobromid
ESI-MS [M+H⁺] = 454.15

### Beispiel 66:

*N*-(2,6-Dimethoxybenzyl)-*N*'-(5-phenyl-1,3-thiazol-2-yl)guanidin Hydrobromid
ESI-MS [M+H⁺] = 369.15

### Beispiel 67:

*N*-[4-(1-Benzofuran-2-yl)-1,3-thiazol-2-yl]-*N*'-(2,6-dimethoxybenzyl)guanidin Hydrobromid
ESI-MS [M+H⁺] = 409.05

### Beispiel 68:

*N*-[4-(3,5-Difluorophenyl)-1,3-thiazol-2-yl]-*N*'-(2,6-dimethoxybenzyl)guanidin Hydrobromid
¹H-NMR (400 MHz, CDCl₃), δ (ppm) = 3.83 (s, 6H), 4.53 (d, 2H), 6.78 (d, 2H), 7.24 (m, 1H), 7.38-7.45 (m, 3H), 7.99 (s, 1H), 8.34 (s breit, 2H), 9.50 (s breit, 1H), 11.90 (s breit, 1H).

### Beispiel 69:

*N*-[4-(1,3-Benzodioxol-5-yl)-1,3-thiazol-2-yl]-*N*'-(2,6-dimethoxybenzyl)guanidin hydrobromid
ESI-MS [M+H⁺] = 413.05

### Beispiel 70:

*N*-(2,6-Dimethoxybenzyl)-*N*'-[4-(2-fluorophenyl)-1,3-thiazol-2-yl]guanidin Hydrobromid
ESI-MS [M+H⁺] = 387.15

### Beispiel 71:

*N*-[4-(2-{[[(2,6-Dimethoxybenzyl)amino](imino)methyl]amino}-1,3-thiazol-4-yl)phenyl]methanesulfonamid Hydrobromid
ESI-MS [M+H⁺] = 462.15

### Beispiel 72:

*N*-(2,6-Dimethoxybenzyl)-*N*'-[4-(3-thienyl)-1,3-thiazol-2-yl]guanidin Acetat
ESI-MS [M+H⁺] = 375.05

### Beispiel 73:

*N*-(2,6-Dimethoxybenzyl)-*N*'-[4-(3-fluorophenyl)-1,3-thiazol-2-yl]guanidin Hydrobromid
ESI-MS [M+H⁺] = 387.15

### Beispiel 74:

*N*-(4-Biphenyl-4-yl-1,3-thiazol-2-yl)-*N*'-(2,6-dimethoxybenzyl)guanidin Hydrobromid
ESI-MS [M+H⁺] = 445.15

### Beispiel 75:

*N*-(2,6-Dimethoxybenzyl)-*N*'-[4-(2-methoxyphenyl)-1,3-thiazol-2-yl]guanidin Hydrobromid
ESI-MS [M+H⁺] = 399.15

### Beispiel 76:

*N*-(2,6-Dimethoxybenzyl)-*N*'-[4-(diphenylmethyl)-1,3-thiazol-2-yl]guanidin Hydrobromid
ESI-MS [M+H⁺] = 459.25

### Beispiel 77:

*N*-[4-(5-Chloro-2-thienyl)-1,3-thiazol-2-yl]-*N*'-(2,6-dimethoxybenzyl)guanidin Hydrobromid
ESI-MS [M+H⁺] = 409.05

### Beispiel 78:

*N*-[4-(1-Benzothien-2-yl)-1,3-thiazol-2-yl]-*N*'-(2,6-dimethoxybenzyl)guanidin Hydrobromid
ESI-MS [M+H⁺] = 425.05

### Beispiel 79:

*N*-[4-(2-{[[(2,6-Dimethoxybenzyl)amino](imino)methyl]amino}-1,3-thiazol-4-yl)phenyl]acetamid Acetat
ESI-MS [M+H⁺] = 462.15

### Beispiel 80:

*N*-(2,6-Dimethoxybenzyl)-*N*'-8H-indeno[1,2-d][1,3]thiazol-2-ylguanidin Hydrobromid
ESI-MS [M+H⁺] = 381.15

### Beispiel 81:

*N*-(2,6-Dimethoxybenzyl)-*N*'-(5-methyl-4-phenyl-1,3-thiazol-2-yl)guanidin Hydrobromid
ESI-MS [M+H⁺] = 375.05

### Beispiel 82:

*N*-(2,6-Dimethoxybenzyl)-*N*'-{4-[4-(methylsulfonyl)phenyl]-1,3-thiazol-2-yl}guanidin Hydrobromid
ESI-MS [M+H⁺] = 447.05

### Beispiel 83:

*N*-(2,6-Dimethoxybenzyl)-*N*'-[4-(3-phenylisoxazol-5-yl)-1,3-thiazol-2-yl]guanidin Hydrobromid
ESI-MS [M+H⁺] = 436.15

### Beispiel 84:

*N*-(2,6-Dimethoxybenzyl)-*N*'-{4-[2-(trifluoromethyl)phenyl]-1,3-thiazol-2-yl}guanidin Hydrobromid
ESI-MS [M+H⁺] = 437.15

### Beispiel 85:

*N*-[4-(1-Adamantyl)-1,3-thiazol-2-yl]-*N*'-(2,6-dimethoxybenzyl)guanidin Hydrobromid
ESI-MS [M+H⁺] = 427.15

### Beispiel 86:

*N*-(2-Fluoro-6-methoxybenzyl)-*N*'-(4-methyl-1,3-thiazol-2-yl)guanidin
ESI-MS [M+H⁺] = 295.05

### Beispiel 87:

*N*-[4-(3,4-Difluorophenyl)-1,3-thiazol-2-yl]-*N*'-(2,6-dimethoxybenzyl)guanidin Hydrobromid
ESI-MS [M+H⁺] = 405.15

### Beispiel 88:

*N*-[4-(1,3-Benzothiazol-2-yl)-1,3-thiazol-2-yl]-*N*'-(2,6-dimethoxybenzyl)guanidin Hydrobromid
ESI-MS [M+H⁺] = 426.05

### Beispiel 89:

*N*-[4-(3-Chlorophenyl)-1,3-thiazol-2-yl]-*N*'-(2,6-dimethoxybenzyl)guanidin Hydrobromid
ESI-MS [M+H⁺] = 403.05

### Beispiel 90:

*N*-(2,6-Dimethoxybenzyl)-*N*'-[4-(2-thienyl)-1,3-thiazol-2-yl]guanidin Hydrobromid
ESI-MS [M+H⁺] = 375.05

### Beispiel 91:

*N*-[4-(2,4-Difluorophenyl)-1,3-thiazol-2-yl]-*N*'-(2,6-dimethoxybenzyl)guanidin Hydrobromid
ESI-MS [M+H⁺] = 405.15

### Beispiel 92:

*N*-(2-Methoxybenzyl)-*N*'-(1-methyl-1H-benzimidazol-2-yl)guanidin
ESI-MS [M+H⁺] = 310.15

### Beispiel 93:

*N*-1H-Benzimidazol-2-yl-*N*'-(2-methoxybenzyl)guanidin acetat
ESI-MS [M+H⁺] = 296.15

### Beispiel 94:

*N*-(2,6-Dimethoxybenzyl)-*N*'-(4,5,6,7-tetrahydro-1,3-benzothiazol-2-yl)guanidin Acetat
ESI-MS [M+H⁺] = 347.15

### Beispiel 95:

*N*-(2,6-Dimethoxybenzyl)-*N*'-[4-(4-isopropylphenyl)-1,3-thiazol-2-yl]guanidin
ESI-MS [M+H⁺] = 411.15

### Beispiel 96:

*N*-[4-(1-Benzothien-3-yl)-1,3-thiazol-2-yl]-*N*'-(2,6-dimethoxybenzyl)guanidin Hydrobromid
ESI-MS [M+H⁺] = 425.05

### Beispiel 97:

*N*-(4-Cyclohexyl-1,3-thiazol-2-yl)-*N*'-(2,6-dimethoxybenzyl)guanidin
ESI-MS [M+H⁺] = 375.15/376.15

### Beispiel 98:

*N*-[4-(2-Fluorophenyl)-1,3-thiazol-2-yl]-*N*'-(2-methoxybenzyl)guanidin
ESI-MS [M+H+] = 357.05

### Beispiel 99:

N-[4-(3-Fluorophenyl)-1,3-thiazol-2-yl]-N'-(2-methoxybenzyl)guanidin
ESI-MS [M+H+] = 357.05

### Beispiel 100:

N-(2-Methoxybenzyl)-N'-{4-[4-(trifluoromethoxy)phenyl]-1,3-thiazol-2-yl}guanidin
ESI-MS [M+H+] = 423.05

### Beispiel 101:

N-[4-(1,3-Benzodioxol-5-yl)-1,3-thiazol-2-yl]-N'-(2-methoxybenzyl)guanidin
ESI-MS [M+H+] = 383.05

### Beispiel 102:

2-{[[(2,6-Dimethoxybenzyl)amino](imino)methyl]amino}-N,N,4-trimethyl-1,3-thiazole-5-carboxamid
ESI-MS [M+H+] = 378.15

### Beispiel 103:

N-(2-Methoxybenzyl)-N'-{4-[2-(trifluoromethyl)phenyl]-1,3-thiazol-2-yl}guanidin
ESI-MS [M+H+] = 407.05

### Beispiel 104:

N-{4-[2-({Imino[(2-methoxybenzyl)amino]methyl}amino)-1,3-thiazol-4-yl]phenyl}methanesulfonamid
ESI-MS [M+H+] = 432.05

### Beispiel 105:

*N*'-(2,6-Dimethoxybenzyl)-*N*-(3-phenyl-1,2,4-thiadiazol-5-yl)guanidin
x 0,5 (2E)-But-2-endioat; ESI-MS[M+H⁺] = 370.1

*N*'-(2,6-Dimethoxybenzyl)-*N*-(3-phenyl-1,2,4-thiadiazol-5-yl)guanidin wurde analog Beispiel 1 hergestellt mit folgenden Variationen: Die Alkylierung von N-(3-Phenyl-1,2,4-thiadiazol-5-yl)thioharnstoff mit Methyliodid wurde in Gegenwart von 1,5 Äquivalenten Triethylamin durchgeführt und die Umsetzung von Methyl N'-(3-phenyl-1,2,4-thiadiazol-5-yl)imidothiocarbamat mit 2,6-Dimethoxybenzylamin erfolgte bei 140°C in der Mikrowelle (120 Watt). Nach chromatographischer Reinigung wurde das Produkt ins (2E)-But-2-endioat überführt.
¹H-NMR (500 MHz, d⁶-DMSO), δ (ppm): 3,83 (s, 6H), 4,42 (d, 2H), 6,63 (s, 1H), 6,74 (d, 2H), 7,24 (sbr, 2H), 7,34 (t, 1H), 7,44 (m, 3H), 7,96 (sbr, 2H). ¹³C-NMR (100,6 MHz, d⁶-DMSO), δ (ppm): 33,80 (t), 55,92 (q), 104.15 (2x d), 112,67 (s), 127,13, (d), 128,53 (2x d), 129,73 (d), 133,14 (s), 134,00 (2x d), 156,13 (s), 158,07 (s), 166, 02 (2x s).

### Beispiel 106:

*N*'-(2-Methoxybenzyl)-*N*-(3-phenyl-1,2,4-thiadiazol-5-yl)guanidin (2E)-But-2-enedioat; ESI-MS[M+H⁺] = 340.1

### Die Synthese erfolgte analog Beispiel 105

### Beispiel 107:

'N-(2,6-Dimethoxybenzyl)-N'-{4-[4-(trifluoromethyl)phenyl]-1,3-thiazol-2-yl}guanidin
Die Herstellung erfolgte analog zu Beispiel 3 durch Umsetzung von 140 mg (0.52 mmol) *N*-[[(2,6-Dimethoxybenzyl)amino](imino)methyl]thioharnstoff mit 140mg (0.52 mmol) 4-(Trifluormethyl)phenacylbromid. Die Edukte wurden in 3ml Dioxan suspendiert, 0.3ml Essigsäure zugesetzt und 40 Minuten lang ind der Mikrowelle erhitzt (Einstrahlung 300W). Anschließend wurde die Mischung eingeengt und das erhaltene Rohprodukt über Chromatographie an Kieselgel (Dichlormethan/Methanol 1-4%) gereinigt. Verrühren des erhaltenen gelblichen Schaum mit Methyl-tert.butylether ergab 120mg eines weißen amorphen Feststoffs
ESI-MS [M+H⁺] = 437.05.

### Analog zu Beispiel 107 wurden hergestellt:

### Beispiel 108:

'N-(2,6-Dimethoxybenzyl)-N'-{4-[3-(trifluoromethyl)phenyl]-1,3-thiazol-2-yl}guanidin
ESI-MS [M+H⁺] = 437.05

### Beispiel 109:

N-{4-[4-(Difluoromethoxy)phenyl]-1,3-thiazol-2-yl}-N'-(2,6-dimethoxybenzyl)guanidin Hydrobromid
ESI-MS [M+H⁺] = 435.05

### Beispiel 110:

N-(2,6-Dimethoxybenzyl)-N'-{4-[4-(dimethylamino)phenyl]-1,3-thiazol-2-yl}guanidin Hydrobromid
ESI-MS [M+H+] = 412.15

### Beispiel 111:

N-(2,6-Dimethoxybenzyl)-N'-[4-(4-fluoro-2-methoxyphenyl)-1,3-thiazol-2-yl]guanidin
ESI-MS [M+H+] = 417.15

### Beispiel 112:

N-(2,6-Dimethoxybenzyl)-N'-[4-(5-fluoro-2-methoxyphenyl)-1,3-thiazol-2-yl]guanidin Hydrobromid
ESI-MS [M+H+] = 417.15

### Beispiel 113:

N-(2,6-Dimethoxybenzyl)-N'-[4-(2,6-dimethoxyphenyl)-1,3-thiazol-2-yl]guanidin
ESI-MS [M+H+] = 429.15

### Beispiel 114:

N-(2,6-Dimethoxybenzyl)-N'-[4-(4-fluoro-1-naphthyl)-1,3-thiazol-2-yl]guanidin
ESI-MS [M+H+] = 437.25

### Beispiel 115:

N-[4-(2,3-Dihydro-1-benzofuran-5-yl)-1,3-thiazol-2-yl]-N'-(2,6-dimethoxybenzyl)guanidin ESI-MS [M+H+] = 411.15

### Beispiel 116:

N-[4-(2-Chloro-4-fluorophenyl)-1,3-thiazol-2-yl]-N'-(2,6-dimethoxybenzyl)guanidin
ESI-MS [M+H+] = 421.05

### Beispiel 117:

N-(2,6-Dimethoxybenzyl)-N'-[4-(2-furyl)-1,3-thiazol-2-yl]guanidin Hydrobromid
ESI-MS [M+H+] = 359.15

### Beispiel 118:

N-(2,6-Dimethoxybenzyl)-N'-[4-(4-fluorophenyl)-5-methyl-1,3-thiazol-2-yl]guanidin
ESI-MS [M+H+] = 401.15

### Beispiel 119:

N-[4-(2,6-Dichlorophenyl)-1,3-thiazol-2-yl]-N'-(2,6-dimethoxybenzyl)guanidin
ESI-MS [M+H+] = 439.05

### Beispiel 120:

tert-Butyl 2-(2-{[[(2,6-dimethoxybenzyl)amino](imino)methyl]amino}-1,3-thiazol-4-yl)pyrrolidin-1-carboxylat
ESI-MS [M+H+] = 462.25

### Beispiel 121:

N-(2,6-Dimethoxybenzyl)-N'-[4-(4-methyl-2-thienyl)-1,3-thiazol-2-yl]guanidin
ESI-MS [M+H+] = 289.05

### Beispiel 122:

N-(2,6-Dimethoxybenzyl)-N'-(4-pyrimidin-2-yl-1,3-thiazol-2-yl)guanidin Hydrobromid ESI-MS [M+H+] = 370.85

### Beispiel 123:

N-[4-(5-Chloropyridin-2-yl)-1,3-thiazol-2-yl]-N'-(2,6-dimethoxybenzyl)guanidin Acetat ESI-MS [M+H+] = 404.25

### Beispiel 124:

N-[4-(4-Chloro-2-thienyl)-1,3-thiazol-2-yl]-N'-(2,6-dimethoxybenzyl)guanidin
ESI-MS [M+H+] = 409.05

### Beispiel 125:

tert-Butyl [(2-{[[(2,6-dimethoxybenzyl)amino](imino)methyl]amino}-1,3-thiazol-4-yl)methyl]carbamat
ESI-MS [M+H+] = 422.15

### Beispiel 126:

N-[4-(3,5-Dichloropyridin-2-yl)-1,3-thiazol-2-yl]-N'-(2,6-dimethoxybenzyl)guanidin
ESI-MS [M+H+] = 440.05

### Beispiel 127:

N-(4,5-Dihydronaphtho[1,2-d][1,3]thiazol-2-yl)-N'-(2,6-dimethoxybenzyl)guanidin
ESI-MS [M+H+] = 395.15

### Beispiel 128:

N-(2,6-Dimethoxybenzyl)-N'-[5-(4-fluorophenyl)-1,3-thiazol-2-yl]guanidin
ESI-MS [M+H+] =387.15

### Analog zu Beispiel 2 wurden hergestellt:

### Beispiel 129:

N-(5-Fluoro-2-methoxybenzyl)-N'-(4-phenyl-1,3-thiazol-2-yl)guanidin
ESI-MS [M+H+] = 357.05

### Beispiel 130:

N-(5-Fluoro-2-methoxybenzyl)-N'-(4-methyl-1,3-thiazol-2-yl)guanidin Fumarat
ESI-MS [M+H+] = 295.05

### Beispiel 131:

N-(2,6-Dimethoxybenzyl)-N'-(4-isopropyl-1,3-thiazol-2-yl)guanidin
ESI-MS [M+H+] = 335.15

### Beispiel 132:

### N-(2-Chloro-6-methoxybenzyl)-N'-(4-phenyl-1,3-thiazol-2-yl)guanidin

### 132.1 2-Chloro-6-methoxy-benzylbromid

Zu 13.0g (84.92mmol) 3-Chlor2-methylanisol in 80ml CCl₄ wurden bei Rückfluß erst 0.4g Dibenzoylperoxid, anschließend portionsweise mit eine Mischung aus 15.2g N-Bromsuccinimid und 0.4g Dibenzoylperoxid gegeben. Nach beendeter Umsetzung wurde die Mischung eingedampft, der Rückstand in Dichlormethan aufgenommen, nacheinander mit Wasser und ges. NaCl-Lösung gewaschen, über MgSO₄ getrocknet, filtriert und erneut eingedampft. 20.8 gelbes Öl.

### 132.2 2-Chloro-6-methoxy-benzylamin Hydrochlorid

Zu einer Suspension von 24g NaH (60% Dispersion in Mineralöl; entölt mit n-Pentan) in 20ml DMF wurden bei 5°C 13g Di-tert.butyliminodicarboxylat, gelöst in 40ml DMF, zugetropft. Nach 1 h wurden 15g 2-Chloro-6-methoxy-benzylbromid (Rohprodukt 131.1), gelöst in 40ml DMF, zugegeben, die Mischung mit weiteren 100ml DMF versetzt und über Nacht bei Raumtemperatur gerührt. Zur Aufarbeitung wurde überschüssiges NaH durch Zugabe von 20ml DMF-Wasser 1:1 zerstört, anschließend zur Trockene eingedampft, der erhaltene Rückstand in Dichlormethan aufgenommen, nacheinander mit 0.1 n HCl- und gesättigter NaCl-Lösung gewaschen, über MgSO₄ getrocknet, filtriert und erneut eingedampft. Verrühren des so erhaltenen öligen Rückstands mit Cyclohexan ergab 11.9g beige Festkörper, die direkt in ohne weitere Aufreinigung weiter umgesetzt wurden.
Zur Boc-Abspaltung wurden 11.6g Festkörper in 60ml Dichlormethan mit 60ml 4n HCL in Dioxan versetzt und 2h auf 70°C erhitzt. Eindampfen der Reaktionsmischung und Behandlung des erhaltenen Öls mit n-Pentan ergab 6.0g 2-Chloro-6-methoxy-benzylamin Hydrochlorid als amorphe Festkörper, ESI-MS [M+H+] = 172.05. Für die weiteren Umsetzungen wurde das Hydrochlorid in die frei Base überführt.

### 132.3 N-(2-Chloro-6-methoxybenzyl)-N'-(4-phenyl-1,3-thiazol-2-yl)guanidin

Umsetzung von 0.35g Methyl-N'-(4-phenyl-1,3-thiazol-2-yl)imidothiocarbamat Hydroiodid (0.93 mmol) und 0.5 g 2-Chloro-6-methoxy-benzylamin (2.91 mmol) analog zu Beispiel 2, 2.4, ergab 220mg N-(2-Chloro-6-methoxybenzyl)-N'-(4-phenyl-1,3-thiazol-2-yl)guanidin; ESI-MS [M+H+] = 373.05

### Analog zu Beispiel 132 wurden hergestellt:

### Beispiel 133:

N-(2-Chloro-6-methoxybenzyl)-N'-(4-pyridin-2-yl-1,3-thiazol-2-yl)guanidin
ESI-MS [M+H+] = 374.05

### Beispiel 134:

N-(2-Chloro-6-methoxybenzyl)-N'-[4-(5-chloropyridin-2-yl)-1,3-thiazol-2-yl]guanidin
ESI-MS [M+H+] = 410.05

### Beispiel 135:

N-(2-Chloro-6-methoxybenzyl)-N'-(4-pyrimidin-2-yl-1,3-thiazol-2-yl)guanidin
ESI-MS [M+H+] = 375.05

### Beispiel 136:

N-(2-Chloro-6-methoxybenzyl)-N'-[4-(4-fluorophenyl)-1,3-thiazol-2-yl]guanidin
ESI-MS [M+H+] = 391.05

### Beispiel 137:

### N-[4-(5-Chloropyridin-2-yl)-1,3-thiazol-2-yl]-N'-(2-methoxy-6-methylbenzyl)guanidin

### 137.1 2-Methoxy-6-methylbenzylamin Hydrochlorid

Die Herstellung erfolgte analog zu Beispiel 2; 2.4, ausgehend von 7.1 g (41.61 mmol) 2-Methoxy-6-methylbenzylchlorid. Boc-Spaltung ergab 1.2g 2-Methoxy-6-methylbenzylamin Hydrochlorid als weißen Feststoff; Für die weiteren Umsetzungen wurde das Hydrochlorid in die frei Base überführt.
¹H-NMR (400 MHz, DMSO-d6), δ (ppm) = 2.38 (s, 3H), 3.29 (s, 3H), 3.98 (s, 2H), 6.86 (d, 1H), 6.92 (d, 1H), 7.29 (t, 1H), 7.45 (s breit, 3H).

### 137.2 N-[4-(5-Chloropyridin-2-yl)-1,3-thiazol-2-yl]-N'-(2-methoxy-6-methylbenzyl)guanidin

Die Umsetzung erfolgte analog zu Beispiel 2, 2.4, ausgehend von 260mg (0.63 mmol) Methyl-N'-[(4-(5-chloropyridin-2-yl)-1,3-thiazol-2-yl]imidothiocarbamat Hydroiodid mit 260mg (1.39mmol) 2-Methoxy-6-methylbenzylamin Hydrochlorid; nach Aufreinigung wurden 64mg des Zielprodukts als weißer Feststoff erhalten. ¹H-NMR (400 MHz, DMSO-d6), δ (ppm) = 2.48 (s, 3H), 3.85 (s, 3H), 4.75 (d, 2H), 6.89 (d, 1H), 6.99 (d, 1H), 7.29 (m, 1H), 7.40 (m, 1H), 7.73 (s, 1H), 7.91 (dd, 1H), 8.61 (d, 1H), 9.81 (s breit, 1H), 11.68 (s breit, 1H).

### Analog wurden hergestellt:

### Beispiel 138:

N-[4-(4-Fluorophenyl)-1,3-thiazol-2-yl]-N'-(2-methoxy-6-methylbenzyl)guanidin
ESI-MS [M+H+] = 371.15

### Beispiel 139:

N-(2-Methoxy-6-methylbenzyl)-N'-(4-pyrimidin-2-yl-1,3-thiazol-2-yl)guanidin
ESI-MS [M+H+] = 355.05

### Beispiel 140:

N-(2-Methoxy-6-methylbenzyl)-N'-(4-pyridin-2-yl-1,3-thiazol-2-yl)guanidin
ESI-MS [M+H+] = 354.15

### Beispiel 141:

N-(2-Fluoro-6-methoxybenzyl)-N'-[4-(4-fluorophenyl)-1,3-thiazol-2-yl]guanidin
ESI-MS [M+H+] = 375.15

### Beispiel 142:

### N-(2,6-Dimethoxybenzyl)-N'-[4-(4-fluoro-2-hydroxyphenyl)-1,3-thiazol-2-yl]guanidin

### 142.1 2-Brom-1-(4-fluoro-2-hydroxyphenyl)ethanon

450mg (2.92 mmol) 2-Fluorohydroxyacetophenon in 5ml Diethoxyethan wurden mit 2.5g CuBr₂ versetzt und 1 Stunde lang bei 120°C in der Mikrowelle erhitzt. Filtration der Mischung über Celite und Eindampfen ergaben 870mg rotes Öl, das direkt weiter eingesetzt wurde.

### 142.2 N-(2,6-Dimethoxybenzyl)-N'-[4-(4-fluoro-2-hydroxyphenyl)-1,3-thiazol-2-yl]guanidin

Umsetzung analog zu Beispiel 107 ergab das Zielprodukt; 188mg; ESI-MS [M+H+] = 403.25.

### Beispiel 143:

N-(2,6-Dimethoxybenzyl)-N'-[4-(4-fluorobenzyl)-1,3-thiazol-2-yl]guanidin Herstellung analog zu Beispiel 3, 3.2; das isolierte Rohprodukt wurde durch Chromatographie an RP-Kieselgel (Chromabond-Säule, Acetonitril/Wasser + 0.1% Eisessig; 0-100%) gereinigt. Nach Lyophilisieren wurde 1 mg des Zielprodukts als weißer Feststoff erhalten; ESI-MS [M+H+] = 401.15.

### Beispiel 144:

### N-(2,6-Dimethoxybenzyl)-N'-[4-(5-fluoropyridin-2-yl)-1,3-thiazol-2-yl]guanidin

### 144.1 1-(5-Fluoropyridin-2-yl)ethanon

1g (5.68mmol) 2-Brom-5-fluoropyridin, 160mg Dichlorbis(triphenylphosphin)-palladium und 170mg Cul wurden im ausgeheizten Kolben unter Schutzgas in 30ml Acetonitril suspendiert, 6.09g (16.87mmol) (1-Ethoxyvinyl)-tributylstannan zugesetzt, die Mischung 8 Stunden lang auf Rückfluß erhitzt, anschließend 200ml 1.5n HCl zugegeben und erneut 1 Stunde refluxiert. Zur Aufarbeitung wurde mit ges. NaHCO₃-Lösung neutralisiert, 3x mit Ethylacetat extrahiert, die vereinigten org. Phasen mit ges. NaCl-Lösung gewaschen und mit MgSO₄ getrocknet. Nach Filtration wurde die Mischung mit 30ml ges. KF-Lösung versetzt, über Celite filtriert und eingedampft. Reinung durch Chromatographie an Kieselgel (Dichlormethan/Methanol 0-3%) ergab 120mg 1-(5-Fluoropyridin-2-yl)ethanon als Öl, das direkt weiter umgesetzt wurde.
¹H-NMR (400 MHz, DMSO-d6), δ (ppm) = 2.65 (s, 3H), 7.92 (m, 1H), 8.18 (dd, 1H), 8.74 (d, 1H).

### 144.2 2-Brom-1-(5-fluoropyridin-2-yl)ethanon

100mg 1-(5-Fluoropyridin-2-yl)ethanon, 500mg polymergebundenes Tribromid (1mmol Br₃⁻/g, Fa. Aldrich) und 0.05ml Eisessig in 5ml THFwurden ca. 24 Stunden lang bei Raumtemperatur geschüttelt. Filtration und Eindampfen ergab 150mg des gewünschten Bromids als gelbes Öl, das ohne weitere Reinigung weiter umgesetzt wurde.

### 144.3 N-(2,6-Dimethoxybenzyl)-N'-[4-(5-fluoropyridin-2-yl)-1,3-thiazol-2-yl]guanidin

Die Umsetzung erfolgte analog zu Beispiel 107; es wurden 83mg des Zielprodukts isoliert; ESI-MS [M+H+] = 388.15.

### Beispiel 145:

### N-[4-(3,5-Difluoropyridin-2-yl)-1,3-thiazol-2-yl]-N'-(2,6-dimethoxybenzyl)guanidin

### 145.1 1-(3,5-Difluoropyridin-2-yl)ethanon

Zu einer Lösung aus 15g (10.71mmol) 2-Cyano-3,5-difluoropyridin in 100ml THF wurde bei 0°C Methylmagnesiumbromid (10ml einer 3N Lösung in Diethylether) unter rühren zugetropft, anschließend wurde bis zur vollständigen Umsetzung bei Raumtemperatur nachgerührt. Zur Aufarbeitung wurde mit 10% H₂SO₄ auf pH 4 angesäuert, anschließend mit 25% NH₄OH basisch gestellt, 2x mit Dichlormethan extrahiert, und die vereinigten org. Phasen mit MgSO₄ getrocknet. Reinung durch Chromatographie an Kieselgel (Dichlormethan/Methanol 0-5%) ergab 500mg eines hellen Öls, das beim Stehen durchkristallisierte.
¹H-NMR (400 MHz, DMSO-d6), δ (ppm) = 2.62 (sm 3H), 8.11 (m, 1H), 8.86 (d, 1H).

### 145.2 2-Brom-1-(3,5-difluoropyridin-2-yl)ethanon

Die Bromierung wurde analog zu Beispiel 144.2 durchgeführt, das erhaltene Bromid wurde direkt weiter umgesetzt.

### 145.3 N-[4-(3,5-Difluoropyridin-2-yl)-1,3-thiazol-2-yl]-N'-(2,6-dimethoxybenzyl)guanidin

Die Umsetzung erfolgte analog zu Beispiel 107; es wurden 90mg des Zielprodukts als heller Feststoff isoliert; ESI-MS [M+H+] = 406.05.

### Beispiel 146:

### N-[(2-Methoxy-1-naphthyl)methyl]-N'-(4-phenyl-1,3-thiazol-2-yl)guanidin

Die Herstellung erfolgte analog zu Beispiel 2; ESI-MS [M+H+] = 389.15.

### Beispiel 147:

N-(2,6-Dimethoxybenzyl)-N'-(4-pyrrolidin-2-yl-1,3-thiazol-2-yl)guanidin Hydrochlorid 490mg (1.06mmol) tert-Butyl 2-(2-{[[(2,6-dimethoxybenzyl)amino](imino)methyl]amino}-1,3-thiazol-4-yl)pyrrolidin-1-carboxylat aus Beispiel 120 in 20ml Dioxan wurden bei Raumtemperatur mit 5ml 4N HCl in Dioxan versetzt und 3 Stunden lang gerührt. Chromatographie des nach Einengen erhaltenen Rohprodukts über RP-Kieselgel (Chromabond-Säule, Acetonitril/Wasser + 0.1% Eisessig; 0-100%) ergab 240mg des Zielprodukts als hellen Feststoff; ESI-MS [M+H+] = 362.15.

### Beispiel 148:

### N-[4-(1-Acetylpyrrolidin-2-yl)-1,3-thiazol-2-yl]-N'-(2,6-dimethoxybenzyl)guanidin

Eine Mischung aus 100mg (0.25mmol) N-(2,6-Dimethoxybenzyl)-N'-(4-pyrrolidin-2-yl-1,3-thiazol-2-yl)guanidin Hydrochlorid, Beispiel 147, 0.021ml Acetylchlorid und 0.04ml Pyridin in 10ml THF wurde 1 Stunde bei 5°C, dann 4 Stunden bei Raumtemperatur gerührt. Chromatographie des Rohprodukts über RP-Kieselgel (Chromabond-Säule, Acetonitril/Wasser + 0.1 % Eisessig; 0-100%) ergab 33mg des gewünschten Produkts; ESI-MS [M+H+] = 404.15.

### Beispiel 149:

### N-(2,6-Dimethoxybenzyl)-N'-[4-(1-methylpyrrolidin-2-yl)-1,3-thiazol-2-yl]guanidin

72mg (0.2mmol) N-(2,6-Dimethoxybenzyl)-N'-(4-pyrrolidin-2-yl-1,3-thiazol-2-yl)guanidin Hydrochlorid, Beispiel 147,und 20mg Formalin (37% wässr. Lösung) wurden bei 10°C mit 51mg Natriumtriacetoxyborhydrid versetzt, 30 Minuten bei 10°C und 2 Stunden bei Raumtemperatur gerührt. Zur Aufarbeitung wurde die Mischung eingedampft, der Rückstand in Dichlormethan aufgenommen, mit Wasser gewaschen, getrocknet und erneut eingeengt. Nach Chromatographie des Rohprodukts über RP-Kieselgel (Chromabond-Säule, Acetonitril/Wasser + 0.1% Eisessig; 0-100%) wurden 22mg weiße Festkörper erhalten, ESI-MS [M+H+] = 376.15.

### Beispiel 150:

### N-(2,6-Dimethoxybenzyl)-N'-{4-[1-(phenylsulfonyl)pyrrolidin-2-yl]-1,3-thiazol-2-yl}guanidin

Umsetzung von 170mg (0.38mmol) N-(2,6-Dimethoxybenzyl)-N'-(4-pyrrolidin-2-yl-1,3-thiazol-2-yl)guanidin Hydrochlorid, Beispiel 147, mit 68.85mg Benzolsulfonsäurechlorid und 0.12ml Triethylamin in 15ml Acetonitril und Reinigung des Rohprodukts durch Chromatographie an Kieselgel (Dichlormethan/Methanol 0-5%) ergab 70mg des gewünschten Produkts als weiße Festkörper; ESI-MS [M+H+] = 502.45

### Beispiel 151:

### N-[4-(1-Benzylpyrrolidin-2-yl)-1,3-thiazol-2-yl]-N'-(2,6-dimethoxybenzyl)guanidin

177mg N-(2,6-Dimethoxybenzyl)-N'-(4-pyrrolidin-2-yl-1,3-thiazol-2-yl)guanidin, Beispiel 147 freie Base, 100mg Benzylbromid und 870mg polymergebundenes Triazabicyclodecen (1.3mmol/g, Fa. Argonaut) in 20ml Acetonitril wurden über Nacht bei Raumtemperatur geschüttelt. Chromatographie des nach Eindampfen erhaltenen Rückstands an Kieselgel (Dichlormethan/Methanol 0-5%) ergab 112mg weiße Festkörper; ESI-MS [M+H+] = 452.15.

### Beispiel 152:

### N-(2-Chloro-6-methoxybenzyl)-N'-[4-(3,5-difluoropyridin-2-yl)-1,3-thiazol-2-yl]guanidin

Umsetzung von 130mg (0.31mmol) Methyl-N'{[4-(3,5-Difluoropyridin-2-yl)-1,3-thiazol-2-yl]imidothiocarbamat Hydroiodid, Herstellung analog zu Beispiel 2.3, mit 120mg 2-Chlor-6-methoxybenzylamin analog zu Beispiel 2.4 und Verrühren des erhaltenen Rohprodukts in Methyl-tert.butylether ergab 33mg des Zielprodukts.
¹H-NMR (400 MHz, DMSO-d6), δ (ppm) = 3.87 (s, 3H), 4.52 (d, 2H), 7.09 (m, 3H), 7.36 (m, 3H), 8.95 (m, 1H), 8.52 (s, 1H).

### Beispiel 153:

### N-(2,6-Dimethoxybenzyl)-N'-{4-[4-fluoro-2-(2-morpholin-4-ylethoxy)phenyl]-1,3-thiazol-2-yl}guanidin

### 153.1 1-[4-Fluoro-2-(2-morpholin-4-ylethoxy)phenyl]ethanon

0.5g (3.24mmol) 4-Fluoro-2-hydroxyacetophenon, 720mg (3.87mmol) N-(2-Chlorethyl)morpholin Hydrochlorid, 900mg K₂CO₃ und eine kat. Menge Nal in 20ml Aceton wurden 20 Stunden lang auf Rückfluß erhitzt. Die Mischung wurde aufkonzentriert, in Dichlormethan aufgenommen, mit Wasser und ges. NaCl-Lösung gewaschen, getrocknet und erneut eingeengt. Chromatographie des Rohprodukts an Kieselgel (Dichlormethan/Methanol 0-4%) ergab 730mg eines klaren, farblosen Öls; ESI-MS [M+H+] = 268.15.

### 153.2 150mg (0.56mmol) 1-[4-Fluoro-2-(2-morpholin-4-ylethoxy)phenyl]ethanon wurden analog zu Beispiel 144.2 bromiert; das nach Eindampfen erhaltene Öl wurde direkt weiter umgesetzt.

### 153.3 N-(2,6-Dimethoxybenzyl)-N'-{4-[4-fluoro-2-(2-morpholin-4-ylethoxy)phenyl]-1,3-thiazol-2-yl}guanidin

Umsetzung analog zu Beispiel 3 ergab das gewünschte Produkt als weißen Feststoff; 66mg; ESI-MS [M+H+] = 516.15.

### Beispiel 154:

### N-[4-(Aminomethyl)-1,3-thiazol-2-yl]-N'-(2,6-dimethoxybenzyl)guanidin Hydrochlorid

Abspaltung der Boc-Gruppe ausgehend von 675mg (1.6mmol) tert-Butyl [(2-{[[(2,6-dimethoxybenzyl)amino](imino)methyl]amino}-1,3-thiazol-4-yl)methyl]carbamat analog zu Beispiel 147 ergab 570mg Festkörper; ESI-MS [M+H+] = 422.15.

### Beispiel 155:

### N-[(2-{[[(2,6-Dimethoxybenzyl)amino](imino)methyl]amino}-1,3-thiazol-4-yl)methyl]benzamid

Umsetzung von 50mg (0.16mmol) N-[4-(Aminomethyl)-1,3-thiazol-2-yl]-N'-(2,6-dimethoxybenzyl)guanidin Hydrochlorid, Beispiel 154 freie Base, mit 24.2mg Benzoylchlorid in 5ml THF unter Zusatz von 230mg polymergebundenem NMM (1.7mmol/g; Fa. Argonaut) und Chromatographie des Rohprodukts an Kieselgel Dichlormethan/Methanol 0-2%) ergab 20mg; ESI-MS [M+H+] =426.15.

### Beispiel 156:

### N-(2,6-Dimethoxybenzyl)-N'-[4-(1-isopropylpyrrolidin-2-yl)-1,3-thiazol-2-yl]guanidin

Reduktive Aminierung von 100mg (0.28mmol) N-[4-(Aminomethyl)-1,3-thiazol-2-yl]-N'-(2,6-dimethoxybenzyl)guanidin Hydrochlorid, Beispiel 154 freie Base, mit 0.04ml Aceton und 120mg Natriumtriacetoxyborhydrid in 10ml Acetonitril analog zu Beispiel 149 ergab das Zielprodukt als weiße Festkörper; 52mg; ESI-MS [M+H+] = 404.15.

### Beispiel 157:

### N-[(2-{[[(2,6-Dimethoxybenzyl)amino](imino)methyl]amino}-1,3-thiazol-4-yl)methyl]butan-1-sulfonamid

Umsetzung von 100mg (0.33mmol) N-[4-(Aminomethyl)-1,3-thiazol-2-yl]-N'-(2,6-dimethoxybenzyl)guanidin Hydrochlorid, Beispiel 154 freie Base, mit 60.4mg Butansulfonsäurechlorid in 5ml THF unter Zusatz von 480mg polymergebundenem NMM (1.7mmol/g; Fa. Argonaut) und Chromatographie des Rohprodukts an Kieselgel Dichlormethan/Methanol 0-2%) ergab 46mg; ESI-MS [M+H+] =442.05.

### Beispiel 158:

### N-(2,6-Dimethoxybenzyl)-N'-{4-[(dimethylamino)methyl]-1,3-thiazol-2-yl}guanidine acetate

Reduktive Aminierung von N-[4-(Aminomethyl)-1,3-thiazol-2-yl]-N'-(2,6-dimethoxybenzyl)guanidin Hydrochlorid, Beispiel 154 freie Base, analog zu Beispiel 149 in 5ml DMF unter Verwendung von polymergebundenem MP-Triacetoxyborhydrid (2mmol/g, Fa. Argonaut) ergab 24mg Zielprodukt; ESI-MS [M+H+] = 350.15.

### Beispiel 159:

### Methyl[(2-{[[(2,6-dimethoxybenzyl)amino](imino)methyl]amino}-1,3-thiazol-4-yl)methyl]carbamat

360mg (1.12mmol) N-[4-(Aminomethyl)-1,3-thiazol-2-yl]-N'-(2,6-dimethoxybenzyl)guanidin Hydrochlorid, Beispiel 154 freie Base, in 13.5ml THF wurden mit 0.2ml NMM versetzt, und unter Rühren 0.1ml Chlorameisensäuremethylester in 1.5ml THF zugetropft. Nach beendeter Umsetzung wurde die Mischung konzentriert, mit Dichlormethan verdünnt, mit ges. NaCl-Lösung gewaschen, getrocknet und eingeengt. Chromatographie des Rohprodukts an Kieselgel (Dichlormethan/Methanol 0-1%) ergab 140mg; ESI-MS [M+H+] = 380.05.

### Beispiel 160:

### Methyl-2-{[[(2,6-dimethoxybenzyl)amino](imino)methyl]amino}-4-(4-fluorophenyl)-1,3-thiazol-5-carboxylat

Bromierung von 800mg (4.08mmol) Methyl-4-fluorbenzoylacetat analog zu Beispiel 144.2 und weitere Umsetzung zum Thiazol analog zu Beispiel 3 ergab 27mg. ¹H-NMR (400 MHz, DMSO-d6), δ (ppm) = 3.68 (s, 3H), 3.78 (s, 6H), 4.34 (m breit, 2H), 6.69 (d, 2H), 7.20 (m, 2H), 7.32 (m, 1H), überlagert 6.95-7.40 (m, 2H), 7.63 (m breit, 2H).

### Beispiel 161:

*tert*-Butyl 4-(2-{[[(2,6-dimethoxybenzyl)amino](imino)methyl]amino}-1,3-thiazol-4-yl)piperidin-1-carboxylat
ESI-MS [M+H+] = 476.15

### Beispiel 162:

### N-(2,6-Dimethoxybenzyl)-N'-(4-piperidin-4-yl-1,3-thiazol-2-yl)guanidin Hydrochlorid

Abspaltung der Boc-Gruppe ausgehend von 490mg (1.0mmol) *tert*-Butyl 4-(2-{[[(2,6-dimethoxybenzyl)amino](imino)methyl]amino}-1,3-thiazol-4-yl)piperidin-1-carboxylat analog zu Beispiel 147 ergab 380mg Festkörper; ESI-MS [M+H+] = 376.1

### Beispiel 163:

N-(2,6-Dimethoxybenzyl)-N'-{4-[1-(methylsulfonyl)piperidin-4-yl]-1,3-thiazol-2-yl}guanidin
ESI-MS [M+H+] = 454.05

### Beispiel 164:

### N-[4-(3-Chloropyridin-2-yl)-1,3-thiazol-2-yl]-N'-(2,6-dimethoxybenzyl)guanidin

### 164.1 1-(3-Chloropyridin-2-yl)ethanon

Grignard-Reaktion ausgehend von 0.5g (0.61 mmol) 2-Cyano-3-chlorpyridin analog zu Beispiel 145.1 ergab 360mg des gewünschten Produkts als hellgelbes Öl.
¹H-NMR (400 MHz, DMSO-d6), δ (ppm) = 2.65 (s, 3H), 7.66 (m, 1H), 8.08 (m, 1H), 8.64 (m, 1H).

### 164.2 N-[4-(3-Chloropyridin-2-yl)-1,3-thiazol-2-yl]-N'-(2,6-dimethoxybenzyl)guanidin

Bromierung analog zu Beispiel 144.2 und weitere Umsetzung des entsprechenden 2-Brom-1-(3-chloropyridin-2-yl)ethanons analog zu Beispiel 3 ergab 110mg des Zielprodukts als hellen Feststoff; ESI-MS [M+H+] = 404.25

### Beispiel 165:

### N-(2,6-Dimethoxybenzyl)-N'-[4-(8-fluorochinolin-4-yl)-1,3-thiazol-2-yl]guanidin

### 165.1 8-Fluorochinolin-4-yl Trifluoromethanesulfonat

Zu 1.9g (11.65mmol) 8-Fluoro-4-hydroxychinolin und 4.7ml Triethylamin in 15ml Dichlormethan wurden bei 5°C 4.9g Trifluormethansulfonsäureanhydrid, gelöst in 5ml Dichlormethan, zugetropft, und ca. 20 Minuten bei 5°C gerührt. Zur Aufarbeitung wurde mit 30ml Wasser verdünnt, mit Dichlormethan extrahiert und die org. Phase mit ges. NaCl-Lösung gewaschen. Reinigung des nach Trockenen mit MgS04 erhaltenen Rohprodukts durch Chromatographie an Kieselgel (Dichlormethan) ergab 2.3g des Triflats als helles Öl; ESI-MS [M+H+] = 295.5.

### 165.2 1-(8-Fluorochinolin-4-yl)ethanon

1.3g (4.4mmol) 8-Fluorochinolin-4-yl Trifluoromethanesulfonat, 153mg Tetrakistriphenylphosphinpalladium und 600mg LiCl wurden im ausgeheizten Kolben unter Schutzgas in 30ml Dioxan suspendiert, 1.6g (4.43mmol) (1-Ethoxyvinyl)-tributylstannan zugesetzt, die Mischung 2 Stunden lang auf Rückfluß erhitzt. Die Mischung wurde mit Dichlormethan verdünnt, mit Wasser gewaschen, getrocknet (MgSO₄) und eingeengt. Der so erhaltene Rückstand wurde in 30ml THF gelöst und nach Zugabe von 2ml 5N HCL 3 Stunden lang bei Raumtemperatur gerührt. Zur Aufarbeitung wurde mit ges. NaHCO₃-Lösung auf pH 11 gestellt, mit Dichlormethan extrahiert, die vereinigten org. Phasen mit ges. NaCl-Lösung gewaschen und mit MgSO₄ getrocknet. Reinung durch Chromatographie an Kieselgel (Dichlormethan) ergab 700mg; ESI-MS [M+H+] = 190.05.

### 165.3 2-Bromo-1-(8-fluorochinolin-4-yl)ethanon

Zu 480mg (2.54mmol) 1-(8-Fluorochinolin-4-yl)ethanon in 2ml 48% HBr in Wasser wurden bei 90°C insgesamt 0.13ml Br₂ portionsweise zugesetzt und 30 Minuten bei 90°C gerührt. Nach beendeter Reaktion wurde mit Wasser verdünnt, durch Zugabe von festem NaHCO₃ neutralisiert und mit dichlormethan extrahiert. Waschen der vereinigten org. Phasen mit ges. naCl-Lösung, Trocken und Eindampfen ergab 600mg des gewünschten Bromids, das direkt weiter umgesetzt wurde.

### 165.4 N-(2,6-Dimethoxybenzyl)-N'-[4-(8-fluorochinolin-4-yl)-1,3-thiazol-2-yl]guanidin

Umsetzung von 150mg (0.56mmol) 2-Bromo-1-(8-fluorochinolin-4-yl)ethanon analog zu Beispiel 3 ergab 110mg des Zielprodukts als weiß-gelbe Festkörper;
ESI-MS [M+H+] = 438.05.

### Beispiel 166:

### 1-[2-({Imino[(2-methoxybenzyl)amino]methyl}amino)-1,3-thiazol-4(5H)-yliden]piperidinium Chlorid

Umsetzung von 100mg (0.38mmol) *N*-(2-Chloro-1-piperidin-1-ylethyliden)-4-methylbenzolsulfonamid (Herstellung nach: Abdelaal, S.; Bauer, L. J. Het. Chem. 1988, 25 (6), 1849-1856) mit 120mg (0.38mmol) *N*-[[(2-Methoxybenzyl)amino](imino)-methyl]thioharnstoff analog zu Beispiel 3 ergab 12mg des gewünschten Produkts; ESI-MS [M+H+] = 346.15. Die Verbindung liegt laut NMR als Tautomerengemisch von 1-[2-({Imino[(2-methoxybenzyl)amino]methyl}amino)-1,3-thiazol-4(5H)-ylidene]piperidin und *N*-(2-Methoxybenzyl)-*N*'-(4-piperidin-1-yl-1,3-thiazol-2-yl)guanidin vor.
¹³C-NMR (100.61 MHz, DMSO-d6), δ (ppm): 23.61, 25.75, 36.23, 40.91, 49.94, 109.95, 120.28, 125.50, 128.30, 128.80, 157.09, 176.7667, 185.96.

### Beispiel 167:

### 4-[2-{[[(2,6-Dimethoxybenzyl)amino](imino)methyl]amino}-1,3-thiazol-4(5H)-yliden]morpholin-4-ium Chlorid

### 167.1 N-(2-Chloro-1-morpholin-4-ylethyliden)-4-methylbenzolsulfonamid

1g (5.02mmol) 2-Chloro-1-morpholin-4-ylethaniminium Chlorid und 0.7ml Triethylamin in 25ml Acetonitril wurden bei 10°C mit 0.96g p-Toluolsulfonsäurechlorid versetzt, und 2 Stunden lang bei 5-10°C gerührt. Zur Aufarbeitung wurde mit Dichlormethan verdünnt, mit Wasser und ges. NaCl-Lösung gewaschen, mit MgSO₄ getrocknet, filtriert und eingedampft. Chromatographie an Kieselgel (Dichlormethan/Methanol 0-3%) ergab 400mg des Zielprodukts; ESI-MS [M+H+] = 317.05.

### 167.2 4-[2-{[[(2,6-Dimethoxybenzyl)amino](imino)methyl]amino}-1,3-thiazol-4(5H)-yliden]morpholin-4-ium Chlorid

Die Mischung aus 200mg (0.75mmol) *N*-[[(2,6-Dimethoxybenzyl)amino](imino)-methyl]thioharnstoff und 240mg (0.75mmol) *N*-(2-Chloro-1-morpholin-4-ylethyliden)-4-methylbenzolsulfonamid in 20ml 2-Butanon wurde 24 Stunden auf Rückfluß erhitzt. Übliche Aufarbeitung ergab 170mg helle Festkörper; ESI-MS [M+H+] = 378.25. Die Verbindung liegt laut NMR als Tautomerengemisch von 4-[2-{[[(2,6-Dimethoxybenzyl)amino](imino)methyl]amino}-1,3-thiazol-4(5H)-yliden]morpholin und *N*-(2,6-Dimethoxybenzyl)-*N*'-(4-morpholin-4-yl-1,3-thiazol-2-yl)guanidin vor.
¹³C-NMR (100.61 MHz, DMSO-d6), δ (ppm): 34.76, 36.36, 47.81, 55.95, 65.15, 104.1, 110.66, 130.19, 158.11, 160.00, 177.51, 180.89.

### Beispiel 168:

### N-(2-Methoxybenzyl)-N'-(6-methyl-1,3-benzothiazol-2-yl)guanidin Fumarat

102mg (0,62mmol) 6-Methyl-1,3-benzothiazol-2-amin und 200mg (1.24 mmol) 1,1-Di-1H-imidazol-1-ylmethanimin (Wu, Y.-Q.; Hamilton, S. K.; Wilkinson, D. E.; Hamilton, G. S. J. Org. Chem. 2002, 67, 7553) wurden in THF (1ml) vorgelegt und 40 Minuten unter Rühren in der Mikrowelle bei 300 W Einstrahlung auf 130°C erhitzt. Nach Zugabe von 2-Methoxybenzylamin (85mg, 0.62mmol) wurde das Reaktionsgemisch 60 Minuten auf 130°C erhitzt (300 W, heating by cooling). Das THF wurde am Rotationsverdampfer unter Vakuum abdestilliert, der Rückstand wurde chromatographisch gereinigt (Kieselgel, Dichlormethan, Methanol). Der so erhaltene Feststoff wurde mit Dichlormethan und tert-Butylmethylether gewaschen; 20mg, ESI-MS [M+H⁺] = 327.1

### Analog zu Beispiel 168 wurden hergestellt:

### Beispiel 169:

N-(2,6-Dimethoxybenzyl)-N'-(6-methyl-1,3-benzothiazol-2-yl)guanidin Fumarat
ESI-MS [M+H+] = 357.1.

### Beispiel 170:

N-(6-Ethoxy-1,3-benzothiazol-2-yl)-N'-(2-methoxybenzyl)guanidin Fumarat
ESI-MS [M+H+] = 357.1.

### Beispiel 171:

N-(5-Chlor-6-methyl-1,3-benzothiazol-2-yl)-N'-(2,6-dimethoxybenzyl)guanidin Difumarat
ESI-MS [M+H+] = 391.0.

### Beispiel 172:

N-(2,6-Dimethoxybenzyl)-N'-(5,6-dimethyl-1,3-benzothiazol-2-yl)guanidin Fumarat
ESI-MS [M+H+] = 371.1.

Die Verbindungen **181** und **185** wurden durch Umsetzung geeigneter Ausgangsmaterialien der Formeln II und IV analog zu Beispiel 2, hergestellt:

### Beispiel 181:

*N*-(2,6-Dimethoxybenzyl)-*N*'-(5-methyl-4*H*-1,2,4-triazol-3-yl)guanidin

Die Herstellung des *N*-(2,6-Dimethoxybenzyl)-*N*'-(5-methyl-4*H*-1,2,4-triazol-3-yl)guanidins wurde analog Beispiel 2 durchgeführt mit folgenden Variationen: Die Verseifung von *N*-{[(5-Methyl-4*H*-1,2,4-triazol-3-yl)amino]carbonothioyl}-benzamid in methanolischer Natronlauge ließ sich bereits bei Raumtemperatur durchführen. Der entstandene *N*-(5-Methyl-4*H*-1,2,4-triazol-3-yl)thioharnstoff wurde mit Methyliodid bei Raumtemperatur alkyliert, und das resultierende Methyl *N*-(5-methyl-4*H*-1,2,4-triazol-3-yl)imidothiocarbamat wurde durch Extraktion der organischen Phase mit 1 *N* NaOH freigesetzt. Abschließend wurde das Methyl *N-*(5-methyl-4*H*-1,2,4-triazol-3-yl)imidothiocarbamat mit 2,6-Dimethoxybenzylamin in Ethanol bei 130°C in der Mikrowelle (100 Watt) zur Reaktion gebracht, und man isolierte nach chromatographischer Reinigung über präparative HPLC (Fa. Merck: Chromolith RP-18E,100-25; Fließmittel Wasser/Acetonitril/ 0.1 molar Essigsäure) das *N*-(2,6-Dimethoxybenzyl)-*N*'-(5-methyl-4*H*-1,2,4-triazol-3-yl)guanidin.
ESI-MS [M+H⁺] = 291.15

### Beispiel 185:

### N-(2-Methoxybenzyl)-N'-(5-methyl-4H-1,2,4-triazol-3-yl)guanidin Acetat

Die Herstellung wurde analog Beispiel **183** durchgeführt. Hier wurde das intermediär gebildete *N*-[Imino(methylthio)methyl]-5-methyl-4*H*-1,2,4-triazol-3-aminium Iodid direkt mit 2-Methoxybenzylamin in Ethanol und einem 1.5-fachen Überschuß an Diisopropylethylamin zum Zielprodukt umgesetzt.
ESI-MS [M+H⁺] = 261.15

Die Verbindung **192** wurde durch Umsetzung geeigneter Ausgangsmaterialien der Formeln IV und V analog zu Beispiel 107 hergestellt:

### Beispiel 192:

### Ethyl 2-{[[(2,6-dimethoxybenzyl)amino](imino)methyl]amino}-1,3-thiazol-4-carboxylat Acetat

Die Reinigung des Produktes erfolgte über präparative HPLC (Fa. Merck: Chromolith RP-18E,100-25; Fließmittel Wasser/Acetonitril/ 0.1molar Essigsäure).
ESI-MS [M+H⁺] = 365.05

Die erfindungsgemäßen Verbindungen **193**, **195** und **196** wurden nach dem allgemeinen Verfahren, beispielsweise beschrieben in Organikum, VEB Deutscher Verlag der Wissenschaften, Leipzig, Berlin, Heidelberg, 21. Auflage, 2001, 483 oder Synth. Commun. 1982, 12, 989-993 durch Aminolyse von **192** hergestellt:

### Beispiel 196:

### 2-{[[(2,6-Dimethoxybenzyl)amino](imino)methyl]amino}-N-isopropyl-1,3-thiazol-4-carboxamid

0.157g (0.35mmol) Ethyl 2-{[[(2,6-dimethoxybenzyl)amino](imino)methyl]amino}-1,3-thiazol-4-carboxylat Bromid wurden mit 1.50ml (17.28mmol) Diisopropylamin vereinigt und in der Mikrowelle bei 50 bis 70 °C (150 Watt) 2 Stunden lang erwärmt. In diesem Beispiel wurde vollständiger Umsatz erst nach weiterem Erwärmen in der Mikrowelle bei 70 °C (150 Watt) unter "Heating by Cooling" nach 4.5 Stunden erzielt. Die Reaktionsmischung wurde mit 20ml Dichlormethan verdünnt und mit Wasser (3 x 30 ml) gewaschen. Nach Trocknen über MgSO₄, Filtration und Entfernen des organischen Lösungsmittels i. Vak. wurde die Mischung über präparative HPLC (Fa. Merck: Chromolith RP-18E,100-25; Fließmittel Wasser/Acetonitril/ 0.1molar Essigsäure) gereinigt. Man isolierte 26mg an 2-{[[(2,6-Dimethoxybenzyl)amino](imino)methyl]amino}-*N*-isopropyl-1,3-thiazol-4-carboxamid.
ESI-MS [M+H⁺] = 378.15

### Analog zu Beispiel 196wurden hergestellt:

### Beispiel 193:

*N*-Allyl-2-{[[(2,6-dimethoxybenzyl)amino](imino)methyl]amino}-1,3-thiazol-4-carboxamid
ESI-MS [M+H⁺] = 376.15

### Beispiel 194:

*N*-Benzyl-2-{[[(2,6-dimethoxybenzyl)amino](imino)methyl]amino}-1,3-thiazol-4-carboxamid
ESI-MS [M+H⁺] = 426.15

Die Verbindung **175** wurde durch Umsetzung geeigneter Ausgangsmaterialien der Formeln II und IV wie folgt hergestellt:

### Beispiel 175

### N-(2-Methoxybenzyl)-N'-(5-phenyl-1,3,4-thiadiazol-2-yl)guanidin Acetat

### 175.1. N-(5-Phenyl-1,3,4-thiadiazol-2-yl)-1H-imidazol-1-carbothioamid

1.80g (6.56mmol) 5-Phenyl-[1,3,4]thiadiazol-2-yl-ammoniumsulfat wurden in 10ml DMF vorgelegt, 4.99 ml (29.7mmol) Diisopropylethylamin zugegeben und die Mischung solange bei Raumtemperatur gerührt, bis die Suspension in Lösung ging. Es wurde anschließend 1.3g (7.29 mmol) *N*,*N*'-Thiocarbonyldiimidazol in 10ml Acetonitril zugetropft und bei Raumtemperatur 12 Stunden lang nachgerührt. Das Lösungsmittel wurde i. Vak. Entfernt, der Rückstand mit Wasser versetzt und der entstandene Feststoff durch Filtration abgetrennt. Nach Trocknen des Feststoffs isolierte man 2.10g *N*-(5-Phenyl-1,3,4-thiadiazol-2-yl)-1*H*-imidazol-1-carbothioamid als Rohmischung, die ohne weitere Auftrennung eingesetzt wurde.
¹H-NMR ([400 MHz, DMSO-d6): δ (ppm) 7.33 (pt, 1H), 7.33-7.54 (m, 3H), 7.66 (m, 1H, *J* = 1.1 Hz), 7.96 (dd, 2H, *J* = 8.1 Hz, *J* = 1.4 Hz), 8.16 (pt, 1H, *J* = 1.3 Hz), 9.18 (s, 1H),.

### 175.2 N-(5-Phenyl-1,3,4-thiadiazol-2-yl)-thioharnstoff

1.0g der Rohmischung mit *N*-(5-Phenyl-1,3,4-thiadiazol-2-yl)-1*H*-imidazol-1-carbothioamid vereinigte man mit 0.536g (6.96mmol) Ammoniumacetat in 4 ml Ethanol. Die Reaktionsmischung wurde in der Mikrowelle (bei 100 Watt) 30 Minuten lang bei 90°C erhitzt. Nach beendeter Reaktion wurde das Lösungsmittel i. Vak. abdestilliert, und der erhaltene Rückstand mit Wasser versetzt. Nach Extraktion mit CH₂Cl₂ und Trocknen der organischen Phase mit Magnesiumsulfat kristallisierte aus der organischen Lösung das Produkt aus, das nach Filtration noch mit Diethylether gewaschen wurde. Es wurden 0.52g des *N*-(5-Phenyl-1,3,4-thiadiazol-2-yl)-thioharnstoffs erhalten.
ESI-MS [M+H⁺] = 237.05

### 175.3 Methyl N-(5-phenyl-1,3,4-thiadiazol-2-yl)imidothiocarbamat

0.52g (2.22mmol) *N*-(5-Phenyl-1,3,4-thiadiazol-2-yl)-thioharnstoff wurden in 4 ml Methanol gelöst und mit 0.515g (3.63mmol) Methyliodid versetzt. Man rührte das Reaktionsgemisch bei 50°C 2 Stunden lang und weitere 12 Stunden bei Raumtemperatur. Zur Freisetzung des intermediär entstandenden Iodid-Salzes wurde die Mischung mit 0.427g (3.30mmol) Diisopropylethylamin versetzt und 2 Stunden lang bei Raumtemperatur gerührt. Das Lösungsmittel wurde i. Vak. entfernt, und der Rückstand in Dichlormethan gelöst. Die Freisetzung des lodid-Salzes kann auch alternativ durch Extraktion mit 2 *N* Natronlauge erfolgen. Nach Extraktion mit Wasser (3 x 50 ml) wurde die organische Phase über MgSO₄ getrocknet und nach Entfernen des Lösungsmittels i. Vak. wurden 0.37g an Rohprodukt isoliert, das ohne weitere Aufreinigung für die Folgeumsetzung verwandt wurde.
ESI-MS [M+H⁺] = 251.10

### 175.4 N-(2-Methoxybenzyl)-N'-(5-phenyl-1,3,4-thiadiazol-2-yl)guanidin Acetat

0.185g (0.74mmol) Methyl *N*-(5-phenyl-1,3,4-thiadiazol-2-yl)imidothiocarbamat wurden zusammen mit 0.132g (0.96mmol) 2-Methoxybenzylamin in 3ml Ethanol gelöst und in der Mikrowelle (bei 100 Watt) 30 Minuten lang bei 90 °C erhitzt. In den Fällen, in denen die Umsetzung nicht vollständig erfolgte, wurde noch 2 ml Toluol zugegeben und wiederholt in der Mikrowelle (bei 100 Watt) 30 Minuten lang bei 100 °C erhitzt. Nach vollständigem Umsatz wurde das Lösungsmittel i. Vak. entfernt. Den Rückstand trennte man mittels präparativer HPLC (Fa. Merck: Chromolith RP-18E,100-25; Fließmittel Wasser/Acetonitril/ 0.1 molar Essigsäure) auf, und man isolierte 32mg des reinen N-(2-Methoxybenzyl)-N'-(5-phenyl-1,3,4-thiadiazol-2-yl)guanidin Acetats.
ESI-MS [M+H⁺] = 340.15

Die Verbindungen **176-178**, und **183** wurden durch Umsetzung geeigneter Ausgangsmaterialien der Formeln II und IV analog zu Beispiel **175** hergestellt:

### Beispiel 176:

N-(2,6-Dimethoxybenzyl)-N'-(5-phenyl-1,3,4-thiadiazol-2-yl)guanidin
ESI-MS [M+H⁺] = 370.15

### Beispiel 177:

*N*-(2,6-Dimethoxybenzyl)-*N*'-1*H*-imidazol-2-ylguanidin Acetat
ESI-MS [M+H⁺] = 276.15

### Beispiel 178:

*N*-(2-Methoxybenzyl)-*N*'-(5-methyl-1,3,4-thiadiazol-2-yl)guanidin
ESI-MS [M+H⁺] = 278.05

### Beispiel 183:

*N*-1*H*-Imidazol-2-yl-*N*'-(2-methoxybenzyl)guanidin Acetat
ESI-MS [M+H⁺] = 246.05

Die Verbindungen **179** und **180** wurden durch Umsetzung geeigneter Ausgangsmaterialien der Formeln II und IV analog zu Beispiel **175** hergestellt, wobei die entsprechend kommerziell erhältlichen Thioharnstoff-Derivate nach der Umsetzung mit Methyliodid mit *1* N Natronlauge wie in **175.3** behandelt wurden, um das Iodid-Salz freizusetzen. Der Zusatz an Toluol wie in **175.4** war hier nicht erforderlich:

### Beispiel 179:

*N*-1*H*-Indazol-3-yl-*N*'-(2-methoxybenzyl)guanidine Acetat
ESI-MS [M+H⁺] = 296.15

### Beispiel 180:

*N*-(2,6-Dimethoxybenzyl)-*N*'-1*H*-indazol-3-ylguanidin Acetat
ESI-MS [M+H⁺] = 326.15

### Beispiel 182:

### N-1H-Benzimidazol-2-yl-N'-(2,6-dimethoxybenzyl)guanidin Diacetat

### 182.1 N-1H-Benzimidazol-2-ylthioharnstoff

0.535g (3.0mmol) *N*,*N*'-Thiocarbonyldiimidazol löste man in 5 ml Acetonitril, tropfte zu dieser gelben Lösung 0.40g (3.0mmol) 2-Aminobenzimidazol, suspendiert in 5 ml Acetonitril, bei Raumtemperatur zu. Nach 30 Minuten bildete sich ein Niederschlag, wobei noch weitere 12 Stunden lang nachgerührt wurde. Nachdem man dann zu dieser Mischung 0.474g (6.0mmol) Ammoniumacetat in Substanz gegeben hatte, wurde in der Mikrowelle (bei 100 Watt) 30 Minuten lang bei 90°C erhitzt. Lösungsmittel wurde i. Vak. entfernt, der Rückstand mit Wasser versetzt und mit Dichlormethan extrahiert (3 x 30 ml). Die vereinigte organische Phase wurde nochmals mit Wasser gewaschen und nach Trocknung über MgSO₄ das Lösungsmittel i. Vak. entfernt. Das Produkt wurde mittels präparativer HPLC (Fa. Merck: Chromolith RP-18E,100-25; Fließmittel Wasser/Acetonitril/ 0.1molar Essigsäure) gereinigt. In diesem Eintopfverfahren wurde ohne Isolation der Zwischenstufe direkt 0.27g des (*N*-1*H*-Benzimidazol-2-ylthioharnstoffs gewonnen.
ESI-MS [M+H⁺] = 193.05

### 182.2 (1H-Benzimidazol-2-ylamino)(methylthio)methaniminium Iodid

0.27g (1.40mmol) *N*-1*H*-Benzimidazol-2-ylthioharnstoff wurden in 4ml Methanol gelöst und mit 0.115ml (1.83mmol) Methyliodid versetzt. Es wurde 30 Minuten lang unter Rückfluß erhitzt. Nach vollständigem Umsatz wurde das Lösungsmittel i. Vak. entfernt und 0.47g 1*H*-Benzimidazol-2-ylamino)(methylthio)methan iminium iodid ohne weitere Aufreinigung für die Folgeumsetzung verwandt.
ESI-MS [M+H⁺] = 207.05

### 182.3 N-1H-Benzimidazol-2-yl-N'-(2,6-dimethoxybenzyl)guanidin Diacetat

0.470g (1.4mmol) 1*H*-Benzimidazol-2-ylamino)(methylthio)methan iminium iodid wurden in 4ml Ethanol gelöst und nach Zugabe von 0.24ml (1.41mmol) Diisopropylethylamin und 0.282g (1.69mmol) 2,6-Dimethoxybenzylamin zusammen 3 Stunden lang unter Rückfluß erhitzt. Alternativ kann die Umsetzung entsprechend auch in der Mikrowelle (bei 100-200 Watt) 30 Minuten lang bei 90-100 °C erfolgen. Nach vollständigem Umsatz wurde das Lösungsmittel i. Vak. entfernt. Der Rückstand wurde in Wasser aufgenommen und mit Dichlormethan wurde das Produkt in die organische Phase extrahiert. Die organische Phase wurde noch mit *1 N* NaOH (2 x 50 ml) gewaschen, um das Produkt vollständig vom Iodid zu befreien. Nach Trocknen über MgSO₄ und Filtration wurde das organische Solvens i. Vak. entfernt. Es bildete sich ein voluminöser Niederschlag, nachdem der Rückstand in 4 ml Acetonitril / Wasser (1:1) und 0.5 ml Eisessig aufgenommen wurde. Der Feststoff wurde abfiltriert und man erhielt 30mg des gewünschten Produkts.
ESI-MS [M+H⁺] = 326.25

Die Verbindungen **184** und **189** wurden durch Umsetzung geeigneter Ausgangsmaterialien der Formeln II und IV analog zu Beispiel **182** hergestellt:

### Beispiel 184:

*N*-(2-Methoxybenzyl)-*N*'-4*H*-1,2,4-triazol-3-ylguanidin Acetat
ESI-MS [M+H⁺] = 247.05

### Beispiel 189:

*N*-(2,6-Dimethoxybenzyl)-*N*'-4*H*-1,2,4-triazol-3-ylguanidin Acetat
ESI-MS [M+H⁺] = 277.10

### Beispiel 187

### N-(2,6-Dimethoxybenzyl)-N'-(4-phenyl-1H-imidazol-2-yl)guanidin Acetat

### 187.1 N-(4-Phenyl-1H-imidazol-2-yl)thioharnstoff

2.10g (11.78mmol) *N*,*N*'-Thiocarbonyldiimidazol und 2.44g (5.64mmol) Bis(4-phenyl-1*H*-imidazol-2-aminium)sulfat wurden zusammengegeben und 50ml Acetonitril zugefügt. Man erwärmte die Reaktionsmischung auf 50 °C 6 Stunden lang. Nach vollständiger Umsetzung gab man 2.26g (29.29mmol) Ammoniumacetat hinzu und erhitzte die Mischung eine Stunde lang auf 80 °C. Der Ansatz wurde i. Vak. vom Lösungsmittel befreit. Der Rückstand wurde mit Wasser versetzt und mit Dichlormethan (3 x 150 ml) extrahiert. Die vereinigte organische Phase wurde über MgSO₄ getrocknet, filtriert und eingedampft; 1.78g
ESI-MS [M+H⁺] = 219.05

### 187.2 Methyl N-(4-phenyl-1H-imidazol-2-yl)imidothiocarbamat

Die Herstellung erfolgte analog zu **182.2** Es wurden 1.89g an Produkt isoliert.
ESI-MS [M+H⁺] = 233.95

### 187.3 N-(2,6-Dimethoxybenzyl)-N'-(4-phenyl-1H-imidazol-2-yl)guanidin Acetat

Die Herstellung erfolgte analog zu **182.3**. Es wurden nach Reinigung 180mg sauberes Produkt isoliert.
ESI-MS [M+H⁺] = 352.15.

Die Verbindungen **186** und **188** wurden durch Umsetzung geeigneter Ausgangsmaterialien der Formeln II und IV analog zu Beispiel **187** hergestellt:

### Beispiel 186:

*N*-[4-(4-Fluorophenyl)-1*H*-imidazol-2-yl]-*N*'-(2-methoxybenzyl)guanidin
ESI-MS [M+H⁺] = 340.15

### Beispiel 188:

*N*-(2-Methoxybenzyl)-*N*'-(4-phenyl-1*H*-imidazol-2-yl)guanidin acetat
ESI-MS [M+H⁺] = 322.15

Die Verbindung **191** und **195** wurden durch Umsetzung geeigneter Ausgangsmaterialien der Formeln II und IV wie folgt hergestellt :

### Beispiel 191:

### N-(2,6-Dimethoxybenzyl)-N'-[4-(4-fluorphenyl)-1H-imidazol-2-yl]guanidin Acetat

### 191.1 N-[4-(4-Fluorphenyl)-1H-imidazol-2-yl]acetamid

3.00g (13.82mmol) 2-Brom-4'-fluoracetophenon, 2.80g (27.64mmol) Acetylguanidin und 15 ml Acetonitril wurden vereinigt und zusammen in der Mikrowelle (bei 100 Watt) 60 Minuten lang bei 40 °C unter "Heating by Cooling" zur Reaktion gebracht. Der nach Abkühlen entstandene Feststoff wurde vom Lösungsmittel abfiltriert. Zusätzlich wurde aus dem Rückstand der Mutterlauge durch fraktionierte Kristallisation mit Ethanol eine Gesamtmenge von 1.02g *N*-[4-(4-Fluorphenyl)-1*H*-imidazol-2-yl]acetamid isoliert.
ESI-MS [M+H⁺] = 220.05

### 191.2 4-(4-Fluorphenyl)-1H-imidazol-2-aminium chlorid

1.0g (4.56mmol) *N*-[4-(4-Fluorphenyl)-1*H*-imidazol-2-yl]acetamid wurde in 30 ml 2 *N* HCl und 30 ml Ethanol suspendiert und 2 Stunden lang bei 80 °C gerührt. Nach Entfernen des Lösungsmittels i. Vak. erhielt man 0.97g 4-(4-Fluorphenyl)-1*H*-imidazol-2-aminium chlorid, das ohne weiterer Aufreinigung für die folgende Reaktion eingesetzt wurde.
ESI-MS [M+H⁺] = 214.05

### 1919.3 N-[4-(4-Fluorphenyl)-1H-imidazol-2-yl]thioharnstoff

Die Herstellung erfolgte analog zu **187.1**. Es wurden nach Reinigung 0.74g sauberes Produkt isoliert.
ESI-MS [M+H⁺] = 237.05

### 191.4 Methyl N-[4-(4-fluorphenyl)-1H-imidazol-2-yl]imidothiocarbamat

Die Herstellung erfolgte analog zu **175.3**, wobei das intermediär erhaltene 4-(4-Fluorphenyl)-*N*-[imino(methylthio)methyl]-1*H*-imidazol-2-aminium iodid mit 2 *N* Natronlauge behandelt wurde. Es wurden nach Reinigung 0.75g sauberes Produkt isoliert.
ESI-MS [M+H⁺] = 251.05

### 191.5 N-(2,6-Dimethoxybenzyl)-N'-[4-(4-fluorphenyl)-1H-imidazol-2-yl]guanidin Acetat

Die Herstellung erfolgte analog zu **175.4**. Die Reaktion von 0.40g (1.60mmol) Methyl *N*-[4-(4-fluorphenyl)-1*H*-imidazol-2-yl]imidothiocarbamat mit 0.53g (3.20mmol) 2,6-Dimethoxybenzylamin in ethanolischer Lösung wurde in der Mikrowelle 3 Minuten lang bei 150 °C (200 Watt) durchgeführt. Es wurden nach Reinigung 35mg sauberes Produkt isoliert.
ESI-MS [M+H⁺] = 370.15

### Beispiel 195:

*N*-(2-Chlor-6-methoxybenzyl)-*N*'-[4-(4-fluorphenyl)-1*H*-imidazol-2-yl]guanidin Acetat
ESI-MS [M+H⁺] = 374.10 / 376.10

### Beispiel 190:

### N-(2,6-Dimethoxybenzyl)-N'-(4-methyl-1,3-oxazol-2-yl)guanidin Acetat

### 190.1 N-Cyano-N'-(2,6-dimethoxybenzyl)guanidin

5.0g (29.9mmol) 2,6-Dimethoxybenzylamin wurden mit 15 ml 2 N Salzsäure versetzt und anschließend die wäßrige Phase i. Vak. entfernt. Der Rückstand wurde dann in 50ml 1-Butanol aufgenommen und 2.66g (29.9mmol) Natriumdicyanamid zugegeben. Die Reaktionsmischung wurde 5.5 Stunden lang auf Rückfluß erhitzt und 12 Stunden lang bei Raumtemperatur nachgerührt. Der gebildete Niederschlag wurde abfiltriert, mit Diethylether gewaschen; 6.81g.
ESI-MS [M+H⁺] = 235.15

### 190.2 N-(2,6-Dimethoxybenzyl)-N'-(4-methyl-1,3-oxazol-2-yl)guanidin Acetat

0.20g (0.85mmol) *N*-Cyano-*N*'-(2,6-dimethoxybenzyl)guanidin wurden in 20ml Methanol / Wasser (1:1) suspendiert. Anschließend wurden 63.3mg (0.85mmol) Hydroxyaceton zugegeben und die Mischung auf 40 °C erhitzt. Der pH-Wert wurde bei erhöhter Temperatur mit 1 *N* HCl auf pH 2.5 - 3 eingestellt. Insgesamt wurde die Reaktionsmischung 34 Stunden lang bei 40 °C unter stetiger pH-Kontrolle erwärmt, bis keine Edukte mehr über Massenspektrometrie zu detektieren waren. Das Methanol wurde i. Vak. abdestilliert, und es blieb ein weißer Feststoff zurück, der über HPLC (Fa. Merck: Chromolith RP-18E,100-25; Fließmittel Wasser/Acetonitril/ 0.1 molar Essigsäure) gereinigt wurde; 64mg.
ESI-MS [M+H⁺] = 291.15

Die Verbindungen **173**, **174** und **197-199** wurden durch Umsetzung geeigneter Ausgangsmaterialien der Formeln IV und VII analog zu Beispiel **190** hergestellt:

### Beispiel 173:

*N*-(4,5-Dimethyl-1,3-oxazol-2-yl)-*N*'-(2-methoxybenzyl)guanidin
ESI-MS [M+H⁺] = 275.15

### Beispiel 174:

*N*-(2,6-Dimethoxybenzyl)-*N*'-(4,5-dimethyl-1,3-oxazol-2-yl)guanidin Acetat
ESI-MS [M+H⁺] = 305.15

### Beispiel 197:

### N-(2-Methoxybenzyl)-N'-(4-phenyl-1,3-oxazol-2-yl)guanidin

Die Cyclisierung von 0.60g (2.94mmol) *N*-Cyano-*N*'-(2-methoxybenzyl)guanidin mit 0.44g (2.94mmol) 2-Hydroxyacetophenon wurde in 20 ml Acetonitril und Wasser (1 : 1) durchgeführt. Der pH-Wert der Mischung wurde dabei mit 1 *N* Salzsäure auf pH 1.5 eingestellt, und die Mischung daraufhin in der Mikrowelle bei 45 °C (100 Watt) 4.5 Stunden lang erwärmt. Nach Aufarbeitung und Reinigung analog zu **190.2** erhielt man 0.10g reines Produkt.
ESI-MS [M+H⁺] = 323.15

### Beispiel 198:

### N-[4-(4-Fluorophenyl)-1,3-oxazol-2-yl]-N'-(2-methoxybenzyl)guanidin

Die Herstellung des *N*-[4-(4-Fluorophenyl)-1,3-oxazol-2-yl]-N'-(2-methoxybenzyl)guanidins erfolgte analog dem Beispiel **197**.
ESI-MS [M+H⁺] = 341.05

### Beispiel 199:

### N-(2,6-Dimethoxybenzyl)-N'-(4-phenyl-1,3-oxazol-2-yl)guanidin

Die Herstellung des *N*-(2,6-Dimethoxybenzyl)-*N*'-(4-phenyl-1,3-oxazol-2-yl)guanidins erfolgte analog dem Beispiel **197**.
ESI-MS [M+H⁺] = 353.35

### Beispiel 200:

### N-(5-tert-Butyl-1H-pyrazol-3-yl)-N'-(2-methoxy-benzyl)-guanidin

### 200.1 (5-tert-Butyl-1H-pyrazol-3-yl)-thioharnstoff

Zu einer Lösung von 2.0g (11.3 mmol) Thiocarbonyldiimidazol in 25ml Acetonitril wurde bei 0°C langsam 1.5g (11.3mmol) 3-Amino-5-tert-butylpyrazol, gelöst in Acetonitril, zugetropft. Der Ansatz wurde noch 20 Minuten bei 0°C nachgerührt, dann mit Ammoniumacetat (1.6g, 21.5mmol) versetzt und in der Mikrowelle 30 Minuten auf 90°C erhitzt (Einstrahlleistung 200Watt / ohne Kühlung). Der entstandene Feststoff wurde abgesaugt, die Lösung eingeengt und der ölige Rückstand (4.3 g) an Kieselgel grob gereinigt (0.9g gelblicher Feststoff)

### 200.2 1-(5-tert-Butyl-1H-pyrazol-3-yl)-2-methyl-isothioharnstoff Hydroiodid

270mg (1.4 mmol) (5-tert-Butyl-1H-pyrazol-3-yl)-thioharnstoff wurden in 2ml Methanol gelöst, mit Methyliodid (202mg, 1.4mmol) versetzt und über Nacht bei Raumtemperatur gerührt. Die Reaktionslösung wurde eingeengt, der Rückstand mit Dichlormethan versetzt und bei Raumtemperatur gerührt. Der Feststoff wurde abgesaugt und getrocknet (0.43g).

### 200.3 N-(5-tert-Butyl-1H-pyrazol-3-yl)-N'-(2-methoxy-benzyl)-guanidin

120mg (0.35 mmol) 1-(5-tert-Butyl-1H-pyrazol-3-yl)-2-methyl-isothioharnstoff Hydroiodid und 150mg (1.1mmol) 2-Methoxybenzylamin wurden in 1 ml 1-Propanol gelöst, und in der Mikrowelle bei 100°C 30Minuten gerührt (Einstrahlleistung 200Watt / ohne Kühlung).
Die Reaktionslösung wurde eingeengt und das farblose Öl (0.29g) durch Chromatographie an Kieselgel (Chromabond RP18; Wasser/Acetonitril + 0.1% Essigsäure 0-80%) gereinigt, wonach 83mg hellgelber Schaum. erhalten wurden. Verrühren mit Diethylether ergab 53mg weißen Feststoff.
ESI-MS [M+H+] = 303

### Analog zu Beispiel 200 wurden hergestellt

### Beispiel 201:

### N-(2-Methoxybenzyl)-N'-(5-phenyl-1H-pyrazol-3-yl)guanidin

Ausgehend von 100mg 2-Methyl-1-(5-phenyl-1 H-pyrazol-3-yl)-isoharnstoff Hydroiodid; 57mg Zielprodukt.
ESI-MS [M+H+] = 322

### Beispiel 202:

### N-(2,6-Dimethoxybenzyl)-N'-(5-phenyl-1H-pyrazol-3-yl)guanidin

Ausgehend von 100mg 2-Methyl-1-(5-phenyl-1H-pyrazol-3-yl)-isoharnstoff Hydroiodid; 49mg Zielprodukt.
ESI-MS [M+H+] = 352

### Beispiel 203:

### N-(2,6-Dimethoxybenzyl)-N'-1H-pyrazol-3-ylguanidin

Ausgehend von 100mg 2-Methyl-1-(1H-pyrazol-3-yl)-isoharnstoff Hydroiodid, 46mg Zielprodukt.
ESI-MS [M+H+] = 276

### Beispiel 204:

### N-(5-tert-Butyl-1H-pyrazol-3-yl)-N'-(2,6-dimethoxybenzyl)guanidin

Ausgehend von 120mg 1-(5-tert-Butyl-1H-pyrazol-3-yl)-2-methyl-isothioharnstoff Hydroiodid, 82 mg Zielprodukt.
ESI-MS [M+H+] = 332

### Beispiel 205:

### N-(2-Methoxybenzyl)-N'-1H-pyrazol-3-ylguanidin

Ausgehend von 130mg 2-Methyl-1-(1H-pyrazol-3-yl)-isoharnstoff Hydroiodid; 67mg Zielprodukt.
ESI-MS [M+H+] = 246

### Beispiel 206:

### N-(2-Methoxybenzyl)-N'-[5-(4-methylphenyl)-1H-pyrazol-3-yl]guanidin

Ausgehend von 150mg 2-Methyl-1-(5-p-tolyl-1H-pyrazol-3-yl)-isoharnstoff Hydroiodid; 79mg Zielprodukt.
ESI-MS [M+H+] = 336

### Beispiel 207:

### N-(2,6-Dimethoxybenzyl)-N'-[5-(4-methylphenyl)-1H-pyrazol-3-yl]guanidin

Ausgehend von 150mg 2-Methyl-1-(5-p-tolyl-1H-pyrazol-3-yl)-isoharnstoff Hydroiodid; 103mg Zielprodukt.
ESI-MS [M+H+] = 366

### Beispiel 208:

### N-(2-Methoxybenzyl)-N'-[5-(4-methoxyphenyl)-1H-pyrazol-3-yl]guanidin

Ausgehend von 120mg 1-[5-(4-Methoxy-phenyl)-1H-pyrazol-3-yl]-2-methyl-isoharnstoff Hydroiodid; 56mg Zielprodukt.
ESI-MS [M+H+] = 352

### Beispiel 209:

### N-(2,6-Dimethoxybenzyl)-N'-[5-(4-methoxyphenyl)-1H-pyrazol-3-yl]guanidin

Ausgehend von 120mg 1-[5-(4-Methoxy-phenyl)-1H-pyrazol-3-yl]-2-methyl-isoharnstoff Hydroiodid; 54mg Zielprodukt.
ESI-MS [M+H+] = 382

### Beispiel 210:

### N-(5-tert-Butyl-1H-pyrazol-3-yl)-N'-(2-fluoro-6-methoxybenzyl)guanidin

Ausgehend von 170mg 1-(5-tert-Butyl-1H-pyrazol-3-yl)-2-methy-isoharnstoff Hydroiodid; 93mg Zielprodukt.
ESI-MS [M+H+] = 320

### Beispiel 211:

### N-[5-(4-Chlorophenyl)-1H-pyrazol-3-yl]-N'-(2-methoxybenzyl)guanidin

Ausgehend von 150mg 1-[5-(4-Chloro-phenyl)-1H-pyrazol-3-yl]-2-methyl-isoharnstoff Hydroiodid; 30mg Zielprodukt.
ESI-MS [M+H+] = 356 / 358

### Beispiel 212:

### N-[5-(4-Chlorophenyl)-1H-pyrazol-3-yl]-N'-(2,6-dimethoxybenzyl)guanidin

Ausgehend von 140mg 1-[5-(4-Chloro-phenyl)-1H-pyrazol-3-yl]-2-methyl-isoharnstoff Hydroiodid; 71 mg Zielprodukt.
ESI-MS [M+H+] = 386 / 388

### Beispiel 213:

### N-(5-tert-Butyl-1H-pyrazol-3-yl)-N'-(2-chloro-6-methoxybenzyl)guanidin

Ausgehend von 150mg 1-(5-tert-Butyl-1H-pyrazol-3-yl)-2-methy-isoharnstoff Hydroiodid,; 89mg Zielprodukt.
ESI-MS [M+H+] = 336 / 338

### Beispiel 214:

### N-(2-Methoxybenzyl)-N'-(1-methyl-1H-pyrazol-3-yl)guanidin

Ausgehend von 160mg 2-Methyl-1-(1-methyl-1H-pyrazol-3-yl)-isoharnstoff Hydroiodid; 34mg Zielprodukt.
ESI-MS [M+H+] = 260

### Beispiel 215:

### N-(2,6-Dimethoxybenzyl)-N'-(1-methyl-1H-pyrazol-3-yl)guanidin

Ausgehend von 160mg 2-Methyl-1-(1-methyl-1H-pyrazol-3-yl)-isoharnstoff Hydroiodid, 56mg Zielprodukt.
ESI-MS [M+H+] = 290

### Beispiel 216:

### N-(5-tert-Butyl-1H-pyrazol-3-yl)-N'-(2-methoxy-6-methylbenzyl)guanidin

Ausgehend von 130mg 1-(5-tert-Butyl-1H-pyrazol-3-yl)-2-methy-isoharnstoff Hydroiodid; 47mg Zielprodukt.
ESI-MS [M+H+] = 316

### Beispiel 217:

### N-(2-Methoxybenzyl)-N'-(4-phenyl-1H-pyrazol-3-yl)guanidin

Ausgehend von 150mg 2-Methyl-1-(4-phenyl-1H-pyrazol-3-yl)-isoharnstoff Hydroiodid; 76mg Zielprodukt.
ESI-MS [M+H+] = 322

### Beispiel 218:

### N-(2,6-Dimethoxybenzyl)-N'-(4-phenyl-1H-pyrazol-3-yl)guanidin

Ausgehend von 160mg 2-Methyl-1-(4-phenyl-1H-pyrazol-3-yl)-isoharnstoff Hydroiodid; 83mg Zielprodukt.
ESI-MS [M+H+] = 352

### Beispiel 219:

### N-(2,6-Dimethoxybenzyl)-N'-(1-phenyl-1H-pyrazol-3-yl)guanidin

Ausgehend von 160mg 2-Methyl-1-(1-phenyl-1H-pyrazol-3-yl)-isoharnstoff Hydroiodid; 34mg Zielprodukt.
ESI-MS [M+H+] = 352

### Beispiel 220:

### N-(2-Methoxybenzyl)-N'-(1-phenyl-1H-pyrazol-3-yl)guanidin

Ausgehend von 130mg 2-Methyl-1-(1-phenyl-1H-pyrazol-3-yl)- isoharnstoff Hydroiodid; 47mg Zielprodukt.
ESI-MS [M+H+] = 322

### Beispiel 221:

### N-[2-Methoxy-5-(trifluoromethyl)benzyl]-N'-(4-phenyl-1,3-thiazol-2-yl)guanidin

### 221.1 Die Herstellung von 2-Methoxy-5-trifluoromethyl-benzylamin erfolgte ausgehend von 2-Methoxy-5-trifluoromethyl-benzonitril durch Reduktion mit

Lithiumaluminiumhydrid in Tetrahydrofuran unter Standardbedingungen.

### 221.2 Ausgehend von 100mg 2-Methyl-1-(4-phenyl-thiazol-2-yl)-isoharnstoff Hydroiodid; 41mg Zielprodukt.

ESI-MS [M+H+] = 407

### Beispiel 222:

### N-4H-Chromeno[4,3-d][1,3-d]thiazol-2-yl-N'-(2,6-dimethoxybenzyl)guanidin

Umsetzung analog zu Beispiel 107 ausgehend von 210mg 3-Brom-2,3-dihydro-4*H-*chromen-4-on (0.92mmol) ergab 80mg des gewünschten produkts als weißen Feststoff; ESI-MS [M+H⁺] = 399.1.

### Beispiel 223:

### N-(2,6-Dimethoxybenzyl)-N'-(4,5,6,7-tetrahydro[1,3]thiazolo[5,4-c]pyridin-2-yl)guanidin 223.1 -3-Bromo-4-oxopiperidin-1-carboxylat

Zu 1g N-Benzyl-4-oxopiperidin-1-carboxylat (4.3mmol) in 20ml THF wurden bei 10°C 0.5ml Eisessig und 0.22ml Brom zugegeben, und die Mischung 1 Stunde lang bei Raumtemparatur nachgerührt. Zur Aufarbeitung wurde mit 20ml Wasser versetzt, mit NaHCO₃ neutralisiert und anschließend mit Dichlormethan mehrmals extrahiert. Die vereinigten organischen Phasen wurden dann mit ges. NaCl-Lösung gewaschen, getrocknet und eingeengt. Auf diese Weise wurden 1.2g des gewünschten Bromids als gelbliches Öl erhalten, das ohne weitere Aufreinigung eingesetzt wurde.

### 223.2 Benzyl-2-{[[(2,6-dimethoxybenzyl)amino](imino)methyl]amino}-6,7-dihydro[1,3]thiazolo[5,4-c]pyridine-5(4H)-carboxylat

Umsetzung von 180mg Benzyl-3-bromo-4-oxopiperidin-1-carboxylat analog zu Beispiel 107 ergab 40mg des gewünschten Produkts; ESI-MS [M+H+] = 482.1.

### 223.3 N-(2,6-Dimethoxybenzyl)-N'-(4,5,6,7-tetrahydro[1,3]thiazolo[5,4-c]pyridin-2-yl)guanidin

150mg Benzyl-2-{[[(2,6-dimethoxybenzyl)amino](imino)methyl]amino}-6,7-dihydro[1,3]thiazolo[5,4-c]pyridine-5(4H)-carboxylat (0.28mmol) in 2ml Eisessig wurden mit 0.2ml HBr (33% in Eisessig) versetzt und 2 Stunden lang bei Raumtemparatur gerührt. Nach beendeter Umsetzung wurde die Mischung eingedampft, über C18-Chromabond filtriert (Wasser/CH₃CN+0.1% Eisessig; 0-30%), und durch Rühren mit polymergebundenem Carbonat in Methanol die freie Base erhalten. 60mg; ESI-MS [M+H+] = 348.15.

### Beispiel 224:

### N-(5-Acetyl-4,5,6,7-tetrahydro[1,3]thiazolo[5,4-c]pyridin-2-yl)-N'-(2,6-dimethoxybenzyl)guanidin

Acetylierung von 110mg N-(2,6-Dimethoxybenzyl)-N'-(4,5,6,7-tetrahydro[1,3]thiazolo[5,4-c]pyridin-2-yl)guanidin (0.26mmol, Beispiel 221.3) unter Standarbedingungen mit Acetylchlorid und Triethylamin in 15ml THF und anschließende Aufreinigung ergab 16mg des gewünschten Produkts als weißen Feststoff; ESI-MS [M+H+] = 390.15.

### Beispiel 225:

### N-(2,6-Dimethoxybenzyl)-N'-[5-(phenylsulfonyl)-4,5,6,7-tetrahydro[1,3]thiazolo[5,4-c]pyridin-2-yl]guanidin

Umsetzung von 30mg N-(2,6-Dimethoxybenzyl)-N'-(4,5,6,7-tetrahydro[1,3]thiazolo[5,4-c]pyridin-2-yl)guanidin (0.09mmol, Beispiel 221.3) mit 18mg Benzolsulfonsäurechlorid und 40mg polymergebundenem DMAP (Fa. Argonaut; 1.06mmol/g) in 5ml Dichlormethan und anschließende chromatographische Aufreinigung (Dichlormethan/Methanol 2-4%) ergab 15mg des Zielprodukts; ESI-MS [M+H+] = 488.15.

### Beispiel 226:

### N-(5-Benzyl-4,5,6,7-tetrahydro[1,3]thiazolo[5,4-c]pyridin-2-yl)-N'-(2,6-dimethoxybenzyl)guanidin

### 226.1 1-Benzyl-3-bromopiperidin-4-on Hydrobromid

Bromierung von 3g N-Benzyl-4-oxopiperidin (15.85mmol) analog zu Beispiel 221.1 ergab 5.2g des Bromids als hellgelben Feststoff.

### 226.2 Freisetzung der Base vom Hydrobromid und Umsetzung von 200mg 1-Benzyl-3-bromopiperidin-4-on (0.75mmol) analog zu Beispiel 107 ergab 93mg des gewünschten Produkts; ESI-MS [M+H+] = 438.3.

### Biologische Tests

### 1. [³H]5-CT Bindungsassay h5-HT_{5A}

Membranen von HEK293-Zellen, die das h5-HT_{5A}-Rezeptorgen permanent exprimieren, werden in 100 mM Tris-HCl-Puffer (pH 7,7), der 1mM EDTA enthält, in Gegenwart von 2,5 nM [³H]5-CT inkubiert (600 µl Gesamtvolumen). Die Gesamtbindung ist definiert durch die Bindung, die beobachtet wird, wenn die Membranen in Gegenwart des Radioliganden allein inkubiert werden. Die durch die Verbindung induzierte Hemmung wird bestimmt durch Inkubieren von Zellmembranen in Gegenwart des Radioliganden und von verschiedenen Konzentrationen der interessierenden Verbindung. Die unspezifische Bindung ist definiert durch die [³H]5-CT-Bindung, die durch Inkubieren der Membranen wie für die Gesamtbindung, aber in Gegenwart von 10 µM Methiothepine erhalten wird. Im Anschluss an eine Inkubation von 75 min bei 30°C wird die Membransuspension durch GF/B-Filter, eingehüllt mit 0,03 % PEI, filtriert, wobei ein Skatron^{R}-Erntesystem verwendet wird. Die in dem Filter zurückgehaltene Radioaktivität wird durch Flüssigszintillationszählung quantifiziert.

### 2. Funktioneller Assay für menschliche 5-HT5A-Rezeptorliganden -Serotonin-induzierte Zunahme der GTP-Europium-Bindung

### Allgemeine Beschreibung:

Eine Stimulierung von G Protein-gekoppelten Rezeptoren durch geeignete Agonisten führt zur Bindung von GTP an die α-Untereinheit von trimeren G-Proteinen, gefolgt von der Dissoziation der GTP-gebundenen α-Untereinheit von den βγ-Untereinheiten und der Aktivierung der Signaltransduktion. Durch Verwenden eines Europium-markierten GTP-Analogons, GTP-Eu, kann die Aktivierung eines G-Protein-gekoppelten Rezeptors durch einen Agonisten als eine Zunahme der Bindung von GTP-Eu an den Rezeptor-G-Protein-Komplex verfolgt werden. Nach dem Entfernen von ungebundenem GTP-Eu kann gebundenes GTP-Eu durch Messen der zeitaufgelösten Fluoreszenzemission in geeigneten Nachweisvorrichtungen quantifiziert werden.

Zelllinie: h5HT5A_18.2_SH-sy-5y, eine menschliche Neuroblastomzelllinie, die den menschlichen 5-HT5A-Rezeptor stabil exprimiert.

Membranpräparation: Zellmembranen werden gemäß einer Standardvorschrift in Gegenwart von Proteaseinhibitoren hergestellt und werden durch zwei aufeinanderfolgende Zentrifugationsschritte bei 40000xg teilweise aufgereinigt. Aliquots werden bei -80°C aufbewahrt.

### Assay:

Der Assay wird in Filterplatten mit 96 Vertiefungen (AcroWell-96, Pall Corp.) durchgeführt. Die in Assaypuffer (2,5 µM GDP, 100 mM NaCl, 3 mM MgCl₂, 50 mM HEPES pH 7,4) verdünnten Rezeptormembranen werden zu der Filterplatte zugegeben (5 µg Rezeptormembran/Vertiefung). Testverbindungen werden in 100 % DMSO gelöst und Reihenverdünnungen werden zu den Rezeptormembranen zugegeben (DMSO-Endkonzentration 0,5 %). Die Reaktion wird durch die Zugabe von Serotonin (Endkonzentration 1 µM, gesamtes Assayvolumen 100µl) gestartet. Nach einem ersten Inkubationszeitraum von 30 min bei 30°C wird GTP-Eu (Endkonzentration 10 nM) zugegeben, gefolgt von einem zweiten Inkubationszeitraum von 30 min bei 30°C. Die Reaktion wird durch schnelle Vakuumfiltration angehalten und die Vertiefungen werden zweimal mit eiskaltem Assaypuffer gewaschen. Gebundenes GTP-Eu wird in einem VICTOR-Multilabel-Counter (PerkinElmerCorp.) unter Verwendung der zeitaufgelösten Europium-Einstellungen gemessen. Die Daten werden bezüglich der unspezifischen Bindung korrigiert und IC50-Werte werden mit PRISM4.0 (GraphPad Inc.) unter Verwendung von standardmäßigen nicht-linearen Kurvenanpassungsalgorithmen berechnet. Kb-Werte werden aus IC50-Werten unter Verwendung der Cheng-Prusoff-Näherung berechnet.

In beiden Assays werden verschiedene Konzentrationen der Testsubstanzen eingesetzt, und die Ki-bzw. die IC50-Werte bestimmt. Die Affinität ausgewählter Verbindungen ist in nachfolgender Tabelle gezeigt:

**Tabelle 1 Affinität an 5-HT5A (Ki)**

| **Beispiel #** | **Bindungsaffinität 5-HT5A (Ki)** |
|---|---|
| 1 | +++ |
| 2 | +++ |
| 3 | +++ |
| 4 | ++ |
| 5 | +++ |
| 6 | +++ |
| 7 | + |
| 8 | +++ |
| 9 | +++ |
| 11 | ++ |
| 12 | + |
| 13 | + |
| 14 | ++ |
| 15 | + |
| 16 | + |
| 17 | ++ |
| 18 | + |
| 19 | ++ |
| 20 | + |
| 21 | +++ |
| 22 | + |
| 23 | + |
| 24 | + |
| 26 | + |
| 27 | +++ |
| 28 | ++ |
| 29 | ++ |
| 30 | ++ |
| 31 | ++ |
| 32 | +++ |
| 33 | +++ |
| 34 | +++ |
| 35 | + |
| 36 | +++ |
| 37 | ++ |
| 38 | ++ |
| 39 | +++ |
| 40 | +++ |
| 41 | +++ |
| 42 | +++ |
| 43 | ++ |
| 44 | +++ |
| 45 | +++ |
| 46 | +++ |
| 47 | +++ |
| 48 | ++ |
| 49 | +++ |
| 50 | +++ |
| 51 | +++ |
| 52 | + |
| 53 | ++ |
| 54 | +++ |
| 55 | +++ |
| 56 | +++ |
| 57 | +++ |
| 58 | +++ |
| 59 | +++ |
| 60 | +++ |
| 61 | +++ |
| 62 | ++ |
| 63 | +++ |
| 64 | +++ |
| 65 | +++ |
| 66 | +++ |
| 67 | +++ |
| 68 | +++ |
| 69 | +++ |
| 70 | +++ |
| 71 | +++ |
| 72 | +++ |
| 73 | +++ |
| 74 | +++ |
| 75 | +++ |
| 76 | +++ |
| 77 | +++ |
| 78 | +++ |
| 79 | +++ |
| 80 | +++ |
| 81 | +++ |
| 82 | ++ |
| 83 | +++ |
| 84 | +++ |
| 85 | +++ |
| 86 | +++ |
| 87 | +++ |
| 88 | +++ |
| 89 | +++ |
| 90 | +++ |
| 91 | +++ |
| 92 | ++ |
| 93 | +++ |
| 94 | +++ |
| 95 | ++ |
| 96 | +++ |
| 97 | +++ |
| 98 | ++ |
| 99 | ++ |
| 100 | +++ |
| 101 | ++ |
| 102 | +++ |
| 103 | ++ |
| 104 | + |
| 105 | + |
| 106 | + |
| 107 | ++ |
| 108 | + |
| 109 | ++ |
| 110 | +++ |
| 111 | +++ |
| 112 | +++ |
| 113 | +++ |
| 114 | +++ |
| 115 | +++ |
| 116 | +++ |
| 117 | +++ |
| 118 | +++ |
| 119 | +++ |
| 120 | ++ |
| 121 | +++ |
| 122 | +++ |
| 123 | +++ |
| 124 | +++ |
| 125 | +++ |
| 126 | +++ |
| 127 | +++ |
| 128 | +++ |
| 129 | ++ |
| 130 | ++ |
| 131 | +++ |
| 132 | +++ |
| 133 | +++ |
| 134 | ++ |
| 135 | +++ |
| 136 | ++ |
| 137 | + |
| 138 | +++ |
| 139 | +++ |
| 140 | +++ |
| 141 | ++ |
| 142 | +++ |
| 143 | +++ |
| 144 | +++ |
| 145 | +++ |
| 146 | ++ |
| 147 | +++ |
| 148 | +++ |
| 149 | +++ |
| 150 | +++ |
| 151 | +++ |
| 152 | +++ |
| 153 | +++ |
| 154 | +++ |
| 155 | +++ |
| 156 | ++ |
| 157 | +++ |
| 158 | +++ |
| 159 | +++ |
| 160 | +++ |
| 162 | +++ |
| 163 | +++ |
| 164 | +++ |
| 165 | +++ |
| 166 | + |
| 167 | ++ |
| 168 | ++ |
| 169 | +++ |
| 170 | ++ |
| 171 | ++ |
| 172 | +++ |
| 173 | ++ |
| 174 | +++ |
| 175 | + |
| 176 | ++ |
| 177 | +++ |
| 178 | ++ |
| 179 | +++ |
| 180 | +++ |
| 181 | +++ |
| 182 | +++ |
| 183 | ++ |
| 184 | + |
| 185 | + |
| 186 | +++ |
| 187 | +++ |
| 188 | +++ |
| 189 | +++ |
| 190 | +++ |
| 191 | +++ |
| 192 | +++ |
| 193 | +++ |
| 194 | +++ |
| 195 | +++ |
| 196 | +++ |
| 197 | ++ |
| 198 | +++ |
| 199 | +++ |
| 200 | ++ |
| 201 | +++ |
| 202 | +++ |
| 203 | +++ |
| 204 | +++ |
| 205 | ++ |
| 206 | +++ |
| 207 | +++ |
| 208 | +++ |
| 209 | +++ |
| 210 | +++ |
| 211 | +++ |
| 212 | +++ |
| 213 | +++ |
| 214 | +++ |
| 215 | +++ |
| 216 | +++ |
| 217 | + |
| 218 | + |
| 219 | +++ |
| 220 | +++ |
| 221 | + |
| 222 | +++ |
| 223 | +++ |
| 222 | +++ |
| 223 | +++ |
| 224 | +++ |
| 225 | +++ |
| 226 | +++ |

| | |
|---|---|
| + bedeutet eine Affinität >500nM ++ bedeutet eine Affinität zwischen 50 und 500nM +++ bedeutet eine Affinität <50nM | |

Die Erfindung betrifft weiterhin die folgenden 22 Gegenstände:
1. Guanidinverbindung der allgemeinen Formel I 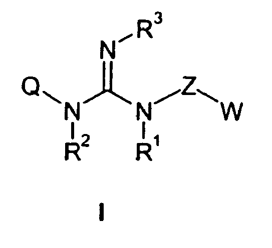 entsprechende enantiomere, diastereomere und/oder tautomere Formen davon sowie pharmazeutisch annehmbare Salze davon, wobei die angegebenen Reste die folgenden Definitionen besitzen:
   W:
      einen Rest der allgemeinen Formel W1 oder W2 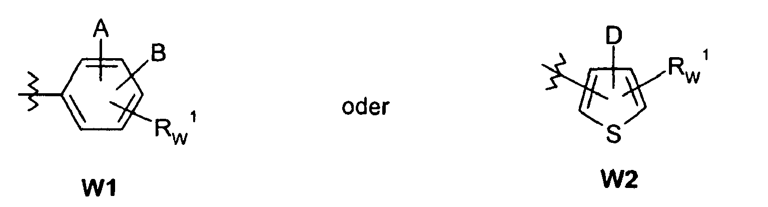
      worin
   A:
      NO₂, NH₂, OH, CN, CF₃, OCF₃, CHF₂, OCHF₂, COOH, O-CH₂-COOH, Halogen, SH, oder
      jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Hetaryl oder C₁-C₄-AlkylenAryl, oder
      O-R_{A}¹, CO-R_{A}¹, S-R_{A}¹, SO-R_{A}¹, CO-O-R_{A}¹, NR_{A}⁴-CO-O-R_{A}¹, O-CH₂-COO-R_{A}¹, NR_{A}²R_{A}³, CONH₂, SO₂NH₂, NR_{A}⁴-CO-R_{A}¹, SO₂-R_{A}¹, NR_{A}⁴-SO₂-R_{A}¹, SO₂-NR_{A}²R_{A}³ oder CO-NR_{A}²R_{A}³;
   **R_{A}¹:**
      jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₂-C₆-Alkenylen-Aryl oder C₁-C₆-Alkylen-Hetaryl;
   **R_{A}²:**
      Wasserstoff, OH, CN, oder
      jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
   **R_{A}³:**
      jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
      oder die Reste **R_{A}²** und **R_{A}³** zusammen mit dem Stickstoff einen 3 bis 7-gliedrigen, gegebenenfalls substituierten, gesättigten oder aromatischen Hetero-cyclus, der ein, zwei oder drei weitere verschiedene oder gleiche Heteroatome aus der Gruppe O, N, S enthalten kann, bilden; wobei gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und wobei der dadurch gebildete Cyclus gegebenenfalls substituiert sein kann oder an diesem Cyclus ein weiterer, gegebenenfalls substituierter Cyclus ankondensiert sein kann;
   **R_{A}⁴:**
      Wasserstoff, oder
      jeweils gegebenenfalls substituiertes C₁-C₆-Alkyl, C₁-C₆-Alkylen-O-C₁-C₆-Alkyl, C₂-C₆-Alkenyl, C₃-C₁₂-Alkinyl, CO-C₁-C₆-Alkyl, CO-O-C₁-C₆-Alkyl, SO₂-C₁-C₆-Alkyl, C₃-C₇-Cycloalkyl, Aryl, C₁-C₄-Alkylen-Aryl, CO-O-Arylalkyl, CO-C₁-C₄-Alkylen-Aryl, CO-Aryl, SO₂-Aryl, Hetaryl, CO-Hetaryl oder SO₂-C₁-C₄-AlkylenAryl;
   **B:**
      Wasserstoff oder wie Rest **A** definiert ist,
      oder jeweils unabhängig voneinander zwei der Reste **A**, **B** oder **R_{w}¹** zusammen einen 3 bis 7-gliedrigen, gegebenenfalls substituierten, gesättigten oder ungesättigten Carbocyclus oder einen gegebenenfalls substituierten, gesättigten oder ungesättigten oder aromatischen Heterocyclus, der ein, zwei oder drei weitere verschiedene oder gleiche Heteroatome aus der Gruppe O, N, S enthalten kann, bilden; wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
   **R_{w}¹:**
      Wasserstoff, OH, Halogen, NO₂, NH₂, CN, CF₃, CHF₂, O-CF₃, O-CHF₂, oder jeweils gegebenenfalls substituiertes C₁-C₆-Alkyl, C₃-C₇-Cycloalkyl, C₁-C₆-Alkylen-O-C₁-C₆-Alkyl, C₁-C₆-Thioalkyl, Aryl, Hetaryl, O-C₁-C₆-Alkyl, O-Aryl, O-Benzyl, C₁-C₆-Alkylamino, C₁-C₆-Dialkylamino, Pyrrolidinyl, Piperidinyl, Morpholinyl, CO-C₁-C₆-Alkyl, SO₂-C₁-C₆-Alkyl, CO-Aryl, SO₂-Aryl, CO-C₁-C₄-AlkylenAryl, SO₂-C₁-C₄-Alkylen-Aryl, SO-Aryl, CONH₂, CONH-C₁-C₆-Alkyl, SO₂NH-C₁-C₆-Alkyl, CON-(C₁-C₆-Alkyl)₂, SO₂N-(C₁-C₆-Alkyl)₂, NH-SO₂-C₁-C₆-Alkyl oder NH-CO-C₁-C₆-Alkyl;
   **D**:
      wie Rest A definiert ist;
   **Z**:
      einen Rest der allgemeinen Formel Z1 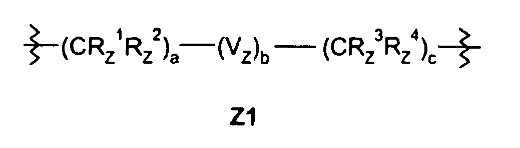
      mit den Indizes
      a=0-4
      b = 0,1
      c=0-4
      wobei die Summe aus a, b und c mindestens 1 und höchstens 5 beträgt;
   **R_{z}¹, R_{z}², R_{z}³**, **R_{z}⁴** unabhängig voneinander:
      Wasserstoff, Halogen, OH, oder
      jeweils gegebenenfalls substituiertes C₁-C₆-Alkyl, C₂-C₆-Alkenyl, C₂-C₆-Alkinyl, C₁-C₆-Alkylen-C₃-C₇-Cycloalkyl, C₃-C₇-Cycloalkyl, Aryl, C₁-C₄-Alkylen-Aryl, Hetaryl oder C₁-C₄-Alkylen-Hetaryl, oder
      jeweils unabhängig voneinander zwei Reste **R_{z}¹** und **R_{z}²** oder **R_{z}³** und **R_{z}⁴** zusammen einen 3 bis 7-gliedrigen, gegebenenfalls substituierten, gesättigten oder ungesättigten Carbo- oder Heterocyclus bilden, wobei der Heterocyclus bis zu drei Heteroatome aus der Gruppe O, N oder S enthalten kann;
   **V_{z}:**
      -CO-, -CO-NR_{z}⁵-, -NR_{z}⁵-CO-, -O-, -S-, -SO-, -SO₂-, -SO₂-NR_{z}⁵-, -NR_{z}⁵-SO₂-, -CS-, -CS-NR_{z}⁵-, -NR_{z}⁵-CS-, -CS-O-, -O-CS-, -CO-O-, -O-CO-, -O-, Ethinylen, -C(=CR_{z}⁶R_{z}⁷)-, -CR_{z}⁶=CR_{z}⁷-, -NR_{z}⁵-CO-NR_{z}^{5*}-, -O-CO-NR_{z}⁵-, -NR_{z}⁵-;
   **R_{z}⁵, R_{z}⁵*** unabhängig voneinander:
      Wasserstoff oder
      jeweils gegebenenfalls substituiertes C₁-C₆-Alkyl, C₁-C₆-Alkylen-O-C₁-C₆-Alkyl, C₂-C₆-Alkenyl, C₃-C₁₂-Alkinyl, CO-C₁-C₆-Alkyl, CO-O-C₁-C₆-Alkyl, SO₂-C₁-C₆-Alkyl, C₃-C₇-Cycloalkyl, Aryl, C₁-C₄-Alkylen-Aryl, CO-O-C₁-C₄-Alkylen-Aryl, CO-C₁-C₄-Alkylen-Aryl, CO-Aryl, SO₂-Aryl, Hetaryl, CO-Hetaryl oder SO₂-C₁-C₄-Alkylen-Aryl;
   **R_{z}⁶**, **R_{z}⁷** unabhängig voneinander:
      Wasserstoff, OH oder
      jeweils gegebenenfalls substituiertes C₁-C₆-Alkyl, C₁-C₄-Alkoxy, C₂-C₆-Alkenyl, C₂-C₆-Alkinyl, C₁-C₆-Alkylen-C₃-C₇-Cycloalkyl, C₃-C₇-Cycloalkyl, Aryl, C₁-C₄-Alkylen-Aryl, Hetaryl oder C₁-C₄-Alkylen-Hetaryl;
   **R¹**, **R², R³** unabhängig voneinander:
      Wasserstoff, OH, CN, oder
         jeweils gegebenenfalls substituiertes C₁-C₆-Alkyl, O-C₁-C₆-Alkyl, C₁-C₆-Alkylen-O-C₁-C₆-Alkyl, C₃-C₇-Cycloalkyl, O-C₃-C₇-Cycloalkyl, Aryl, Hetaryl, C₁-C₄-Alkylen-Aryl, C₁-C₄-Alkylen-Hetaryl, O-Aryl, O-C₁-C₄-Alkylen-Aryl, O-Hetaryl, O-C₁-C₄-Alkylen-Hetaryl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-Hetaryl, CO-O-C₁-C₄-Alkylen-Aryl, SO₂-C₁-C₆-Alkyl, SO₂-Aryl, SO₂-Hetaryl, SO₂-C₁-C₄-Alkylen-Aryl, OCO-C₁-C₆-Alkyl, OCO-Aryl, OCO-Hetaryl, OCO-C₁-C₄-Alkylen-Aryl, OCO-C₁-C₄-Alkylen-Hetaryl, SO₂-C₁-C₆-Alkyl, SO₂-Aryl, SO₂-Hetaryl oder SO₂-C₁-C₄-Alkylen-Aryl, oder
      jeweils unabhängig vom dritten Rest zwei Reste von **R¹, R²** oder **R³** zusammen einen 5 bis 7-gliedrigen, gegebenenfalls substituierten, gesättigten oder ungesättigten Carbocyclus, oder einen gegebenenfalls substituierten, gesättigten oder ungesättigten Heterocyclus, der ein, zwei oder drei weitere verschiedene oder gleiche Heteroatome aus der Gruppe O, N, S enthalten kann, bilden, wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
   Q:
      einen zweifach substituierten 5-gliedrigen Hetaryl-Rest, ausgewählt aus Q1 bis Q7 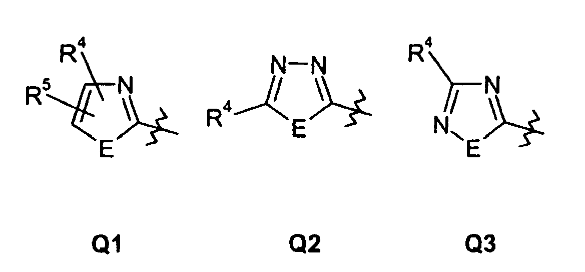 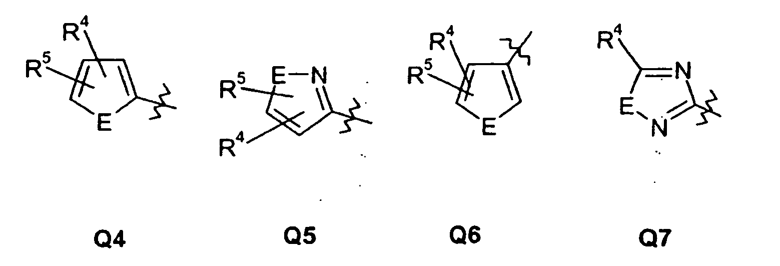
   **E**: O, N-R_{Q}¹ oder S;
   **R_{Q}¹:**
      Wasserstoff, oder
      jeweils gegebenenfalls substituiertes C₁-C₄-Alkyl, CO-C₁-C₄-Alkyl, SO₂-C₁-C₄-Alkyl, CO-O-C₁-C₄-Alkyl, Aryl, C₁-C₄-Alkylen-Aryl, CO-Aryl, CO-Hetaryl, SO₂-Aryl, SO₂-Hetaryl, CO-O-Aryl, CO-C₁-C₄-Alkylen-Aryl, SO₂-C₁-C₄-Alkylen-Aryl oder CO-O-C₁-C₄-Alkylen-Aryl;
   **R⁴, R⁵** jeweils unabhängig voneinander einen Rest, ausgewählt aus den Gruppen **1.)**, **2.), 3.), 4.), 5.), 6.)** oder **7.):**
      **1.)** Wasserstoff, Halogen, CN, CF₃, CHF₂, oder
         jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryl, C₁-C₄-Alkylen-Hetaryl, C₁-C₆-Alkylen-O-C₁-C₆-Alkyl, C₁-C₆-Alkylen-O-Aryl, COO-C₁-C₄-Alkyl oder C₁-C₄-Alkylen-COO-C₁-C₄-Alkyl;
      **2.)** Phenyl oder Naphthyl, die jeweils mit **R_{Q}², R_{Q}³** und **R_{Q}⁴** substituiert sind, wobei
         **R_{Q}², R_{Q}³** und **R_{Q}⁴** jeweils unabhängig voneinander einen Substituenten aus der folgenden Gruppe darstellen:
            Wasserstoff, NO₂. NH₂, OH, CN, CF₃, CHF₂, OCF₃, OCHF₂, COOH, O-CH₂-COOH, SH, Halogen, oder
               jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-Alkylen-Hetaryl, oder
               **O-R_{Q}⁵,** S-R_{Q}⁵, NR_{Q}⁷R_{Q}⁸, CO-OR_{Q}⁶, NR_{Q}⁸-CO-O-R_{Q}⁶, O-CH₂-COO-R_{Q}⁶, NR_{Q}⁸-CO-R_{Q}⁶, SO₂-R_{Q}⁶, NR_{Q}⁸-SO₂-R_{Q}⁶, SO₂NH₂, CONH₂, SO₂-NR_{Q}⁷R_{Q}⁸ oder CO-NR_{Q}⁷R_{Q}⁸, oder
            jeweils zwei der Reste aus **R_{Q}², R_{Q}³** oder **R_{Q}⁴** zusammen einen 3 bis 7-gliedrigen, gegebenenfalls substituierten, gesättigten, ungesättigten oder aromatischen Carbocyclus oder einen gegebenenfalls substituierten, gesättigten, ungesättigten aromatischen Heterocyclus, der bis zu drei weitere verschiedene oder gleiche Heteroatome O, N, S enthalten kann, bilden, und gegebenenfalls zwei an diesem Heterocyclus substituierte Reste können zusammen einen anellierten, gesättigten, ungesättigten oder aromatischen Carbocyclus oder Heterocyclus bilden, wobei der Heterocyclus bis zu drei verschiedene oder gleiche Heteroatome O, N, S enthalten kann, und der gebildete Cyclus kann gegebenenfalls substituiert sein oder an diesem Cyclus kann ein weiterer, gegebenenfalls substituierter Cyclus ankondensiert sein,
         **R_{Q}⁵** jeweils gegebenenfalls substituiertes C₂-C₆-Alkenyl, C₂-C₆-Alkinyl, C₁-C₄-Alkylen-C₃-C₇-Cycloalkyl, C₁-C₄-Alkylen-Heterocycloalkyl, Heterocycloalkyl oder Hetaryl, oder
            C₁-C₆-Alkyl, das gegebenenfalls substituiert ist mit einem Substituenten aus der Gruppe, bestehend aus Halogen, NO₂, NH₂, OH, CN, CF₃, CHF₂, OCF₃, OCHF₂, NH-(C₁-C₆-Alkyl) und N(C₁-C₆-Alkyl)₂;
         **R_{Q}⁶** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl oder C₁-C₆-Alkylen-O-C₁-C₆-Alkyl;
         **R_{Q}⁷** Wasserstoff, OH, CN, oder
            jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, C₁-C₄-Alkylen-Aryl, C₁-C₄-Alkylen-Hetaryl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
         **R_{Q}⁸** Wasserstoff oder
            jeweils gegebenenfalls substituiertes C₁-C₆-Alkyl. C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
            oder die Reste R_{Q}⁷ und R_{Q}⁸ zusammen mit dem Stickstoff einen 3 bis 7-gliedrigen, gegebenenfalls substituierten, gesättigten oder aromatischen Heterocyclus, der ein, zwei oder drei weitere verschiedene oder gleiche Heteroatome O, N, S enthalten kann, bilden; und gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und der gebildete Cyclus gegebenenfalls substituiert sein kann oder an diesem Cyclus ein weiterer, gegebenenfalls substituierter Cyclus ankondensiert sein kann;
         3.) einen 5- oder 6-gliedrigen, gegebenenfalls mit 1 oder 2 Substituenten substituierten Hetaryl-Rest aus der Gruppe, bestehend aus:
            2-Pyrrolyl, 3-Pyrrolyl, 2-Thiazolyl, 4-Thiazolyl, 5-Thiazolyl, 2-Oxazolyl, 4-Oxazolyl, 5-Oxazolyl, 2-Pyrimidyl, 4-Pyrimidyl, 5-Pyrimidyl, 6-Pyrimidyl, 3-Pyrazolyl, 4-Pyrazolyl, 5-Pyrazolyl, 3-Isothiazolyl, 4-Isothiazolyl, 5-Isothiazolyl, 2-Imidazolyl, 4-Imidazolyl, 5-Imidazolyl, 3-Pyridazinyl, 4-Pyridazinyl, 5-Pyridazinyl, 6-Pyridazinyl, 3-Isoxazolyl, 4-Isoxazolyl, 5-Isoxazolyl, Thiadiazolyl, Oxadiazolyl oder Triazinyl oder deren anellierten Derivate Indazolyl, Benzothiophenyl, Benzofuranyl, Indolinyl, Benzimidazolyl, Benzthiazolyl, Benzoxazolyl, Chinolinyl und Isochinolinyl; oder
            **gegebenenfalls** mit 1 oder 2 Substituenten substituiertes 2-Thienyl oder 3-Thienyl, wobei die Substituenten ausgewählt sind aus der Gruppe, bestehend aus Halogen, NO₂, NH₂, OH, CN, CF₃, OCF₃, CHF₂, O-CHF₂, C₁-C₆-Alkyl, O-C₁-C₆-Alkyl, NH-(C₁-C₆-Alkyl), N(C₁-C₆-Alkyl)₂, NHCO-C₁-C₄-Alkyl, NHSO₂-C₁-C₄-Alkyl und SO₂-C₁-C₄-Alkyl;
         **4.)** beide Reste **R⁴** und **R⁵** zusammen einen 4 bis 7-gliedrigen, gegebenenfalls substituierten, gesättigten oder ungesättigten oder aromatischen Carbocyclus oder einen 5- oder 6-gliedrigen gegebenenfalls substituierten, gesättigten oder ungesättigten oder aromatischen Heterocyclus, der bis zu drei weitere verschiedene oder gleiche Heteroatome O, N, S enthalten kann, bilden, und mit bis zu zwei weiteren Resten substituiert sein kann, wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
         **5.)** einen C₅-C₁₈- bi- oder tricyclischen, gesättigten Kohlenwasserstoffrest;
         **6.)** jeweils gegebenenfalls substituiertes C₁-C₈-Alkyl-NH₂, C₁-C₈-Alkyl-NR_{Q}⁷R_{Q}⁸, C₁-C₈-Alkyl-CO-NR_{Q}⁷R_{Q}⁸, C₁-C8-Alkyl-SO₂NR_{Q}⁷R_{Q}⁸, C₁-C₈-Alkyl-CO-NH₂, C₁-C₈-Alkyl-SO₂NH₂, CO-NH₂, CO-NR_{Q}⁷R_{Q}⁸, SO₂NH₂, SO₂NR_{Q}⁷R_{Q}⁸, NR_{Q}⁷R_{Q}⁸;
         **7.)** einen 4-7-gliedrigen mono- oder bicyclischen gesättigten oder ungesättigten Heterocyclus, der bis zu zwei verschiedene oder gleiche Heteroatome aus der Gruppe O, N oder S enthalten kann, wobei dieser Cyclus ebenfalls mehrfach substituiert sein kann. Für den Fall, dass der Heterocyclus ein N-Atom enthält, kann dies mit einem Rest R_{Q}⁷ substituiert sein.
2. Guanidinverbindung der allgemeinen Formel I 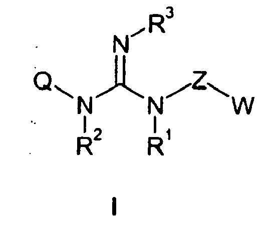 entsprechende enantiomere, diastereomere und/oder tautomere Formen davon sowie pharmazeutisch annehmbare Salze davon, wobei die angegebenen Reste die folgenden Definitionen besitzen:
   **W:**
      einen Rest der allgemeinen Formel **W1** oder **W2** 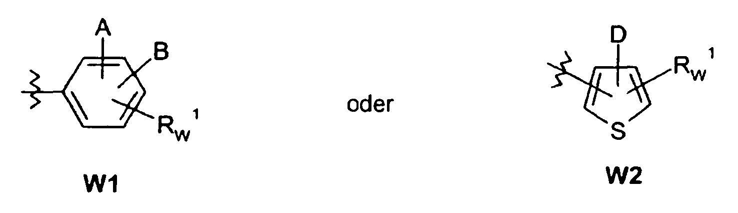 worin
   **A:**
      NO₂. NH₂. OH, CN, CF₃, OCF₃, CHF₂, OCHF₂, COOH, O-CH₂-COOH, Halogen, SH, oder
      jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Hetaryl oder C₁-C₄-AlkylenAryl, oder
      O-R_{A}¹, CO-R_{A}¹, S-R_{A}¹, SO-R_{A}¹, CO-O-R_{A}¹, NR_{A}⁴-CO-O-R_{A}¹, O-CH₂-COO-R_{A}¹, NR_{A}²R_{A}³, CONH₂, SO₂NH₂, NR_{A}⁴-CO-R_{A}¹, SO₂-R_{A}¹, NR_{A}⁴-SO₂-R_{A}¹, SO₂-NR_{A}²R_{A}³ oder CO-NR_{A}²R_{A}³;
   R_{A}¹:
      jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₂-C₆-Alkenylen-Aryl oder C₁-C₆-Alkylen-Hetaryl;
   **R_{A}²:**
      Wasserstoff, OH, CN, oder
      jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
   **R_{A}³:**
      jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, **CO-O-Hetaryl,** CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
      oder die Reste **R_{A}²** und **R_{A}³** zusammen mit dem Stickstoff einen 3 bis 7-gliedrigen, gegebenenfalls substituierten, gesättigten oder aromatischen Hetero-cyclus, der ein, zwei oder drei weitere verschiedene oder gleiche Heteroatome aus der Gruppe O, N, S enthalten kann, bilden; wobei gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und wobei der dadurch gebildete Cyclus gegebenenfalls substituiert sein kann oder an diesem Cyclus ein weiterer, gegebenenfalls substituierter Cyclus ankondensiert sein kann;
   **R_{A}⁴:**
      Wasserstoff, oder
      jeweils gegebenenfalls substituiertes C₁-C₆-Alkyl, C₁-C₆-Alkylen-O-C₁-C₆-Alkyl. C₂-C₆-Alkenyl, C₃-C₁₂-Alkinyl, CO-C₁-C₆-Alkyl, CO-O-C₁-C₆-Alkyl, SO₂-C₁-C₆-Alkyl, C₃-C₇-Cycloalkyl, Aryl, C₁-C₄-Alkylen-Aryl, CO-O-Arylalkyl, CO-C₁-C₄-Alkylen-Aryl, CO-Aryl, SO₂-Aryl, Hetaryl, CO-Hetaryl oder SO₂-C₁-C₄-AlkylenAryl;
   **B:**
      Wasserstoff oder wie Rest **A** definiert ist,
      oder jeweils unabhängig voneinander zwei der Reste **A**, **B** oder **R_{w}¹** zusammen einen 3 bis 7-gliedrigen, gegebenenfalls substituierten, gesättigten oder ungesättigten Carbocyclus oder einen gegebenenfalls substituierten, gesättigten oder ungesättigten oder aromatischen Heterocyclus, der ein, zwei oder drei weitere verschiedene oder gleiche Heteroatome aus der Gruppe O, N, S enthalten kann, bilden; wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
   **R_{w}¹:**
      Wasserstoff, OH, Halogen, NO₂, NH₂, CN, CF₃, CHF₂, O-CF₃, O-CHF₂, oder
      jeweils gegebenenfalls substituiertes C₁-C₆-Alkyl, C₃-C₇-Cycloalkyl, C₁-C₆-Alkylen-O-C₁-C₆-Alkyl, C₁-C₆-Thioalkyl, Aryl, Hetaryl, O-C₁-C₆-Alkyl, O-Aryl, O-Benzyl, C₁-C₆-Alkylamino, C₁-C₆-Dialkylamino, Pyrrolidinyl, Piperidinyl, Morpholinyl, CO-C₁-C₆-Alkyl, SO₂-C₁-C₆-Alkyl. CO-Aryl, SO₂-Aryl, CO-C₁-C₄-AlkylenAryl, SO₂-C₁-C₄-Alkylen-Aryl, SO-Aryl, CONH₂, CONH-C₁-C₆-Alkyl, SO₂NH-C₁-C₆-Alkyl, CON-(C₁-C₆-Alkyl)₂, SO₂N-(C₁-C₆-Alkyl)₂, NH-SO₂-C₁-C₆-Alkyl oder NH-CO-C₁-C₆-Alkyl;
   **D:**
      wie Rest **A** definiert ist;
   **Z:**
      einen Rest der allgemeinen Formel Z1 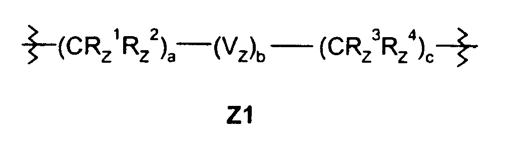 mit den Indizes
         a = 0 - 4
         b = 0 , 1
         c = 0 - 4
         wobei die Summe aus a, b und c mindestens 1 und höchstens 5 beträgt;
   **R_{z}¹, R_{z}², R_{z}³, R_{z}⁴** unabhängig voneinander:
      Wasserstoff, Halogen, OH, oder
      jeweils gegebenenfalls substituiertes C₁-C₆-Alkyl, C₂-C₆-Alkenyl, C₂-C₆-Alkinyl, C₁-C₆-Alkylen-C₃-C₇-Cycloalkyl, C₃-C₇-Cycloalkyl, Aryl, C₁-C₄-Alkylen-Aryl, Hetaryl oder C₁-C₄-Alkylen-Hetaryl, oder
      jeweils unabhängig voneinander zwei Reste **R_{z}¹** und R_{z}² oder **R_{z}³** und **R_{z}⁴** zusammen einen 3 bis 7-gliedrigen, gegebenenfalls substituierten, gesättigten oder ungesättigten Carbo- oder Heterocyclus bilden, wobei der Heterocyclus bis zu drei Heteroatome aus der Gruppe O, N oder S enthalten kann;
   **V_{z}:**
      -CO-, -CO-NR_{z}⁵-, -NR_{z}⁵-CO-, -O-, -S-, -SO-, -SO₂-, -SO₂-NR_{z}⁵-, -NR_{z}⁵-SO₂-, -CS-, -CS-NR_{z}⁵-, -NR_{z}⁵-CS-, -CS-O-, -O-CS-, -CO-O-, -O-CO-, -O-, Ethinylen, -C(=CR_{z}⁶R_{z}⁷)-, -CR_{z}⁶=CR_{z}⁷-, -NR_{z}⁵-CO-NR_{z}^{5*}-, -O-CO-NR_{z}⁵-, -NR_{z}⁵-;
   **R_{z}⁵, R_{z}⁵*** unabhängig voneinander:
      Wasserstoff oder
      jeweils gegebenenfalls substituiertes C₁-C₆-Alkyl, C₁-C₆-Alkylen-O-C₁-C₆-Alkyl, C₂-C₆-Alkenyl, C₃-C₁₂-Alkinyl, CO-C₁-C₆-Alkyl, CO-O-C₁-C₆-Alkyl, SO₂-C₁-C₆-Alkyl, C₃-C₇-Cycloalkyl, Aryl, C₁-C₄-Alkylen-Aryl, CO-O-C₁-C₄-Alkylen-Aryl, CO-C₁-C₄-Alkylen-Aryl, CO-Aryl, SO₂-Aryl, Hetaryl, CO-Hetaryl oder SO₂-C₁-C₄-Alkylen-Aryl;
   **R_{z}⁶, R_{z}⁷** unabhängig voneinander:
      Wasserstoff, OH oder
      jeweils gegebenenfalls substituiertes C₁-C₆-Alkyl, C₁-C₄-Alkoxy, C₂-C₆-Alkenyl, C₂-C₆-Alkinyl, C₁-C₆-Alkylen-C₃-C₇-Cycloalkyl, C₃-C₇-Cycloalkyl, Aryl, C₁-C₄-Alkylen-Aryl, Hetaryl oder C₁-C₄-Alkylen-Hetaryl;
   **R¹, R², R³** unabhängig voneinander:
      Wasserstoff, OH, CN, oder
      jeweils gegebenenfalls substituiertes C₁-C₆-Alkyl, O-C₁-C₆-Alkyl, C₁-C₆-Alkylen-O-C₁-C₆-Alkyl, C₃-C₇-Cycloalkyl, O-C₃-C₇-Cycloalkyl, Aryl, Hetaryl, C₁-C₄-Alkylen-Aryl, C₁-C₄-Alkylen-Hetaryl, O-Aryl, O-C₁-C₄-Alkylen-Aryl, O-Hetaryl, O-C₁-C₄-Alkylen-Hetaryl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-Hetaryl, CO-O-C₁-C₄-Alkylen-Aryl, SO₂-C₁-C₆-Alkyl, SO₂-Aryl, SO₂-Hetaryl, SO₂-C₁-C₄-Alkylen-Aryl, OCO-C₁-C₆-Alkyl, OCO-Aryl, OCO-Hetaryl, OCO-C₁-C₄-Alkylen-Aryl, OCO-C₁-C₄-Alkylen-Hetaryl, SO₂-C₁-C₆-Alkyl, SO₂-Aryl, SO₂-Hetaryl oder SO₂-C₁-C₄-Alkylen-Aryl, oder
      jeweils unabhängig vom dritten Rest zwei Reste von **R¹, R²** oder **R³** zusammen einen 5 bis 7-gliedrigen, gegebenenfalls substituierten, gesättigten oder ungesättigten Carbocyclus, oder einen gegebenenfalls substituierten, gesättigten oder ungesättigten Heterocyclus, der ein, zwei oder drei weitere verschiedene oder gleiche Heteroatome aus der Gruppe O, N, S enthalten kann, bilden, wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
   **Q**:
      einen zweifach substituierten 5-gliedrigen Hetaryl-Rest, ausgewählt aus Q1 bis **Q6** 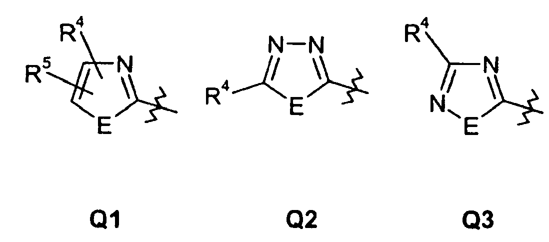 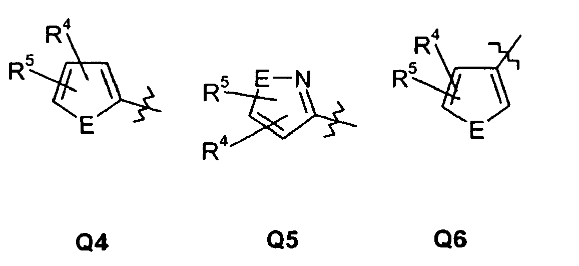
   **E:** O, N-R_{Q}¹ oder S;
   **R_{Q}¹:**
      Wasserstoff, oder
      jeweils gegebenenfalls substituiertes C₁-C₄-Alkyl, CO-C₁-C₄-Alkyl, SO₂-C₁-C₄-Alkyl, CO-O-C₁-C₄-Alkyl, Aryl, C₁-C₄-Alkylen-Aryl, CO-Aryl, CO-Hetaryl, SO₂-Aryl, SO₂-Hetaryl, CO-O-Aryl, CO-C₁-C₄-Alkylen-Aryl, SO₂-C₁-C₄-Alkylen-Aryl oder CO-O-C₁-C₄-Alkylen-Aryl;
   **R⁴, R⁵** jeweils unabhängig voneinander einen Rest, ausgewählt aus den Gruppen **1.), 2.), 3.), 4.)** oder **5.):**
      **1.)** Wasserstoff, Halogen, CN, CF₃, CHF₂, oder
         jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryl, C₁-C₄-Alkylen-Hetaryl, C₁-C₆-Alkylen-O-C₁-C₆-Alkyl, C₁-C₆-Alkylen-O-Aryl, COO-C₁-C₄-Alkyl oder C₁-C₄-Alkylen-COO-C₁-C₄-Alkyl;
      **2.)** Phenyl oder Naphthyl, die jeweils mit **R_{Q}², R_{Q}³** und **R_{Q}⁴** substituiert sind, wobei
         **R_{Q}², R_{Q}³** und **R_{Q}⁴** jeweils unabhängig voneinander einen Substituenten aus der folgenden Gruppe darstellen:
            Wasserstoff, NO₂, NH₂, OH, CN, CF₃, CHF₂, OCF₃, OCHF₂, COOH, O-CH₂-COOH, SH, Halogen, oder
               jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-Alkylen-Hetaryl, oder
               O-R_{Q}⁵, S-R_{Q}⁵, NR_{Q}⁷R_{Q}⁸, CO-OR_{Q}⁶, NR_{Q}⁸-CO-O-R_{Q}⁶, O-CH₂-COO-R_{Q}⁶, NR_{Q}⁸-CO-R_{Q}⁶, SO₂-R_{Q}⁶, NR_{Q}⁸-SO₂-R_{Q}⁶, SO₂NH₂, CONH₂, SO₂-NR_{Q}⁷R_{Q}⁸ oder CO-NR_{Q}⁷R_{Q}⁸, oder jeweils zwei der Reste aus **R_{Q}², R_{Q}³** oder **R_{Q}⁴** zusammen einen 3 bis 7-gliedrigen, gegebenenfalls substituierten, gesättigten, ungesättigten oder aromatischen Carbocyclus oder einen gegebenenfalls substituierten, gesättigten, ungesättigten aromatischen Heterocyclus, der bis zu drei weitere verschiedene oder gleiche Heteroatome O, N, S enthalten kann, bilden, und gegebenenfalls zwei an diesem Heterocyclus substituierte Reste können zusammen einen anellierten, gesättigten, ungesättigten oder aromatischen Carbocyclus oder Heterocyclus bilden, wobei der Heterocyclus bis zu drei verschiedene oder gleiche Heteroatome O, N, S enthalten kann, und der gebildete Cyclus kann gegebenenfalls substituiert sein oder an diesem Cyclus kann ein weiterer, gegebenenfalls substituierter Cyclus ankondensiert sein
         **R_{Q}⁵** jeweils gegebenenfalls substituiertes C₂-C₆-Alkenyl, C₂-C₆-Alkinyl, C₁-C₄-Alkylen-C₃-C₇-Cycloalkyl, C₁-C₄-Alkylen-Heterocycloalkyl, Heterocycloalkyl oder Hetaryl, oder
            C₁-C₆-Alkyl, das gegebenenfalls substituiert ist mit einem Substituenten aus der Gruppe, bestehend aus Halogen, NO_{2,} NH₂, OH, CN, CF₃, CHF₂, OCF₃, OCHF₂, NH-(C₁-C₆-Alkyl) und N(C₁-C₆-Alkyl)₂;
         **R_{Q}⁶** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl oder C₁-C₆-Alkylen-O-C₁-C₆-Alkyl;
         **R_{Q}⁷** Wasserstoff, OH, CN, oder
            jeweils gegebenenfalls substituiertes C₁-C₈-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₃-Alkyl, CO-C₁-C₆-Alkyl, C₁-C₄-Alkylen-Aryl, C₁-C₄-Alkylen-Hetaryl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
         **R_{Q}⁸** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
            oder die Reste **R_{Q}⁷** und **R_{Q}⁸** zusammen mit dem Stickstoff einen 3 bis 7-gliedrigen, gegebenenfalls substituierten, gesättigten oder aromatischen Heterocyclus, der ein, zwei oder drei weitere verschiedene oder gleiche Heteroatome O, N, S enthalten kann, bilden; und gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und der gebildete Cyclus gegebenenfalls substituiert sein kann oder an diesem Cyclus ein weiterer, gegebenenfalls substituierter Cyclus ankondensiert sein kann;
      **3.)** einen 5- oder 6-gliedrigen, gegebenenfalls mit 1 oder 2 Substituenten substituierten Hetaryl-Rest aus der Gruppe, bestehend aus:
         2-Pyrrolyl, 3-Pyrrolyl, 2-Thiazolyl, 4-Thiazolyl, 5-Thiazolyl, 2-Oxazolyl, 4-Oxazolyl, 5-Oxazolyl, 2-Pyrimidyl, 4-Pyrimidyl, 5-Pyrimidyl, 6-Pyrimidyl, 3-Pyrazolyl, 4-Pyrazolyl, 5-Pyrazolyl, 3-Isothiazolyl, 4-Isothiazolyl, 5-Isothiazolyl, 2-Imidazolyl, 4-Imidazolyl, 5-Imidazolyl, 3-Pyridazinyl, 4-Pyridazinyl, 5-Pyridazinyl, 6-Pyridazinyl, 3-Isoxazolyl, 4-Isoxazolyl, 5-Isoxazolyl, Thiadiazolyl, Oxadiazolyl oder Triazinyl oder deren anellierten Derivate Indazolyl, Benzothiophenyl, Benzofuranyl, Indolinyl, Benzimidazolyl, Benzthiazolyl, Benzoxazolyl, Chinolinyl und Isochinolinyl; oder
         gegebenenfalls mit 1 oder 2 Substituenten substituiertes 2-Thienyl oder 3-Thienyl, wobei die Substituenten ausgewählt sind aus der Gruppe, bestehend aus Halogen, NO₂, NH₂, OH, CN, CF₃, OCF₃, CHF₂, O-CHF₂, C₁-C₆-Alkyl, O-C₁-C₆-Alkyl, NH-(C₁-C₆-Alkyl), N(C₁-C₆-Alkyl)₂, NHCO-C₁-C₄-Alkyl, NHSO₂-C₁-C₄-Alkyl und SO₂-C₁-C₄-Alkyl;
      **5.)** beide Reste **R⁴** und **R⁵** zusammen einen 4 bis 7-gliedrigen, gegebenenfalls substituierten, gesättigten oder ungesättigten oder aromatischen Carbocyclus oder einen 5- oder 6-gliedrigen gegebenenfalls substituierten, gesättigten oder ungesättigten oder aromatischen Heterocyclus, der bis zu drei weitere verschiedene oder gleiche Heteroatome O, N, S enthalten kann, bilden, und mit bis zu zwei weiteren Resten substituiert sein kann, wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
      **6.)** einen C₅-C₁₈- bi- oder tricyclischen, gesättigten Kohlenwasserstoffrest.
3. Guanidinverbindung nach Gegenstand 1 oder 2, wobei die angegebenen Reste die folgenden Definitionen besitzen:
   **W: W1;**
   **A:** Halogen, OH, CN, CF₃, CHF₂, OCF₃, OCHF₂, oder
      jeweils gegebenenfalls substituiertes C₁-C₆-Alkyl oder C₂-C₆-Alkenyl, O-CH₂-COO-R_{A}¹, O-R_{A}¹, S-R_{A}¹, NR_{A}²R_{A}³, NR_{A}⁴-CO-R_{A}¹ oder CO-NR_{A}⁴R_{A}¹;
   **R_{A}¹:** jeweils gegebenenfalls substituiertes C₁-C₄-Alkyl, C₃-C₇-Cycloalkyl, Phenyl oder Benzyl;
   **R_{A}²:** Wasserstoff, oder
      jeweils gegebenenfalls substituiertes C₁-C₄-Alkyl, Phenyl, Benzyl, Phenethyl, CO-C₁-C₄-Alkyl, CO-Aryl, CO-O-C₁-C₄-Alkyl, SO₂-C₁-C₄-Alkyl, SO₂-Aryl, SO₂-Hetaryl oder SO₂-C₁-C₄-Alkylen-Aryl;
   **R_{A}³:** jeweils gegebenenfalls substituiertes C₁-C₄-Alkyl, Phenyl, Benzyl, Phenethyl, CO-C₁-C₄-Alkyl, CO-Aryl, CO-O-C₁-C₄-Alkyl, SO₂-C₁-C₄-Alkyl, SO₂-Aryl, SO₂-Hetaryl, oder SO₂-C₁-C₄-Alkylen-Aryl;
      oder die Reste **R_{A}²** und **R_{A}³** zusammen einen gegebenenfalls substituierten 5-oder 6-gliedrigen gesättigten oder ungesättigten Ring, der bis zu zwei gleiche oder verschiedene Heteroatome aus der Gruppe O und N enthalten kann, bilden;
   **R_{A} ⁴:** Wasserstoff oder einen gegebenenfalls substituierten C₁-C₄-Alkylrest;
   **B:** Wasserstoff oder wie Rest **A** definiert ist;
   **Rw¹:** Wasserstoff, F, Cl, CN, CF₃, O-CF₃, oder
      jeweils gegebenenfalls substituiertes C₁-C₄-Alkyl, Aryl, C₁-C₆-Alkylamino oder C₁-C₆-Dialkylamino;
   in der Formel **Z1** die Summe aus a, b und c 1, 2 oder 3 ist;
   **R_{Z}¹. R_{Z}², R_{Z}³, R_{Z}⁴** unabhängig voneinander:
      Wasserstoff, Halogen, OH, gegebenenfalls substituiertes C₁-C₆-Alkyl;
   **V_{Z}:** -CO-, -CO-NR_{z}⁵-, -NR_{z}⁵-CO-, -O-, -S-;
   **R_{Z}⁵:** Wasserstoff, CH₃;
   **R¹, R², R³** unabhängig voneinander:
      Wasserstoff, OH, CN, C₁-C₄-Alkyl, C₁-C₆-Alkylen-O-C₁-C₆-Alkyl, substituiertes Aryl, Benzyl, CO-C₁-C₆-Alkyl, CO-Aryl, CO-C₁-C₄-Alkylen-Aryl, OCO-C₁-C₆-Alkyl, OCO-Aryl oder OCO-C₁-C₄-Alkylen-Hetaryl;
   **Q** ausgewählt ist aus der Gruppe, bestehend aus **Q1, Q2** und **Q3;**
   **R_{Q}¹:** Wasserstoff, gegebenenfalls substituiertes C₁-C₄-Alkyl, im Arylrest gegebenenfalls substituiertes Benzyl, CO-C₁-C₄-Alkyl, gegebenenfalls substituiertes Benzoyl, SO₂-C₁-C₄-Alkyl oder im Arylrest gegebenenfalls substituiertes SO₂-Aryl.
4. Guanidinverbindung nach mindestens einem der Gegenstände 1 bis 3, wobei die angegebenen Reste die folgenden Definitionen besitzen:
   **A:** OH, F, Cl, OCF₃, OCHF₂, gegebenenfalls substituiertes C₁-C₄-Alkyl,O-C₁-C₄-Alkyl oder S-C₁-C₄-Alkyl;
   **B:** Wasserstoff, OH, F, Cl, CF₃, OCF₃, OCHF₂, gegebenenfalls substituiertes C₁-C₄-Alkyl, O-C₁-C₄-Alkyl oder S-C₁-C₄-Alkyl;
      Wasserstoff, F, Cl, CN, CF₃ oder O-CF₃;
   **Z:** jeweils gegebenenfalls substituiertes C₁-C₄-Alkyl oder C₁-C₄-Alkylen-O-C₁-C₄-Alkyl;
   **R_{Z}¹. R_{Z}², R_{Z}³' R_{Z}⁴** jeweils unabhängig voneinander:
      Wasserstoff, F, CH₃;
   **R¹, R², R³** unabhängig voneinander:
      Wasserstoff, OH, CN, O-Methyl, O-Phenyl, Acetyl, Benzoyl, O-Acetyl, O-Benzoyl;
   **Q** ist ausgewählt aus der Gruppe, bestehend aus 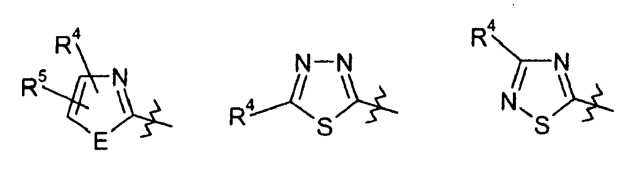
   **R_{Q}¹:** Wasserstoff, CH₃, Methansulfonyl, Phenylsulfonyl oder Tosyl.
5. Guanidinverbindung nach mindestens einem der Gegenstände 1 bis 4, wobei die angegebenen Reste die folgenden Definitionen besitzen:
   **A:** OH, OCF₃, OCH₃, O-Ethyl, O-Propyl oder O-iPropyl;
   **Z:** -CH₂-, -CH₂-O-, -CH₂-CH₂- oder -CH₂-CH₂-O-;
   jeweils zwei der Reste **R¹, R²,** oder **R³** Wasserstoff sind, und der dritte Rest Wasserstoff, OH, Acetyl oder Benzoyl ist;
   **Q:** 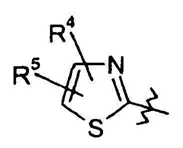
6. Guanidinverbindung nach mindestens einem der Gegenstände 1 bis 5, wobei R4 und/oder **R⁵** jeweils unabhängig voneinander einen Rest ausgewählt aus den Gruppen **1.), 2.), 3.), 4.)** oder **5.)** darstellen:
   **1.)** Wasserstoff, F, Cl, CN, CF₃, oder
      jeweils gegebenenfalls substituiertes C₁-C₆-Alkyl, C₂-C₆-Alkenyl, C₁-C₆-Alkylen-O-C₁-C₆-Alkyl oder C₃-C₇-Cycloalkyl;
   **2.) R_{Q}¹, R_{Q}²** und **R_{Q}³** unabhängig voneinander
      Wasserstoff, CN, CF₃, CHF₂, OCF₃, OCHF₂, F, Cl, OH oder
      jeweils gegebenenfalls substituiertes Phenyl oder Hetaryl, C₁-C₄-Alkyl, C₅-C₇-Cycloalkyl, O-R_{Q}⁵, NR_{Q}⁷R_{Q}⁸, CO-OR_{Q}⁶, NR_{Q}⁸-CO-O-R_{Q}⁶, O-CH₂-COO-R_{Q}⁶, NR_{Q}⁸-CO-R_{Q}⁶, SO₂-R_{Q}⁶, NR_{Q}⁸-SO₂-R_{Q}⁶, NR_{Q}⁸-CO-O-R_{Q}⁶, SO₂NH₂, CONH₂, SO₂-NR_{Q}⁷R_{Q}⁸ oder CO-NR_{Q}⁷R_{Q}⁸;
      **R_{Q}⁵:** C₁-C₄-Alkyl, das gegebenenfalls substituiert ist mit einem Substituenten aus der Gruppe, bestehend aus F, Cl, OH, CN, CF₃, OCF₃, NH-(C₁-C₄-Alkyl) und N(C₁-C₄-Alkyl)_{2;}
      **R_{Q}⁶:** jeweils gegebenenfalls substituiertes C₁-C₃-Alkyl, Aryl, Hetaryl oder Phenyl;
      **R_{Q}⁷_{:}** Wasserstoff, jeweils gegebenenfalls substituiertes C₁-C₄-Alkyl, Allyl, Aryl, Hetaryl, Benzyl, Phenethyl oder CH₂-Hetaryl;
      **R_{Q}⁸_{:}** jeweils gegebenenfalls substituiertes C₁-C₄-Alkyl, Allyl, Aryl, Hetaryl, Benzyl, Phenethyl oder CH₂-Hetaryl;
      oder R_{Q}⁷ und R_{Q}⁸ bilden einen gegebenenfalls substituierten 3-oder 7-gliedrigen gesättigten oder ungesättigten Ring, der bis zu zwei gleiche oder verschiedene Heteroatome aus der Gruppe O und N enthalten kann;
   **3.)** Benzothiophenyl, Benzofuranyl, Chinolinyl oder Isochinolinyl;
   **4.)** beide Reste **R⁴** und **R⁵** zusammen einen der folgenden Ringe bilden: 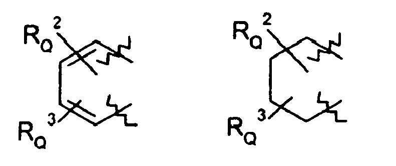 wobei R_{Q}² und R_{Q}³ wie unter **2.)** definiert sind;
   **5.)** Adamantyl.
7. Guanidinverbindung nach Gegenstand 1 oder 2, wobei die angegebenen Reste die folgenden Definitionen besitzen:
   **W: W1;**
   **A:** Halogen, OH, CN, CF₃, CHF₂, OCF₃, OCHF₂, oder
      jeweils gegebenenfalls substituiertes C₁-C₆-Alkyl oder C₂-C₆-Alkenyl, O-CH₂-COO-R_{A}¹, O-R_{A}¹, S-R_{A}¹, NR_{A}²R_{A}³, NR_{A}⁴-CO-R_{A}¹, SO₂NH₂, NR_{A}⁴-SO₂-R_{A}¹, SO₂-NR_{A}²R_{A}³ oder CO-NR_{A}⁴R_{A}¹;
   **R_{A}¹:** jeweils gegebenenfalls substituiertes C₁-C₄-Alkyl, C₃-C₇-Cycloalkyl, Phenyl oder Benzyl;
   **R_{A}²_{:}** Wasserstoff, oder
      jeweils gegebenenfalls substituiertes C₁-C₄-Alkyl, Phenyl, Benzyl, Phenethyl, CO-C₁-C₄-Aklyl, CO-Aryl, CO-O-C₁-C₄-Alkyl, SO₂-C₁-C₄-Alkyl, SO₂-Aryl, SO₂-Hetaryl oder SO₂-C₁-C₄-Alkylen-Aryl;
   **R_{A}**³: jeweils gegebenenfalls substituiertes C₁-C₄-Alkyl, Phenyl, Benzyl, Phenethyl, CO-C₁-C₄-Alkyl, CO-Aryl, CO-O-C₁-C₄-Alkyl, SO₂-C₁-C₄-Alkyl, SO₂-Aryl, SO₂-Hetaryl, oder SO₂-C₁-C₄-Alkylen-Aryl;
      oder die Reste **R_{A}²** und **R_{A}³** zusammen einen gegebenenfalls substituierten 5-oder 6-gliedrigen gesättigten oder ungesättigten Ring, der bis zu zwei gleiche oder verschiedene Heteroatome aus der Gruppe O und N enthalten kann, bilden;
   **R_{A}⁴:** Wasserstoff oder einen gegebenenfalls substituierten C₁-C₄-Alkylrest;
   **B:** Wasserstoff oder wie Rest A definiert ist;
   **R_{w}¹:** Wasserstoff, F, Cl, CN, CF₃, O-CF₃, oder
      jeweils gegebenenfalls substituiertes C₁-C₄-Alkyl, Aryl, C₁-C₆-Alkylamino oder C₁-C₆-Dialkylamino;
   in der Formel **Z1** die Summe aus a, b und c 1, 2 oder 3 ist;
   **R_{Z}¹, R_{Z}², R_{Z}^{3,} R_{Z}⁴** unabhängig voneinander:
      Wasserstoff, Halogen, OH, gegebenenfalls substituiertes C₁-C₃,-Alkyl;
   **V_{z}:** -CO-, -CO-NR_{z}⁵-, -NR_{Z}⁵-CO-, -O-, -S-;
   **R_{z}⁵:** Wasserstoff, CH₃;
   **_{R}¹, R², R³** unabhängig voneinander:
      Wasserstoff, OH, CN, C₁-C₄-Alkyl, C₁-C₆-Alkylen-O-C₁-C₆-Alkyl, substituiertes Aryl, Benzyl, CO-C₁-C₆-Alkyl, CO-Aryl, CO-C₁-C₄-Alkylen-Aryl, OCO-C₁-C₆-Alkyl, OCO-Aryl oder OCO-C₁-C₄-Alkylen-Hetaryl;
   **Q** ausgewählt ist aus der Gruppe, bestehend aus **Q1, Q2, Q3** und **Q5;**
   **R_{Q}¹:** Wasserstoff, gegebenenfalls substituiertes C₁-C₄-Alkyl, im Arylrest gegebenenfalls substituiertes Benzyl, CO-C₁-C₄-Alkyl, gegebenenfalls substituiertes Benzoyl, SO₂-C₁-C₄-Alkyl oder im Arylrest gegebenenfalls substituiertes SO₂-Aryl.
8. Guanidinverbindung nach mindestens einem der Gegenstände 1, 2 oder 7, wobei die angegebenen Reste die folgenden Definitionen besitzen:
   **A:** OH, F, Cl, OCF₃, OCHF₂, gegebenenfalls substituiertes C₁-C₄-Alkyl, O-C₁-C₄-Alkyl oder S-C₁-C₄-Alkyl;
   **B:** Wasserstoff, OH, F, Cl, CF₃, OCF₃, OCHF₂, gegebenenfalls substituiertes C₁-C₄-Alkyl, O-C₁-C₄-Alkyl oder S-C₁-C₄-Alkyl;
   **R_{W}¹:** Wasserstoff, F, Cl, CN, CF₃ oder O-CF₃;
   **Z:** jeweils gegebenenfalls substituiertes C₁-C₄-Alkyl oder C₁-C₄-Alkylen-O-C₁-C₄-Alkyl;
   **R_{Z}¹, R_{Z}², R_{Z}^{3,} R_{Z}⁴** jeweils unabhängig voneinander:
      Wasserstoff, F, CH₃;
   **R¹, R², R³** unabhängig voneinander:
      Wasserstoff, OH, CN, O-Methyl, O-Phenyl, Acetyl, Benzoyl, O-Acetyl, O-Benzoyl;
   Q ist ausgewählt aus der Gruppe, bestehend aus 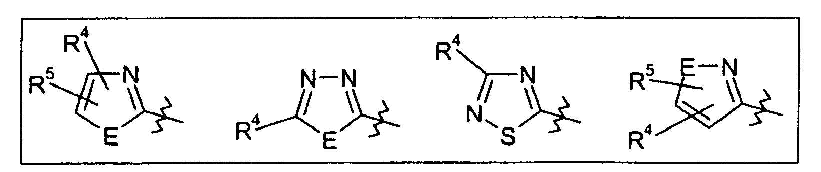
   **R_{Q}¹:** Wasserstoff, CH₃, Phenyl, Benzyl, Methansulfonyl, Phenylsulfonyl oder Tosyl.
9. Guanidinverbindung nach mindestens einem der Gegenstände 1, 2, 7 oder 8, wobei die angegebenen Reste die folgenden Definitionen besitzen:
   **A:** OH, OCF₃, OCH₃, O-Ethyl, O-Propyl oder O-iPropyl;
   **Z:** -CH₂-, -CH₂-O-, -CH₂-CH₂- oder -CH₂-CH₂-O-;
      jeweils zwei der Reste **R¹, R²,** oder **R³** Wasserstoff sind, und der dritte Rest Wasserstoff, OH, Acetyl oder Benzoyl ist;
   **Q:** 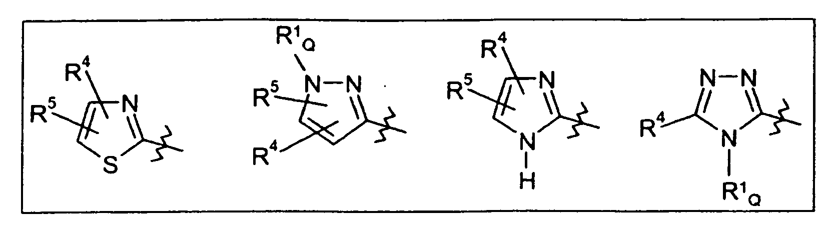
   **R_{Q}¹:** Wasserstoff, CH₃, Phenyl, Benzyl, Methansulfonyl, Phenylsulfonyl oder Tosyl.
10. Guanidinverbindung nach mindestens einem der Gegenstände 1, 2 oder 7 bis 9, wobei **R⁴** und/oder **R⁵** jeweils unabhängig voneinander einen Rest ausgewählt aus den Gruppen **1.), 2.), 3.), 4.), 5.)** oder **7.)** darstellen:
   **1.)** Wasserstoff, F, Cl, CN, CF₃, oder
      jeweils gegebenenfalls substituiertes C₁-C₆-Alkyl, C₂-C₆-Alkenyl, C₁-C₆-Alkylen-O-C₁-C₆-Alkyl oder C₃-C₇-Cycloalkyl;
   **2.) R_{Q}1, R_{Q}² und R_{Q}³** unabhängig voneinander
      Wasserstoff, CN, CF₃, CHF₂, OCF₃, OCHF₂, F, Cl, OH oder
      jeweils gegebenenfalls substituiertes Phenyl oder Hetaryl, C₁-C₄-Alkyl, C₅-C₇-Cycloalkyl, O-R_{Q}⁵, NR_{Q}⁷R_{Q}⁸, CO-OR_{Q}⁶, NR_{Q}⁸-CO-O-R_{Q}⁶, O-CH₂-COO-R_{Q}⁶, NR_{Q}⁸-CO-R_{Q}⁶, SO₂-R_{Q}⁶, NR_{Q}⁸-SO₂-R_{Q}⁶, NR_{Q}⁸-CO-O-R_{Q}⁶, SO₂NH₂, CONH₂, SO₂-NR_{Q}⁷R_{Q}⁸ oder CO-NR_{Q}⁷R_{Q}⁸;
      **R_{Q}⁵:** C₁-C₄-Alkyl, das gegebenenfalls substituiert ist mit einem Substituenten aus der Gruppe, bestehend aus F, Cl, OH, CN, CF₃, OCF₃, NH-(C₁-C₄-Alkyl) und N(C₁-C₄-Alkyl)₂;
      **R_{Q}⁶:** jeweils gegebenenfalls substituiertes C₁-C₆-Alkyl, Aryl, Hetaryl oder Phenyl;
      **R_{Q}⁷:** Wasserstoff, jeweils gegebenenfalls substituiertes C₁-C₄-Alkyl, Allyl, Aryl, Hetaryl, Benzyl, Phenethyl oder CH₂-Hetaryl;
      **R_{Q}⁸:** Wasserstoff, jeweils gegebenenfalls substituiertes C₁-C₄-Alkyl, Allyl, Aryl, Hetaryl, Benzyl, Phenethyl oder CH₂-Hetaryl;
      oder R_{Q}⁷ und R_{Q}⁸ bilden einen gegebenenfalls substituierten 3-oder 7-gliedrigen gesättigten oder ungesättigten Ring, der bis zu zwei gleiche oder verschiedene Heteroatome aus der Gruppe O und N enthalten kann;
   **3.)** Benzothiophenyl, Benzofuranyl, Chinolinyl oder Isochinolinyl;
   **4.)** beide Reste **R⁴** und **R⁵** zusammen einen der folgenden Ringe bilden: 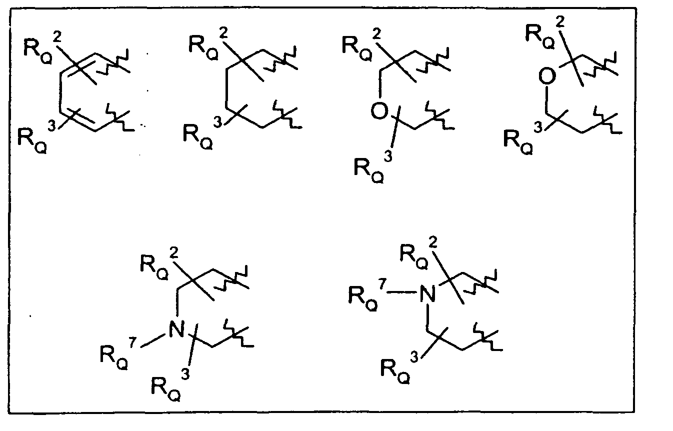 wobei R_{Q}² und R_{Q}³ wie unter **2.)** definiert sind; ; oder zusammen einen annelierten 5- oder 6-gliedrigen Ring bilden können;
   **5.)** Adamantyl;
   **7.)** jeweils gegebenenfalls substituiertes Azetidin-3-yl, Pyrrolidin-2-yl, Pyrrolidin-3-yl, Piperidin-2-yl, Piperidin-3-yl, Piperidin-4-yl, Tetrahydro-2H-pyran-4-yl, Tetrahydrofuran-3-yl, Azepan-4-yl, Azepan-3-yl, Azepan-2-yl, 1,4-Diazepan-5-yl, 1,2,3,6-Tetrahydropyridin-4-yl, 2,5-Dihydro-1H-pyrrol-3-yl.
11. Guanidinverbindung nach mindesten einem der Gegenstände 1 bis 10, wobei ein Rest von **R⁴** und **R⁵** ausgewählt wird aus Gruppe 1.), und der andere Rest von **R⁴** und **R⁵** ausgewählt wird aus Gruppe 1.), 2.) oder 3.).
12. Guanidinverbindung nach mindestens einem der Gegenstände 1 bis 11 als Arzneimittel.
13. Pharmazeutische Zusammensetzung, enthaltend mindestens eine Guanidinverbindung nach einem der Gegenstände 1 bis 12 sowie einen pharmazeutisch annehmbaren Träger oder Verdünnungsmittel.
14. Verwendung von Verbindungen der allgemeinen Formel IVA zur Herstellung von 5HT5A-Rezeptorliganden:

   W - Z - NH₂ **IVA**
15. Verwendung nach Gegenstand 14 zur Herstellung der Guanidinverbindungen nach einem der Gegenstände 1 bis 12.
16. Verwendung einer Guanidinverbindung der allgemeinen Formel **IA** 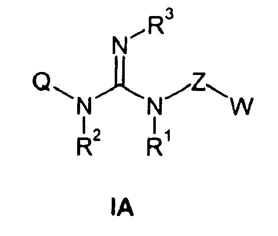 der entsprechenden enantiomeren, diastereomeren und/oder tautomeren Formen davon sowie pharmazeutisch annehmbare Salze davon,
   wobei die angegebenen Reste die folgenden Definitionen besitzen:
   **W:**
      einen Rest der allgemeinen Formel **W1** oder **W2** 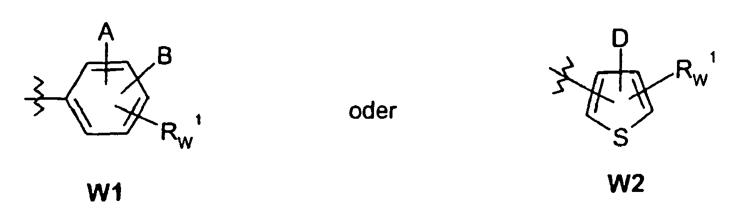
   **A:**
      NO₂, NH₂, OH, CN, CF₃, OCF₃, CHF₂, OCHF₂, COOH, O-CH₂-COOH, Halogen, SH, oder
         jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl oder C₁-C₄-Alkylen-Heterocycloalkyl, Aryl, Hetaryl, Heterocycloalkyl, C₁-C₄-Alkylen-Hetaryl oder C₁-C₄-Alkylen-Aryl, oder
         O-R_{A}¹, CO-R_{A}¹, S-R_{A}¹, SO-R_{A}¹, CO-O-R_{A}¹, NR_{A}⁴-CO-O-R_{A}¹, O-CH₂-COO-R_{A}¹, NR_{A}²R³, CONH₂, SO₂NH₂, NR_{A}⁴-CO-R_{A}¹, SO₂-R_{A}¹, NR_{A}⁴-SO₂-R_{A}¹, SO₂-NR_{A}²R_{A}³ oder CO-NR_{A}²R_{A}³;
   **R_{A}¹:**
      jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₂-C₆-Alkenylen-Aryl oder C₁-C₆-Alkylen-Hetaryl;
   **R_{A}2:**
      Wasserstoff, OH, CN, oder
         jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
   **R_{A}³_{:}**
      jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
      oder die Reste **R_{A}²** und **R_{A}³** zusammen mit dem Stickstoff einen 3 bis 7-gliedrigen, gegebenenfalls substituierten, gesättigten oder aromatischen Hetero-cyclus, der ein, zwei oder drei weitere verschiedene oder gleiche Heteroatome aus der Gruppe O, N, S enthalten kann, bilden; wobei gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und wobei der dadurch gebildete Cyclus gegebenenfalls substituiert sein kann oder an diesem Cyclus ein weiterer, gegebenenfalls substituierter Cyclus ankondensiert sein kann;
   **R_{A}⁴:**
      Wasserstoff, oder
         jeweils gegebenenfalls substituiertes C₁-C₆-Alkyl, C₁-C₆-Alkylen-O-C₁-C₆-Alkyl, C₂-C₆-Alkenyl, C₃-C₁₂-Alkinyl, CO-C₁-C₆-Alkyl, CO-O-C₁-C₆-Alkyl, SO₂-C₁-C₆-Alkyl, C₃-C₇-Cycloalkyl, Aryl, C₁-C₄-Alkylen-Aryl, CO-O-Arylalkyl, CO-C₁-C₄-Alkylen-Aryl, CO-Aryl, SO₂-Aryl, Hetaryl, CO-Hetaryl oder SO₂-C₁-C₄-AlkylenAryl;
   **B:**
      Wasserstoff oder wie Rest **A** definiert ist,
      oder jeweils unabhängig voneinander zwei der Reste **A, B** oder **R_{W}¹** zusammen einen 3 bis 7-gliedrigen, gegebenenfalls substituierten, gesättigten oder ungesättigten Carbocyclus oder einen gegebenenfalls substituierten, gesättigten oder ungesättigten oder aromatischen Heterocyclus, der ein, zwei oder drei weitere verschiedene oder gleiche Heteroatome aus der Gruppe O, N, S enthalten kann, bilden; wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
   **R_{W}¹:**
      Wasserstoff, OH, Halogen, NO₂, NH₂, CN, CF₃, CHF₂, O-CF₃, O-CHF₂, oder jeweils gegebenenfalls substituiertes C₁-C₆-Alkyl, C₃-C₇-Cycloalkyl, C₁-C₆-Alkylen-O-C₁-C₆-Alkyl, C₁-C₆-Alkylen-S-C₁-C₆-Alkyl, Aryl, Hetaryl, O-C₁-C₆-Alkyl, O-Aryl, O-Benzyl, C₁-C₆-Alkylamino, C₁-C₆-Dialkylamino, Pyrrolidinyl, Piperidinyl, Morpholinyl, CO-C₁-C₆-Alkyl, SO₂-C₁-C₆-Alkyl, CO-Aryl, SO₂-Aryl, CO-C₁-C₄-Alkylen-Aryl, SO₂-C₁-C₄-Alkylen-Aryl, SO-Aryl, CONH₂, CONH-C₁-C₆-Alkyl, SO₂NH-C₁-C₆-Alkyl, CON(C₁-C₆-Alkyl)₂, SO₂N(C₁-C₆-Alkyl)₂, NH-SO₂-C₁-C₆-Alkyl oder NH-CO-C₁-C₆-Alkyl;
   **D:**
      wie Rest **A** definiert ist;
   **Z:**
      einen Rest der allgemeinen Formel **Z1** 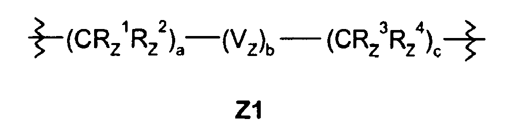 mit den Indizes
         a = 0 - 4
         b = 0, 1
         c = 0 - 4
         wobei die Summe aus a, b und c höchstens 5 beträgt;
   **R_{Z}¹, R_{z}², R_{Z}³, R_{Z}⁴** unabhängig voneinander :
      Wasserstoff, Halogen, OH, oder
      jeweils gegebenenfalls substituiertes C₁-C₆-Alkyl, C₂-C₆-Alkenyl, C₂-C₆-Alkinyl, C₁-C₆-Alkylen-C₃-C₇-Cycloalkyl, C₃-C₇-Cycloalkyl, Aryl, C₁-C₄-Alkylen-Aryl, Hetaryl oder C₁-C₄-Alkylen-Hetaryl, oder
      jeweils unabhängig voneinander zwei Reste **R_{z}¹** und **R_{Z}²** oder **R_{z}³** und **R_{z}⁴** zusammen einen 3 bis 7-gliedrigen, gegebenenfalls substituierten, gesättigten oder ungesättigten Carbo- oder Heterocyclus, der bis zu drei Heteroatome aus der Gruppe O, N oder S enthalten kann, bilden;
   **V_{Z}**:
      -CO-, -CO-NR_{z}⁵-, -NR_{z}⁵-CO-, -O-, -S-, -SO-, -SO₂-, -SO₂-NR_{z}⁵-, -NR_{z}⁵-SO₂-, -CS-, -CS-NR_{z}⁵-, -NR_{z}⁵-CS-, -CS-O-, -O-CS-, -CO-O-, -O-CO-, -O-, Ethinylen, -C(=CR_{Z}⁶R_{z}⁷)-, -CR_{Z}⁶=CR_{Z}⁷-, -NR_{z}⁵-CO-NR_{z}^{5*}-, -O-CO-NR_{z}⁵-, -NR_{z}⁵-;
   **R_{z}⁵, R_{z}⁵*** unabhängig voneinander:
      Wasserstoff, oder
         jeweils gegebenenfalls substituiertes C₁-C₆-Alkyl, C₁-C₆-Alkylen-O-C₁-C₆-Alkyl, C₂-C₆-Alkenyl, C₃-C₁₂-Alkinyl, CO-C₁-C₆-Alkyl, CO-O-C₁-C₆-Alkyl, SO₂-C₁-C₆-Alkyl, C₃-C₇-Cycloalkyl, Aryl, C₁-C₄-Alkylen-Aryl, CO-O-C₁-C₄-Alkylen-Aryl, CO-C₁-C₄-Alkylen-Aryl, CO-Aryl, SO₂-Aryl, Hetaryl, CO-Hetaryl oder SO2-C₁C₄-Alkylen-Aryl;
   **R_{z}⁶, R_{z}⁷** unabhängig voneinander:
      Wasserstoff, OH, oder
         jeweils gegebenenfalls substituiertes C₁-C₆-Alkyl, C₁-C₄-Alkoxy, C₂-C₆-Alkenyl, C₂-C₆-Alkinyl, C₁-C₆-Alkylen-C₃-C₇-Cycloalkyl, C₃-C₇-Cycloalkyl, Aryl, C₁-C₄-Alkylen-Aryl, Hetaryl oder C₁-C₄-Alkylen-Hetaryl;
   **R¹, R² , R³** unabhängig voneinander:
      Wasserstoff, OH, CN, oder
         jeweils gegebenenfalls substituiertes C₁-C₆-Alkyl, O-C₁-C₆-Alkyl, C₁-C₆-Alkylen-O-C₁-C₆-Alkyl, C₃-C₇-Cycloalkyl, O-C₃-C₇-Cycloalkyl, Aryl, Hetaryl, C₁-C₄-Alkylen-Aryl, C₁-C₄-Alkylen-Hetaryl, O-Aryl, O-C₁-C₄-Alkylen-Aryl, O-Hetaryl, O-C₁-C₄-Alkylen-Hetaryl, CO-C₁-C₆-Alkyl, CO-Aryl, CO-Hetaryl, CO-C₁-C₄-AlkylenAryl, CO-C₁-C₄-Alkylen-Hetaryl, CO-O-C₁-C₆-Alkyl, CO-O-Aryl, CO-O-Hetaryl, CO-O-C₁-C₄-Alkylen-Aryl, SO₂-C₁-C₆-Alkyl, SO₂-Aryl, SO₂-Hetaryl, SO₂-C₁-C₄-Alkylen-Aryl, OCO-C₁-C₆-Alkyl, OCO-Aryl, OCO-Hetaryl, OCO-C₁-C₄-AlkylenAryl, OCO-C₁-C₄-Alkylen-Hetaryl, SO₂-C₁-C₆-Alkyl, SO₂-Aryl, SO₂-Hetaryl oder SO₂-C₁-C₄-Alkylen-Aryl, oder
      jeweils unabhängig vom dritten Rest zwei Reste von **R¹, R²** oder **R³** zusammen einen 5 bis 7-gliedrigen, gegebenenfalls substituierten, gesättigten oder ungesättigten Carbocyclus, oder einen gegebenenfalls substituierten, gesättigten oder ungesättigten, der ein, zwei oder drei weitere verschiedene oder gleiche Heteroatome aus der Gruppe O, N, S enthalten kann, bilden, wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyctus gegebenenfalls substituiert sein kann oder an diesem Cyclus ein weiterer, gegebenenfalls substituierter Cyclus ankondensiert sein kann;
   **Q:**
      einen zweifach substituierten 5-gliedrigen Hetaryl-Rest, augewählt aus **Q1** bis **Q7** 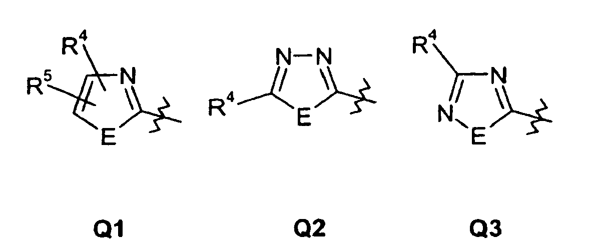 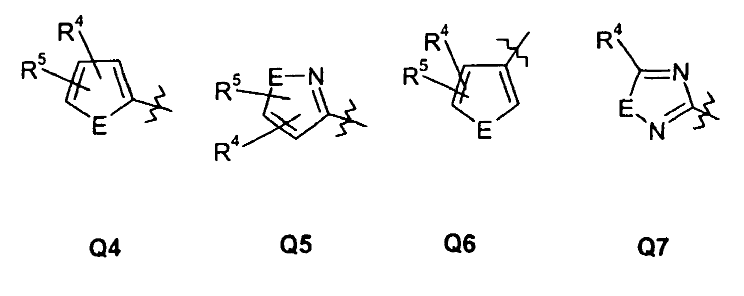
   **E:** O, N-R_{Q}¹ oder S;
   **R_{Q}¹:**
      Wasserstoff, oder
         jeweils gegebenenfalls substituiertes C₁-C₄-Alkyl, CO-C₁-C₄-Alkyl, SO₂-C₁-C₄-Alkyl, CO-O-C₁-C₄-Alkyl, Aryl, C₁-C₄-Alkylen-Aryl, CO-Aryl, CO-Hetaryl, SO₂-Aryl, SO₂-Hetaryl, CO-O-Aryl, CO-C₁-C₄-Alkylen-Aryl, SO₂-C₁-C₄-Alkylen-Aryl oder CO-O-C₁-C₄-Alkylen-Aryl;
   **R⁴, R⁵** jeweils unabhängig voneinander einen Rest ausgewählt aus den Gruppen **1.), 2.), 3.), 4.), 5.), 6.)** oder **7.):**
      **1.)** Wasserstoff, Halogen, CN, CF₃, CHF₂, oder
         jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryl, C₁-C₄-Alkylen-Hetaryl, C₁-C₆-Alkylen-O-C₁-C₆-Alkyl, C₁-C₆-Alkylen-O-Aryl, COO-C₁-C₄-Alkyl oder C₁-C₄-Alkylen-COO-C₁-C₄-Alkyl;
      2.) Phenyl oder Naphthyl, die jeweils mit **R_{Q}², R_{Q}³** und **R_{Q}⁴** substituiert sind, wobei
         **R_{Q}², R_{Q}³** und **R_{Q}⁴** jeweils unabhängig voneinander einen Substituenten aus der folgenden Gruppe darstellen:
            Wasserstoff, NO₂, NH₂. OH, CN, CF₃, CHF₂, OCF₃, OCHF₂, COOH, O-CH₂-COOH, SH, Halogen, oder jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocydoalkyl, C₁-C₄-Alkylen-Aryl oder C₁-C₄-Alkylen-Hetaryl, oder

               O-R_{Q}⁵, S-R_{Q}⁵, NR_{Q}⁷R_{Q}⁸, CO-OR_{Q}⁶, NR_{Q}⁸-CO-O-R_{Q}⁶, O-CH₂-COO-R_{Q}⁶, NR_{Q}⁸-CO-R_{Q}⁶, SO₂-R_{Q}⁶, NR_{Q}⁸-SO₂-R_{Q}⁶, SO₂NH₂, CONH₂, SO₂-NR_{Q}⁷-R_{Q}⁸ oder CO-NR_{Q}⁷R_{Q}⁸, oder
            jeweils zwei der Reste aus **R_{Q}², R_{Q}³** oder **R_{Q}⁴** zusammen einen 3 bis 7-gliedrigen, gegebenenfalls substituierten, gesättigten, ungesättigten Carbocyclus oder einen gegebenenfalls substituierten, gesättigten, ungesättigten aromatischen Heterocyclus, der bis zu drei weitere verschiedene oder gleiche Heteroatome O, N, S enthalten kann, bilden, und gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und der gebildete Cyclus gegebenenfalls substituiert sein kann oder an diesem Cyclus ein weiterer, gegebenenfalls substituierter Cyclus ankondensiert sein kann;
         **R_{Q}⁵** jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Heterocycloalkyl, Aryl oder Hetaryl;
         **R_{Q}⁶** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl oder C₁-C₆-Alkylen-O-C₁-C₆-Alkyl;
         **R_{Q}⁷** Wasserstoff, OH, CN, oder
            jeweils gegebenenfalls substituiertes C₁-C₆-Alkyl, C₂-C₆-Alkenyl, C₂-C₆-Alkinyl, C₃-C₇-Cycloalkl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, C₁-C₄-Alkylen-Aryl, C₁-C₄-Alkylen-Hetaryl, CO-Aryl, CO-Hetaryl, CO-C₁-C₄-Alkylen-Aryl, CO-C₁-C₄-Alkylen-Hetaryl, CO-O-Cₗ-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
         **R_{Q}⁸** Wasserstoff oder
            jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂Aryl, SO₂-Hetaryl, SO₂-C₁-C₄-Alkylen-Aryl oder SO₂-C₁-C₄-Alkylen-Hetaryl,
         oder beide Reste **R_{Q}⁷** und **R_{Q}⁸** zusammen mit dem Stickstoff einen 3 bis 7-gliedrigen, gegebenenfalls substituierten, gesättigten oder aromatischen Heterocyclus, der ein, zwei oder drei weitere verschiedene oder gleiche Heteroatome O, N, S enthalten kann- bilden; und gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und der gebildete Cyclus gegebenenfalls substituiert sein kann oder an diesem Cyclus ein weiterer, gegebenenfalls substituierter Cyclus ankondensiert sein kann;
      **3.)** einen 5- oder 6-gliedrigen, gegebenenfalls mit 1 oder 2 Substituenten substituierten Hetaryl-Rest aus der Gruppe, bestehend aus:
         2-Furyl, 3-Furyl, 2-Pyrrolyl, 3-Pyrrolyl, 2-Thienyl, 3-Thienyl, 2-Pyridyl, 3-Pyridyl, 4-Pyridyl, 2-Thiazolyl, 4-Thiazolyl, 5-Thiazolyl, 2-Oxazolyl, 4-Oxazolyl, 5-Oxazolyl, 2-Pyrimidyl, 4-Pyrimidyl, 5-Pyrimidyl, 6-Pyrimidyl, 3-Pyrazolyl, 4-Pyrazolyl, 5-Pyrazolyl, 3-Isothiazolyl, 4-Isothiazolyl, 5-Isothiazolyl, 2-Imidazolyl, 4-Imidazolyl, 5-Imidazolyl, 3-Pyridazinyl, 4-Pyridazinyl, 5-Pyridazinyl, 6-Pyridazinyl, 3-Isoxazolyl, 4-Isoxazolyl, 5-Isoxazolyl, Thiadiazolyl, Oxadiazolyl oder Triazinyl oder deren anellierten Derivate Indazolyl, Indolyl, Benzothiophenyl, Benzofuranyl, Indolinyl, Benzimidazolyl, Benzthiazolyl, Benzoxazolyl, Chinolinyl und Isochinolinyl;
      **4.)** beide Reste **R⁴** und **R⁵** zusammen einen 4 bis 7-gliedrigen, gegebenenfalls substituierten, gesättigten oder ungesättigten oder aromatischen Carbocyclus oder einen 5- oder 6-gliedrigen gegebenenfalls substituierten, gesättigten oder ungesättigten oder aromatischen Heterocyclus, der bis zu drei weitere verschiedene oder gleiche Heteroatome O, N, S enthalten kann, bilden, und mit bis zu zwei weiteren Resten substituiert sein kann, wobei gegebenenfalls zwei an diesem Carbo- oder Heterocyclus substituierte Reste zusammen einen anellierten, gesättigten, ungesättigten oder aromatischen Carbocyclus
         oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
      **5.)** einen C₆-C₁₀- bi- oder tricyclischen, gesättigten Kohlenwasserstoffrest;
      **6.)** jeweils gegebenenfalls substituiertes C₁-C₈-Alkyl-NH₂, C₁-C₈-Alkyl-NR_{Q}⁷R_{Q}⁸, C₁-C₈-Alkyl-CO-NR_{Q}⁷R_{Q}⁸, C₁-C₈-Alkyl-SO₂NR_{Q}⁷RQ⁸, C₁-C₈-Alkyl-CO-NH₂, C₁-C₈-Alkyl-SO₂NH₂, CO-NH₂, CO-NR_{Q}⁷R_{Q}⁸, SO₂NH₂, SO₂NR_{Q}⁷R_{Q}⁸, NR_{Q}⁷R_{Q}⁸;
      **7.)** einen 4-7-gliedrigen mono- oder bicyclischen gesättigten oder ungesättigten Heterocyclus, der bis zu zwei verschiedene oder gleiche Heteroatome aus der Gruppe O, N oder S enthalten kann, wobei dieser Cyclus ebenfalls mehrfach substituiert sein kann. Für den Fall, dass der Heterocyclus ein N-Atom enthält, kann dies mit einem Rest R_{Q}⁷ substituiert sein.
      zur Herstellung eines Medikaments zur Behandlung von Krankheiten, die durch eine 5-HT5-Rezeptoraktivität moduliert werden.
17. Verwendung einer Guanidinverbindung der allgemeinen Formel **IA** 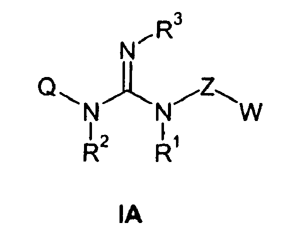 der entsprechenden enantiomeren, diastereomeren und/oder tautomeren Formen davon sowie pharmazeutisch annehmbare Salze davon,
   wobei die angegebenen Reste die folgenden Definitionen besitzen:
   **W:**
      einen Rest der allgemeinen Formel **W1** oder **W2** 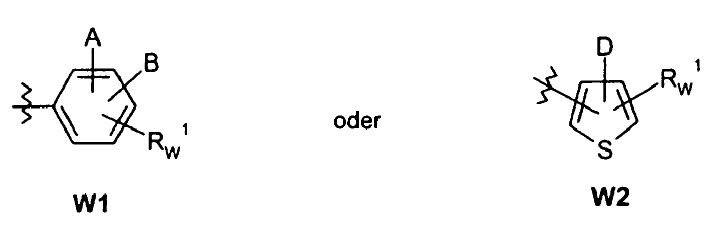
   **A:**
      NO₂, NH₂. OH, CN, CF₃, OCF₃, CHF₂, OCHF₂, COOH, O-CH₂-COOH, Halogen, SH, oder
         jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl oder C₁-C₄-Alkylen-Heterocycloalkyl, Aryl, Hetaryl, Heterocycloalkyl, C₁-C₄-Alkylen-Hetaryl oder C₁-C₄-Alkylen-Aryl, oder
         O-R_{A}¹, CO-R_{A}¹, S-R_{A}¹, SO-R_{A}¹, CO-O-R_{A}¹, NR_{A}⁴-CO-O-R_{A}¹, O-CH₂-COO-R_{A}¹, NR_{A}²R_{A}³, CONH₂, SO₂NH₂, NR_{A}⁴-CO-R_{A}¹, SO₂-R_{A}¹, NR_{A}⁴-SO₂-R_{A}¹,SO₂-NR_{A}²R_{A}³ oder CO-NR_{A}²R_{A}³;
   **R_{A}¹:**
      jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-cycloalkyl, C₁-C₄-Alkylen-Heterocycloalkyl, Aryl, Hetaryl, Heterocycloalkyl, C₁-C₄-Alkylen-Aryl, C₂-C₆-Alkenylen-Aryl oder C₁-C₆-Alkylen-Hetaryl;
   **R_{A}²:**
      Wasserstoff, OH, CN, oder
         jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
   **R_{A}³:**
      jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
      oder die Reste **R_{A}²** und **R_{A}³** zusammen mit dem Stickstoff einen 3 bis 7-gliedrigen, gegebenenfalls substituierten, gesättigten oder aromatischen Hetero-cyclus, der ein, zwei oder drei weitere verschiedene oder gleiche Heteroatome aus der Gruppe O, N, S enthalten kann, bilden; wobei gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und wobei der dadurch gebildete Cyclus gegebenenfalls substituiert sein kann oder an diesem Cyclus ein weiterer, gegebenenfalls substituierter Cyclus ankondensiert sein kann;
   **R_{A}⁴:**
      Wasserstoff, oder
         jeweils gegebenenfalls substituiertes C₁-C₆-Alkyl, C₁-C₆-Alkylen-O-C₁-C₆-Alkyl, C₂-C₆-Alkenyl, C₃-C₁₂-Alkinyl, CO-C₁-C₆-Alkyl, CO-O-C₁-C₆-Alkyl, SO₂-C₁-C₆-Alkyl, C₃-C₇-Cycloalkyl, Aryl, C₁-C₄-Alkylen-Aryl, CO-O-Arylalkyl, CO-C₁-C₄-Alkylen-Aryl, CO-Aryl, SO₂-Aryl, Hetaryl, CO-Hetaryl oder SO₂-C₁-C₄-AlkylenAryl;
   **B:**
      Wasserstoff oder wie Rest **A** definiert ist,
      oder jeweils unabhängig voneinander zwei der Reste **A, B** oder **R_{w}¹** zusammen einen 3 bis 7-gliedrigen, gegebenenfalls substituierten, gesättigten oder ungesättigten Carbocyclus oder einen gegebenenfalls substituierten, gesättigten oder ungesättigten oder aromatischen Heterocyclus, der ein, zwei oder drei weitere verschiedene oder gleiche Heteroatome aus der Gruppe O, N, S enthalten kann, bilden; wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
   **R_{w}¹:**
      Wasserstoff, OH, Halogen, NO₂, NH₂, CN, CF₃, CHF₂, O-CF₃, O-CHF₂, oder **jeweils** gegebenenfalls substituiertes C₁-C₆-Alkyl, C₃-C₇-Cycloalkyl, C₁-C₆-Alkylen-O-C₁-C₆-Alkyl, C₁-C₆-Alkylen-S-C₁-C₆-Alkyl, Aryl, Hetaryl, O-C₁-C₆-Alkyl, O-Aryl, O-Benzyl, C₁-C₆-Alkylamino, C₁-C₆-Dialkylamino, Pyrrolidinyl, Piperidinyl, Morpholinyl, CO-C₁-C₆-Alkyl, SO₂-C₁-C₆-Alkyl, CO-Aryl, SO₂-Aryl, CO-C₁-C₄-Alkylen-Aryl, SO₂-C₁-C₄-Alkylen-Aryl, SO-Aryl, CONH₂, CONH-C₁-C₆-Alkyl. S0₂NH-Cᵢ-C₆-Alkyl, CON(C₁-C₆-Alkyl)₂, SO₂N(C₁-C₆-Alkyl)₂, NH-SO₂-C₁-C₆-Alkyl oder NH-CO-C₁-C₆-Alkyl;
   **D:**
      wie Rest **A** definiert ist;
   **Z:**
      einen Rest der allgemeinen Formel **Z1** 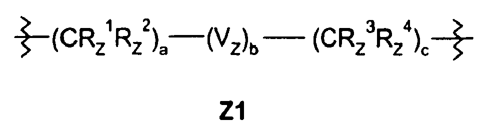 mit den Indizes
         a = 0 - 4
         b = 0, 1
         c = 0 - 4
         wobei die Summe aus a, b und c höchstens 5 beträgt;
   **R_{z}¹, R_{z}², R_{z}³, R_{z}⁴** unabhängig voneinander:
      Wasserstoff, Halogen, OH, oder
         jeweils gegebenenfalls substituiertes C₁-C₆-Alkyl, C₂-C₆-Alkenyl, C₂-C₆-Alkinyl, C₁-C₆-Alkylen-C₃-C₇-Cycloalkyl, C₃-C₇-Cycloalkyl, Aryl, C₁-C₄-Alkylen-Aryl, Hetaryl oder C₁-C₄-Alkylen-Hetaryl, oder
         jeweils unabhängig voneinander zwei Reste **R_{z}¹** und **R_{z}²** oder **R_{z}³** und **R_{z}⁴** zusammen einen 3 bis 7-gliedrigen, gegebenenfalls substituierten, gesättigten oder ungesättigten Carbo- oder Heterocyclus, der bis zu drei Heteroatome aus der Gruppe O, N oder S enthalten kann, bilden;
   **V_{z}:**
      -CO-, -CO-NR_{z}⁵-, -NR_{z}⁵-CO-, -O-, -S-, -SO-, -SO₂-, -SO₂-NR_{z}⁵-, -NR_{z}⁵-SO₂-, -CS-, -CS-NR_{z}⁵-, -NR_{z}⁵-CS-, -CS-O-, -O-CS-, -CO-O-, -O-CO-, -O-, Ethinylen, -C(=CR_{z}⁶R_{z}⁷)-, -CR_{z}⁶=CR_{z}⁷-, -NR_{z}⁵-CO-NR_{z}⁵*-, -O-CO-NR_{z}⁵-, -NR_{z}⁵-;
   **R_{z}⁵, R_{z}⁵*** unabhängig voneinander.
      Wasserstoff, oder
         jeweils gegebenenfalls substituiertes C₁-C₆-Alkyl, C₁-C₆-Alkylen-O-C₁-C₆-Alkyl, C₂-C₆-Alkenyl, C₃-C₁₂-Alkinyl, CO-C₁-C₆-Alkyl, CO-O-C₁-C₆-Alkyl, SO₂-C₁-C₆-Alkyl, C₃-C₇-Cycloalkyl, Aryl, C₁-C₄-Alkylen-Aryl, CO-O-C₁-C₄-Alkylen-Aryl, CO-C₁-C₄-Alkylen-Aryl, CO-Aryl, SO₂-Aryl, Hetaryl, CO-Hetaryl oder SO₂-C₁-C₄-Alkylen-Aryl;
   **R_{z}⁵, R_{z}⁷** unabhängig voneinander:
      Wasserstoff, OH, oder
         jeweils gegebenenfalls substituiertes C₁-C₆-Alkyl, C₁-C₄-Alkoxy, C₂-C₆-Alkenyl. C₂-C₆-Alkinyl, C₁-C₆-Alkylen-C₃-C₇-Cycloalkyl, C₃-C₇-Cycloalkyl, Aryl, C₁-C₄-Alkylen-Aryl, Hetaryl oder C₁-C₄-Alkylen-Hetaryl;
   **R¹, R², R³** unabhängig voneinander:
      Wasserstoff, OH, CN, oder
         jeweils gegebenenfalls substituiertes C₁-C₆-Alkyl, O-C₁-C₆-Alkyl, C₁-C₆-Alkylen-O-C₁-C₆-Alkyl, C₃-C₇-Cycloalkyl, O-C₃-C₇-Cycloalkyl, Aryl, Hetaryl, C₁-C₄-Alkylen-Aryl, C₁-C₄-Alkylen-Hetaryl, O-Aryl, O-C₁-C₄-Alkylen-Aryl, O-Hetaryl, O-C₁-C₄-Alkylen-Hetaryl, CO-C₁-C₆-Alkyl, CO-Aryl, CO-Hetaryl, CO-C₁-C₄-AlkylenAryl, CO-C₁-C₄-Alkylen-Hetaryl, CO-O-C₁-C₆-Alkyl, CO-O-Aryl, CO-O-Hetaryl, CO-O-C₁-C₄-Alkylen-Aryl, SO₂-C₁-C₆-Alkyl, SO₂-Aryl, SO₂-Hetaryl, SO₂-C₁-C₄-Alkylen-Aryl, OCO-C₁-C₆-Alkyl, OCO-Aryl, OCO-Hetaryl, OCO-C₁-C₄-AlkylenAryl, OCO-C₁-C₄-Alkylen-Hetaryl, SO₂-C₁-C₆-Alkyl, SO₂-Aryl, SO₂-Hetaryl oder SO₂-C₁-C₄-Alkylen-Aryl, oder
      jeweils unabhängig vom dritten Rest zwei Reste von **R¹, R²** oder **R³** zusammen einen 5 bis 7-gliedrigen, gegebenenfalls substituierten, gesättigten oder ungesättigten Carbocyclus, oder einen gegebenenfalls substituierten, gesättigten oder ungesättigten, der ein, zwei oder drei weitere verschiedene oder gleiche Heteroatome aus der Gruppe O, N, S enthalten kann, bilden, wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
   **Q:**
      einen zweifach substituierten 5-gliedrigen Hetaryl-Rest, augewählt aus **Q1** bis **Q6** 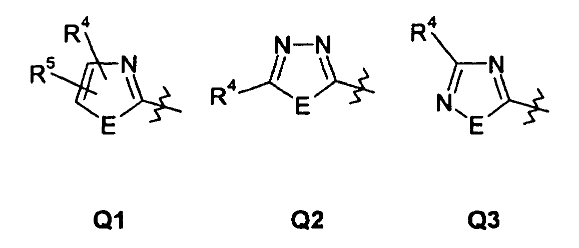 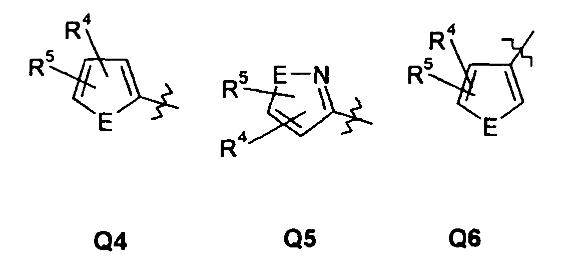
   **E:** O, N-R_{Q}¹ oder S;
   **R_{Q}¹:**
      Wasserstoff, oder
         jeweils gegebenenfalls substituiertes C₁-C₄-Alkyl, CO-C₁-C₄-Alkyl, SO₂-C₁-C₄-Alkyl, CO-O-C₁-C₄-Alkyl, Aryl, C₁-C₄-Alkylen-Aryl, CO-Aryl, CO-Hetaryl, SO₂-Aryl, SO₂-Hetaryl, CO-O-Aryl, CO-C₁-C₄-Alkylen-Aryl, SO₂-C₁-C₄-Alkylen-Aryl oder CO-O-C₁-C₄-Alkylen-Aryl;
   **R⁴**, **R⁵** jeweils unabhängig voneinander einen Rest ausgewählt aus den Gruppen 1.), **2.)**, **3.)**, **4.)** oder **5.)**:
      **1.)** Wasserstoff, Halogen, CN, CF₃, CHF₂, oder
         jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryl, C₁-C₄-Alkylen-Hetaryl, C₁-C₆-Alkylen-O-C₁-C₆-Alkyl, C₁-C₆-Alkylen-O-Aryl, COO-C₁-C₄-Alkyl oder C₁-C₄-Alkylen-COO-C₁-C₄-Alkyl;
      **3.)** Phenyl oder Naphthyl, die jeweils mit **R_{Q}², R_{Q}³** und **R_{Q}⁴** substituiert sind, wobei
         **R_{Q}², R_{Q}³** und **R_{Q}⁴** jeweils unabhängig voneinander einen Substituenten aus der folgenden Gruppe darstellen:
            Wasserstoff, NO₂, NH₂, OH, CN, CF₃, CHF₂, OCF₃, OCHF₂, COOH, O-CH₂-COOH, SH, Halogen, oder
               jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-Alkylen-Hetaryl, oder
               O-R_{Q}⁵, S-R_{Q}⁵, NR_{Q}⁷R_{Q}⁸, CO-OR_{Q}⁶, NR_{Q}⁸-CO-O-R_{Q}⁶, O-CH₂-COO-R_{Q}⁶, NR_{Q}⁸-CO-R_{Q}⁶, SO₂-R_{Q}⁶, NR_{Q}⁸-SO₂-R_{Q}⁶, SO₂NH₂, CONH₂, SO₂-NR_{Q}⁷R_{Q}⁸ oder CO-NR_{Q}⁷R_{Q}⁸, oder jeweils zwei der Reste aus **R_{Q}², R_{Q}³** oder **R_{Q}⁴** zusammen einen 3 bis 7-gliedrigen, gegebenenfalls substituierten, gesättigten, ungesättigten Carbocyclus oder einen gegebenenfalls substituierten, gesättigten, ungesättigten aromatischen Heterocyclus, der bis zu drei weitere verschiedene oder gleiche Heteroatome O, N, S enthalten kann, bilden, und gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und der gebildete Cyclus gegebenenfalls substituiert sein kann oder an diesem Cyclus ein weiterer, gegebenenfalls substituierter Cyclus ankondensiert sein kann;
         **R_{Q}⁵** jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Heterocycloalkyl, Aryl oder Hetaryl;
         **R_{Q}⁶** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl oder C₁-C₆-Alkylen-O-C₁-C₆-Alkyl;
         **R_{Q}⁷** Wasserstoff, OH, CN, oder
            jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, C₁-C₄-Alkylen-Aryl, C₁-C₄-Alkylen-Hetaryl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
         **R_{Q}⁸** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
         oder beide Reste **R_{Q}⁷** und **R_{Q}⁸** zusammen mit dem Stickstoff einen 3 bis 7-gliedrigen, gegebenenfalls substituierten, gesättigten oder aromatischen Heterocyclus, der ein, zwei oder drei weitere verschiedene oder gleiche Heteroatome O, N, S enthalten kann- bilden; und gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und der gebildete Cyclus gegebenenfalls substituiert sein kann oder an diesem Cyclus ein weiterer, gegebenenfalls substituierter Cyclus ankondensiert sein kann;
      **3.)** einen 5- oder 6-gliedrigen, gegebenenfalls mit 1 oder 2 Substituenten substituierten Hetaryl-Rest aus der Gruppe, bestehend aus:
         2-Furyl, 3-Furyl, 2-Pyrrolyl, 3-Pyrrolyl, 2-Thienyl, 3-Thienyl, 2-Pyridyl, 3-Pyridyl, 4-Pyridyl, 2-Thiazolyl, 4-Thiazolyl, 5-Thiazolyl, 2-Oxazolyl, 4-Oxazolyl, 5-Oxazolyl, 2-Pyrimidyl, 4-Pyrimidyl, 5-Pyrimidyl, 6-Pyrimidyl, 3-Pyrazolyl, 4-Pyrazolyl, 5-Pyrazolyl, 3-Isothiazolyl, 4-Isothiazolyl, 5-Isothiazolyl, 2-Imidazolyl, 4-Imidazolyl, 5-Imidazolyl, 3-Pyridazinyl, 4-Pyridazinyl, 5-Pyridazinyl, 6-Pyridazinyl, 3-Isoxazolyl, 4-Isoxazolyl, 5-Isoxazolyl, Thiadiazolyl, Oxadiazolyl oder Triazinyl oder deren anellierten Derivate Indazolyl, Indolyl, Benzothiophenyl, Benzofuranyl, Indolinyl, Benzimidazolyl, Benzthiazolyl, Benzoxazolyl, Chinolinyl und Isochinolinyl;
      **4.)** beide Reste **R⁴** und **R⁵** zusammen einen 4 bis 7-gliedrigen, gegebenenfalls substituierten, gesättigten oder ungesättigten oder aromatischen Carbocyclus oder einen 5- oder 6-gliedrigen gegebenenfalls substituierten, gesättigten oder ungesättigten oder aromatischen Heterocyclus, der bis zu drei weitere verschiedene oder gleiche Heteroatome O, N, S enthalten kann, bilden, und mit bis zu zwei weiteren Resten substituiert sein kann, wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
      **5.)** einen C₆-C₁₀- bi- oder tricyclischen, gesättigten Kohlenwasserstoffrest;
      zur Herstellung eines Medikaments zur Behandlung von Krankheiten, die durch eine 5-HT5-Rezeptoraktivität moduliert werden.
18. Verwendung nach Gegenstand 16, wobei R4 und/oder R5 die folgenden Bedeutungen besitzen:
   2-Pyridyl, 3-Pyridyl, 4-Pyridyl, 2-Thienyl, 3-Thienyl, Benzothiophenyl, Benzofuranyl, Chinolinyl oder Isochinolinyl, die gegebenenfalls mit 1 oder 2 Resten substituiert sein können.
19. Verwendung nach Gegenstand 16 oder 17 zur Behandlung von neuropathologischen, neuropsychiatrischen und neurodegenerativen Störungen, Symptomen und Fehifunktionen.
20. Verwendung nach mindestens einem der Gegenstände 16 bis 18 zur Behandlung von Migräne und Gehirnschädigungen.
21. Verwendung nach Gegenstand 18 zur Behandlung von neuropathologischen, neuropsychiatrischen und neurodegenerativen Krankheiten, ausgewählt aus der Gruppe, bestehend aus cerebraler Ischämie, Schlaganfall, Epilepsie und Anfälle im allgemeinen, Psychosen, Schizophrenie, Autismus, OCD-Syndrom, cognitiven Erkrankungen, Aufmerksamkeitsstörungen, Depressionen, bipolaren und/oder unipolaren Depressionen, Angstzuständen, Demenz, seniler Demenz, Alzheimer Demenz, demyelinisierenden Erkrankungen, Multipler Sklerose und Gehirntumoren.
22. Verwendung nach Gegenstand 16 oder 17 zur Behandlung von Krankheiten ausgewählt aus der Gruppe, bestehend aus cerebrovaskulären Störungen, Schmerz, Schmerz-bedingten Störungen, Abhängigkeit, Drogen-bedingten Störungen, Amnesie, Alkoholmissbrauch, Drogenmissbrauch, Störungen des circadianen Rhythmus und Cushing Syndrom.

## Patentansprüche

1. Guanidinverbindung der allgemeinen Formel I 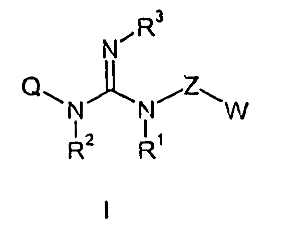 entsprechende enantiomere, diastereomere und/oder tautomere Formen davon sowie pharmazeutisch annehmbare Salze davon, wobei die angegebenen Reste die folgenden Definitionen besitzen:
**W:**
einen Rest der allgemeinen Formel W1 oder W2 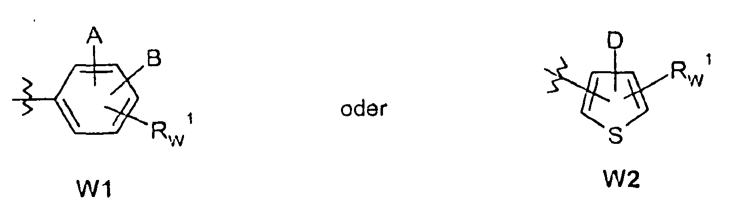
worin
**A:**
NO_{2,} NH_{2,} OH, CN, CF₃, OCF₃, CHF₂, OCHF₂, COOH, O-CH₂-COOH, Halogen, SH, oder
jeweils gegebenenfalls substituiertes C₁-C₆-Alkyl, C₂-C₈-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Hetaryl oder C₁-C₄-AlkylenAryl, oder
O-R_{A}¹, CO-R_{A}¹, S-R_{A}¹, SO-R_{A}¹, CO-O-R_{A}¹, NR_{A}⁴-CO-O-R_{A}¹, O-CH₂-COO-R_{A}¹, NR_{A}²R_{A}³, CONH₂, SO₂NH₂, NR_{A}¹-CO-R_{A}¹, SO₂R_{A}¹, NR_{A}¹-SO₂-R_{A}¹, SO₂-NR_{A}²R_{A}³ oder CO-NR_{A}²-R_{A}⁴;
**R_{A}¹:**
jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₂-C₆-Alkenylen-Aryl oder C₁-C₆-Alkylen-Hetaryl;
**R_{A}²:**
Wasserstoff, OH, CN, oder
jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl,SO₂-Aryl, SO₂-Hetaryl, SO₂-C₁-C₄-Alkylen-Aryl oder SO₂-C₁-C₄-Alkylen-Hetaryl;
**R_{A}³:**
jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
oder die Reste **R_{A}²** und **R_{A}³** zusammen mit dem Stickstoff einen 3 bis 7-gliedrigen, gegebenenfalls substituierten, gesättigten oder aromatischen Hetero-cyclus, der ein, zwei oder drei weitere verschiedene oder gleiche Heteroatome aus der Gruppe O, N, S enthalten kann, bilden; wobei gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und wobei der dadurch gebildete Cyclus gegebenenfalls substituiert sein kann oder an diesem Cyclus ein weiterer, gegebenenfalls substituierter Cyclus ankondensiert sein kann;
**R_{A}⁴:**
Wasserstoff, oder
jeweils gegebenenfalls substituiertes C₁-C₆-Alkyl, C₁-C₆-Alkylen-O-C₁-C₆-Alkyl, C₂-C₆-Alkenyl, C₃-C₁₂-Alkinyl, CO-C₁-C₆-Alkyl, CO-O-C₁-C₆-Alkyl, SO₂-C₁-C₆-Alkyl, C₃-C₇-Cycloalkyl, Aryl, C₁-C₄-Alkylen-Aryl, CO-O-Arylalkyl, CO-C₁-C₄-Alkylen-Aryl, CO-Aryl, SO₂-Aryl, Hetaryl, CO-Hetaryl oder SO₂-C₁-C₄-AlkylenAryl;
**B:**
Wasserstoff oder wie Rest **A** definiert ist,
oder jeweils unabhängig voneinander zwei der Reste **A, B** oder **R_{w}¹** zusammen einen 3 bis 7-gliedrigen, gegebenenfalls substituierten, gesättigten oder ungesättigten Carbocyclus oder einen gegebenenfalls substituierten, gesättigten oder ungesättigten oder aromatischen Heterocyclus, der ein, zwei oder drei weitere verschiedene oder gleiche Heteroatome aus der Gruppe O, N, S enthalten kann, bilden; wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
**R_{w}¹:**
Wasserstoff, OH, Halogen, NO₂, NH₂, CN, CF₃, CHF₂, O-CF₃, O-CHF₂, oder
jeweils gegebenenfalls substituiertes C₁-C₆-Alkyl, C₃-C₇-Cycloalkyl, C₁-C₆-Alkylen-O-C₁-C₆-Alkyl, C₁-C₆-Thioalkyl, Aryl, Hetaryl, O-C₁-C₆-Alkyl, O-Aryl, O-Benzyl, C₁-C₆-Alkylamino, C₁-C₆-Dialkylamino, Pyrrolidinyl, Piperidinyl, Morpholinyl, CO-C₁-C₆-Alkyl, SO₂-C₁-C₆-Alkyl, CO-Aryl, SO₂-Aryl, CO-C₁-C₄-AlkylenAryl, SO₂-C₁-C₄-Alkylen-Aryl, SO-Aryl, CONH₂, CONH-C₁-C₆-Alkyl, SO₂NH-C₁-C₆-Alkyl, CON-(C₁-C₆-Alkyl)₂, SO₂N-(C₁-C₆-Alkyl)₂, NH-SO₂-C₁-C₆-Alkyl oder NH-CO-C₁-C₆-Alkyl;
**D:**
wie Rest **A** definiert ist;
**Z:**
einen Rest der allgemeinen Formel **Z1** 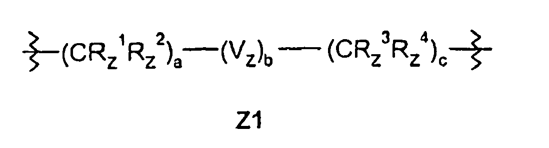 mit den Indizes
a=0-4
b=0,1 1
c=0-4
wobei die Summe aus a, b und c mindestens 1 und höchstens 5 beträgt;
**R_{z}¹, R_{z}², R_{z}³, R_{z}⁴** unabhängig voneinander:
Wasserstoff, Halogen, OH, oder
jeweils gegebenenfalls substituiertes C₁-C₆-Alkyl, C₂-C₆-Alkenyl, C₂-C₆-Alkinyl, C₁-C₆-Alkylen-C₃-C₇-Cycloalkyl, C₃-C₇-Cycloalkyl, Aryl, C₁-C₄-Alkylen-Aryl, Hetaryl oder C₁-C₄-Alkylen-Hetaryl, oder
jeweils unabhängig voneinander zwei Reste **R_{z}¹** und **R_{z}²** oder **R_{z}³** und **R_{z}⁴** zusammen einen 3 bis 7-gliedrigen, gegebenenfalls substituierten, gesättigten oder ungesättigten Carbo- oder Heterocyclus bilden, wobei der Heterocyclus bis zu drei Heteroatome aus der Gruppe O, N oder S enthalten kann;
**V_{z}:**
-CO-, -CO-NR_{z}⁵-, -NR_{z}⁵-CO-, -O-, -S-, -SO-, -SO₂-, -SO₂-NR_{z}⁵-, -NR_{z}⁵-SO₂-, -CS-, -CS-NR_{z}⁵-, -NR_{z}⁵-CS-, -CS-O-, -O-CS-, -CO-O-, -O-CO-, -O-, Ethinylen, -C(=CR_{z}⁶R_{z}⁷)-, -CR_{z}⁶=CR_{z}⁷-, -NR_{z}⁵-CO-NR_{z}^{5*}-, -O-CO-NR_{z}⁵-, -NR_{z}⁵-;
**R_{z}⁵, R_{z}^{5*}** unabhängig voneinander:
Wasserstoff oder
jeweils gegebenenfalls substituiertes C₁-C₆-Alkyl, C₁-C₆-Alkylen-O-C₁-C₆-Alkyl, C₂-C₆-Alkenyl, C₃-C₁₂-Alkinyl, CO-C₁-C₆-Alkyl, CO-O-C₁-C₆-Alkyl, SO₂-C₁-C₆-Alkyl, C₃-C₇-Cycloalkyl, Aryl, C₁-C₄-Alkylen-Aryl, CO-O-C₁-C₄-Alkylen-Aryl, CO-C₁-C₄-Alkylen-Aryl, CO-Aryl, SO₂-Aryl, Hetaryl, CO-Hetaryl oder SO₂-C₁-C₄-Alkylen-Aryl;
**R_{z}⁶, R_{z}⁷** unabhängig voneinander:
Wasserstoff, OH oder
jeweils gegebenenfalls substituiertes C₁-C₆-Alkyl, C₁-C₄-Alkoxy, C₂-C₆-Alkenyl, C₂-C₆-Alkinyl, C₁-C₆-Alkylen-C₃-C₇-Cycloalkyl, C₃-C₇-Cycloalkyl, Aryl, C₁-C₄-Alkylen-Aryl, Hetaryl oder C₁-C₄-Alkylen-Hetaryl;
**R¹, R², R³** unabhängig voneinander:
Wasserstoff, OH, CN, oder
jeweils gegebenenfalls substituiertes C₁-C₆-Alkyl, O-C₁-C₆-Alkyl, C₁-C₆-Alkylen-O-C₁-C₆-Alkyl, C₃-C₇-Cycloalkyl, O-C₃-C₇-Cycloalkyl, Aryl, Hetaryl, C₁-C₄-Alkylen-Aryl, C₁-C₄-Alkylen-Hetaryl, O-Aryl, O-C₁-C₄-Alkylen-Aryl, O-Hetaryl, O-C₁-C₄-Alkylen-Hetaryl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-Hetaryl, CO-O-C₁-C₄-Alkylen-Aryl, SO₂-C₁-C₆-Alkyl, SO₂-Aryl, SO₂-Hetaryl, SO₂-C₁-C₄-Alkylen-Aryl, OCO-C₁-C₆-Alkyl, OCO-Aryl, OCO-Hetaryl, OCO-C₁-C₄-Alkylen-Aryl, OCO-C₁-C₄-Alkylen-Hetaryl, SO₂-C₁-C₆-Alkyl, SO₂-Aryl, SO₂-Hetaryl oder SO₂-C₁-C₄-Alkylen-Aryl, oder
jeweils unabhängig vom dritten Rest zwei Reste von **R¹, R²** oder **R³** zusammen einen 5 bis 7-gliedrigen, gegebenenfalls substituierten, gesättigten oder ungesättigten Carbocyclus, oder einen gegebenenfalls substituierten, gesättigten oder ungesättigten Heterocyclus, der ein, zwei oder drei weitere verschiedene oder gleiche Heteroatome aus der Gruppe O, N, S enthalten kann, bilden, wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
**Q:**
einen zweifach substituierten 5-gliedrigen Hetaryl-Rest, ausgewählt aus **Q1** bis **Q7** 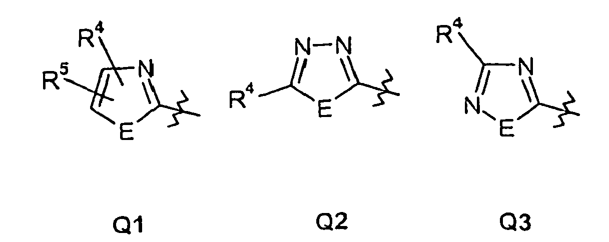 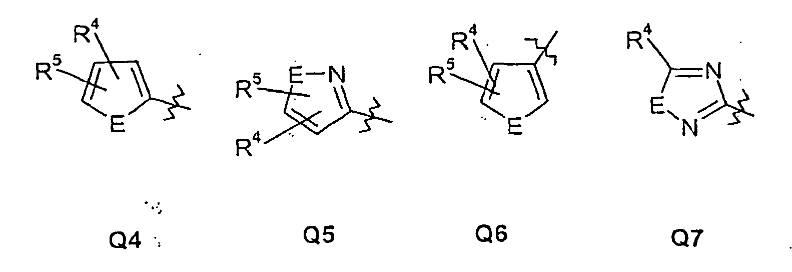
**E:** O, N-R_{Q}¹ oder S;
**R_{Q}¹:**
Wasserstoff, oder
jeweils gegebenenfalls substituiertes C₁-C₄-Alkyl, CO-C₁-C₄-Alkyl, SO₂-C₁-C₄-Alkyl, CO-O-C₁-C₄-Alkyl, Aryl, C₁-C₄-Alkylen-Aryl, CO-Aryl, CO-Hetaryl, SO₂-Aryl, SO₂-Hetaryl, CO-O-Aryl, CO-C₁-C₄-Alkylen-Aryl, SO₂-C₁-C₄-Alkylen-Aryl oder CO-O-C₁-C₄-Alkylen-Aryl;
**R⁴, R⁵** jeweils unabhängig voneinander einen Rest, ausgewählt aus den Gruppen **1.), 2.), 3.), 4.), 5.), 6.)** oder **7.):**
1.) Wasserstoff, Halogen, CN, CF₃, CHF₂, oder
jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryl, C₁-C₄-Alkylen-Hetaryl, C₁-C₆-Alkylen-O-C₁-C₆-Alkyl, C₁-C₆-Alkylen-O-Aryl, COO-C₁-C₄-Alkyl oder C₁-C₄-Alkylen-COO-C₁-C₄-Alkyl;
2.) Phenyl oder Naphthyl, die jeweils mit R_{Q}², R_{Q}³ und R_{Q}⁴ substituiert sind, wobei
R_{Q}², R_{Q}³ und R_{Q}⁴ jeweils unabhängig voneinander einen Substituenten aus der folgenden Gruppe darstellen:
Wasserstoff, NO₂, NH₂. OH, CN, CF₃, CHF₂, OCF₃, OCHF₂, COOH, O-CH₂-COOH, SH, Halogen, oder
jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-Alkylen-Hetaryl, oder
O-R_{Q}⁵, S-R_{Q}⁵, NR_{Q}⁷R_{Q}⁸, CO-OR_{Q}⁶, NR_{Q}⁸-CO-O-R_{Q}⁶, O-CH₂-COO-R_{Q}³, NR_{Q}⁸-CO-R_{Q}⁶, SO₂-R_{Q}⁶, NR_{Q}⁸-SO₂-R_{Q}⁶, SO₂NH₂, CONH₂, SO₂-NR_{Q}⁷R_{Q}⁸ oder CO-NR_{Q}⁷R_{Q}⁸, oder
jeweils zwei der Reste aus R_{Q}², R_{Q}³ oder R_{Q}⁴ zusammen einen 3 bis 7-gliedrigen, gegebenenfalls substituierten, gesättigten, ungesättigten oder aromatischen Carbocyclus oder einen gegebenenfalls substituierten, gesättigten, ungesättigten aromatischen Heterocyclus, der bis zu drei weitere verschiedene oder gleiche Heteroatome O, N, S enthalten kann, bilden, und gegebenenfalls zwei an diesem Heterocyclus substituierte Reste können zusammen einen anellierten, gesättigten, ungesättigten oder aromatischen Carbocyclus oder Heterocyclus bilden, wobei der Heterocyclus bis zu drei verschiedene oder gleiche Heteroatome O, N, S enthalten kann, und der gebildete Cyclus kann gegebenenfalls substituiert sein oder an diesem Cyclus kann ein weiterer, gegebenenfalls substituierter Cyclus ankondensiert sein;
**R_{Q}⁵** jeweils gegebenenfalls substituiertes C₂-C₆-Alkenyl, C₂-C₆-Alkinyl, C₁-C₄-Alkylen-C₃-C₇-Cycloalkyl, C₁-C₄-Alkylen-Heterocycloalkyl, Heterocycloalkyl oder Hetaryl, oder
C₁-C₆-Alkyl, das gegebenenfalls substituiert ist mit einem Substituenten aus der Gruppe, bestehend aus Halogen, NO₂, NH₂, OH, CN, CF₃, CHF₂, OCF₃, OCHF₂, NH-(C₁-C₆-Alkyl) und N(C₁-C₆-Alkyl)₂;
**R_{Q}⁶** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl. Heterocycloalkyl oder C₁-C₈-Alkylen-O-C₁-C₈-Alkyl;
**R_{Q}⁷** Wasserstoff, OH, CN, oder
jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, C₁-C₄-Alkylen-Aryl, C₁-C₄-Alkylen-Hetaryl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
**R_{Q}⁸** Wasserstoff oder
jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
oder die Reste **R_{Q}⁷** und **R_{Q}⁸** zusammen mit dem Stickstoff einen 3 bis 7-gliedrigen, gegebenenfalls substituierten, gesättigten oder aromatischen Heterocyclus, der ein, zwei oder drei weitere verschiedene oder gleiche Heteroatome O, N, S enthalten kann, bilden; und gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und der gebildete Cyclus gegebenenfalls substituiert sein kann oder an diesem Cyclus ein weiterer, gegebenenfalls substituierter Cyclus ankondensiert sein kann;
3.) einen 5- oder 6-gliedrigen, gegebenenfalls mit 1 oder 2 Substituenten substituierten Hetaryl-Rest aus der Gruppe, bestehend aus:
2-Pyrrolyl, 3-Pyrrolyl, 2-Thiazolyl, 4-Thiazolyl, 5-Thiazolyl, 2-Oxazolyl, 4-Oxazolyl, 5-Oxazolyl, 2-Pyrimidyl, 4-Pyrimidyl, 5-Pyrimidyl, 6-Pyrimidyl, 3-Pyrazolyl, 4-Pyrazolyl, 5-Pyrazolyl, 3-Isothiazolyl, 4-Isothiazolyl, 5-Isothiazolyl, 2-Imidazolyl, 4-Imidazolyl, 5-Imidazolyl, 3-Pyridazinyl, 4-Pyridazinyl, 5-Pyridazinyl, 6-Pyridazinyl, 3-Isoxazolyl, 4-Isoxazolyl, 5-Isoxazolyi, Thiadiazolyl, Oxadiazolyl oder Triazinyl oder deren anellierten Derivate Indazolyl, Benzothiophenyl, Benzofuranyl, Indolinyl, Benzimidazolyl, Benzthiazolyl, Benzoxazolyl, Chinolinyl und Isochinolinyl; oder
gegebenenfalls mit 1 oder 2 Substituenten substituiertes 2-Thienyl oder 3-Thienyl, wobei die Substituenten ausgewählt sind aus der Gruppe, bestehend aus Halogen, NO₂, NH₂, OH, CN, CF₃, OCF₃, CHF₂, O-CHF₂, C₁-C₆-Alkyl, O-C₁-C₆-Alkyl, NH-(C₁-C₆-Alkyl), N(C₁-C₆-Alkyl)₂, NHCO-C₁-C₄-Alkyl, NHSO₂-C₁-C₄-Alkyl und SO₂-C₁-C₄-Alkyl;
4.) beide Reste **R⁴** und **R⁵** zusammen einen 4 bis 7-gliedrigen, gegebenenfalls substituierten, gesättigten oder ungesättigten oder aromatischen Carbocyclus oder einen 5- oder 6-gliedrigen gegebenenfalls substituierten, gesättigten oder ungesättigten oder aromatischen Heterocyclus, der bis zu drei weitere verschiedene oder gleiche Heteroatome O, N, S enthalten kann, bilden, und mit bis zu zwei weiteren Resten substituiert sein kann, wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
5.) einen C₅-C₁₈- bi- oder tricyclischen, gesättigten Kohlenwasserstoffrest;
6.) jeweils gegebenenfalls substituiertes C₁-C₈-Alkyl-NH₂, C₁-C₈-Alkyl-NR_{Q}⁷R_{Q}⁸, C₁-C₈-Alkyl-CO-NR_{Q}⁷R_{Q}⁸, C₁-C₈-Alkyl-SO₂NR_{Q}⁷R_{Q}⁸, C₁-C₈-Alkyl-CO-NH₂, C₁-C₈-Alkyl-SO₂NH₂, CO-NH₂, CO-NR_{Q}⁷R_{Q}⁸, SO₂NH₂, SO₂NR_{Q}⁷R_{Q}⁸, NR_{Q}⁷R_{Q}⁸;
7.) einen 4-7-gliedrigen mono- oder bicyclischen gesättigten oder ungesättigten Heterocyclus, der bis zu zwei verschiedene oder gleiche Heteroatome aus der Gruppe O, N oder S enthalten kann, wobei dieser Cyclus ebenfalls mehrfach substituiert sein kann. Für den Fall, dass der Heterocyclus ein N-Atom enthält, kann dies mit einem Rest R_{Q}⁷ substituiert sein.

2. Guanidinverbindung der allgemeinen Formel I 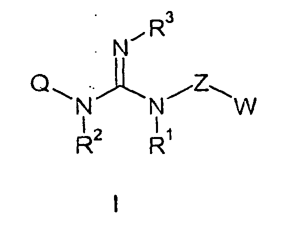 entsprechende enantiomere, diastereomere und/oder tautomere Formen davon sowie pharmazeutisch annehmbare Salze davon, wobei die angegebenen Reste die folgenden Definitionen besitzen:
W:
einen Rest der allgemeinen Formel W1 oder W2 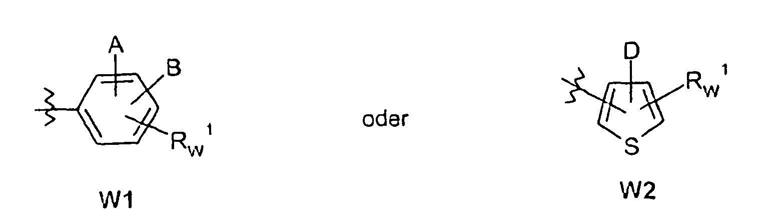
worin
A:
NO₂, NH₂, OH, CN, CF₃, OCF₃, CHF₂, OCHF₂, COOH, O-CH₂-COOH, Halogen, SH, oder
jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Hetaryl oder C₁-C₄-AlkylenAryl, oder
O-R_{A}¹, CO-R_{A}¹, S-R_{A}¹, SO-R_{A}¹, CO-O-R_{A}¹, NR_{A}⁴-CO-O-R_{A}¹, O-CH₂-COO-R_{A}¹, NR_{A}²R_{A}³, CONH₂, SO₂NH₂, NR_{A}⁴-CO-R_{A}¹, SO₂-R_{A}¹, NR_{A}⁴-SO₂R_{A}¹, SO₂-NR_{A}²R_{A}³ oder CO-NR_{A}²R_{A}³;
R_{A}¹:
jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₂-C₆-Alkenylen-Aryl oder C₁-C₆-Alkylen-Hetaryl;
R_{A}²:
Wasserstoff, OH, CN, oder
jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
R_{A}³:
jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂C₁-C₄-Alkylen-Hetaryl;
oder die Reste R_{A}² und R_{A}³ zusammen mit dem Stickstoff einen 3 bis 7-gliedrigen, gegebenenfalls substituierten, gesättigten oder aromatischen Hetero-cyclus, der ein, zwei oder drei weitere verschiedene oder gleiche Heteroatome aus der Gruppe O, N, S enthalten kann, bilden; wobei gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und wobei der dadurch gebildete Cyclus gegebenenfalls substituiert sein kann oder an diesem Cyclus ein weiterer, gegebenenfalls substituierter Cyclus ankondensiert sein kann;
**R_{A}⁴:**
Wasserstoff, oder
jeweils gegebenenfalls substituiertes C₁-C₆-Alkyl, C₁-C₆-Alkylen-O-C₁-C₆-Alkyl, C₂-C₆-Alkenyl, C₃-C₁₂-Alkinyl, CO-C₁-C₈-Alkyl, CO-O-C₁-C₆-Alkyl, SO₂-C₁-C₆-Alkyl, C₃-C₇-Cycloalkyl, Aryl, C₁-C₄-Alkylen-Aryl, CO-O-Arylalkyl, CO-C₁-C₄-Alkylen-Aryl, CO-Aryl, SO₂-Aryl, Hetaryl, CO-Hetaryl oder SO₂-C₁-C₄-AlkylenAryl;
**B:**
Wasserstoff oder wie Rest **A** definiert ist,
oder jeweils unabhängig voneinander zwei der Reste **A, B** oder **R**_{w}¹ zusammen einen 3 bis 7-gliedrigen, gegebenenfalls substituierten, gesättigten oder ungesättigten Carbocyclus oder einen gegebenenfalls substituierten, gesättigten oder ungesättigten oder aromatischen Heterocyclus, der ein, zwei oder drei weitere verschiedene oder gleiche Heteroatome aus der Gruppe O, N, S enthalten kann, bilden; wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
**R_{w}¹:**
Wasserstoff. OH, Halogen, NO₂, NH₂, CN, CF₃, CHF₂, O-CF₃, O-CHF₂, oder jeweils gegebenenfalls substituiertes C₁-C₈-Alkyl, C₃-C₇-Cycloalkyl, C₁-C₆-Alkylen-O-C₁-C₆-Alkyl, C₁-C₆-Thioalkyl. Aryl, Hetaryl, O-C₁-C₆-Alkyl, O-Aryl, O-Benzyl, C₁-C₆-Alkylamino, C₁-C₆-Dialkylamino, Pyrrolidinyl, Piperidinyl, Morpholinyl, CO-C₁-C₆-Alkyl, SO₂-C₁-C₆-Alkyl, CO-Aryl, SO₂-Aryl, CO-C₁-C₄-AlkylenAryl, SO₂-C₁-C₄-Alkylen-Aryl, SO-Aryl, CONH₂, CONH-C₁-C₆-Alkyl, SO₂NH-C₁-C₆-Alkyl, CON-(C₁-C₆-Alkyl)₂, SO₂N-(C₁-C₆-Alkyl)₂, NH-SO₂-C₁-C₆-Alkyl oder NH-CO-C₁-C₆-Alkyl;
**D:**
wie Rest **A** definiert ist;
**Z:**
einen Rest der allgemeinen Formel Z1 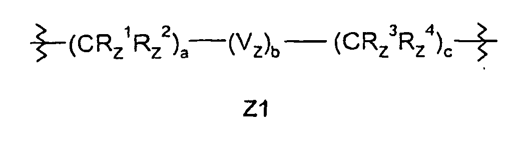 mit den Indizes
a = 0 - 4
b = 0, 1
c = 0 - 4
wobei die Summe aus a, b und c mindestens 1 und höchstens 5 beträgt;
R_{z}¹, R_{z}², R_{z}³, R_{z}⁴ unabhängig voneinander :
Wasserstoff, Halogen, OH, oder
jeweils gegebenenfalls substituiertes C₁-C₆-Alkyl, C₂-C₆-Alkenyl, C₂-C₆-Alkinyl, C₁-C₆-Alkylen-C₃-C₇-Cycloalkyl, C₃-C₇-Cycloalkyl, Aryl, C₁-C₄-Alkylen-Aryl, Hetaryl oder C₁-C₄-Alkylen-Hetaryl, oder
jeweils unabhängig voneinander zwei Reste R_{z}¹ und R_{z}² oder R_{z}³ und R_{z}⁴ zusammen einen 3 bis 7-gliedrigen, gegebenenfalls substituierten, gesättigten oder ungesättigten Carbo- oder Heterocyclus bilden, wobei der Heterocyclus bis zu drei Heteroatome aus der Gruppe O, N oder S enthalten kann;
**V_{z}:**
-CO-, -CO-NR_{z}⁵-, -NR_{z}⁵-CO-, -O-, -S-, -SO-, -SO₂-, -SO₂-NR_{z}⁵-, -NR_{z}⁵-SO₂-, -CS-, -CS-NR_{z}⁵-, -NR_{z}⁵-CS-, -CS-O-, -O-CS-, -CO-O-, -O-CO-, -O-, Ethinylen, -C(=CR_{z}⁶R_{z}⁷)-, -CR_{z}⁶=CR_{z}⁷-, -NR_{z}⁵-CO-NR_{z}^{5*}-, -O-CO-NR_{z}⁵-, -NR_{z}⁵-;
**R_{z}⁵, R_{z}⁵*** unabhängig voneinander:
Wasserstoff oder
jeweils gegebenenfalls substituiertes C₁-C₆-Alkyl, C₁-C₆-Alkylen-O-C₁-C₆-Alkyl, C₂-C₆-Alkenyl, C₃-C₁₂-Alkinyl, CO-C₁-C₆-Alkyl, CO-O-C₁-C₆-Alkyl, SO₂-C₁-C₆-Alkyl, C₃-C₇-Cycloalkyl, Aryl, C₁-C₄-Alkylen-Aryl, CO-O-C₁-C₄-Alkylen-Aryl, CO-C₁-C₄-Alkylen-Aryl, CO-Aryl, SO₂-Aryl, Hetaryl, CO-Hetaryl oder SO₂-C₁-C₄-Alkylen-Aryl;
**R_{z}⁶, R_{z}⁷** unabhängig voneinander:
Wasserstoff, OH oder
jeweils gegebenenfalls substituiertes C₁-C₆-Alkyl, C₁-C₄-Alkoxy, C₂-C₆-Alkenyl, C₂-C₆-Alkinyl, C₁-C₆-Alkylen-C₃-C₇-Cycloalkyl, C₃-C₇-Cycloalkyl, Aryl, C₁-C₄-Alkylen-Aryl, Hetaryl oder C₁-C₄-Alkylen-Hetaryl;
**R¹, R², R³** unabhängig voneinander:
Wasserstoff, OH, CN, oder
jeweils gegebenenfalls substituiertes C₁-C₆-Alkyl, O-C₁-C₆-Alkyl, C₁-C₆-Alkylen-O-C₁-C₆-Alkyl, C₃-C₇-Cycloalkyl, O-C₃-C₇-Cycloalkyl, Aryl, Hetaryl, C₁-C₄-Alkylen-Aryl, C₁-C₄-Alkylen-Hetaryl, O-Aryl, O-C₁-C₄-Alkylen-Aryl, O-Hetaryl, O-C₁-C₄-Alkylen-Hetaryl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-Hetaryl, CO-O-C₁-C₄-Alkylen-Aryl, SO₂-C₁-C₆-Alkyl, SO₂-Aryl, SO₂-Hetaryl, SO₂-C₁-C₄-Alkylen-Aryl, OCO-C₁-C₆-Alkyl, OCO-Aryl, OCO-Hetaryl, OCO-C₁-C₄-Alkylen-Aryl, OCO-C₁-C₄-Alkylen-Hetaryl, SO₂-C₁-C₆-Alkyl, SO₂-Aryl, SO₂-Hetaryl oder SO₂-C₁-C₄-Alkylen-Aryl, oder
jeweils unabhängig vom dritten Rest zwei Reste von **R¹, R²** oder **R³** zusammen einen 5 bis 7-gliedrigen, gegebenenfalls substituierten, gesättigten oder ungesättigten Carbocyclus, oder einen gegebenenfalls substituierten, gesättigten oder ungesättigten Heterocyclus, der ein, zwei oder drei weitere verschiedene oder gleiche Heteroatome aus der Gruppe O, N, S enthalten kann, bilden, wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
**Q:**
einen zweifach substituierten 5-gliedrigen Hetaryl-Rest, ausgewählt aus **Q1** bis **Q6** 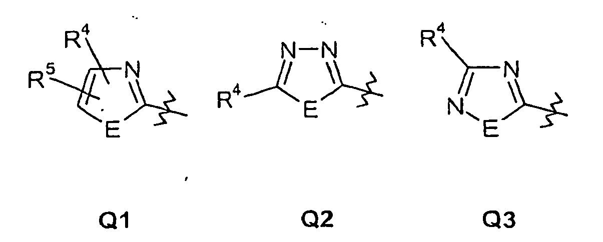 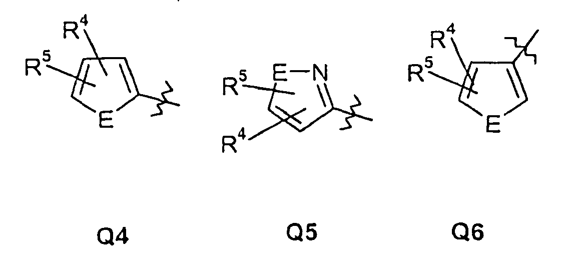
**E:** O, N-R_{Q}¹ oder S;
R_{Q}¹:
Wasserstoff, oder
jeweils gegebenenfalls substituiertes C₁-C₄-Alkyl, CO-C₁-C₄-Alkyl, SO₂-C₁-C₄-Alkyl, CO-O-C₁-C₄-Alkyl, Aryl, C₁-C₄-Alkylen-Aryl, CO-Aryl, CO-Hetaryl, SO₂-Aryl, SO₂-Hetaryl, CO-O-Aryl, CO-C₁-C₄-Alkylen-Aryl, SO₂-C₁-C₄-Alkylen-Aryl oder CO-O-C₁-C₄-Alkylen-Aryl;
**R⁴, R⁵** jeweils unabhängig voneinander einen Rest, ausgewählt aus den Gruppen 1.), 2.), 3.), 4.) oder 5.):
1.) Wasserstoff, Halogen, CN, CF₃, CHF₂, oder
jeweils gegebenenfalls substituiertes C₁-C₁₀Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryl, C₁-C₄-Alkylen-Hetaryl, C₁-C₆-Alkylen-O-C₁-C₆-Alkyl, C₁-C₆-Alkylen-O-Aryl, COO-C₁-C₄-Alkyl oder C₁-C₄-Alkylen-COO-C₁-C₄-Alkyl;
2.) Phenyl oder Naphthyl, die jeweils mit R_{Q}², R_{Q}³ und R_{Q}⁴ substituiert sind, wobei
R_{Q}², R_{Q}³ und R_{Q}⁴ jeweils unabhängig voneinander einen Substituenten aus der folgenden Gruppe darstellen:
Wasserstoff, NO₂, NH₂. OH, CN, CF₃, CHF₂, OCF₃, OCHF₂, COOH, O-CH₂-COOH, SH, Halogen, oder jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-Alkylen-Hetaryl, oder
O-R_{Q}⁵, S-R_{Q}⁵, NR_{Q}⁷R_{Q}⁸, CO-OR_{Q}⁶, NR_{Q}⁸-CO-O-R_{Q}⁶, O-CH₂-COO-R_{Q}⁸, NR_{Q}⁸-CO-R_{Q}⁶, SO₂-R_{Q}⁶, NR_{Q}⁸-SO₂-R_{Q}⁶, SO₂NH₂, CONH₂, SO₂-NR_{Q}⁷R_{Q}⁸ oder CO-NR_{Q}⁷R_{Q}⁸, oder
jeweils zwei der Reste aus **R_{Q}², R_{Q}³** oder **R_{Q}⁴** zusammen einen 3 bis 7-gliedrigen, gegebenenfalls substituierten, gesättigten, ungesättigten oder aromatischen Carbocyclus oder einen gegebenenfalls substituierten, gesättigten, ungesättigten aromatischen Heterocyclus, der bis zu drei weitere verschiedene oder gleiche Heteroatome O, N, S enthalten kann, bilden, und gegebenenfalls zwei an diesem Heterocyclus substituierte Reste können zusammen einen anellierten, gesättigten, ungesättigten oder aromatischen Carbocyclus oder Heterocyclus bilden, wobei der Heterocyclus bis zu drei verschiedene oder gleiche Heteroatome O, N, S enthalten kann, und der gebildete Cyclus kann gegebenenfalls substituiert sein oder an diesem Cyclus kann ein weiterer, gegebenenfalls substituierter Cyclus ankondensiert sein;
**R_{Q}⁵** jeweils gegebenenfalls substituiertes C₂-C₆-Alkenyl, C₂-C₆-Alkinyl, C₁-C₄-Alkylen-C₃-C₇-Cycloalkyl, C₁-C₄-Alkylen-Heterocycloalkyl, Heterocycloalkyl oder Hetaryl, oder
C₁-C₆-Alkyl, das gegebenenfalls substituiert ist mit einem Substituenten aus der Gruppe, bestehend aus Halogen, NO₂, NH₂, OH, CN, CF₃, CHF₂, OCF₃, OCHF₂, NH-(C₁-C₆-Alkyl) und N(C₁-C₆-Alkyl)₂;
**R_{Q}⁶** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl oder C₁-C₆-Alkylen-O-C₁-C₆-Alkyl;
**R_{Q}⁷** Wasserstoff, OH, CN, oder
jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, C₁-C₄-Alkylen-Aryl, C₁-C₄-Alkylen-Hetaryl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Ayl, CO-O-Hetaryl, CO-O-C₁-C₄-Alkylen-Hetayl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
**R_{Q}⁸** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
oder die Reste R_{Q}⁷ und R_{Q}⁸ zusammen mit dem Stickstoff einen 3 bis 7-gliedrigen, gegebenenfalls substituierten, gesättigten oder aromatischen Heterocyclus, der ein, zwei oder drei weitere verschiedene oder gleiche Heteroatome O, N, S enthalten kann, bilden; und gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und der gebildete Cyclus gegebenenfalls substituiert sein kann oder an diesem Cyclus ein weiterer, gegebenenfalls substituierter Cyclus ankondensiert sein kann;
3.) einen 5- oder 6-gliedrigen, gegebenenfalls mit 1 oder 2 Substituenten substituierten Hetaryl-Rest aus der Gruppe, bestehend aus:
2-Pyrrolyl, 3-Pyrrolyl, 2-Thiazolyl, 4-Thiazolyl, 5-Thiazolyl, 2-Oxazolyl, 4-Oxazolyl, 5-Oxazolyl, 2-Pyrimidyl, 4-Pyrimidyl, 5-Pyrimidyl, 6-Pyrimidyl, 3-Pyrazolyl, 4-Pyrazolyl, 5-Pyrazolyl, 3-Isothiazolyl, 4-Isothiazolyl, 5-Isothiazolyl, 2-Imidazolyl, 4-Imidazolyl, 5-Imidazolyl, 3-Pyridazinyl, 4-Pyridazinyl, 5-Pyridazinyl, 6-Pyridazinyl, 3-Isoxazolyl, 4-Isoxazolyl, 5-Isoxazolyl, Thiadiazolyl, Oxadiazolyl oder Triazinyl oder deren anellierten Derivate Indazolyl, Benzothiophenyl, Benzofuranyl, Indolinyl, Benzimidazolyl, Benzthiazolyl, Benzoxazolyl, Chinolinyl und Isochinolinyl; oder
gegebenenfalls mit 1 oder 2 Substituenten substituiertes 2-Thienyl oder 3-Thienyl, wobei die Substituenten ausgewählt sind aus der Gruppe, bestehend aus Halogen, NO₂, NH₂, OH, CN, CF₃, OCF₃, CHF₂, O-CHF₂, C₁-C₆-Alkyl, O-C₁-C₆-Alkyl, NH-(C₁-C₆-Alkyl), N(C₁-C₆-Alkyl)₂, NHCO-C₁-C₄-Alkyl, NHSO₂-C₁-C₄-Alkyl und SO₂-C₁-C₄-Alkyl;
5.) beide Reste R⁴ und R⁵ zusammen einen 4 bis 7-gliedrigen, gegebenenfalls substituierten, gesättigten oder ungesättigten oder aromatischen Carbocyclus oder einen 5- oder 6-gliedrigen gegebenenfalls substituierten, gesättigten oder ungesättigten oder aromatischen Heterocyclus, der bis zu drei weitere verschiedene oder gleiche Heteroatome O, N, S enthalten kann, bilden, und mit bis zu zwei weiteren Resten substituiert sein kann, wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
6.) einen C₅-C₁₈- bi- oder tricyclischen, gesättigten Kohlenwasserstoffrest.

3. Guanidinverbindung nach Anpruch 1 oder 2, wobei die angegebenen Reste die folgenden Definitionen besitzen:
**W:** W1;
**A:** Halogen, OH, CN, CF₃, CHF₂, OCF₃, OCHF₂, oder
jeweils gegebenenfalls substituiertes C₁-C₆-Alkyl oder C₂-C₆-Alkenyl, O-CH₂-COO-R_{A}¹, O-R_{A}¹, S-R_{A}¹, NR_{A}²R_{A}³, NR_{A}⁴-CO-R_{A}¹ oder CO-NR_{A}⁴R_{A}¹;
**R_{A}¹:** jeweils gegebenenfalls substituiertes C₁-C₄-Alkyl, C₃-C₇-Cycloalkyl, Phenyl oder Benzyl;
**R_{A}²:** Wasserstoff, oder
jeweils gegebenenfalls substituiertes C₁-C₄-Alkyl, Phenyl, Benzyl, Phenethyl, CO-C₁-C₄-Alkyl, CO-Aryl, CO-O-C₁-C₄-Alkyl, SO₂-C₁-C₄-Alkyl, SO₂-Aryl, SO₂-Hetaryl oder SO₂-C₁-C₄-Alkylen-Aryl;
**R_{A}³:** jeweils gegebenenfalls substituiertes C₁-C₄-Alkyl, Phenyl, Benzyl, Phenethyl, CO-C₁-C₄-Alkyl, CO-Aryl, CO-O-C₁-C₄-Alkyl, SO₂-C₁-C₄-Alkyl, SO₂-Aryl, SO₂-Hetaryl, oder SO₂-C₁-C₄-Alkylen-Aryl;
oder die Reste **R_{A}²** und **R_{A}³** zusammen einen gegebenenfalls substituierten 5-oder 6-gliedrigen gesättigten oder ungesättigten Ring, der bis zu zwei gleiche oder verschiedene Heteroatome aus der Gruppe O und N enthalten kann, bilden;
**R_{A}⁴:** Wasserstoff oder einen gegebenenfalls substituierten C₁-C₄-Alkylrest;
**B:** Wasserstoff oder wie Rest A definiert ist;
**R_{w}¹:** Wasserstoff, F, Cl, CN, CF₃, O-CF₃, oder
jeweils gegebenenfalls substituiertes C₁-C₄-Alkyl, Aryl, C₁-C₆-Alkylamino oder C₁-C₆-Dialkylamino;
in der Formel **Z1** die Summe aus a, b und c 1, 2 oder 3 ist;
**R_{z}^{1,} R_{z}², R_{z}³, R_{z}⁴** unabhängig voneinander:
Wasserstoff, Halogen, OH, gegebenenfalls substituiertes C₁-C₆-Alkyl;
**V_{z}:** -CO-, -CO-NR_{z}⁵-, -NR_{z}⁵-CO- -O-, -S-;
**R_{z}⁵:** Wasserstoff, CH₃;
**R¹, R², R³** unabhängig voneinander:
Wasserstoff, OH, CN, C₁-C₄-Alkyl, C₁-C₆-Alkylen-O-C₁-C₆-Alkyl, substituiertes Aryl, Benzyl, CO-C₁-C₆-Alkyl, CO-Aryl, CO-C₁-C₄-Alkylen-Aryl, OCO-C₁-C₆-Alkyl, OCO-Aryl oder OCO-C₁-C₄-Alkylen-Hetaryl-,
**Q** ausgewählt ist aus der Gruppe, bestehend aus **Q1, Q2** und **Q3;**
**R_{Q}¹:** Wasserstoff, gegebenenfalls substituiertes C₁-C₄-Alkyl, im Arylrest gegebenenfalls substituiertes Benzyl, CO-C₁-C₄-Alkyl, gegebenenfalls substituiertes Benzoyl, SO₂-C₁-C₄-Alkyl oder im Arylrest gegebenenfalls substituiertes SO₂-Aryl.

4. Guanidinverbindung nach mindestens einem der Ansprüche 1 bis 3, wobei die angegebenen Reste die folgenden Definitionen besitzen:
**A:** OH, F, Cl, OCF₃, OCHF₂, gegebenenfalls substituiertes C₁-C₄-Alkyl, O-C₁-C₄-Alkyl oder S-C₁-C₄-Alkyl;
**B:** Wasserstoff, OH, F, Cl, CF₃, OCF₃, OCHF₂, gegebenenfalls substituiertes C₁-C₄-Alkyl, O-C₁-C₄-Alkyl oder S-C₁-C₄-Alkyl;
R_{W}¹: Wasserstoff, F, Cl, CN, CF₃ oder O-CF₃;
Z: jeweils gegebenenfalls substituiertes C₁-C₄-Alkyl oder C₁-C₄-Alkylen-O-C₁-C₄-Alkyl;
R_{Z}^{1,} R_{z}², R_{Z}^{3,} R_{Z}⁴ jeweils unabhängig voneinander:
Wasserstoff, F, CH₃;
R¹, R², R³ unabhängig voneinander:
Wasserstoff, OH, CN, O-Methyl, O-Phenyl, Acetyl, Benzoyl, O-Acetyl, O-Benzoyl;
Q ist ausgewählt aus der Gruppe, bestehend aus 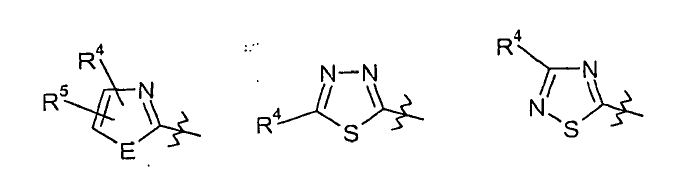
R_{Q}¹: Wasserstoff, CH₃, Methansulfonyl, Phenylsulfonyl oder Tosyl.

5. Guanidinverbindung nach mindestens einem der Ansprüche 1 bis 4, wobei die angegebenen Reste die folgenden Definitionen besitzen:
A: OH, OCF₃, OCH₃, O-Ethyl, O-Propyl oder O-iPropyl;
Z: -CH₂-, -CH₂-O-, -CH₂-CH₂- oder -CH₂CH₂-O-;
jeweils zwei der Reste R¹, R², oder R³ Wasserstoff sind, und der dritte Rest Wasserstoff, OH, Acetyl oder Benzoyl ist;
Q:

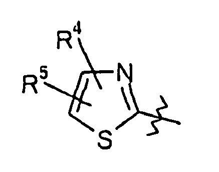

6. Guanidinverbindung nach mindestens einem der Ansprüche 1 bis 5, wobei R⁴ und/oder R⁵ jeweils unabhängig voneinander einen Rest ausgewählt aus den Gruppen 1.), 2.), 3.), 4.) oder 5.) darstellen:
1.) Wasserstoff, F, Cl, CN, CF₃, oder
jeweils gegebenenfalls substituiertes C₁-C₆-Alkyl, C₂-C₆-Alkenyl, C₁-C₆-Alkylen-O-C₁-C₆-Alkyl oder C₃-C₇-Cycloalkyl;
2.) R_{Q}¹, R_{Q}² und R_{Q}³ unabhängig voneinander
Wasserstoff, CN, CF₃, CHF₂, OCF₃, OCHF₂, F, Cl, OH oder
jeweils gegebenenfalls substituiertes Phenyl oder Hetaryl, C₁-C₄-Alkyl, C₅-C₇-Cycloalkyl, O-R_{Q}⁵, NR_{Q}⁷R_{Q}⁸, CO-OR_{Q}⁶, NR_{Q}⁸-CO-O-R_{Q}⁶, O-CH₂-COO-R_{Q}⁶, NR_{Q}⁸-CO-R_{Q}⁶, SO₂-R_{Q}⁶, NR_{Q}⁸-SO₂-R_{Q}⁶, NR_{Q}⁸-CO-O-R_{Q}⁶, SO₂NH_{2,} CONH₂, SO₂-NR_{Q}⁷R_{Q}⁸ oder CO-NR_{Q}⁷R_{Q}⁸;
R_{Q}⁵: C₁-C₄-Alkyl, das gegebenenfalls substituiert ist mit einem Substituenten aus der Gruppe, bestehend aus F, Cl, OH, CN, CF₃, OCF₃, NH-(C₁-C₄-Alkyl) und N(C₁-C₄-Alkyl)₂;
R_{Q}⁶: jeweils gegebenenfalls substituiertes C₁-C₆-Alkyl, Aryl, Hetaryl oder Phenyl;
R_{Q}⁷: Wasserstoff, jeweils gegebenenfalls substituiertes C₁-C₄-Alkyl, Allyl, Aryl, Hetaryl, Benzyl, Phenethyl oder CH₂-Hetaryl;
R_{Q}⁸: jeweils gegebenenfalls substituiertes C₁-C₄-Alkyl, Allyl, Aryl, Hetaryl, Benzyl, Phenethyl oder CH₂-Hetaryl;
oder R_{Q}⁷ und R_{Q}⁸ bilden einen gegebenenfalls substituierten 3-oder 7-gliedrigen gesättigten oder ungesättigten Ring, der bis zu zwei gleiche oder verschiedene Heteroatome aus der Gruppe O und N enthalten kann;
3.) Benzothiophenyl, Benzofuranyl, Chinolinyl oder Isochinolinyl;
4.) beide Reste R⁴ und R⁵ zusammen einen der folgenden Ringe bilden: 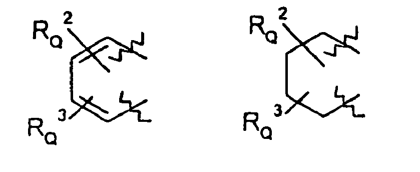 wobei R_{Q}² und R_{Q}³ wie unter 2.) definiert sind;
5.) Adamantyl.

7. Guanidinverbindung nach Anpruch 1 oder 2, wobei die angegebenen Reste die folgenden Definitionen besitzen:
W: W1;
A: Halogen, OH, CN, CF₃, CHF₂, OCF₃, OCHF₂, oder
jeweils gegebenenfalls substituiertes C₁-C₆-Alkyl oder C₂-C₆-Alkenyl, O-CH₂-COO-R_{A}¹, O-R_{A}¹, S-R_{A}¹, NR_{A}²R_{A}³, NR_{A}⁴-CO-R_{A}¹, SO₂NH₂, NR_{A}⁴-SO₂-R_{A}¹, SO₂-NR_{A}²R_{A}³ oder CO-NR_{A}⁴R_{A}¹;
R_{A}¹: jeweils gegebenenfalls substituiertes C₁-C₄-Alkyl, C₃-C₇-Cycloalkyl, Phenyl oder Benzyl;
R_{A}²: Wasserstoff, oder
jeweils gegebenenfalls substituiertes C₁-C₄-Alkyl, Phenyl, Benzyl, Phenethyl, CO-C₁-C₄-Alkyl, CO-Aryl, CO-O-C₁-C₄-Alkyl, SO₂-C₁-C₄-Alkyl, SO₂-Aryl, SO₂-Hetaryl oder SO₂-C₁-C₄-Alkylen-Aryl;
R_{A}³: jeweils gegebenenfalls substituiertes C₁-C₄-Alkyl, Phenyl, Benzyl, Phenethyl, CO-C₁-C₄-Alkyl, CO-Aryl, CO-O-C₁-C₄-Alkyl, SO₂-C₁-C₄-Alkyl, SO₂-Aryl, SO₂-Hetaryl, oder SO₂-C₁-C₄-Alkylen-Aryl;
oder die Reste R_{A}² und R_{A}³ zusammen einen gegebenenfalls substituierten 5-oder 6-gliedrigen gesättigten oder ungesättigten Ring, der bis zu zwei gleiche oder verschiedene Heteroatome aus der Gruppe O und N enthalten kann, bilden;
R_{A}⁴: Wasserstoff oder einen gegebenenfalls substituierten C₁-C₄-Alkylrest;
B: Wasserstoff oder wie Rest A definiert ist;
R_{W}¹: Wasserstoff, F, Cl, CN, CF₃, O-CF₃, oder
jeweils gegebenenfalls substituiertes C₁-C₄-Alkyl, Aryl, C₁-C₆-Alkylamino oder C₁-C₆-Dialkylamino;
in der Formel Z1 die Summe aus a, b und c 1, 2 oder 3 ist;
R_{Z}^{1,} R_{Z}², R_{z}³, R_{Z}⁴ unabhängig voneinander:
Wasserstoff, Halogen, OH, gegebenenfalls substituiertes C₁-C₆-Alkyl;
V_{z}: -CO-, -CO-NR_{z}⁵-, -NR_{z}⁵-CO-, -O-, -S-;
R_{Z}⁵; Wasserstoff, CH₃;
R¹, R², R³ unabhängig voneinander:
Wasserstoff, OH, CN, C₁-C₄-Alkyl, C₁-C₆-Alkylen-O-C₁-C₆-Alkyl, substituiertes Aryl, Benzyl, CO-C₁-C₆-Alkyl, CO-Aryl, CO-C₁-C₄-Alkylen-Aryl, OCO-C₁-C₆-Alkyl, OCO-Aryl oder OCO-C₁-C₄-Alkylen-Hetaryl;
Q ausgewählt ist aus der Gruppe, bestehend aus Q1, Q2, Q3 und Q5;
R_{Q}¹: Wasserstoff, gegebenenfalls substituiertes C₁-C₄-Alkyl, im Arylrest gegebenenfalls substituiertes Benzyl, CO-C₁-C₄-Alkyl, gegebenenfalls substituiertes Benzoyl, SO₂-C₁-C₄-Alkyl oder im Arylrest gegebenenfalls substituiertes SO₂-Aryl.

8. Guanidinverbindung nach mindestens einem der Ansprüche 1, 2 oder 7, wobei die angegebenen Reste die folgenden Definitionen besitzen:
A: OH, F, Cl, OCF₃, OCHF₂, gegebenenfalls substituiertes C₁-C₄-Alkyl, O-C₁-C₄-Alkyl oder S-C₁-C₄-Alkyl;
B: Wasserstoff, OH, F, Cl, CF₃, OCF₃, OCHF₂, gegebenenfalls substituiertes C₁-C₄-Alkyl, O-C₁-C₄-Alkyl oder S-C₁-C₄-Alkyl;
R_{w}¹: Wasserstoff, F, Cl, CN, CF₃ oder O-CF₃;
Z: jeweils gegebenenfalls substituiertes C₁-C₄-Alkyl oder C₁-C₄-Alkylen-O-C₁-C₄-Alkyl;
R_{Z}^{1,} R_{z}², R_{Z}^{3,} R_{Z}⁴ jeweils unabhängig voneinander:
Wasserstoff, F, CH₃;
R¹, R², R³ unabhängig voneinander:
Wasserstoff, OH, CN, O-Methyl, O-Phenyl, Acetyl, Benzoyl, O-Acetyl, O-Benzoyl;
Q ist ausgewählt aus der Gruppe, bestehend aus 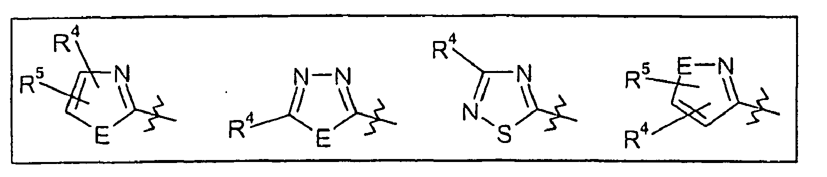
R_{Q}¹: Wasserstoff, CH₃, Phenyl, Benzyl, Methansulfonyl, Phenylsulfonyl oder Tosyl.

9. Guanidinverbindung nach mindestens einem der Ansprüche 1, 2, 7 oder 8, wobei die angegebenen Reste die folgenden Definitionen besitzen:
A: OH, OCF₃, OCH₃, O-Ethyl, O-Propyl oder O-iPropyl;
Z: -CH₂-, -CH₂-O-, -CH₂-CH₂- oder :CH₂-CH₂-O-;
jeweils zwei der Reste R¹, R², oder R³ Wasserstoff sind, und der dritte Rest Wasserstoff, OH, Acetyl oder Benzoyl ist;
Q: 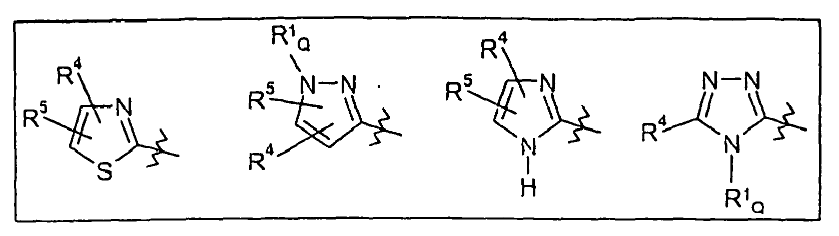
R_{Q}¹: Wasserstoff, CH₃, Phenyl, Benzyl, Methansulfonyl, Phenylsulfonyl oder Tosyl.

10. Guanidinverbindung nach mindestens einem der Ansprüche 1, 2 oder 7 bis 9, wobei R⁴ und/oder R⁵ jeweils unabhängig voneinander einen Rest ausgewählt aus den Gruppen 1.), 2.), 3.), 4.), 5.) oder 7.) darstellen:
1.) Wasserstoff, F, Cl, CN, CF₃, oder
jeweils gegebenenfalls substituiertes C₁-C₆-Alkyl, C₂-C₆-Alkenyl, C₁-C₆-Alkylen-O-C₁-C₆-Alkyl oder C₃-C₇-Cycloalkyl;
2.) R_{Q}¹, R_{Q}² und R_{Q}³ unabhängig voneinander
Wasserstoff, CN, CF₃, CHF₂, OCF₃, OCHF₂, F, Cl, OH oder
jeweils gegebenenfalls substituiertes Phenyl oder Hetaryl, C₁-C₄-Alkyl, C₅-C₇-Cycloalkyl, O-R_{Q}⁵, NR_{Q}⁷R_{Q}⁶, CO-OR_{Q}⁸, NR_{Q}⁸-CO-O-R_{Q}⁶, O-CH₂-COO-R_{Q}⁶, NR_{Q}⁸-CO-R_{Q}⁶, SO₂-R_{Q}⁶, NR_{Q}⁸-SO₂-R_{Q}⁶, NR_{Q}⁸-CO-O-R_{Q}⁶, SO₂NH₂, CONH₂, SO₂-NR_{Q}⁷R_{Q}⁸ oder CO-NR_{Q}⁷R_{Q}⁸;
R_{Q}⁵: C₁-C₄-Alkyl, das gegebenenfalls substituiert ist mit einem Substituenten aus der Gruppe, bestehend aus F, Cl, OH, CN, CF₃, OCF₃, NH-(C₁-C₄-Alkyl) und N(C₁-C₄-Alkyl)₂;
R_{Q}⁶: jeweils gegebenenfalls substituiertes C₁-C₆-Alkyl, Aryl, Hetaryl oder Phenyl;
R_{Q}⁷: Wasserstoff, jeweils gegebenenfalls substituiertes C₁-C₄-Alkyl, Allyl, Aryl, Hetaryl, Benzyl, Phenethyl oder CH₂-Hetaryl;
R_{Q}⁸_{:} Wasserstoff, jeweils gegebenenfalls substituiertes C₁-C₄-Alkyl, Allyl, Aryl, Hetaryl, Benzyl, Phenethyl oder CH₂-Hetaryl;
oder R_{Q}⁷ und R_{Q}⁸ bilden einen gegebenenfalls substituierten 3-oder 7-gliedrigen gesättigten oder ungesättigten Ring, der bis zu zwei gleiche oder verschiedene Heteroatome aus der Gruppe O und N enthalten kann;
3.) Benzothiophenyl, Benzofuranyl, Chinolinyl oder Isochinolinyl;
4.) beide Reste R⁴ und R⁵ zusammen einen der folgenden Ringe bilden: 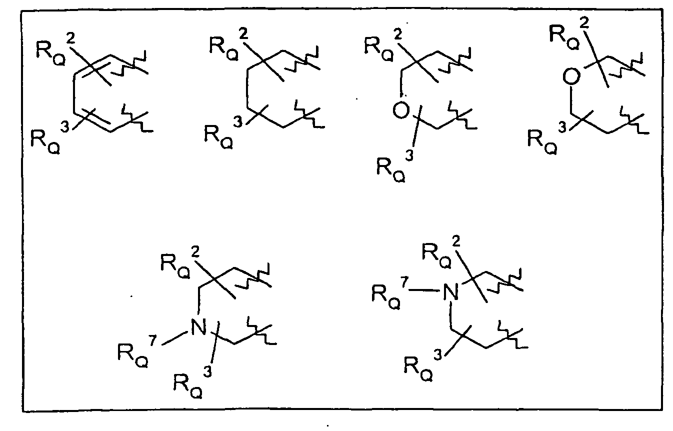 wobei R_{Q}² und R_{Q}³ wie unter 2.) definiert sind; ; oder zusammen einen annelierten 5- oder 6-gliedrigen Ring bilden können;
5.) Adamantyl;
7.) jeweils gegebenenfalls substituiertes Azetidin-3-yl, Pyrrolidin-2-yl, Pyrrolidin-3-yl, Piperidin-2-yl, Piperidin-3-yl, Piperidin-4-yl, Tetrahydro-2H-pyran-4-yl, Tetrahydrofuran-3-yl, Azepan-4-yl, Azepan-3-yl, Azepan-2-yl, 1,4-Diazepan-5-yl, 1,2,3,6-Tetrahydropyridin-4-yl, 2,5-Dihydro-1H-pyrrol-3-yl.

11. Guanidinverbindung nach mindesten einem der Ansprüche 1 bis 10, wobei ein Rest von R⁴ und R⁵ ausgewählt wird aus Gruppe 1.), und der andere Rest von R⁴ und R⁵ ausgewählt wird aus Gruppe 1.), 2.) oder 3.).

12. Guanidinverbindung nach mindestens einem der Ansprüche 1 bis 11 als Arzneimittel.

13. Pharmazeutische Zusammensetzung, enthaltend mindestens eine Guanidinverbindung nach einem der Ansprüche 1 bis 12 sowie einen pharmazeutisch annehmbaren Träger oder Verdünnungsmittel.

14. Verwendung von Verbindungen der allgemeinen Formel IVA zur Herstellung von 5HT5A-Rezeptorliganden:
W-Z-NH₂ IVA

15. Verwendung nach Anspruch 14 zur Herstellung der Guanidinverbindungen nach einem der Ansprüche 1 bis 12.

16. Verwendung einer Guanidinverbindung der allgemeinen Formel IA 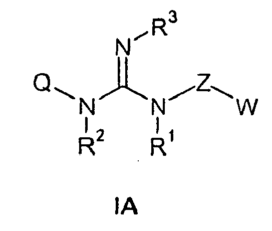 der entsprechenden enantiomeren, diastereomeren und/oder tautomeren Formen davon sowie pharmazeutisch annehmbare Salze davon,
wobei die angegebenen Reste die folgenden Definitionen besitzen:
W:
einen Rest der allgemeinen Formel W1 oder W2 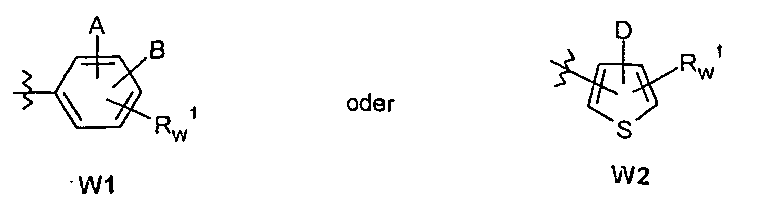
A:
NO₂, NH₂, OH, CN, CF₃, OCF₃, CHF₂, OCHF₂, COOH, O-CH₂-COOH, Halogen, SH, oder
jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl oder C₁-C₄-Alkylen-Heterocycloalkyl, Aryl, Hetaryl, Heterocycloalkyl, C₁-C₄-Alkylen-Hetaryl oder C₁-C₄-Alkylen-Aryl, oder
O-R_{A}¹, CO-R_{A}¹, S-R_{A}¹,SO-R_{A}¹, CO-O-R_{A}¹, NR_{A}⁴-CO-O-R_{A}¹, O-CH₂-COO-R_{A}¹, NR_{A}²R_{A}³, CONH₂, SO₂NH₂, NR_{A}⁴-CO-R_{A}¹, SO₂-R_{A}¹, NR_{A}⁴-SO₂-R_{A}¹, SO₂-NR_{A}²R_{A}³ oder CO-NR_{A}²R_{A}³;
R_{A}¹:
jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₂-C₆-Alkenylen-Aryl oder C₁-C₆-Alkylen-Hetaryl;
R_{A}²:
Wasserstoff, OH, CN, oder
jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₈-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
R_{A}³:
jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆₋Alkyl, CO-C₁-C₆-Alkyl, CO-Aryl, CO-Hetaryl, CO-C₁-C₄₋Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
oder die Reste R_{A}² und R_{A}³ zusammen mit dem Stickstoff einen 3 bis 7-gliedrigen, gegebenenfalls substituierten, gesättigten oder aromatischen Hetero-cyclus, der ein, zwei oder drei weitere verschiedene oder gleiche Heteroatome aus der Gruppe O, N, S enthalten kann, bilden; wobei gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und wobei der dadurch gebildete Cyclus gegebenenfalls substituiert sein kann oder an diesem Cyclus ein weiterer, gegebenenfalls substituierter Cyclus ankondensiert sein kann;
R_{A}⁴:
Wasserstoff, oder
jeweils gegebenenfalls substituiertes C₁-C₆-Alkyl, C₁-C₆-Alkylen-O-C₁-C₆-Alkyl, C₂-C₆-Alkenyl, C₃-C₁₂-Alkinyl, CO-C₁-C₆-Alkyl, CO-O-C₁-C₆-Alkyl, O₂-C₁-C₆-Alkyl, C₃-C₇-Cycloalkyl, Aryl, C₁-C₄-Alkylen-Aryl, CO-O-Arylalkyl, CO-C₁-C₄-Alkylen-Aryl, CO-Aryl, SO₂-Aryl, Hetaryl, CO-Hetaryl oder SO₂-C₁-C₄-AlkylenAryl;
B:
Wasserstoff oder wie Rest A definiert ist,
oder jeweils unabhängig voneinander zwei der Reste A, B oder R_{W}¹ zusammen einen 3 bis 7-gliedrigen, gegebenenfalls substituierten, gesättigten oder ungesättigten Carbocyclus oder einen gegebenenfalls substituierten, gesättigten oder ungesättigten oder aromatischen Heterocyclus, der ein, zwei oder drei weitere verschiedene oder gleiche Heteroatome aus der Gruppe O, N, S enthalten kann, bilden; wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
R_{W}¹:
Wasserstoff, OH, Halogen, NO₂, NH₂, CN, CF₃, CHF₂, O-CF₃, O-CHF₂, oder jeweils gegebenenfalls substituiertes C₁-C₆-Alkyl, C₃-C₇-Cycloalkyl, C₁-C₆-Alkylen-O-C₁-C₆-Alkyl, C₁-C₆-Alkylen-S-C₁-C₆-Alkyl, Aryl, Hetaryl, O-C₁-C₆-Alkyl, O-Aryl, O-Benzyl, C₁-C₆-Alkylamino, C₁-C₆-Dialkylamino, Pyrrolidinyl, Piperidinyl, Morpholinyl, CO-C₁-C₆-Alkyl, SO₂-C₁-C₆-Alkyl, CO-Aryl, SO₂-Aryl, CO-C₁-C₄-Alkylen-Aryl, SO₂-C₁-C₄-Alkylen-Aryl, SO-Aryl, CONH₂, CONH-C₁-C₆-Alkyl, SO₂NH-C₁-C₆-Alkyl, CON(C₁-C₆-Alkyl)₂, SO₂N(C₁-C₆-Alkyl)₂, NH-SO₂-C₁-C₆-Alkyl oder NH-CO-C₁-C₆-Alkyl;
D:
wie Rest A definiert ist;
Z:
einen Rest der allgemeinen Formel Z1 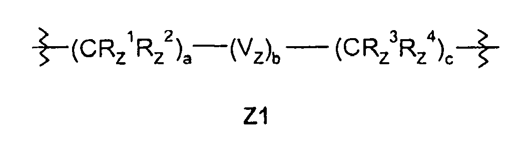 mit den Indizes
a = 0 - 4
b = 0, 1
c = 0 - 4
wobei die Summe aus a, b und c höchstens 5 beträgt;
R_{Z}¹, R_{Z}², R_{z}³, R_{Z}⁴ unabhängig voneinander:
Wasserstoff, Halogen, OH, oder
jeweils gegebenenfalls substituiertes C₁-C₆-Alkyl, C₂-C₆-Alkenyl, C₂-C₆-Alkinyl, C₁-C₆-Alkylen-C₃-C₇-Cycloalkyl, C₃-C₇-Cycloalkyl, Aryl, C₁-C₄-Alkylen-Aryl, Hetaryl oder C₁-C₄-Alkylen-Hetaryl, oder
jeweils unabhängig voneinander zwei Reste R_{Z}¹ und R_{Z}² oder R_{Z}³ und R_{Z}⁴ zusammen einen 3 bis 7-gliedrigen, gegebenenfalls substituierten, gesättigten oder ungesättigten Carbo- oder Heterocyclus, der bis zu drei Heteroatome aus der Gruppe O, N oder S enthalten kann, bilden;
V_{z}:
-CO-, -CO-NR_{z}⁵-, -NR_{z}⁵-CO-, -O-, -S-, -SO-, -SO₂-. -SO₂-NR_{z}⁵-, -NR_{z}⁵-SO₂-, -CS-, -CS-NR_{z}⁵-, -NR_{z}⁵-CS-, -CS-O-, -O-CS-, -CO-O-, -O-CO-, -O-, Ethinylen, -C(=CR_{Z}⁶R_{z}⁷)-, -CR_{Z}⁶=CR_{Z}⁷-, -NR_{z}⁵-CO-NR_{z}⁵-, -O-CO-NR_{z}⁵-, -NR_{z}⁵-;
R_{Z}⁵, R_{Z}⁵* unabhängig voneinander:
Wasserstoff, oder
jeweils gegebenenfalls substituiertes C₁-C₆-Alkyl, C₁-C₆-Alkylen-O-C₁-C₆-Alkyl, C₂-C₆-Alkenyl, C₃-C₁₂-Alkinyl, CO-C₁-C₆-Alkyl, CO-O-C₁-C₆-Alkyl, SO₂-C₁-C₆-Alkyl, C₃-C₇-Cycloalkyl, Aryl, C₁-C₄-Alkylen-Aryl, CO-O-C₁-C₄-Alkylen-Aryl, CO-C₁-C₄-Alkylen-Aryl, CO-Aryl, SO₂-Aryl, Hetaryl, CO-Hetaryl oder SO₂-C₁-C₄-Alkylen-Aryl;
R_{Z}⁶, R_{Z}⁷ unabhängig voneinander:
Wasserstoff, OH, oder
jeweils gegebenenfalls substituiertes C₁-C₆-Alkyl, C₁-C₄-Alkoxy, C₂-C₆-Alkenyl, C₂-C₆-Alkinyl, C₁-C₆-Alkylen-C₃-C₇-Cycloalkyl, C₃-C₇-Cycloalkyl, Aryl, C₁-C₄-Alkylen-Aryl, Hetaryl oder C₁-C₄-Alkylen-Hetaryl;
R¹, R², R³ unabhängig voneinander:
Wasserstoff, OH, CN, oder
jeweils gegebenenfalls substituiertes C₁-C₆-Alkyl, O-C₁-C₆-Alkyl, C₁-C₆-Alkylen-O-C₁-C₆-Alkyl, C₃-C₇-Cycloalkyl, O-C₃-C₇-Cycloalkyl, Aryl, Hetaryl, C₁-C₄-Alkylen-Aryl, C₁-C₄-Alkylen-Hetaryl, O-Aryl, O-C₁-C₄-Alkylen-Aryl, O-Hetaryl, O-C₁-C₄-Alkylen-Hetaryl, CO-C₁-C₆-Alkyl, CO-Aryl, CO-Hetaryl, CO-C₁-C₄-AlkylenAryl, CO-C₁-C₄-Alkylen-Hetaryl, CO-O-C₁-C₆-Alkyl, CO-O-Aryl, CO-O-Hetaryl, CO-O-C₁-C₄-Alkylen-Aryl, SO₂-C₁-C₆-Alkyl, SO₂-Aryl, SO₂-Hetaryl, SO₂-C₁-C₄-Alkylen-Aryl, OCO-C₁-C₆-Alkyl, OCO-Aryl, OCO-Hetaryl, OCO-C₁-C₄-AlkylenAryl, OCO-C₁-C₄-Alkylen-Hetaryl, SO₂-C₁-C₆-Alkyl, SO₂-Aryl, SO₂-Hetaryl oder SO₂-C₁-C₄-Alkylen-Aryl, oder
jeweils unabhängig vom dritten Rest zwei Reste von R¹, R² oder R³ zusammen einen 5 bis 7-gliedrigen, gegebenenfalls substituierten, gesättigten oder ungesättigten Carbocyclus, oder einen gegebenenfalls substituierten, gesättigten oder ungesättigten, der ein, zwei oder drei weitere verschiedene oder gleiche Heteroatome aus der Gruppe O, N, S enthalten kann, bilden, wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder
gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
Q:
einen zweifach substituierten 5-gliedrigen Hetaryl-Rest, augewählt aus Q1 bis Q7 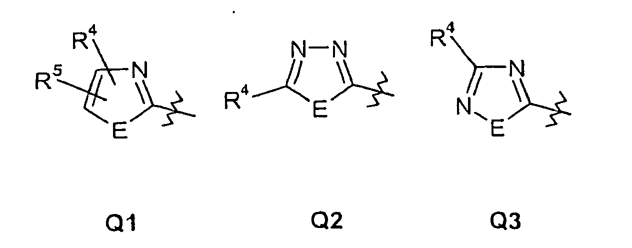 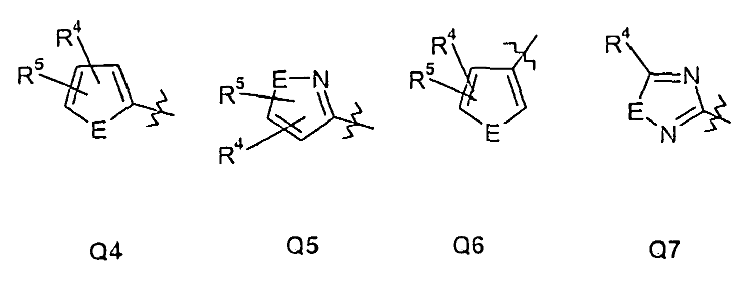
E: O, N-R_{Q}¹ oder S;
R_{Q}¹:
Wasserstoff, oder
jeweils gegebenenfalls substituiertes C₁-C₄-Alkyl, CO-C₁-C₄-Alkyl, SO₂-C₁-C₄-Alkyl, CO-O-C₁-C₄-Alkyl, Aryl, C₁-C₄-Alkylen-Aryl, CO-Aryl, CO-Hetaryl, SO₂-Aryl, SO₂-Hetaryl, CO-O-Aryl, CO-C₁-C₄-Alkylen-Aryl, SO₂-C₁-C₄-Alkylen-Aryl oder CO-O-C₁-C₄-Alkylen-Aryl;
R⁴, R⁵ jeweils unabhängig voneinander einen Rest ausgewählt aus den Gruppen 1.), 2.), 3.), 4.), 5.), 6.) oder 7.):
1.) Wasserstoff, Halogen, CN, CF₃, CHF₂, oder jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇-Cycloalkyl, C₁-C₄-Alkylen-Aryl,
C₁-C₄-Alkylen-Hetaryl, C₁-C₆-Alkylen-O-C₁-C₆-Alkyl, C₁-C₆-Alkylen-O-Aryl, COO-C₁-C₄-Alkyl oder C₁-C₄-Alkylen-COO-C₁-C₄-Alkyl;
2.) Phenyl oder Naphthyl, die jeweils mit R_{Q}², R_{Q}³ und R_{Q}⁴ substituiert sind, wobei
R_{Q}², R_{Q}² und R_{Q}⁴ jeweils unabhängig voneinander einen Substituenten aus der folgenden Gruppe darstellen:
Wasserstoff, NO₂, NH₂, OH, CN, CF₃, CHF₂, OCF₃, OCHF₂, COOH, O-CH₂-COOH, SH, Halogen, oder
jeweils gegebenenfalls substituiertes Aryl, Hetaryl, Heterocycloalkyl, C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-Alkylen-Hetaryl, oder
O-R_{Q}⁵, S-R_{Q}⁵, NR_{Q}⁷R_{Q}⁸, CO-OR_{Q}⁶, NR_{Q}⁸-CO-O-R_{Q}⁶, O-CH₂-COO-R_{Q}⁶, NR_{Q}⁸-CO-R_{Q}⁶, SO₂-R_{Q}⁶, NR_{Q}⁸-SO₂-R_{Q}⁶, SO₂NH₂, CONH₂, SO₂-NR_{Q}⁷R_{Q}⁸ oder CO-NR_{Q}⁷R_{Q}⁸, oder
jeweils zwei der Reste aus R_{Q}², R_{Q}³ oder R_{Q}⁴ zusammen einen 3 bis 7-gliedrigen, gegebenenfalls substituierten, gesättigten, ungesättigten Carbocyclus oder einen gegebenenfalls substituierten, gesättigten, ungesättigten aromatischen Heterocyclus, der bis zu drei weitere verschiedene oder gleiche Heteroatome O, N, S enthalten kann, bilden, und gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und der gebildete Cyclus gegebenenfalls substituiert sein kann oder an diesem Cyclus ein weiterer, gegebenenfalls substituierter Cyclus ankondensiert sein kann;
R_{Q}⁵ jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Heterocycloalkyl, Aryl oder Hetaryl;
R_{Q}⁶ jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl; Aryl, Hetaryl, Heterocycloalkyl oder C₁-C₆-Alkylen-O-C₁-C₆-Alkyl;
R_{Q}⁷ Wasserstoff, OH, CN, oder
jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, C₁-C₄-Alkylen-Aryl, C₁-C₄-Alkylen-Hetaryl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
R_{Q}⁸ Wasserstoff oder
jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
oder beide Reste R_{Q}⁷ und R_{Q}⁸ zusammen mit dem Stickstoff einen 3 bis 7-gliedrigen, gegebenenfalls substituierten, gesättigten oder aromatischen Heterocyclus, der ein, zwei oder drei weitere verschiedene oder gleiche Heteroatome O, N, S enthalten kann- bilden; und gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und der gebildete Cyclus gegebenenfalls substituiert sein kann oder an diesem Cyclus ein weiterer, gegebenenfalls substituierter Cyclus ankondensiert sein kann;
3.) einen.5- oder 6-gliedrigen, gegebenenfalls mit 1 oder 2 Substituenten substituierten Hetaryl-Rest aus der Gruppe, bestehend aus:
2-Furyl, 3-Furyl, 2-Pyrrolyl, 3-Pyrrolyl, 2-Thienyl, 3-Thienyl, 2-Pyridyl, 3-Pyridyl, 4-Pyridyl, 2-Thiazolyl, 4-Thiazolyl, 5-Thiazolyl, 2-Oxazolyl, 4-Oxazolyl, 5-Oxazolyl, 2-Pyrimidyl, 4-Pyrimidyl, 5-Pyrimidyl, 6-Pyrimidyl, 3-Pyrazolyl, 4-Pyrazolyl, 5-Pyrazolyl, 3-Isothiazolyl, 4-Isothiazolyl, 5-Isothiazolyl, 2-Imidazolyl, 4-Imidazolyl, 5-Imidazolyl, 3-Pyridazinyl, 4-Pyridazinyl, 5-Pyridazinyl, 6-Pyridazinyl, 3-Isoxazolyl, 4-Isoxazolyl, 5-Isoxazolyl, Thiadiazolyl, Oxadiazolyl oder Triazinyl oder deren anellierten Derivate Indazolyl, Indolyl, Benzothiophenyl, Benzofuranyl, Indolinyl, Benzimidazolyl, Benzthiazolyl, Benzoxazolyl, Chinolinyl und Isochinolinyl;
4.) beide Reste R⁴ und R⁵ zusammen einen 4 bis 7-gliedrigen, gegebenenfalls substituierten, gesättigten oder ungesättigten oder aromatischen Carbocyclus oder einen 5- oder 6-gliedrigen gegebenenfalls substituierten, gesättigten Oder ungesättigten oder aromatischen Heterocyclus, der bis zu drei weitere verschiedene oder gleiche Heteroatome O, N, S enthalten kann, bilden, und mit bis zu zwei weiteren Resten substituiert sein kann, wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
5.) einen C₆-C₁₀- bi- oder tricyclischen, gesättigten Kohlenwasserstoffrest;
6.) jeweils gegebenenfalls substituiertes C₁-C₈-Alkyl-NH₂, C₁-C₈-Alkyl-NR_{Q}⁷R_{Q}⁸, C₁-C₈-Alkyl-CO-NR_{Q}⁷R_{Q}⁸, C₁-C₈-Alkyl-SO₂NRa⁷R_{Q}⁸, C₁-C₈-Alkyl-CO-NH₂, C₁-C₈-Alkyl-SO₂NH₂, CO-NH₂, CO-NR_{Q}⁷R_{Q}⁸, SO₂NH₂, SO₂NR_{Q}⁷R_{Q}⁸, NR_{Q}⁷R_{Q}⁸;
7.) einen 4-7-gliedrigen mono- oder bicyclischen gesättigten oder ungesättigten Heterocyclus, der bis zu zwei verschiedene oder gleiche Heteroatome aus der Gruppe O, N oder S enthalten kann, wobei dieser Cyclus ebenfalls mehrfach substituiert sein kann. Für den Fall, dass der Heterocyclus ein N-Atom enthält, kann dies mit einem Rest R_{Q}⁷ substituiert sein.
zur Herstellung eines Medikaments zur Behandlung von Krankheiten, die durch eine 5-HT5-Rezeptoraktivität moduliert werden.

17. Verwendung einer Guanidinverbindung der allgemeinen Formel IA 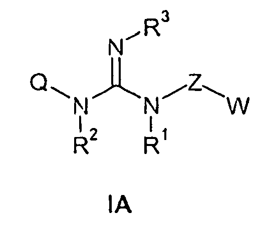 der entsprechenden enantiomeren, diastereomeren und/oder tautomeren Formen davon sowie pharmazeutisch annehmbare Salze davon,
wobei die angegebenen Reste die folgenden Definitionen besitzen:
W:
einen Rest der allgemeinen Formel W1 oder W2 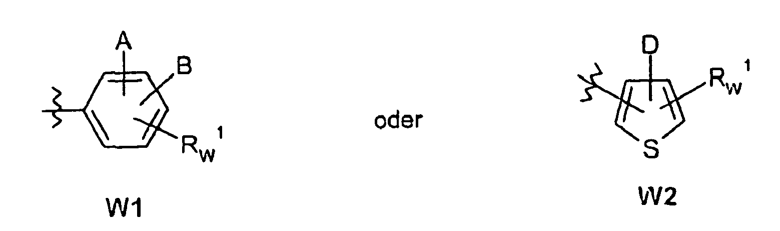
**A:**
NO₂, NH₂, OH, CN, CF₃, OCF₃, CHF₂, OCHF₂, COOH, O-CH₂-COOH, Halogen, SH, oder
jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl oder C₁-C₄-Alkylen-Heterocycloalkyl, Aryl, Hetaryl, Heterocycloalkyl, C₁-C₄-Alkylen-Hetaryl oder C₁-C₄-Alkylen-Aryl, oder
O-R_{A}¹, CO-R_{A}¹, S-R_{A}¹, SO-R_{A}¹, CO-O-R_{A}¹, NR_{A}⁴-CO-O-R_{A}¹, O-CH₂-COO-R_{A}¹, NR_{A}²R_{A}³, CONH₂, SO₂NH₂, NR_{A}⁴-CO-R_{A}¹, SO₂-R_{A}¹, NR_{A}⁴-SO₂-R_{A}¹, SO₂-NR_{A}²R_{A}³ oder CO-NR_{A}²R_{A}³;
**R_{A}¹:**
jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₂-C₆-Alkenylen-Aryl oder C₁-C₆-Alkylen-Hetaryl;
**R_{A}²:**
Wasserstoff, OH, CN, oder
jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
**R_{A}³:**
jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Aryl, Hetaryl, Heterocycloalkyl, C₁-C₄-Alkylen-Aryl, C₁-C₄-Alkylen-Hetaryl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
oder die Reste **R_{A}²** und **R_{A}³** zusammen mit dem Stickstoff einen 3 bis 7-gliedrigen, gegebenenfalls substituierten, gesättigten oder aromatischen Hetero-cyclus, der ein, zwei oder drei weitere verschiedene oder gleiche Heteroatome aus der Gruppe O, N, S enthalten kann, bilden; wobei gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und wobei der dadurch gebildete Cyclus gegebenenfalls substituiert sein kann oder an diesem Cyclus ein weiterer, gegebenenfalls substituierter Cyclus ankondensiert sein kann;
**R_{A}⁴:**
Wasserstoff, oder
jeweils gegebenenfalls substituiertes C₁-C₆-Alkyl, C₁-C₆-Alkylen-O-C₁-C₆-Alkyl, C₂-C₆-Alkenyl, C₃-C₁₂-Alkinyl, CO-C₁-C₆-Alkyl, CO-O-C₁-C₆-Alkyl, SO₂-C₁-C₆-Alkyl, C₃-C₇-Cycloalkyl, Aryl, C₁-C₄-Alkylen-Aryl, CO-O-Arylalkyl, CO-C₁-C₄-Alkylen-Aryl, CO-Aryl, SO₂-Aryl, Hetaryl, CO-Hetaryl oder SO₂-C₁-C₄-AlkylenAryl;
**B:**
Wasserstoff oder wie Rest A definiert ist,
oder jeweils unabhängig voneinander zwei der Reste A, B oder R_{w}¹ zusammen einen 3 bis 7-gliedrigen, gegebenenfalls substituierten, gesättigten oder ungesättigten Carbocyclus oder einen gegebenenfalls substituierten, gesättigten oder ungesättigten oder aromatischen Heterocyclus, der ein, zwei oder drei weitere verschiedene oder gleiche Heteroatome aus der Gruppe O, N, S enthalten kann, bilden; wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
**Rw¹:**
Wasserstoff, OH, Halogen, NO₂, NH₂, CN, CF₃, CHF₂, O-CF₃, O-CHF₂, oder
jeweils gegebenenfalls substituiertes C₁-C₆-Alkyl, C₃-C₇-Cycloalkyl, C₁-C₆-Alkylen-O-C₁-C₆-Alkyl, C₁-C₆-Alkylen-S-C₁-C₆-Alkyl, Aryl, Hetaryl, O-C₁-C₆-Alkyl, O-Aryl, O-Benzyl, C₁-C₆-Alkylamino, C₁-C₆-Dialkylamino, Pyrrolidinyl, Piperidinyl, Morpholinyl, CO-C₁-C₆-Alkyl, SO₂-C₁-C₆,-Alkyl, CO-Aryl, SO₂Aryl, CO-C₁-C₄-Alkylen-Aryl; SO₂-C₁-C₄-Alkylen-Aryl, SO-Aryl, CONH₂, CONH-C₁-C₆-Alkyl, SO₂NH-C₁-C₆-Alkyl, CON(C₁-C₆-Alkyl)₂, SO₂N(C₁-C₆-Alkyl-)₂, NH-SO₂-C₁-C₆-Alkyl oder NH-CO-C₁-C₆-Alkyl;
**D:**
wie Rest **A** definiert ist;
**Z:**
einen Rest der allgemeinen Formel **Z1** 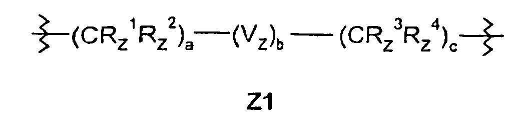 mit den Indizes
a=0-4
b = 0, 1
c=0-4
wobei die Summe aus a, b und c höchstens 5 beträgt;
**R_{z}¹, R_{z}², R_{z}³, R_{z}⁴** unabhängig voneinander:
Wasserstoff, Halogen, OH, oder
jeweils gegebenenfalls substituiertes C₁-C₆-Alkyl, C₂-C₆-Alkenyl, C₂-C₆-Alkinyl, C₁-C₆-Alkylen-C₃-C₇-Cycloalkyl, C₃-C₇-Cycloalkyl, Aryl, C₁-C₄-Alkylen-Aryl. Hetaryl oder C₁-C₄-Alkylen-Hetaryl, oder
jeweils unabhängig voneinander zwei Reste **R_{z}¹** und **R_{z}²** oder **R_{z}³** und **R_{z}⁴** zusammen einen 3 bis 7-gliedrigen, gegebenenfalls substituierten, gesättigten oder ungesättigten Carbo- oder Heterocyclus, der bis zu drei Heteroatome aus der Gruppe O, N oder S enthalten kann, bilden;
**V_{z}:**
-CO-, -CO-NR_{z}⁵-, -NR_{z}⁵-CO-, -O-, -S-, -SO-, -SO₂-, -SO₂-NR_{z}⁵-, -NR_{z}⁵-SO₂-, -CS-, -CS-NR_{z}⁵-, -NR_{z}⁵-CS-, -CS-O-, -O-CS-, -CO-O-, -O-CO-, -O-, Ethinylen, -C(=CR_{z}⁶R_{z}⁷)-, -CR_{z}⁶=CR_{z}⁷-, -NR_{z}⁵-CO-NR_{z}^{5*}-, -O-CO-NR_{z}⁵-, -NR_{z}⁵-;
**R_{z}⁵, R_{z}⁵*** unabhängig voneinander.
Wasserstoff, oder
jeweils gegebenenfalls substituiertes C₁-C₆-Alkyl, C₁-C₆-Alkylen-O-C₁-₆-Alkyl, C₂-C₆-Alkenyl, C₃-C₁₂-Alkinyl, CO-C₁-C₆-Alkyl, CO-O-C₁-C₆-Alkyl, SO₂-C₁-C₆,-Alkyl, C₃-C₇-Cycloalkyl, Aryl, C₁-C₄-Alkylen-Aryl, CO-O-C₁-C₄-Alkylen-Aryl, CO-C₁-C₄-Alkylen-Aryl, CO-Aryl, SO₂-Aryl, Hetaryl, CO-Hetaryl oder SO₂-C₁-C₄-Alkylen-Aryl;
**R_{z}⁶, R_{z}⁷** unabhängig voneinander:
Wasserstoff, OH, oder
jeweils gegebenenfalls substituiertes C₁-C₆-Alkyl, C₁-C₄-Alkoxy, C₂-C₆-Alkenyl, C-₂-C₆-Alkinyl, C₁-C₆-Alkylen-C₃-C₇-Cycloalkyl, C₃-C₇-Cycloalkyl, Aryl, C₁-C₄-Alkylen-Aryl, Hetaryl oder C₁-C₄-Alkylen-Hetaryl;
**R¹, R², R³** unabhängig voneinander:
Wasserstoff. OH, CN, oder
jeweils gegebenenfalls substituiertes C₁-C₆-Alkyl, O-C₁-C₆-Alkyl, C₁-C₆-Alkylen-O-C₁-C₅-Alkyl, C₃-C₇-Cycloalkyl, O-C₃-C₇-Cycloalkyl, Aryl, Hetaryl, C₁-C₄-Alkylen-Aryl, C₁-C₄-Alkylen-Hetaryl, O-Aryl, O-C₁-C₄-Alkylen-Aryl, O-Hetaryl, O-C₁-C₄-Alkylen-Hetaryl, CO-C₁-C₅-Alkyl, CO-Aryl, CO-Hetaryl, CO-C₁-C₄-AlkylenAryl, CO-C₁-C₄-Alkylen-Hetaryl, CO-O-C₁-C₈-Alkyl, CO-O-Aryl, CO-O-Hetaryl, CO-O-C₁-C₄-Alkylen-Aryl, SO₂C₁-C₆-Alkyl, SO₂-Aryl, SO₂-Hetaryl, SO₂-C₁-C₄-Alkylen-Aryl, OCO-C₁-C₆-Alkyl, OCO-Aryl, OCO-Hetaryl, OCO-C₁-C₄-AlkylenAryl, OCO-C₁-C₄-Alkylen-Hetaryl, SO₂-C₁-C₆-Alkyl, SO₂-Aryl, SO₂-Hetaryl oder SO₂-C₁-C₄-Alkylen-Aryl, oder
jeweils unabhängig vom dritten Rest zwei Reste von **R¹, R²** oder **R³** zusammen einen 5 bis 7-gliedrigen, gegebenenfalls substituierten, gesättigten oder ungesättigten Carbocyclus, oder einen gegebenenfalls substituierten, gesättigten oder ungesättigten, der ein, zwei oder drei weitere verschiedene oder gleiche Heteroatome aus der Gruppe O, N, S enthalten kann, bilden, wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
**Q:**
einen zweifach substituierten 5-gliedrigen Hetaryl-Rest, augewählt aus **Q1** bis **Q6** 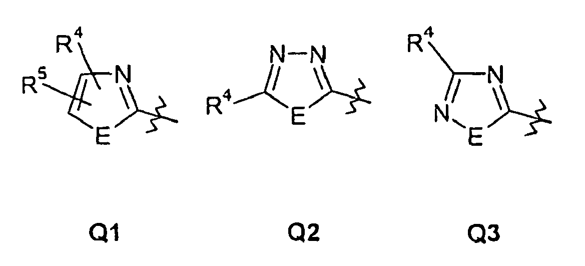 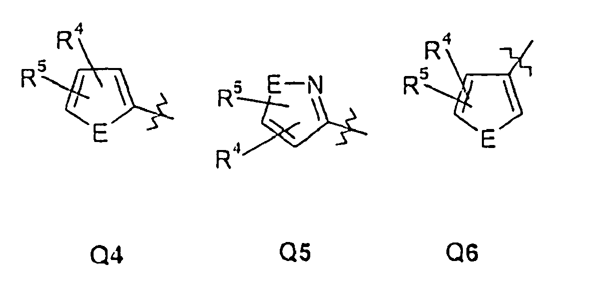
**E:** O, N-R_{Q}¹ oder S;
**R_{Q}¹:**
Wasserstoff, oder
jeweils gegebenenfalls substituiertes C₁-C₄-Alkyl, CO-C₁-C₄-Alkyl, SO₂-C₁-C₄-Alkyl, CO-O-C₁-C₄-Alkyl, Aryl, C₁-C₄-Alkylen-Aryl, CO-Aryl, CO-Hetaryl, SO₂-Aryl, SO₂-Hetaryl, CO-O-Aryl, CO-C₁-C₄-Alkylen-Aryl, SO₂-C₁-C₄-Akylen-Aryl oder CO-O-C₁-C₄-Alkylen-Aryl;
**R⁴, R⁵** jeweils unabhängig voneinander einen Rest ausgewählt aus den Gruppen **1.), 2.), 3.), 4.)** oder **5.):**
**1.)** Wasserstoff, Halogen, CN, CF₃, CHF₂, oder
jeweils gegebenenfalls substituiertes C₁-C₁₀-Alkyl, C₂-C₁₀-Alkenyl, C₂-C₁₀-Alkinyl, C₃-C₇-Cycloalkyl, C₁-C₆-Alkylen-C₃-C₇Cycloalkyl, C₁-C₄-Alkylen-Aryl, C₁-C₄-Alkylen-Hetaryl, C₁-C₆-Alkylen-O-C₁-C₆-Alkyl, C₁-C₆-Alkylen-O-Aryl, COO-C₁-C₄-Alkyl oder C₁-C₄-Alkylen-COO-C₁-C₄-Alkyl;
**3.)** Phenyl oder Naphthyl, die jeweils mit **R_{Q}², R_{Q}³** und **R_{Q}⁴** substituiert sind, wobei
**R_{Q}², R_{Q}³** und **R_{Q}⁴** jeweils unabhängig voneinander einen Substituenten aus der folgenden Gruppe darstellen:
Wasserstoff, NO₂, NH₂, OH, CN, CF₃, CHF₂, OCF₃, OCHF₂, COOH, O-CH₂-COOH, SH, Halogen, oder
jeweils gegebenenfalls substituiertes Aryl, Hetaryl, Heterocycloalkyl, C₁-C₆-Alkyl, C₂-C₆-Alkenyl, C₂-C₆-Alkinyl. C₃-C₇-Cycloalkyl, C₁-C₄-Alkylen-C₃-C₇-Cycloalkyl, C₁-C₄-Alkylen-Heterocycloalkyl, C₁-C₄-Alkylen-Aryl oder C₁-C₄-Alkylen-Hetaryl, oder
O-R_{Q}⁵, S-R_{Q}⁵, NR_{Q}⁷R_{Q}⁸, CO-OR_{Q}⁶, NR_{Q}⁸-CO-O-R_{Q}⁶, O-CH₂-COO-R_{Q}⁶, NR_{Q}⁸-CO-R_{Q}⁶, SO₂-R_{Q}⁶, NR_{Q}⁸-SO₂-R_{Q}⁶, SO₂NH₂, CONH₂, SO₂-NR_{Q}⁷R_{Q}⁸ oder CO-NR_{Q}⁷R_{Q}⁸, oder
jeweils zwei der Reste aus **R_{Q}², R_{Q}³** oder **R_{Q}⁴** zusammen einen 3 bis 7-gliedrigen, gegebenenfalls substituierten, gesättigten, ungesättigten Carbocyclus oder einen gegebenenfalls substituierten, gesättigten, ungesättigten aromatischen Heterocyclus, der bis zü drei weitere verschiedene oder gleiche Heteroatome O, N, S enthalten kann, bilden, und gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und der gebildete Cyclus gegebenenfalls substituiert sein kann oder an diesem Cyclus ein weiterer, gegebenenfalls substituierter Cyclus ankondensiert sein kann:
**R_{Q}⁵** jeweils gegebenenfalls substituiertes C₁-C₆-Alkyl, C₂-C₆-Alkenyl, C₂-C₆-Alkinyl, C₁-C₄-Alkylen-C₃-C₇-Cycloalkyl, C₁-C₄-Alkylen-Heterocycloalkyl, Heterocycloalkyl, Aryl oder Hetaryl;
**R_{Q}⁶** jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl oder C₁-C₆-Alkylen-O-C₁-C₆-Alkyl;
**R_{Q}⁷** Wasserstoff, OH, CN, oder
jeweils gegebenenfalls substituiertes C₁-C₆-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, C₁-C₄-Alkylen-Aryl, C₁-C₄-Alkylen-Hetaryl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂C₁-C₄-Alkylen-Hetaryl;
**R_{Q}⁸** jeweils gegebenenfalls substituiertes C₁-C₈-Alkyl, C₂-C₆-Alkenyl, C₂-C₆-Alkinyl, C₃-C₇-Cycloalkyl, C₁-C₄-Alkylen-C₃-C₇-Cycloalkyl, C₁-C₄-Alkylen-Heterocycloalkyl, Aryl, Hetaryl, Heterocycloalkyl, C₁-C₆-Alkylen-O-C₁-C₆-Alkyl, CO-C₁-C₆-Alkyl, CO-Aryl, CO-Hetaryl, CO-C₁-C₄-Alkylen-Aryl, CO-C₁-C₄-Alkylen-Hetaryl, CO-O-C₁-C₆-Alkyl, CO-O-Aryl, CO-O-C₁-C₄-Alkylen-Aryl, CO-O-Hetaryl, CO-O-C₁-C₄-Alkylen-Hetaryl, SO₂-C₁-C₆-Alkyl, SO₂-Aryl, SO₂-Hetaryl, SO₂-C₁-C₄-Alkylen-Aryl oder SO₂-C₁-C₄-Alkylen-Hetaryl;
oder belde Reste **R_{Q}⁷** und **R_{Q}⁸** zusammen mit dem Stickstoff einen 3 bis 7-gliedrigen, gegebenenfalls substituierten, gesättigten oder aromatischen Heterocyclus, der ein, zwei oder drei weitere verschiedene oder gleiche Heteroatome O, N, S enthalten kann- bilden; und gegebenenfalls zwei an diesem Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann, und der gebildete Cyclus gegebenenfalls substituiert sein kann oder an diesem Cyclus ein weiterer, gegebenenfalls substituierter Cyclus ankondensiert sein kann;
**3.)** einen 5- oder 6-gliedrigen, gegebenenfalls mit 1 oder 2 Substituenten substituierten Hetaryl-Rest aus der Gruppe, bestehend aus:
2-Furyl, 3-Furyl, 2-Pyrrolyl, 3-Pyrrolyl, 2-Thienyl, 3-Thienyl, 2-Pyridyl, 3-Pyridyl, 4-Pyridyl, 2-Thiazolyl, 4-Thiazolyl, 5-Thiazolyl, 2-Oxazolyl, 4-Oxazolyl, 5-Oxazolyl, 2-Pyrimidyl, 4-Pyrimidyl, 5-Pyrimidyl, 6-Pyrimidyl, 3-Pyrazolyl, 4-Pyrazolyl, 5-Pyrazolyl, 3-Isothiazolyl, 4-Isothiazolyl, 5-Isothiazolyl, 2-Imidazolyl, 4-Imidazolyl, 5-Imidazolyl, 3-Pyridazinyl, 4-Pyridazinyl, 5-Pyridazinyl, 6-Pyridazinyl, 3-Isoxazolyl, 4-Isoxazolyl, 5-Isoxazolyl, Thiadiazolyl, Oxadiazolyl oder Triazinyl oder deren anellierten Derivate Indazolyl, Indolyl, Benzothiophenyl, Benzofuranyl, Indolinyl, Benzimidazolyl, Benzthiazolyl, Benzoxazolyl, Chinolinyl und Isochinolinyl;
**4.)** beide Reste **R⁴** und **R⁵** zusammen einen 4 bis 7-gliedrigen, gegebenenfalls substituierten, gesättigten oder ungesättigten oder aromatischen Carbocyclus oder einen 5- oder 6-gliedrigen gegebenenfalls substituierten, gesättigten oder ungesättigten oder aromatischen Heterocyclus, der bis zu drei weitere verschiedene oder gleiche Heteroatome O, N, S enthalten kann, bilden, und mit bis zu zwei weiteren Resten substituiert sein kann, wobei gegebenenfalls zwei an diesem Carbo- oder Heterocyclus substituierte Reste zusammen einen anellierten, gesättigten, ungesättigten oder aromatischen Carbocyclus oder Heterocyclus bilden können, wobei der Heterocyclus bis zu drei verschiedene oder gleiche Heteroatome O, N, S enthalten kann und wobei der gebildete Cyclus gegebenenfalls substituiert sein kann oder an diesem Cyclus ein weiterer, gegebenenfalls substituierter Cyclus ankondensiert sein kann;
**5.)** einen C₆-C₁₀- bi- oder tricyclischen, gesättigten Kohlenwasserstoffrest;
zur Herstellung eines Medikaments zur Behandlung von Krankheiten, die durch eine 5-HT5-Rezeptoraktivität moduliert werden.

18. Verwendung nach Anspruch 16, wobei **R⁴** und/oder **R⁵** die folgenden Bedeutungen besitzen:
2-Pyridyl, 3-Pyridyl, 4-Pyridyl, 2-Thienyl, 3-Thienyl, Benzothiophenyl, Benzofuranyl, Chinolinyl oder Isochinolinyl, die gegebenenfalls mit 1 oder 2 Resten substituiert sein können.

19. Verwendung nach Anspruch 16 oder 17 zur Behandlung von neuropathologischen, neuropsychiatrischen und neurodegenerativen Störungen, Symptomen und Fehlfunktionen.

20. Verwendung nach mindestens einem der Ansprüche 16 bis 18 zur Behandlung von Migräne und Gehirnschädigungen.

21. Verwendung nach Anspruch 18 zur Behandlung von neuropathologischen, neuropsychiatrischen und neurodegenerativen Krankheiten, ausgewählt aus der Gruppe, bestehend aus cerebraler Ischämie, Schlaganfall, Epilepsie und Anfälle im allgemeinen, Psychosen, Schizophrenie, Autismus, OCD-Syndrom, cognitiven Erkrankungen, Aufmerksamkeitsstörungen, Depressionen, bipolaren und/oder unipolaren Depressionen, Angstzuständen, Demenz, seniler Demenz, Alzheimer Demenz, demyelinisierenden Erkrankungen, Multipler Sklerose und Gehirntumoren.

22. Verwendung nach Anspruch 16 oder 17 zur Behandlung von Krankheiten ausgewählt aus der Gruppe, bestehend aus cerebrovaskulären Störungen, Schmerz, Schmerzbedingten Störungen, Abhängigkeit, Drogen-bedingten Störungen, Amnesie, Alkoholmissbrauch, Drogenmissbrauch, Störungen des circadianen Rhythmus und Cushing Syndrom.
